# FASCICULE DE BREVET EUROPEEN

(11) **EP 1 246 807 B1**
(45) Date de publication et mention de la délivrance du brevet: **02.11.2005**
(21) Numéro de dépôt: 00958634.8
(22) Date de dépôt: 28.07.2000
(51) Int. Cl.: C07D 233/86, C07D 233/78, C07D 403/06, C07D 403/04, C07D 403/14, C07D 409/14, C07D 407/14, C07D 413/14, C07D 417/14, A61K 31/415, A61K 31/505, A61P 5/02, C07D 405/14

(54) **DERIVES D'HYDANTOINES, DE THIOHYDANTOINES, DE PYRIMIDINEDIONES ET DE THIOXOPYRIMIDINONES, LEURS PROCEDES DE PREPARATION ET LEUR APPLICATION A TITRE DE MEDICAMENTS**
HYDANTOIN-, THIOHYDANTOIN-, PYRIMIDINEDION- UND THIOXOPYRIMIDINON-DERIVATE, VERFAHREN ZUR IHRER HERSTELLUNG UND IHRE ANWENDUNG ALS ARZTNEIMITTEL
NOVEL HYDANTOIN, THIOHYDANTOIN, PYRIMIDINEDIONE AND THIOXOPYRIMIDINONE DERIVATIVES, PREPARATION METHOD AND USE AS MEDICINES

(30) Priorité: 30.07.1999 FR 9909886
(43) Date de publication de la demande: 09.10.2002
(73) Titulaire: SOCIETE DE CONSEILS DE RECHERCHES ET D'APPLICATIONS SCIENTIFIQUES (S.C.R.A.S.), 75016 Paris (FR)
(72) Inventeur: POITOUT, Lydie, F-74270 Le Kremlin-Bicêtre (FR); THURIEAU, Christophe, F-75016 Paris (FR); BRAULT, Valérie, F-91190 Gif sur Yvette (FR)
(74) Mandataire: Bourgouin, André
(86) Numéro de dépôt international: PCT/FR2000/002164
(87) Numéro de publication internationale: WO 2001/009090

(56) Documents cités:
- WO-A-97/43278
- WO-A-98/13045
- WO-A-98/45285
- WO-A-98/58646
- WO-A-99/64401
- WO-A-99/64420
- WO-A-99/65942
- SCICINSKI J J ET AL: "The solid phase synthesis of a series of tri-substituted hydantoin ligands for the somatostatin SST5 receptor" BIOORGANIC & MEDICINAL CHEMISTRY LETTERS,GB,OXFORD, vol. 8, no. 24, 15 décembre 1998 (1998-12-15), pages 3609-3614, XP004150376 ISSN: 0960-894X
- LIU S ET AL: "Nonpeptide somatostatin agonists with sst4 selectivity: syntheses and structure-activity relationships of thioureas" JOURNAL OF MEDICINAL CHEMISTRY,US,AMERICAN CHEMICAL SOCIETY. WASHINGTON, vol. 41, no. 24, 1998, pages 4693-4705, XP002136780 ISSN: 0022-2623
- LIU S ET AL: "2-PYRIDYLTHIOUREAS: NOVEL NONPEPTIDE SOMATOSTATIN AGONISTS WITH SST4 SELECTIVITY" CURRENT PHARMACEUTICAL DESIGN,NL,BENTHAM SCIENCE PUBLISHERS, SCHIPHOL, vol. 5, no. 4, 1999, pages 255-263, XP000882348 ISSN: 1381-6128

## Description

L'invention concerne les nouveaux dérivés d'hydantoïnes, de thiohydantoïnes, de pyrimidinediones et de thioxopyrimidinones de formule générale **(I)** représentée plus loin, leurs procédés de préparation et leur utilisation en tant que médicaments. Ces composés ont une bonne affinité avec certains sous-types de récepteurs de la somatostatine et présentent donc d'intéressantes propriétés pharmacologiques. L'invention concerne également des compositions pharmaceutiques contenant lesdits composés et leur utilisation pour la préparation d'un médicament destiné à traiter les états pathologiques ou les maladies dans lesquels un (ou plusieurs) des récepteurs de la somatostatine est (sont) impliqué(s).

La somatostatine (SST) est un tétradécapeptide cyclique qui a été isolé pour la première fois de l'hypothalamus en tant que substance inhibitrice de l'hormone de croissance (Brazeau P. et al., *Science* 1973, **179**, 77-79). Elle intervient également en tant que neurotransmetteur dans le cerveau (Reisine T. et al., *Neuroseience* 1995, 67, 777-790 ; Reisine et al., *Endocrinology* 1995, **16**, 427-442). Le clonage moléculaire a permis de montrer que la bioactivité de la somatostatine dépend directement d'une famille de cinq récepteurs liés à la membrane.

L'hétérogénéité des fonctions biologiques de la somatostatine a conduit à des études pour essayer d'identifier les relations structure-activité des analogues peptidiques sur les récepteurs de la somatostatine, ce qui a amené la découverte de 5 sous-types de récepteurs (Yamada et al., *Proc. Natl. Acad. Sci. U.S.A*, **89**, 251-255, 1992 ; Raynor, K. et al, *Mol. Pharmacol*., **44**, 385-392, 1993). Les rôles fonctionnels de ces récepteurs sont actuellement activement étudiés. Les affinités avec les différents sous-types de récepteurs de la somatostatine ont été associés au traitement des désordres / maladies suivants. L'activation des sous-types 2 et 5 a été associée à la suppression de l'hormone de croissance (GH) et plus particulièrement à celle des adénomes sécrétant GH (acromégalie) et de ceux sécrétant l'hormone TSH. L'activation du sous-type 2 mais pas du sous-type 5 a été associée au traitement des adénomes sécrétant la prolactine.

D'autres indications associées avec l'activation des sous-types de récepteurs de la somatostatine sont la resténose, l'inhibition de la sécrétion d'insuline et/ou de glucagon et en particulier le diabète mellitus, l'hyperlipidémie, l'insensiblité à l'insuline, le Syndrome X, l'angiopathie, la rétinopathie pmliférative, le phénomène de Dawn et la néphropathie; l'inhibition de la sécrétion d'acide gastrique et en particulier les ulcères peptiques, les fistules entérocutanées et pancréaticocutanées, le syndrome du colon irritable, le syndrome de Dumping, le syndrome des diarrhées aqueuses, les diarrhées reliées au SIDA, les diarrhées induites par la chimiothérapie, la pancréatite aiguë ou chronique et les tumeurs gastrointestinales sécrétrices; le traitement du cancer comme les hépatomes; l'inhibition de l'angiogénèse, le traitement des désordres inflammatoires comme l'arthrite; le rejet chronique des allogreffes; l'angioplastie; la prévention des saignements des vaisseaux greffés et des saignements gastrointestinaux. Les agonistes de la somatostatine peuvent aussi être utilisés pour diminuer le poids d'un patient.

Parmi les désordres pathologiques associés à la somatostatine (Moreau J.P. et al., *Life Sciences,* 1987, **40**, 419 ; Harris A.G. et al., *The European Journal of Medicine,* **1993***,* 2, 97-105), on peut donc citer par exemple : l'acromégalie, les adénomes hypophysaires, la maladie de Cushing, les gonadotrophinomes et les prolactinomes, les effets secondaires cataboliques des glucocorticoïdes, le diabète insulinodépendant, la rétinopathie diabétique, la néphropathie diabétique, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et d'autres domaines thérapeutiques comme, par exemple, les céphalées y compris les céphalées associées aux tumeurs hypophysaires, les douleurs, les accès de panique, la chimiothérapie, la cicatrisation des plaies, l'insuffisance rénale résultant d'un retard de croissance, l'obésité et retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis ainsi que la maladie d'Alzheimer. On peut également citer l'ostéoporose.

La déposante a trouvé que les composés de formule générale **(I)** décrits ci-après présentaient une affinité et une sélectivité pour les récepteurs de la somatostatine. Comme la somatostatine et ses analogues peptidiques ont souvent une mauvaise biodisponibilité par voie orale et une faible sélectivité (Robinson, C., *Drugs of the Future,* 1994, **19**, 992; Reubi, J.C. et al., *TIPS,* 1995, **16**, 110), lesdits composés, agonistes ou antagonistes non-peptidiques de la somatostatine, peuvent être avantageusement utilisés pour traiter les états pathologiques ou les maladies tels que présentés ci-dessus et dans lesquels un (ou plusieurs) des récepteurs de la somatostatine est (sont) impliqué(s). De manière préférentielle, lesdits composés peuvent être utilisés pour le traitement de l'acromégalie, des adénomes hypophysaires ou des tumeurs gastroentéropancréatiques endocriniennes dont le syndrome carcinoïde.

Les composés de la présente invention répondent à la formule générale **(I)** 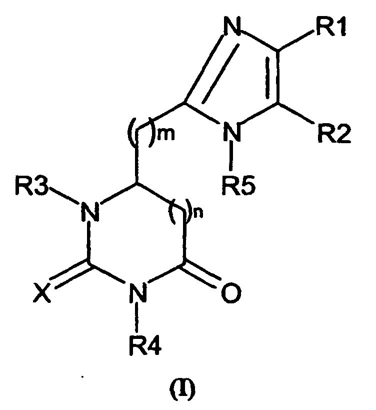 sous forme racémique, d'énantiomère ou toutes combinaisons de ces formes, dans laquelle :
R1 représente un radical phényle éventuellement substitué
R2 représente H,
R3 représente H ou (CH₂)ₚ-Z3;
Z3 représente (C₁-C₁₂)alkyle, (C₁-C₁₂)alkényle, (C₃-C₈)cycloalkyle, un radical aryle carbocyclique ou hétérocyclique éventuellement substitué, un radical hétérocyclique non aromatique éventuellement substitué, un radical *bis*-arylalkyle ou di-arylalkyle ou encore le radical 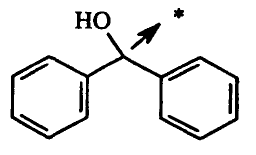
R4 représente -(CH₂)ₚ-Z4 ;
Z4 représente amino, (C₁-C₁₂)alkyle, (C₃-C₈)cycloalkyle, (C₁-C₁₂)alkylamino, N,N-di-(C₁-C₁₂)alkylamino, amino(C₃-C₆)cycloalkyle, amino(C₁-C₆)alkyl(C₃-C₆)cycloalkyl(C₁-C₆)alkyle, aminoaryle carbocyclique ou hétérocyclique, (C₁-C₁₂)alkoxy, (C₁-C₁₂)alkényle, -N-C(O)O(C₁-C₆)alkyle, un radical aryle carbocyclique ou hétérocyclique éventuellement substitué, un radical hétérocyclique non aromatique éventuellement substitué, *bis*-arylalkyle, di-arylalkyle ou l'un des radicaux représentés ci-dessous 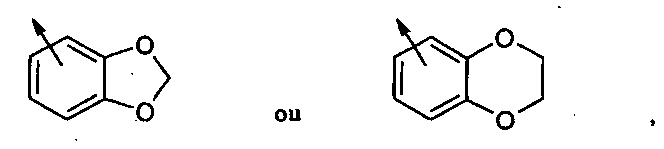 ou encore Z4 représente un radical N(R6)(R7) dans lequel R6 et R7 pris ensemble avec l'atome d'azote qui les porte forment ensemble un hétérocycle de 5 à 7 chaînons ;
R5 représente H, étant entendu qu'un radical éventuellement substitué ou un phényle éventuellement substitué est éventuellement substitué par un ou plus d'un substituant, chacun de préférence choisi indépendamment parmi le groupe constitué des radicaux Cl, F, Br, I, CF₃, NO₂, OH, NH₂, CN, N₃, -OCF₃, (C₁-C₁₂)alkyle, (C₁-C₁₂)alkoxy, -(CH₂)ₚ-phényl-(X1)_{q}, -NH-CO-(C₁-C₆)alkyle, -NH-C(O)O-(C₁-C₆)alkyle, -S-(C₁-C₆)alkyle, -S-phényl-(X1)_{q}, -O-(CH₂)ₚ-phényl-(X1)_{q}, -(CH₂)ₚ-C(O)-O-(C₁-C₆)alkyle, -(CH₂)ₚ-C(O)-(C₁-C₆)alkyle, -O-(CH₂)ₚ-NH₂, -O-(CH₂)ₚ-NH-(C₁-C₆)alkyle, -O-(CH₂)ₚ-N-di-((C₁-C₆)alkyl) et -((C₀-C₁₂))alkyl-(X1)_{q} ;
X1, à chaque fois qu'il intervient, est indépendamment choisi parmi le groupe constitué des radicaux H, Cl, F, Br, I, CF₃, NO₂, OH, NH₂, CN, N₃, -OCF₃, (C₁-C₁₂)alkyle, (C₁-C₁₂)alkoxy, -S-(C₁-C₆)alkyle, -(CH₂)ₚ-amino, -(CH₂)ₚ-NH-(C₁-C₆)alkyle, -(CH₂)ₚ-N-di-((C₁-C₆)alkyle), -(CH₂)ₚ-phényle et -(CH₂)ₚ-NH-(C₃-C₆)cycloalkyle ;
p à chaque fois qu'il intervient est indépendamment 0 ou un entier de 1 à 6 ;
q à chaque fois qu'il intervient est indépendamment un entier de 1 à 5.
X représente O ou S;
n représente 0 ou 1; et enfin
lorsque n représente 0, m représente 1, 2 ou 3, et lorsque n représente 1, m représente 0 ou 1;
ou un sel pharmaceutiquement acceptable d'un tel composé.

Selon une variante préférée de l'invention, les composés de formule générale **(I)** seront tels que R5 représente H.

Les composés de formule générale **(I)** peuvent le cas échéant comporter plus d'un centre asymétrique. Dans cette éventualité, les diastéréomères ou tout mélange de diastéréomères sont également compris dans l'invention. Par exemple, lorsque les composé de formule générale **(I)** possèderont deux centres asymétriques, l'invention comprendra les composés de formule générale **(I)** de configurations "R,S", "S,R", "R,R" et "S,S", ainsi qu'un mélange dans des proportions quelconques de ces derniers.

Dans la présente invention, les radicaux alkyle peuvent être linéaires ou ramifiés. Par alkyle, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un système monocyclique carboné comptant de 3 à 7 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par alkynyle, lorsqu'il n'est pas donné plus de précision, on entend un radical alkyle linéaire ou ramifié comptant de 1 à 6 atomes de carbone et présentant au moins une double insaturation (triple liaison). Par aryle carbocyclique ou hétérocyclique, on entend un système carbocyclique ou hétérocyclique comprenant au moins un cycle aromatique, un système étant dit hétérocyclique lorsque l'un au moins des cycles qui le composent comporte au moins un hétéroatome (O, N ou S). Par aryle, lorsqu'il n'est pas donné plus de précision, on entend un système carbocyclique comprenant au moins un cycle aromatique. Par haloalkyle, on entend un radical alkyle dont au moins l'un des atomes d'hydrogène (et éventuellement tous) est remplacé par un atome halogène. Par radical hétérocyclique non aromatique, on entend un système hétérocyclique ne comprenant aucun cycle aromatique, l'un au moins des cycles composant ledit système comportant au moins un hétéroatome (O, N ou S)

Par radicaux alkylthio, alkoxy, haloalkyle, haloalkoxy, aminoalkyle, alkylamino, alkényle, alkynyle et aralkyle, on entend respectivement les radicaux alkylthio, alkoxy, haloalkyle, haloalkoxy, aminoalkyle, alkylamino, alkényle, alkynyle et aralkyle dont le radical alkyle a la signification indiquée précédemment.

Par radical N,N-di-(C₁-C₁₂)alkylamino, on entend un radical dialkylamino dont les deux radicaux alkyle substituant l'atome d'azote peuvent compter indépendamment de 1 à 12 atomes de carbone.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par cycloalkyle, on entend en particulier les radicaux cyclopropanyle, cyclobutanyle, cyclopentanyle, cyclohexyle et cycloheptanyle. Par aryle carbocyclique ou hétérocyclique, on entend notamment les radicaux phényle, naphtyle, pyridinyle, furannyle, pyrrolyle, thiophènyle, thiazolyle, indanyle, indolyle, imidazolyle, benzofurannyle, benzothiophènyle, phtalimidyle. Par aralkyle carbocyclique ou hétérocyclique, on entend notamment les radicaux benzyle, phényléthyle, phénylpropyle, phénylbutyle, indolylalkyle, phtalimidoalkyle.

Lorsqu'il émane une flèche d'une structure chimique, ladite flèche indique le point d'attache. Par exemple : 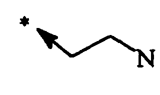 représente le radical aminoéthyle.

Lorsqu'une flèche est dessinée à travers un groupe bi- ou tricyclique, ladite flèche indique que ledit groupe bi- ou tricyclique peut être attaché par n'importe lequel des points d'attache disponibles sur n'importe cycle aromatique dudit groupe. Par exemple : 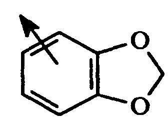 représente un radical qui est attaché par n'importe quelle position du cycle benzénique.

En particulier, les composés de formule générale **(I)** selon l'invention pourront être choisis tels que :
R1 représente un radical phényle éventuellement substitué ;
R2 représente H ;
R3 représente l'un des radicaux ci-après : 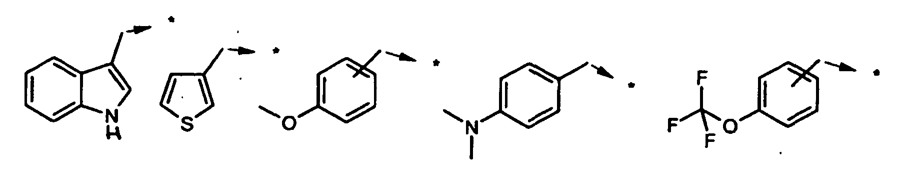 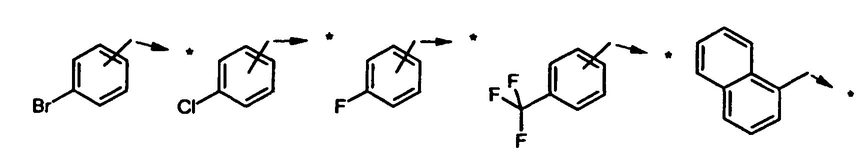 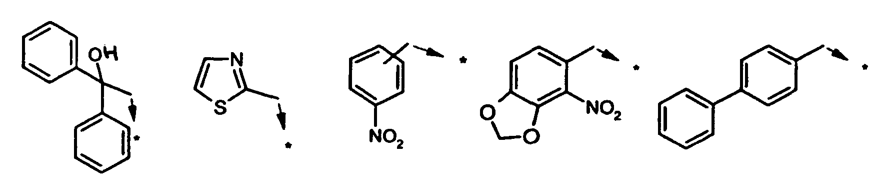 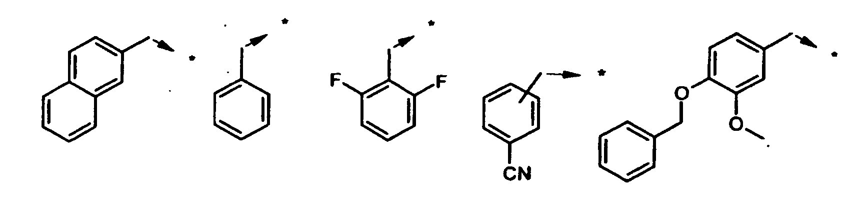 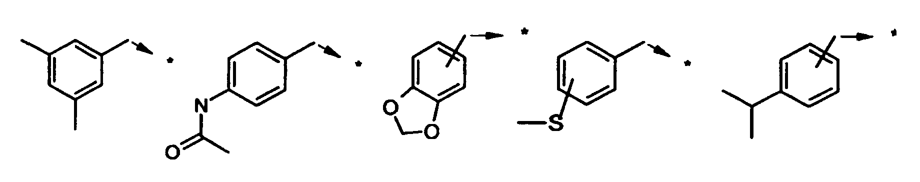 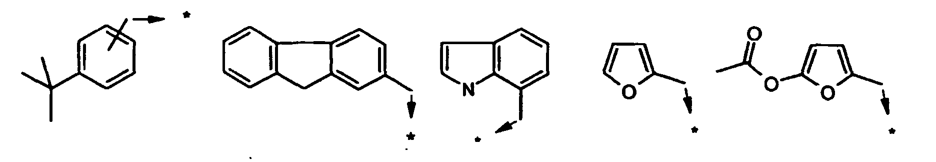 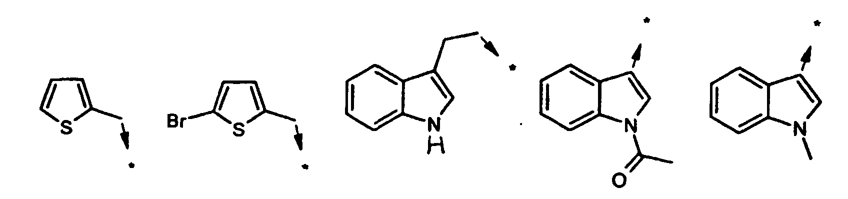 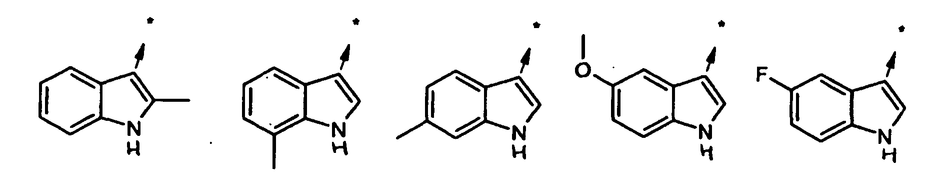 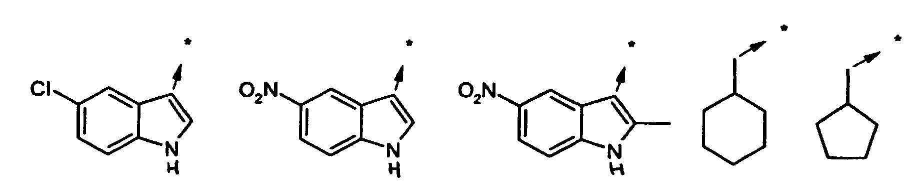 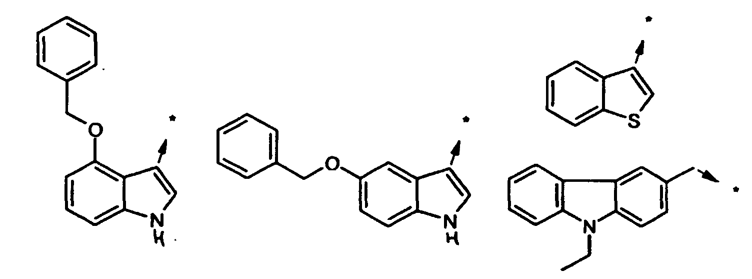 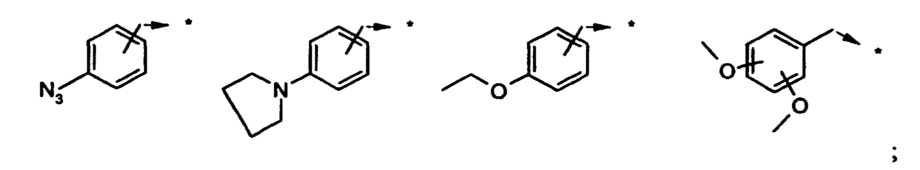
R4 représente l'un des radicaux ci-après : 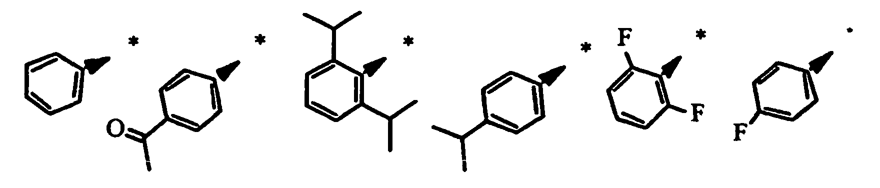 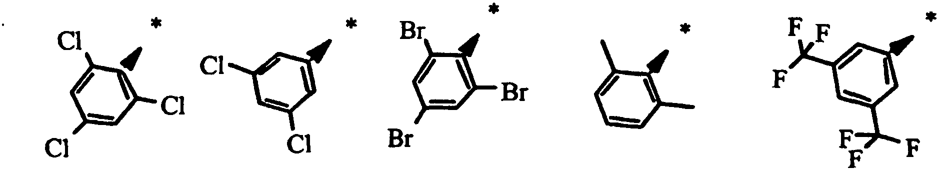 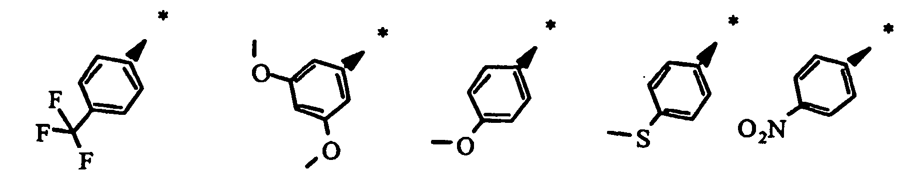 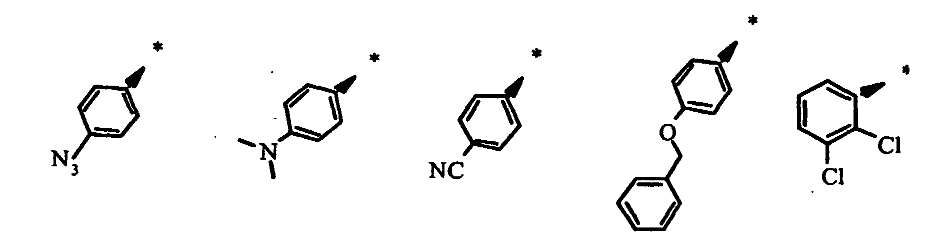 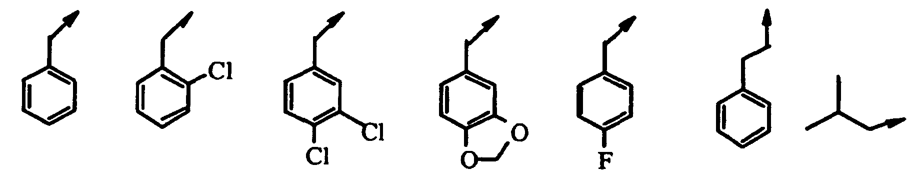 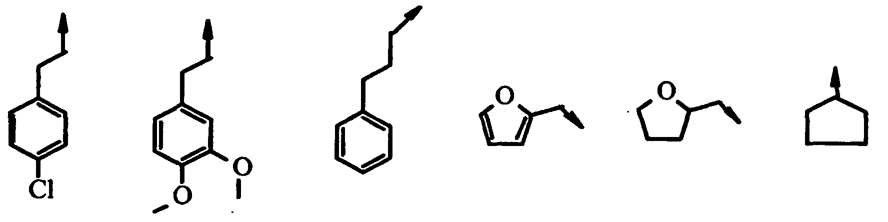 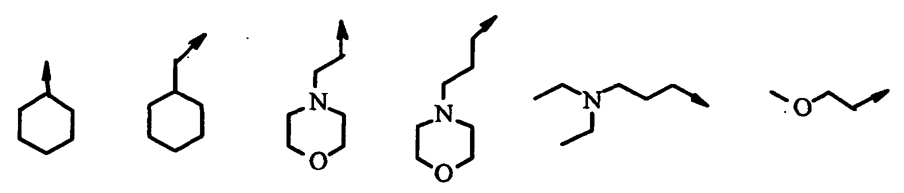 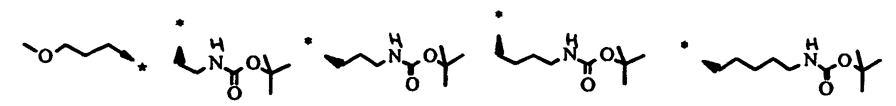 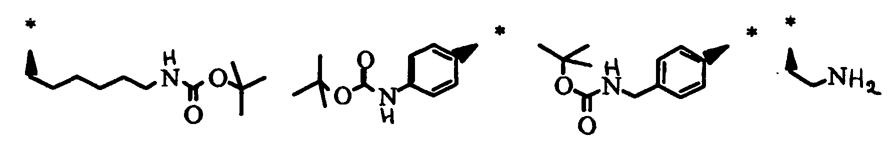 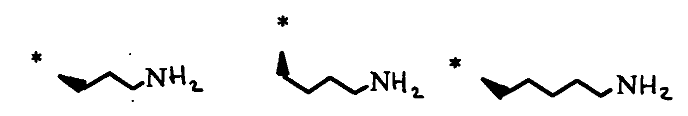 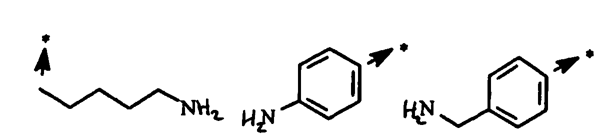 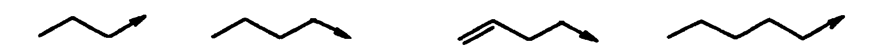 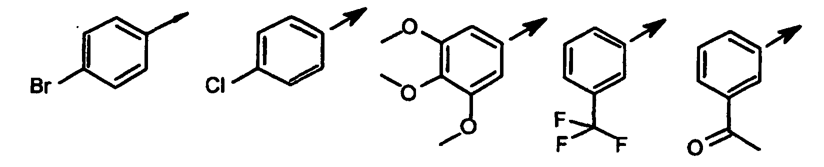 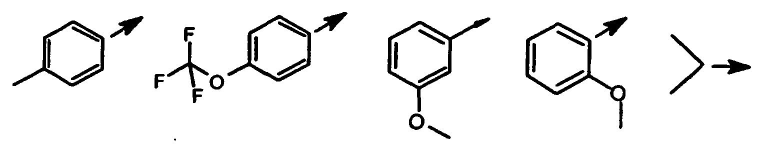 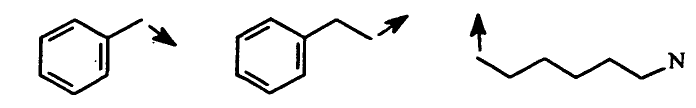 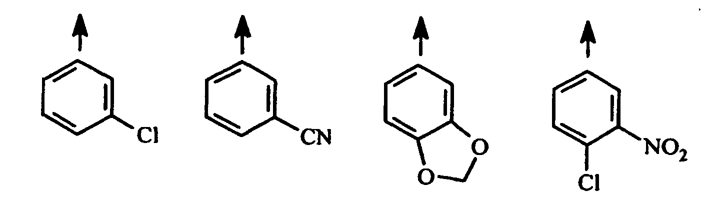 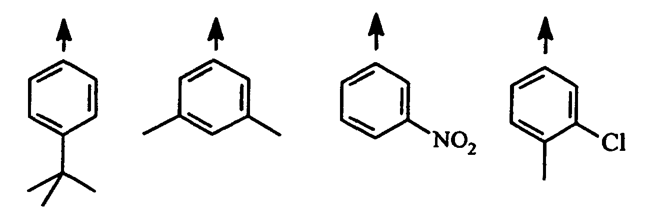 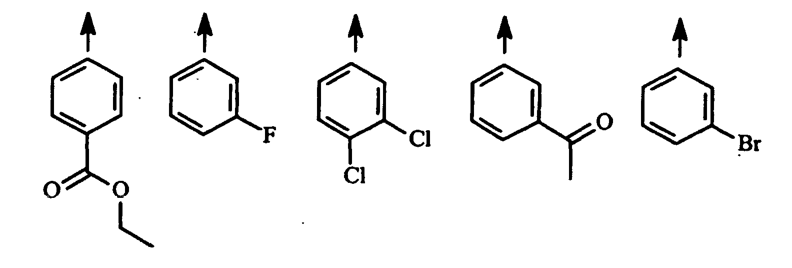 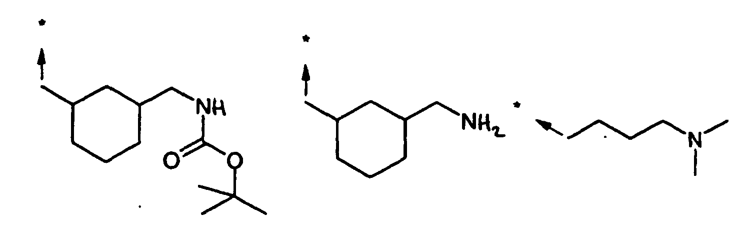 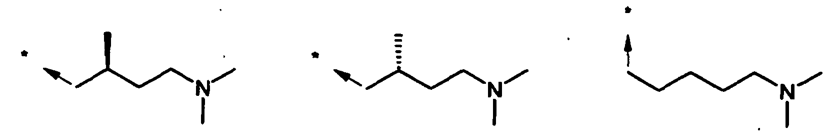 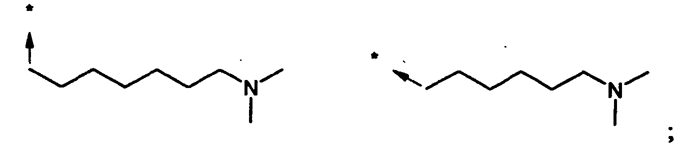
R5 représente H.

De préférence, les composés de formule générale **(I)** seront tels que :
R1 représente le radical phényle éventuellement substitué par un atome halogène ou un radical (C₁-C₁₂)alkyle, (C₁-C₁₂)alkoxy ou nitro ;
R2 et R5 représentent H ;
R3 représente H ou (CH₂)ₚ-Z3;
Z3 représente (C₁-C₁₂)alkyle, (C₃-C₈)cycloalkyle, un radical aryle carbocyclique ou hétérocyclique éventuellement substitué, un radical hétérocyclique non aromatique éventuellement substitué, *bis*-arylalkyle, di-arylalkyle ou l'un des radicaux représentés ci-dessous 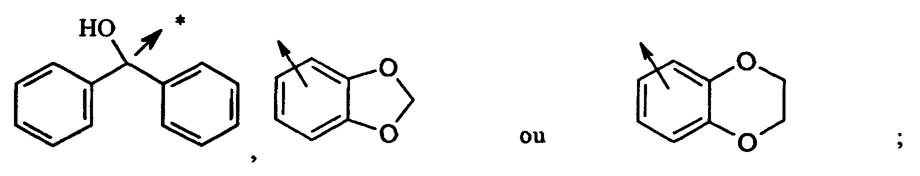
R4 représente (CH₂)ₚ-Z4 ;
Z4 représente amino, (C₃-C₈)cycloalkyle, (C₁-C₁₂)alkylamino, N,N-di-(C₁-C₁₂)alkylamino, amino(C₃-C₆)cycloalkyle, amino(C₁-C₆)alkyl(C₃-C₆)cycloalkyl(C₁-C₆)alkyle, aminoaryle carbocyclique ou hétérocyclique, un radical aryle carbocyclique ou hétérocyclique éventuellement substitué, un radical hétérocyclique non aromatique éventuellement substitué,*bis*-arylalkyle, di-arylalkyle ou l'un des radicaux représentés ci-dessous 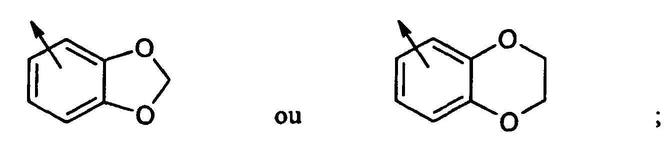 étant entendu qu'un radical éventuellement substitué ou un phényle éventuellement substitué est éventuellement substitué par un ou plus d'un substituant, chacun de préférence choisi indépendamment parmi le groupe constitué des radicaux Cl, F, Br, I, CF₃, NO₂, OH, NH₂, CN, N₃, -OCF₃, (C₁-C₁₂)alkyle, (C₁-C₁₂)alkoxy, -(CH₂)ₚ-phényl-(X1)_{q}, -NH-CO-(C₁-C₆)alkyle, -NH-C(O)O-(C₁-C₆)alkyle, -S-(C₁-C₆)alkyle, -S-phényl-(X1)_{q}, -O-(CH₂)ₚ-phényl-(X1)_{q}, -(CH₂)ₚ-C(O)-O-(C₁-C₆)alkyle, -(CH₂)ₚ-C(O)-(C₁-C₆)alkyle, -O-(CH₂)ₚ-NH₂, -O-(CH₂)ₚ-NH-(C₁-C₆)alkyle, -O-(CH₂)ₚ-N-di-((C₁-C₆)alkyl) et -((C₀-C₁₂))alkyl-(X1)_{q} ;
X1, à chaque fois qu'il intervient, est indépendamment choisi parmi le groupe constitué des radicaux H, Cl, F, Br, I, CF₃, NO₂, OH, NH₂, CN, N₃, -OCF₃, (C₁-C₁₂)alkyle, (C₁-C₁₂)alkoxy, -S-(C₁-C₆)alkyle, -(CH₂)ₚ-amino, -(CH₂)ₚ-NH-(C₁-C₆)alkyle, -(CH₂)ₚ-N-di-((C₁-C₆)alkyl), -(CH₂)ₚ-phényle et -(CH₂)ₚ-NH-(C₃-C₆)cycloalkyle ;
p à chaque fois qu'il intervient est indépendamment 0 ou un entier de 1 à 6 ;
q à chaque fois qu'il intervient est indépendamment un entier de 1 à 5 ;
X représente O ou S ;
n représente 0 ou 1; et enfin
lorsque n représente 0, m représente 1, 2 ou 3, et lorsque n représente 1, m représente 0 ou 1;
ou un sel pharmaceutiquement acceptable d'un tel composé.

Plus préférentiellement, les composés de formule générale **(I)** seront tels que :
R1 représente le radical phényle éventuellement substitué par un atome halogène ou un radical (C₁-C₁₂)alkyle, (C₁-C₁₂)alkoxy ou nitro ;
R2 et R5 représentent H ;
R3 représente (CH₂)ₚ -Z3,
Z3 représentant un radical (C₃-C₈)cycloalkyle ou un radical éventuellement substitué choisi parmi les radicaux phényle, naphtyle, furannyle, thiophène, indolyle, pyrrolyle et benzothiophène ;
R4 représente (CH₂)ₚ-Z4 ;
Z4 représentant amino, (C₁-C₁₂)alkylamino, N,N-di-(C₁-C₁₂)alkylamino ou amino(C₁-C₆)alkyl(C₃-C₆)cycloalkyl-(C₁-C₆)alkyle ;
X représente S ;
p à chaque fois qu'il intervient est indépendamment 0 ou un entier de 1 à 6 ;
m représente 0, 1 ou 2 ; et enfin
n représente 0 ou 1.

Encore plus préférentiellement, les composés de la présente invention seront des composés :
- de sous-formule générale **(I)a** représentée ci-dessous : 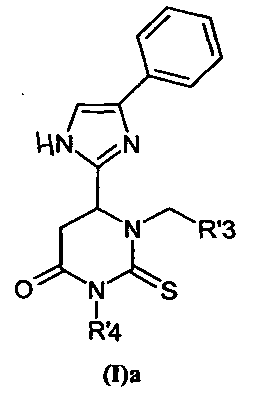 dans laquelle :
   R'3 représente l'un des radicaux représentés ci-dessous : 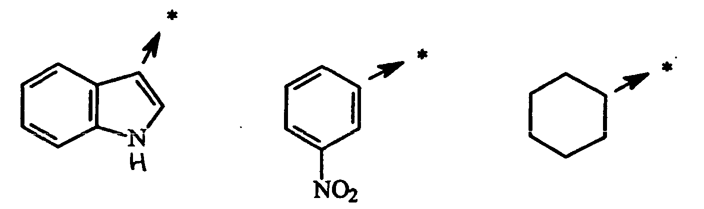 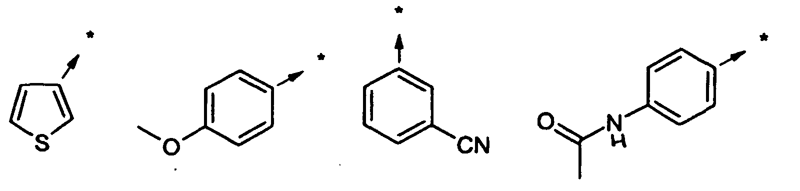
   et R'4 représente l'un des radicaux représentés ci-dessous : 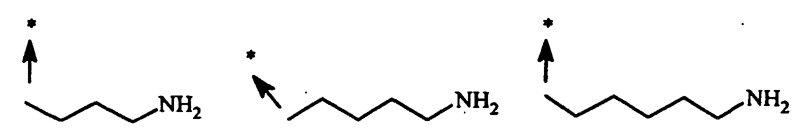 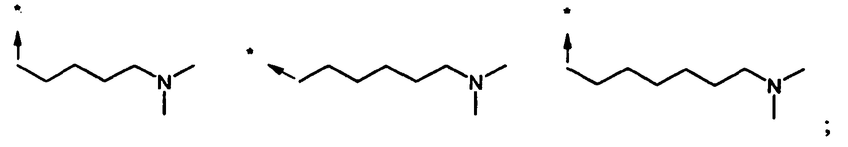
- de sous-formule générale **(I)b** représentée ci-dessous : 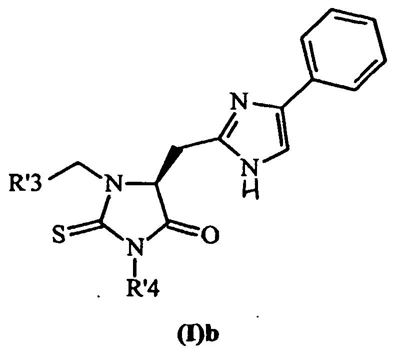 dans laquelle :
   R'3 représente l'un des radicaux représentés ci-dessous : 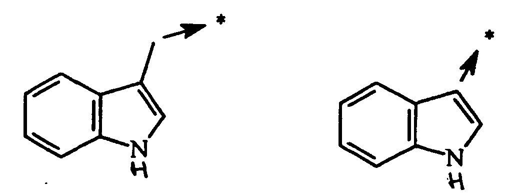
   et R'4 représente l'un des radicaux représentés ci-dessous : 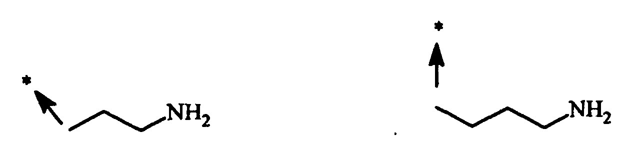 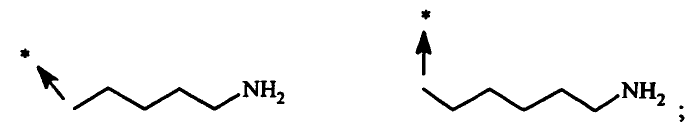
- de sous-formule générale **(I)c** représentée ci-dessous : 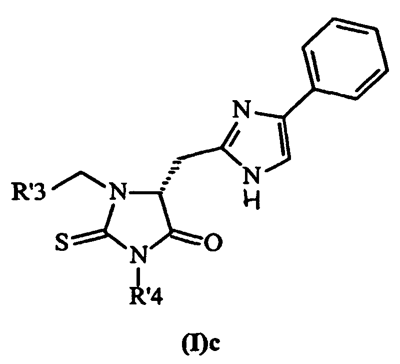 dans laquelle :
   R'3 représente l'un des radicaux représentés ci-dessous : 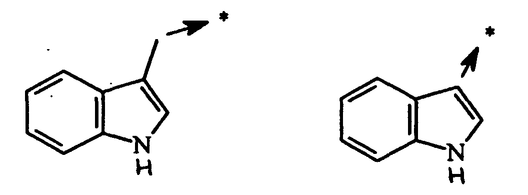
   et **R'4** représente l'un des radicaux représentés ci-dessous : 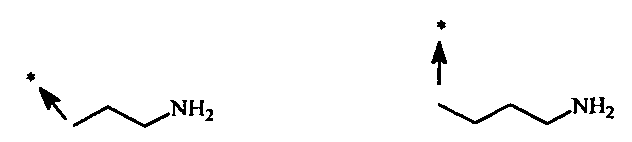 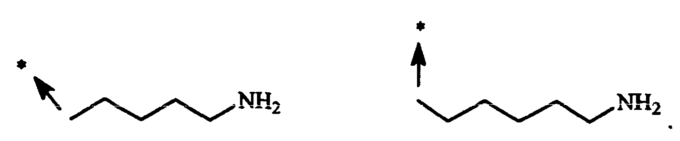
ou un sel pharmaceutiquement acceptable d'un tel composé.

L'invention concerne de plus des procédés de préparation des composés de formule générale **(I)** décrits précédemment (applicables également aux composés correspondants de sous-formules générale **(I)a, (I)b** et **(I)c**).

Les composés de formule générale **(I)** décrite précédemment pour lesquels n représente 0 et X représente O ou S peuvent être préparés par réaction dans un solvant aprotique du composé de formule générale **(II)** représentée ci-dessous 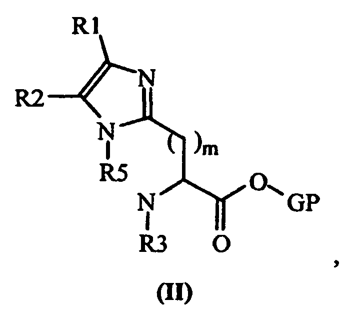 dans laquelle m, R1, R2, R3 et R5 ont la même signification que dans la formule générale **(I)**, et le radical O-GP est un groupe protecteur partant dérivé d'un alcool et en particulier benzyloxy, méthoxy ou tert-butoxy,
avec un isocyanate ou isothiocyanate de formule générale **(III)**

R4-N=C=X , **(III)**

dans laquelle R4 et X ont la même signification que dans la formule générale **(I)**,
de préférence en présence d'une base tertiaire pendant une durée d'environ 1 à 24 heures et à une température de préférence comprise entre 20 et 60 °C.

Les composés de formule générale **(I)** décrite précédemment pour lesquels n représente 1 et X représente O ou S peuvent être préparés par réaction dans un solvant aprotique du composé de formule générale **(IV)** représentée ci-dessous 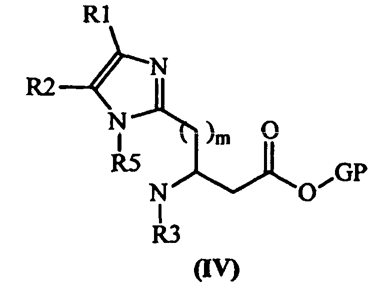 dans laquelle m, R1, R2, R3 et R5 ont la même signification que dans la formule générale **(I)**, et le radical O-GP est un groupe protecteur partant dérivé d'un alcool et en particulier benzyloxy, méthoxy ou tert-butoxy,
avec un isocyanate ou isothiocyanate de formule générale **(III)**

R4-N=C=X **(III)**

dans laquelle R4 et X ont la même signification que dans la formule générale **(I)**,
de préférence en présence d'une base tertiaire pendant une durée d'environ 1 à 48 heures et à une température de préférence comprise entre 20 et 70 °C.

Pour les procédés ci-dessus, le solvant aprotique est de préférence polaire et pourra en particulier être le THF ou le dichlorométhane. La base tertiaire sera par exemple la triéthylamine ou la *N,N*-diisopropyléthylamine.

L'invention offre de plus de nouveaux intermédiaires de synthèse utiles pour la préparation des composés de formule générale **(I)**. Ces composés, précurseurs des composés de formule générale **(II)** et **(IV)**, répondent à la formule générale **(V)** : 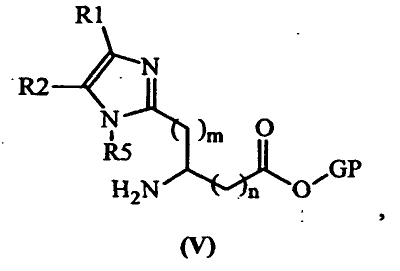 dans laquelle
R1, R2, R5, m et n ont la même signification que dans la formule générale **(I)** ;
et le radical O-GP est un groupe protecteur choisi parmi has groupes benzyloxy, méthoxy ou tert-butoxy.

Les composés suivants répondant à la formule générale **(V)** sont des intermédiaires préférés :
- (2*S*)-2-amino-3-[(4-phényl)-1H-imidazol-2-yl]propanoate de benzyle ;
- (2*R*)-2-amino-3-[(4-phényl)-1H-imidazol-2-yl]propanoate de benzyle ;
- (2*S*)-2-amino-4-[(4-phényl)-1H-imidazol-2-yl]butanoate de benzyle ;
- (2*R*)-2-amino-4-[(4-phényl)-1H-imidazol-2-yl]butanoate de benzyle ;
- (3R)-3-amino-4-[(4-phényl)-1H-imidazol-2-yl]propanoate de benzyle ;
- (3S)-3-arnino-4-[(4-phényl)-1H-imidazol-2-yl]propanoate de benzyle.

L'invention a également pour objet, à titre de médicaments, les composés de formules générales **(I), (I)a, (I)b** et **(I)c** décrits précédemment ou leurs sels pharmaceutiquement acceptables. Elle concerne aussi des compositions pharmaceutiques comprenant lesdits composés ou leurs sels pharmaceutiquement acceptables, et leur utilisation pour la préparation d'un médicament destiné à traiter les états pathologiques ou les maladies dans lesquels un (ou plusieurs) des récepteurs de la somatostatine est (sont) impliqué(s).

En particulier, les composés de formules générales **(I), (I)a, (I)b** et **(I)c** décrits précédemment ou leurs sels pharmaceutiquement acceptables pourront être utilisés pour la préparation d'un médicament destiné à traiter les états pathologiques ou les maladies choisis parmi le groupe composé des états pathologiques ou des maladies qui suivent : l'acromégalie, les adénomes hypophysaires, la maladie de Cushing, les gonadotrophinomes et les prolactinomes, les effets secondaires cataboliques des glucocorticoïdes, le diabète insulinodépendant, la rétinopathie diabétique, la néphropathie diabétique, le syndrome X, le phénomène de Dawn, l'angiopathie, l'angioplastie, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, les ulcères, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, les diarrhées induites par la chimiothérapie, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, le saignement des vaisseaux greffés, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et, dans d'autres domaines thérapeutiques, les céphalées y compris les céphalées associées aux tumeurs hypophysaires, les douleurs, les désordres inflammatoires comme l'arthrite, les accès de panique, la chimiothérapie, la cicatrisation des plaies, l'insuffisance rénale résultant d'un retard de croissance, l'hyperlipidémie, l'obésité et le retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis, le rejet chronique des allogreffes ainsi que la maladie d'Alzheimer et enfin l'ostéoporose.

De préférence, les composés de formules générales **(I), (I)a, (I)b** et **(I)c** décrits précédemment ou leurs sels pharmaceutiquement acceptables pourront être utilisés pour la préparation d'un médicament destinés à traiter les états pathologiques ou les maladies choisis parmi le groupe composé des états pathologiques ou des maladies qui suivent : l'acromégalie, les adénomes hypophysaires ou les tumeurs gastroentéropancréatiques endocriniennes dont le syndrome carcinoïde, et les saignements gastro-intestinaux.

Par sel pharmaceutiquement acceptable, on entend notamment des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthane sulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", *J. Pharm. Sci*. **66**:1 (1977).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau. Les suspensions comprendront en particulier les suspensions de microparticules à libération prolongée chargées en principe actif (notamment des microparticules en polylactide-co-glycolide ou PLGA - cf. par exemple les brevets US 3,773,919, EP 52 510 ou EP 58 481 ou la demande de brevet PCT WO 98/47489), lesquelles permettent l'administration d'une dose journalière déterminée sur une période de plusieurs jours à plusieurs semaines.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Les composés de la présente invention sont préparés selon les procédures suivantes.

### PREPARATION DES COMPOSES DE L'INVENTION

### PREPARATION DE DERIVES IMIDAZOLYLE

### Procédure générale :

### i) Cyclisation pour obtenir le groupe imidazole :

Un aminoacide est converti en son sel de césium en utilisant du carbonate de césium dans un solvant polaire comme un mélange DMF/H₂O (1:1) ou EtOH/H₂O (1:1). Un ester est alors obtenu en utilisant une bromocétone appropriée dans un solvant polaire aprotique comme du DMF anhydre. Le bromure de césium formé est éliminé par filtration et de l'acétate d'ammonium est ajouté dans un solvant aprotique ayant une température d'ébullition élevée comme le xylène ou le toluène ou dans un solvant protique acide comme l'acide acétique. Le mélange est maintenu à reflux en utilisant un piège Dean-Stark pendant une demi-heure à une heure. Dans le schéma immédiatement ci-dessous, PG1 est un groupe protecteur, de préférence un carbamate, comme t-Boc ou benzyl carbamate, et PG2 est également un groupe protecteur, de préférence un groupe benzyle. 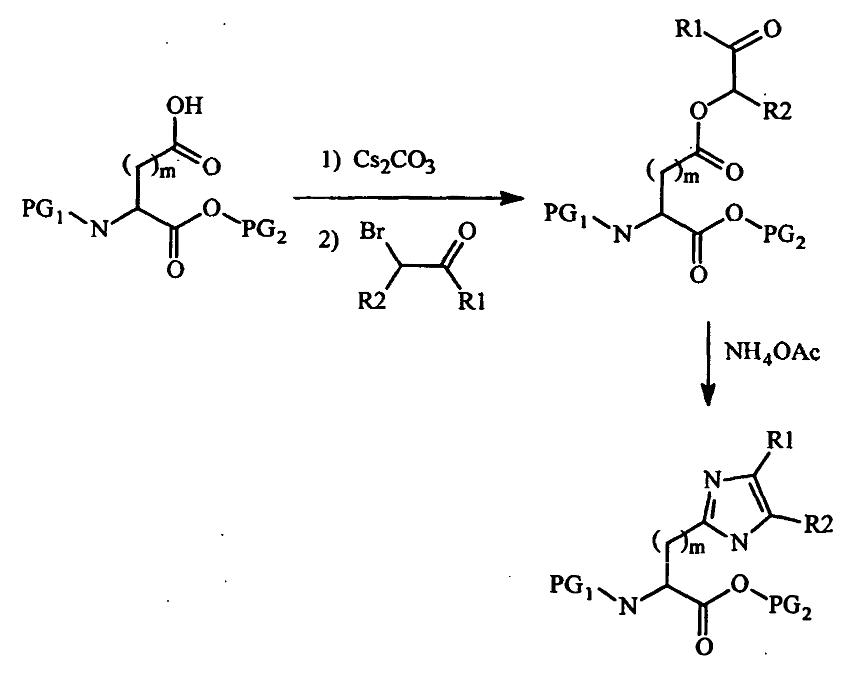 **ou**

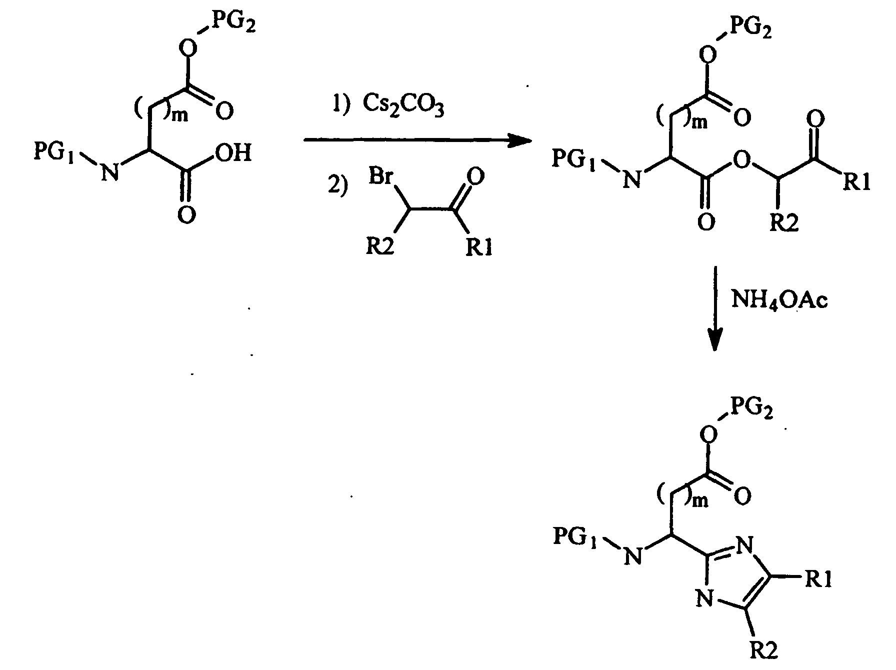

### ii) N-substitution sur le groupe imidazole :

Le cas échéant, pour les composés de formule générale **(I)** pour lesquels R5 ne représente pas H, la N-substitution sur le groupe imidazole est réalisée par la réaction décrite ci-après.

Une solution de l'intermédiaire obtenu à l'étape précédente, un agent alkylant tel qu'une α-bromocétone, un α-bromoester, d'un bromure d'alkyle ou d'aryle, en présence d'une base organique ou inorganique (éventuellement supportée sur une résine telle qu'une résine polystyrène), dans un solvant aprotique tel que le THF, l'acétonitrile ou le DMF, est chauffée à une température de 20 à 80 °C pendant une durée de 2 à 48 heures.

### Préparation du (2S)-2-[(tert-butoxycarbonyl)amino]-3-(4-phényl-1H-imidazol-2-yl)propanoate de benzyle

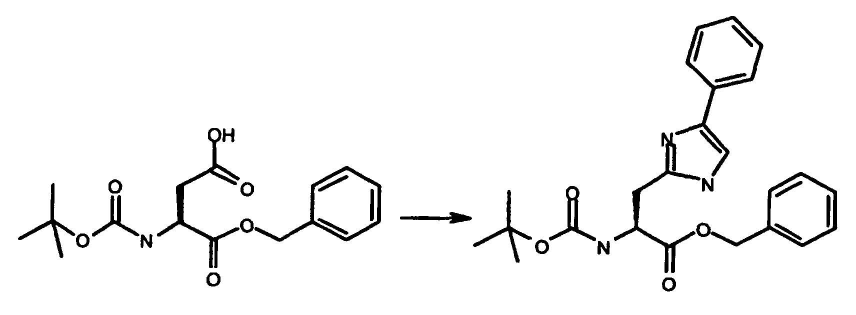

Une solution de Boc-L-Asp-OBn (12 g ; 37,1 mmol) et de carbonate de césium (6,05 g ; 0,5 éq.) dans EtOH/H₂O (1:1, 7 ml) est agitée pendant environ 30 minutes à environ 20 °C, puis concentrée sous pression réduite à environ 40 °C.
25 ml d'une solution de 2-bromoacétophénone (7,38 g ; 1 éq.) dans du DMF sec sont ajoutés au sel résultant dissous dans 130 ml de DMF sec. Le mélange est agité pendant environ 1 h à environ 20 °C sous atmosphère d'argon puis concentré sous pression réduite. De l'acétate d'éthyle est ajouté (100 ml) et le mélange filtré, CsBr étant lavé avec de l'acétate d'éthyle. Le filtrat est alors concentré sous pression réduite.
Une solution du résidu obtenu et d'acétate d'ammonium (58 g ; 20 éq.) dans du xylène (280 ml) est maintenue à reflux pendant environ une demi-heure à environ 140 °C. L'excédent de NH₄OAc et d'eau est éliminé grâce à un piège Dean-Stark. L'avancement de la réaction est suivi par chromatographie sur couche mince (CCM ; éluant : acétate d'éthyle / heptane 1:1). Le mélange est alors ramené à environ 20 °C puis lavé successivement avec de l'eau, une solution saturée en NaHCO₃ solution jusqu'à obtention d'un pH basique puis avec de la saumure jusqu'à pH neutre. La phase organique est alors séchée sur Na₂SO₄ et concentrée sous pression réduite.
La purification du résidu résultant par chromatographie éclair sur gel de silice (éluant : acétate d'éthyle / heptane 1: 1) donne le composé attendu (8,2 g ; 52 %).
RMN (¹H, 400 MH₂, CDCl₃) : 7,64-7,14 (m, 11H, H arom) ; 5,95 (d, 1H, NHBoc) ; 5,21-5,13 (AB, 2H, OCH₂Ph, J_{AB} = 12Hz) ; 4,73 (m, 1H, CH) ; 3,30 (m, 2H, CH₂) ; 1.42 (s, 9H, (CH₃)₃C).
SM/CL : MM calculée = 421,2 ; m/z = 422,2 (M+H).

Les composés suivants sont préparés de façon analogue à la procédure décrite pour le (2S)-2-[(tert-butoxycarbonyl)amino]-3-(4-phényl-1H-imidazol-2-yl)propanoate de benzyle :

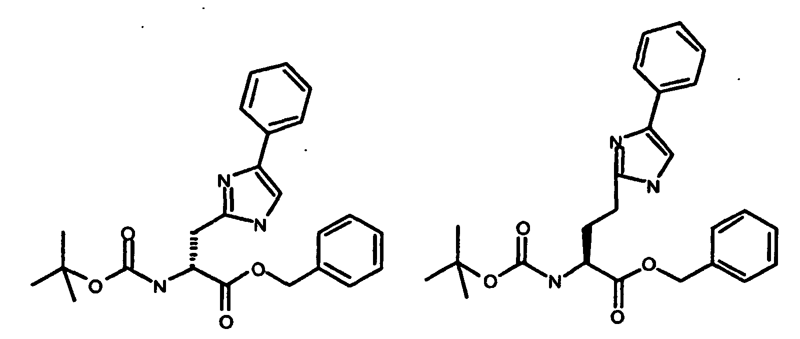

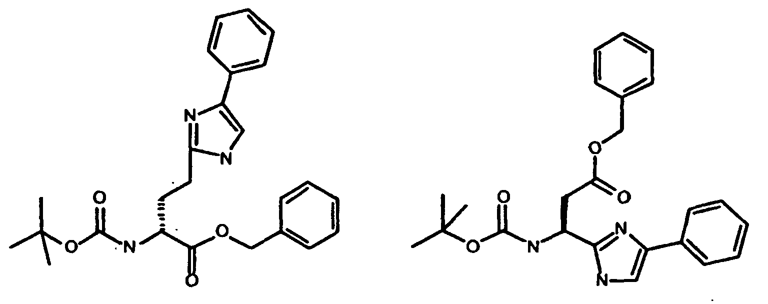

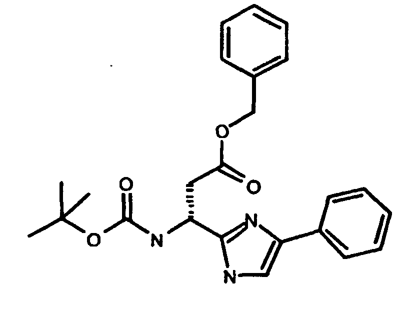

### Etape de déprotection

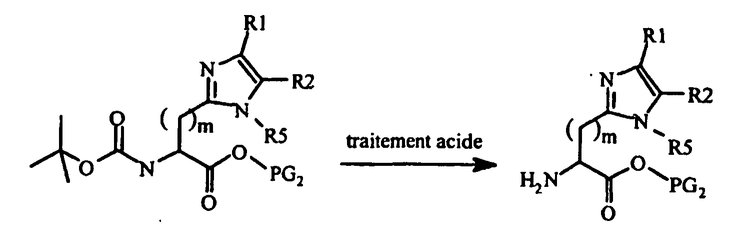

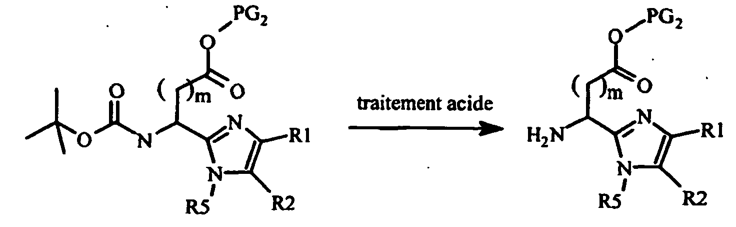

Procédure générale : les dérivés imidazolyle protégés par N-Boc sont traités avec un acide organique ou inorganique comme l'acide trifluoroacétique ou le chlorure d'hydrogène (aqueux ou sous forme gazeuse) dans un solvant aprotique comme le dichlorométhane ou l'acétate d'éthyle à une température comprise entre 0 °C et 25 °C pendant 0,5 à 5 heures.

### Préparation du dichlorhydrate de (3S)-3-(4-phényl-1H-imidazol-2-yl)-3-aminopropanoate de benzyle

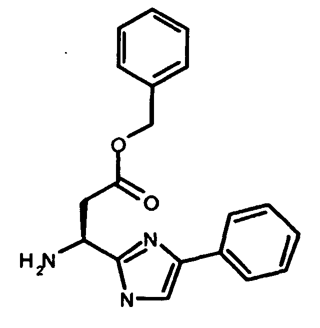

Une solution de (3S)-3-(4-phényl-1H-imidazol-2-yl)-3-[(tert-butoxycarbonyl)amino propanoate de benzyle (5 g) dans de l'acétate d'éthyle (120 ml) à 0 °C est traversée par un flux de HCl sec jusqu'à ce que la CCM (éluant : acétate d'éthyle 100%) montre une disparition complète du composé de départ. Le mélange résultant est alors évaporé sous pression réduite. Du diéthyléther est ajouté au solide obtenu et le mélange est filtré. Le chlorhydrate est lavé plusieurs fois avec du dichlorométhane puis du diéthyléther et séché sous pression réduite pour donner 4,6 g du composé attendu (98 % de rendement). RMN (¹H, 400 MHz, DMSOd6) : 9,21 (s large, 2H, NH) ; 8,03-7,28 (m, H arom, 11H) ; 5,10 (s, 1H, OCH₂Ph) ; 5,04 (m, 1H, CH) ; 3,61 (dd, 1H, CH₂, 3J = 9 Hz, 2J = 17,0 Hz) ; 3,39 (dd, 1H, CH₂', 3J = 5,5 Hz, 2J = 17,0 Hz). SM/CL : MM calculée = 321,2 ; m/z = 322,1 (M+H).

Les composés suivants sont préparés de façon analogue à la procédure décrite pour le dichlorhydrate de (3S)-3-(4-phényl-1H-imidazol-2-yl)-3-amino-propanoate de benzyle.

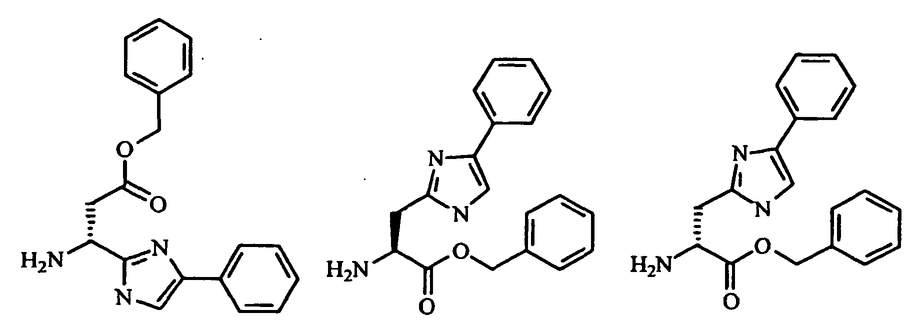

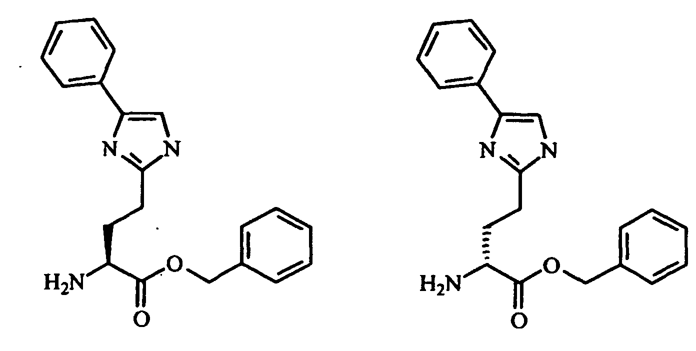

### REACTION DE N-ALKYLATION

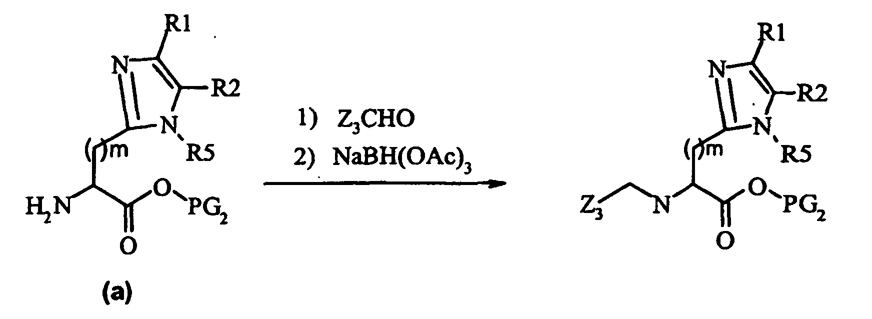

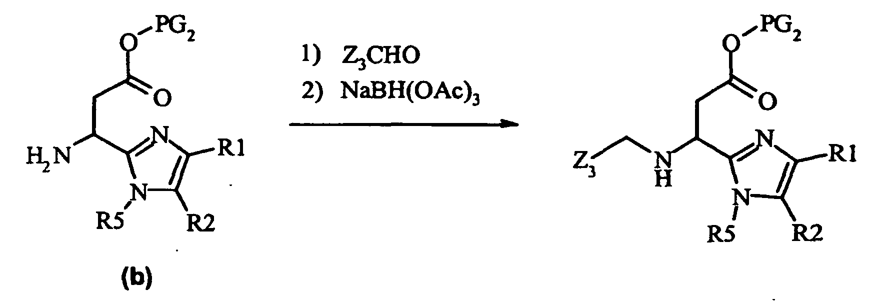

Procédure générale : Une amine libre de formule **(a)** ou **(b)** est traitée avec un aldéhyde dans un solvant protique ou aprotique, de préférence du dichlorométhane ou du tétrahydrofuranne, pendant une durée de 1 à 15 heures à 20-50 °C. L'imine résultante est ensuite réduite grâce à un agent réducteur, de préférence du sodium triacétoxyborohydrure de sodium ou du cyanoborohydrure de sodium avec ou sans la présence d'un acide comme l'acide acétique, à une température comprise entre 20 et 50 °C pendant une durée de 0,2 à 5 heures. Le composé *N*-alkylé est isolé par addition d'eau et extraction suivie d'une chromatographie éclair sur gel de silice ou par cristallisation.

### Préparation du (2S)-4-(4-phényl-1H-imidazol-2-yl)-2-[(3-thiénylméthyl)amino]butanoate de benzyle

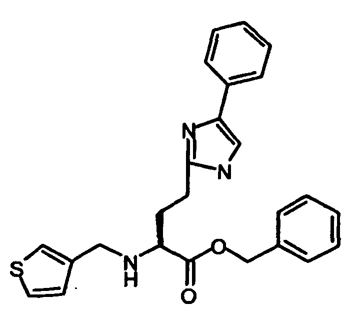

A une solution de (2S)-2-amino-4-(4-phényl-1H-imidazol-2-yl)butanoate de benzyle sous forme de base libre (3,6 g ; 1 éq.) dans du tétrahydrofuranne (ci-après THF, 40 ml) est ajouté du thiophène-3-carboxaldéhyde (1 ml ; 1 éq.). Le mélange est agité pendant 15 heures à environ 20 °C et dilué par addition de 50 ml de tétrahydrofuranne. Du NaBH(OAc)₃ (4,73 g ; 2 éq.) est alors ajouté. Après 1 heure d'agitation à environ 20 °C, la réaction est arrêtée par ajout d'eau (40 ml) et de l'acétate d'éthyle est ensuite ajouté (100 ml). Après décantation et extraction, les phases organiques combinées sont lavées avec de la saumure, séchées sur Na₂SO₄ puis évaporées sous pression réduite à 40 °C. La purification chromatographie éclair sur gel de silice (éluant : acétate d'éthyle / heptane 9:1) donne le composé attendu sous forme d'une huile jaune (3,08 g ; 66 % de rendement).
RMN (¹H, 400 MHz, CDCl₃): 7,62-7,04 (m, 15H, H arom., NH) ; 5,18 (s, 2H, OCH₂) ; 3,87-3,69 (AB, 2H, CH₂NH, 2J_{AB} = 13 Hz) ; 3,38 (dd, 1H, CHNH, 3J = 4,5 Hz, 2J = 8,5 Hz) ; 2,98 (m, 1H, CH₂CH) ; 2,88 (m, 1H, CH₂CH) ; 2,17 (m, 1H, CH₂) ; 1,97 (m, 1H, CH₂).
SM/CL : MM calculée = 431,2 ; m/z = 432,2 (M+H) ; m/z = 430,8 (M-H).

Les composés suivants (dans leurs deux formes énantiomères) sont préparés de façon analogue à la procédure décrite pour le (2S)-4-(4-phényl-1H-imidazol-2-yl)-2-[(3-thiénylméthyl)amino]butanoate de benzyle :

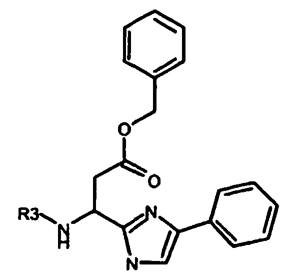

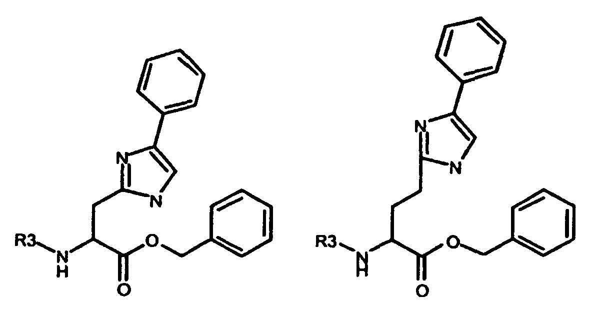

Dans les formules ci-dessus, R3 représente l'un des radicaux ci-après :

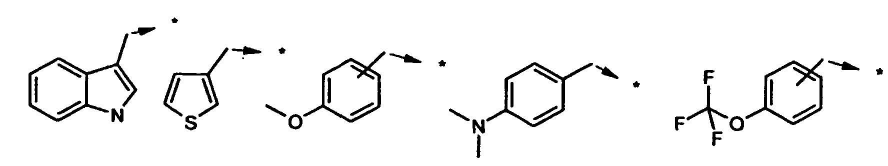

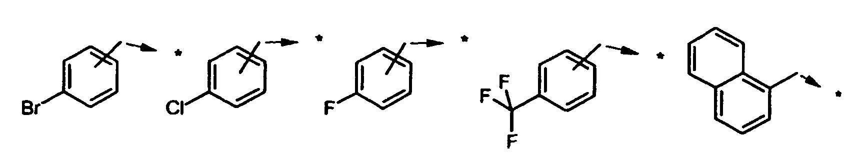

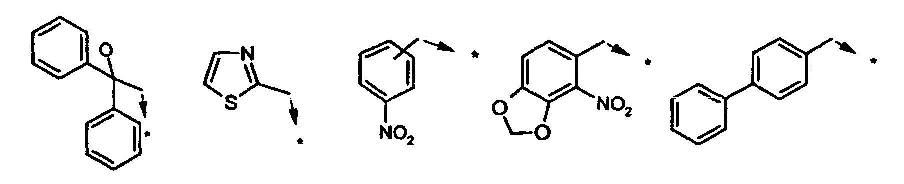

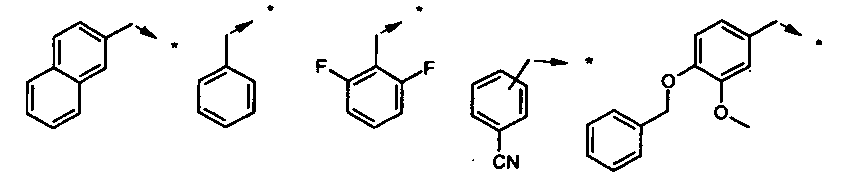

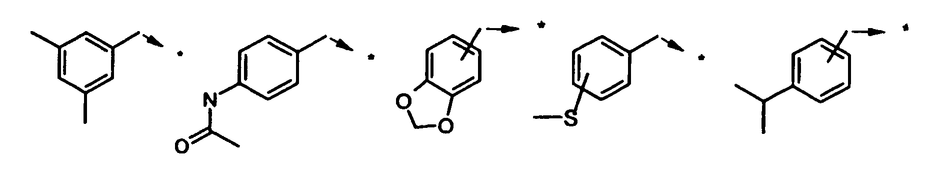

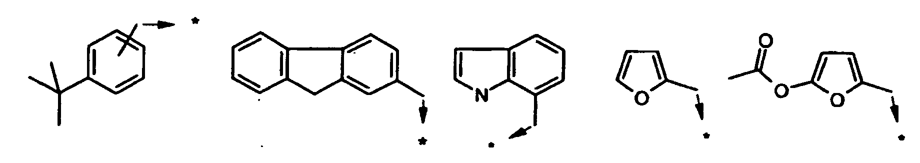

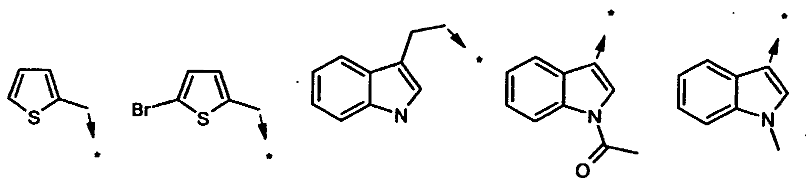

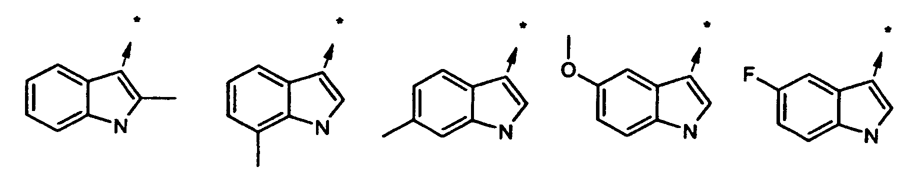

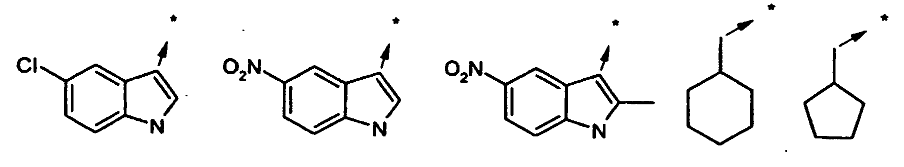

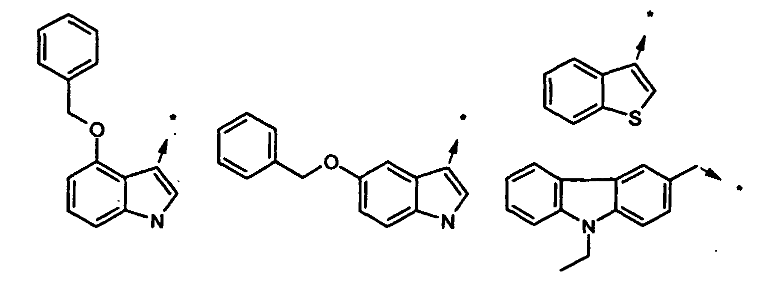

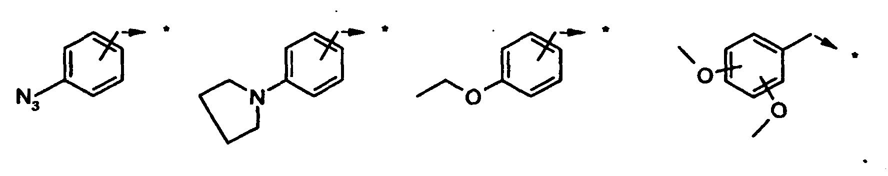

### PREPARATION D'HYDANTOINES ET DE THIOHYDANTOINES

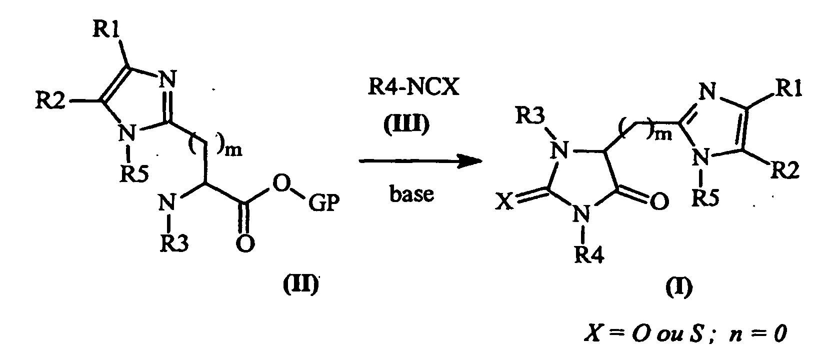

### Procédure générale :

Une amine de formule **(II)**, dans laquelle m, R1, R2, R3 et R5 ont les mêmes significations que dans la formule générale **(I)** et le radical O-GP est un groupe protecteur partant dérivé d'un alcool et en particulier benzyloxy, méthoxy ou tert-butoxy, est traitée avec un isocyanate ou un isothiocyanate de formule générale R4-NCX dans laquelle R4 a la même signification que dans la formule générale **(I)**, en présence ou en absence d'une base tertiaire comme la triéthylamine ou la *N,N*-diisopropyléthylamine, dans un solvant aprotique, de préférence le tétrahydrofuranne ou le dichlorométhané, à une température comprise entre 20 et 60 °C environ et pendant 1 à 24 heures environ. L'hydantoïne ou la thiohydandoïne résultante peut être isolée avec un rendement de 60 à 95 %, soit par chromatographie éclair sur gel de silice, soit par addition au mélange réactionnel d'un réactif nucléophile porté par un polymère comme par exemple une résine aminométhylpolystyrène (acquise auprès de Novabiochem) suivie par la filtration et l'évaporation du filtrat.

Lorsque R4 représente un radical comportant une terminaison amino primaire (par exemple R4 représente aminoéthyle, aminopropyle, etc.), le réactif n'est pas R4-NCX mais le composé correspondant dont le groupe amino est protégé par un groupe protecteur adapté, par exemple un groupe tert-butoxycarbonyle. Il faut donc une étape de déprotection ultérieure (effectuée dans des conditions classiques, à savoir un traitement acide) pour obtenir le composé de formule générale **(I)**.

### Préparation de certains isothiocyanates non commerciaux de formule générale (III) :

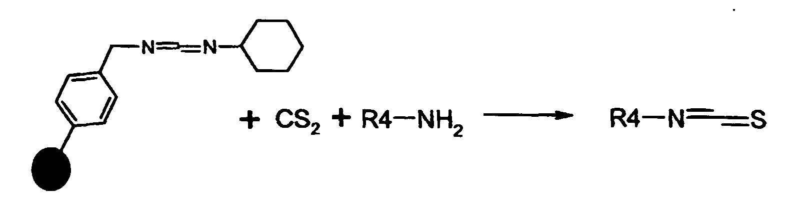

Ces composés sont préparés comme suit : une amine primaire de formule générale R4-NH₂ est traitée avec un mélange de disulfure de carbone et de résine *N*-cyclohexylcarbodiimide *N*-méthyl polystyrène, dans un solvant aprotique, de préférence du tétrahydrofuranne ou du dichlorométhane, pendant une durée de 1 h à 18 h à 20-50°C. L'isothiocyanate résultant est isolé après filtration sur fritté et évaporation du filtrat.

### Préparation de la 6-isothiocyanato-N,N-dimethyl-1-hexanamine

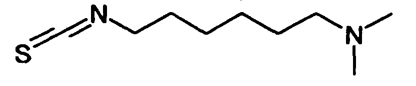

A une suspension de résine *N*-cyclohexylcarbodiimide *N*-méthyl polystyrène (7,8 g ; 1,1 éq ; acquise auprès de Novabiochem ; charge de 1,95 mmol/g) dans du THF anhydre (120 ml) sont successivement additionnés goutte à goutte le disulfure de carbone (8,3 ml ; 10 éq.) et une solution de *N,N*-diméthyl-1,6-hexanediamine (2 g ; 1 éq.) dans le THF (10 ml). La suspension est agitée 2 h à environ 20°C puis filtrée sur fritté. Le filtrat est ensuite concentré à sec sous pression réduite à 40 °C pour donner le dérivé isothiocyanate attendu (2,6 g ; 93% de rendement).
RMN ¹H (400 MHz, CDCl₃, δ) : 3,50 (t, 2H) ; 2,24 (t, 2H) ; 2,20 (s, 6H) ; 1,68 (q, 2H) ; 1,50-1,31 (m, 6H).

Les composés suivants sont préparés de façon analogue à la procédure décrite pour la 6-isothiocyanato-N,N-diméthyl-1-hexanamine :

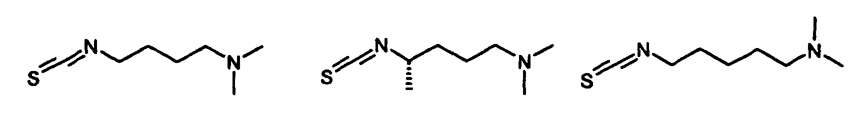

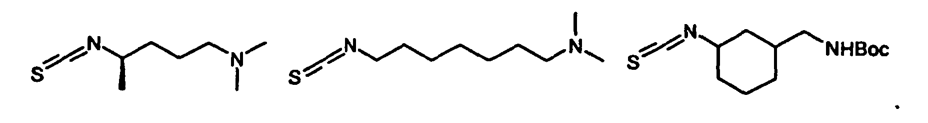

### Préparation de la (5S)-1-(1H-indol-3-ylméthyl)-3-(4-nitrophényl)-5-[2-(4-phényl-1H-imidazol-2-yl)éthyl]-2-thioxo-4-imidazolidinone

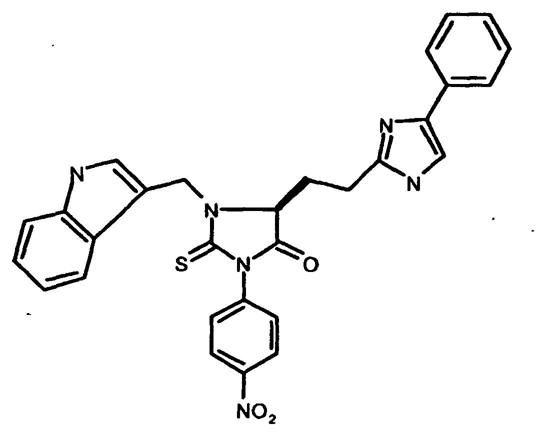

A une solution de (2*S*)-2-[(1*H*-indol-3-ylméthyl)amino]-4-(4-phényl-1*H*-imidazol-2-yl)butanoate de benzyle (93 mg ; 1 éq.) dans du THF (2 ml) est ajouté du 4-nitro-phénylisothiocyanate (43 mg ; 1,2 éq.). Le mélange est agité pendant 2 heures à environ 20 °C puis dilué avec 4 ml de THF. De la résine aminométhylpolystyrène (acquise auprès de Novabiochem, charge de 3,2 mmol/g, 125 mg, 2 éq.) est ajoutée, puis de la triéthylamine (200 µl). Le mélange est agité pendant 15 heures à environ 20 °C puis filtré sur fritté. Le filtrat est concentré à sec sous pression réduite à 40 °C (une coévaporation avec du dichlorométhane est nécessaire pour éliminer l'excès de triéthylamine). La purification du résidu par chromatographie éclair sur gel de silice (éluant: acétate d'éthylelheptane 9:1) donne le composé attendu (90 mg; 84 % de rendement).
RMN (¹H, 400 MHz, CDCl₃) : 8,24-7,09 (m, 17H, H arom, NH) ; 5,88, 4,64 (AB, 2H, CH₂N, 2J_{AB} = 15 Hz) ; 3,38 (dd, 1H, CH, 3J = 3,0 Hz, 2J = 8,5 Hz) ; 2,92 (m, 2H, CH₂CH) ; 2,74 (m, 1 H, CH₂) ; 2,24 (m, 1H, CH₂).
SM/CL : MM calculée = 536,2 ; m/z = 537,1 (M+H).

Les composés suivants (dans leurs deux formes énantiomères) sont préparés de façon analogue à la procédure décrite pour la (5S)-1-(1H-indol-3-ylméthyl)-3-(4-nitrophényl)-5-[2-(4-phényl-1H-imidazol-2-yl)éthyl]-2-thioxo-4-imidazolidinone (hormis la purification finale par chromatographie éclair sur gel de silice qui est facultative) :

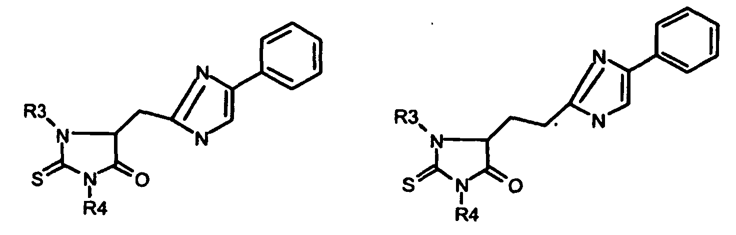

Dans les formules ci-dessus, R3 représente l'un des radicaux ci-après : 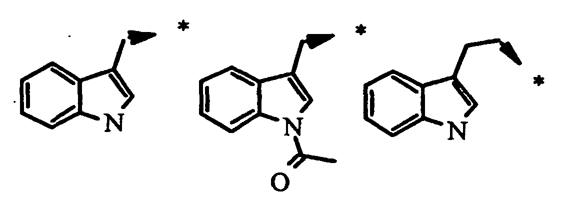 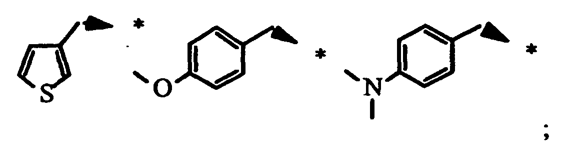 et R4 représente l'un des radicaux ci-après :

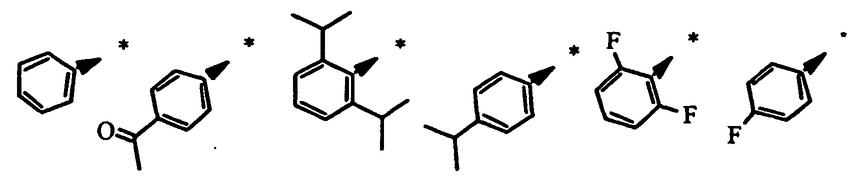

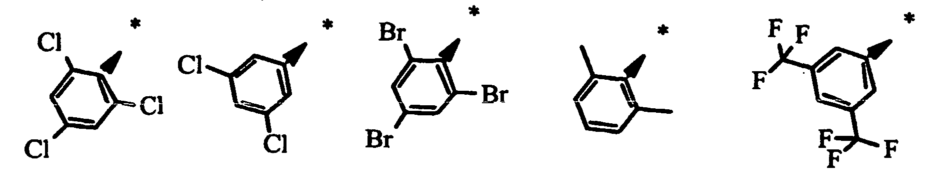

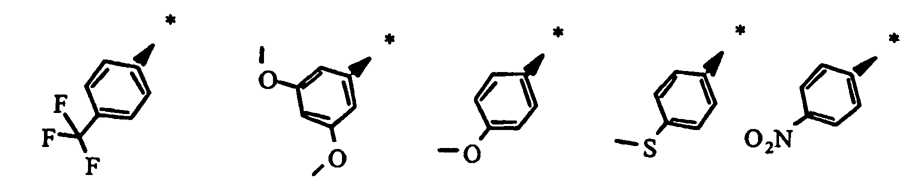

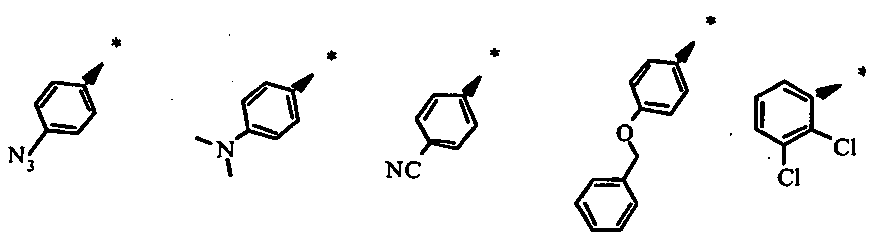

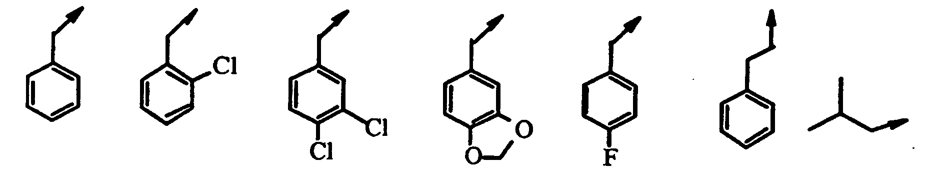

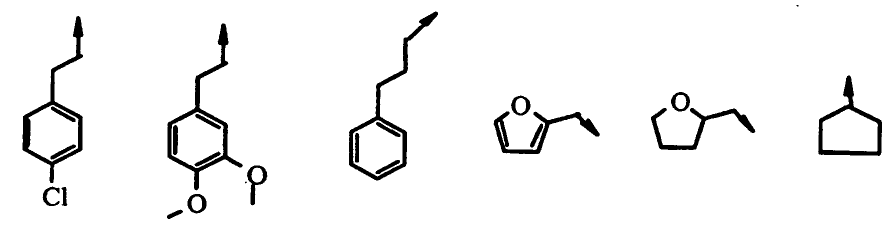

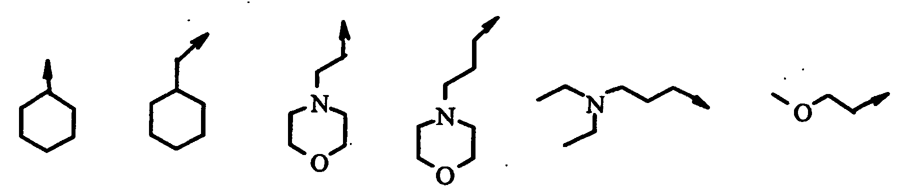

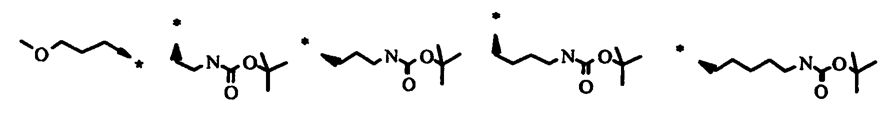

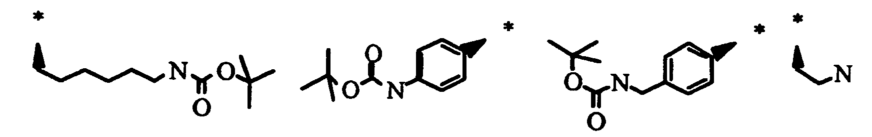

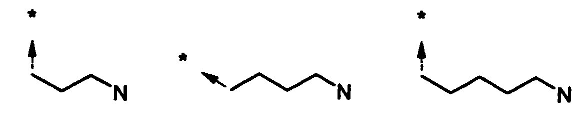

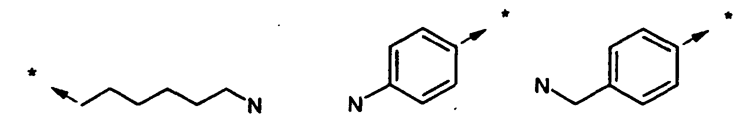

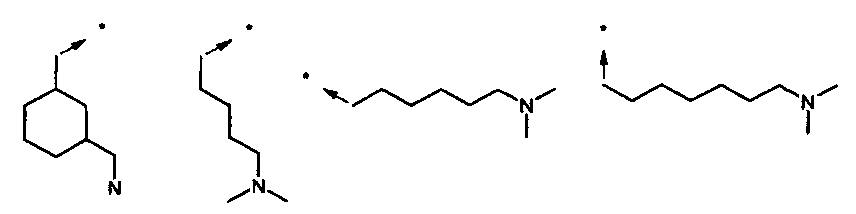

### Préparation de la (5S)-1-(1H-indol-3-ylméthyl)-5-[2-(4-phényl-1H-imidazol-2-yl)éthyl]-3-[3-(trifluorométhyl)phényl]-2,4-imidazolidinedione

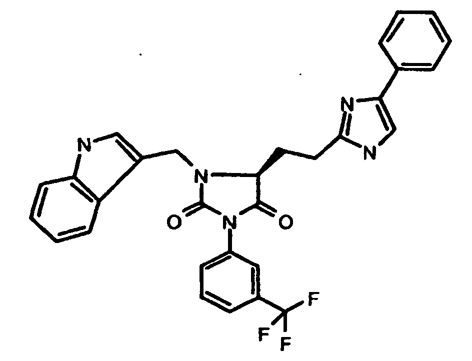

A une solution de (2*S*)-2-[(1*H*-indol-3-ylméthyl)amino]-4-(4-phényl-1*H*-imidazol-2-yl)butanoate de benzyle (23 mg, 1 éq.) dans 2 ml de THF est ajouté du 3-trifluorométhyl-phénylisocyanate (11 mg, 1,2 éq.). Le mélange est agité pendant 2 heures à environ 20 °C puis dilué avec 2 ml de THF. De la résine aminométhylpolystyrène (acquise auprès de Novabiochem, charge de 3,2 mmol/g, 125 mg, 2 éq.) est ajoutée, puis de la triéthylamine (200 µl). Le mélange est agité pendant 15 heures à environ 20 °C puis filtré sur fritté. Le filtrat est ensuite concentré à sec sous pression réduite à 40 °C (une coévaporation avec du dichlorométhane est nécessaire pour éliminer l'excès de triéthylamine) pour donner le composé attendu (25 mg, 92% de rendement).
RMN (¹H, 400 MHz, CDCl₃) : 7,75-6,99 (m, 17H, H arom, NH) ; 5,25, 4,44 (AB, 2H, CH₂N, J_{AB} = 15 Hz); 3,77 (m, 1H, CH); 2,92 (m, 1H, CH₂CH) ; 2,88 (m, 1H, CH₂CH) ; 2,72 (m, 1H, CH₂) ; 2,17 (m, 1H, CH₂).
SM/CL : MM calculée = 543,2 ; m/z = 544,2 (M+H).

Les composés suivants (dans leurs deux formes énantiomères) sont préparés de façon analogue à la procédure décrite pour la (5S)-1-(1H-indol-3-ylméthyl)-5-[2-(4-phényl-1H-imidazol-2-yl)éthyl]-3-[3-(trifluorométhyl)phényl]-2,4-imidazolidinedione :

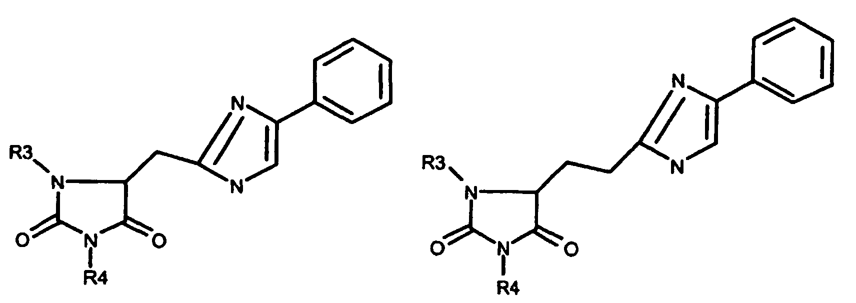

Dans les formules ci-dessus, R3 représente l'un des radicaux suivants : 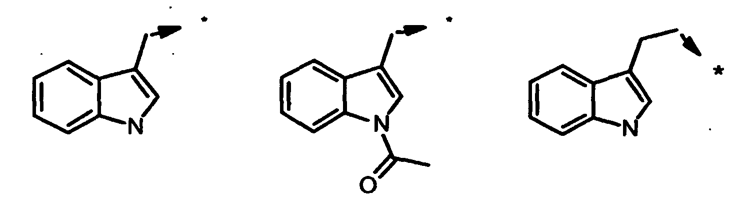 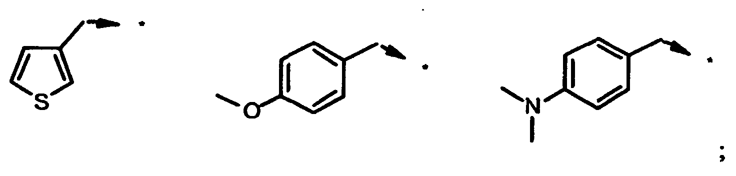 et R4 représente l'un des radicaux suivants :

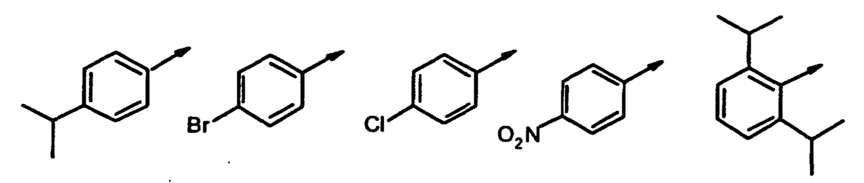

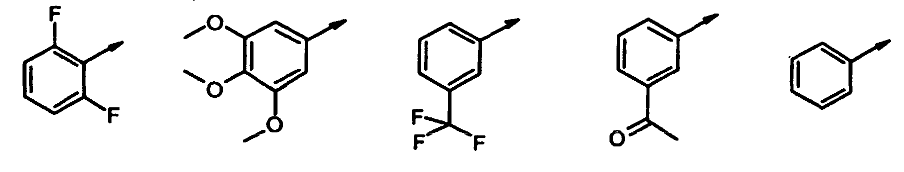

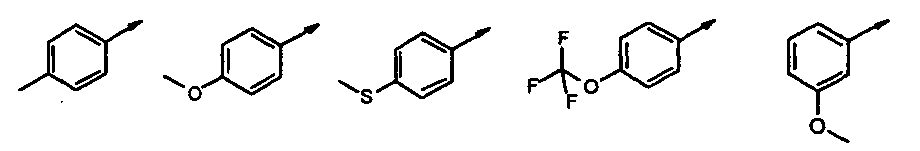

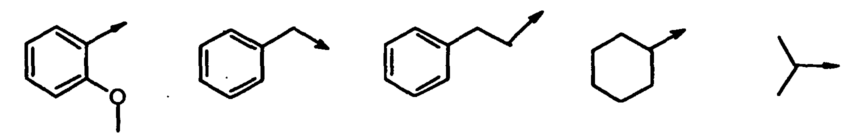

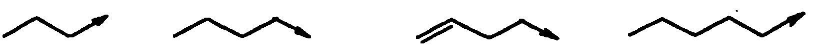

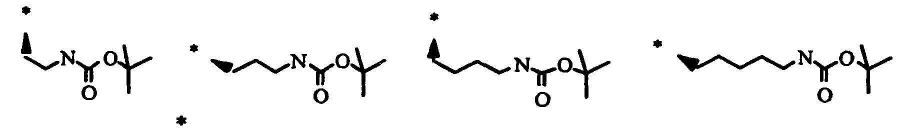

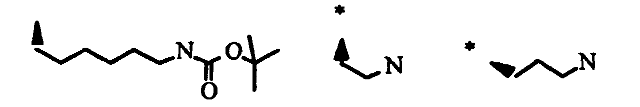

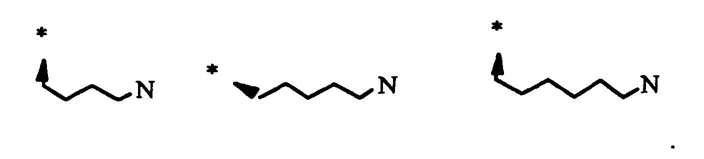

### PREPARATION DE DIHYDROPYRIMIDIN-2,4-DIONES ET DE 2-THIOXO-TETRAHYDRO-4-PYRIMIDINONES

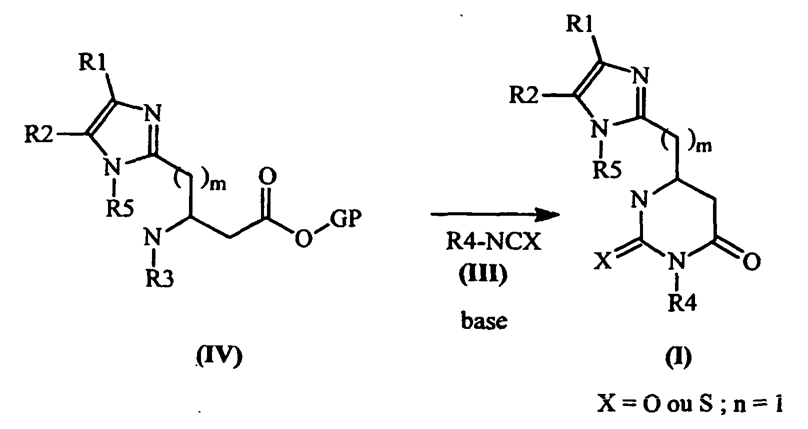

### Procédure générale :

Une amine de formule générale **(IV)**, dans laquelle m, R1, R2, R3 et R5 ont les mêmes significations que dans la formule générale **(I)** et le radical O-GP est un groupe protecteur partant dérivé d'un alcool et en particulier benzyloxy, méthoxy ou tert-butoxy, est traitée avec un isocyanate ou un isothiocyanate R4-NCX, en présence d'une base tertiaire comme la triéthylamine ou la *N,N*-diisopropyléthylamine dans un solvant aprotique, de préférence le THF ou le dichlorométhane, à une température comprise entre 20 et 70 °C pendant 1 à 48 heures. Le composé obtenu peut être isolé avec un rendement de 40 à 90 %, soit par chromatographie éclair sur gel de silice, soit par addition au mélange réactionnel d'un réactif nucléophile porté par un polymère comme par exemple une résine aminométhylpolystyrène (acquise auprès de Novabiochem) suivie par la filtration et l'évaporation du filtrat.

Lorsque R4 représente un radical comportant une terminaison amino primaire (par exemple R4 représente aminoéthyle, aminopropyle, etc.), le réactif n'est pas R4-NCX mais le composé correspondant dont le groupe amino est protégé par un groupe protecteur adapté, par exemple un groupe tert-butoxycarbonyle. Il faut donc une étape de déprotection ultérieure (effectuée dans des conditions classiques, à savoir un traitement acide) pour obtenir le composé de formule générale **(I)**.

### Préparation de la (6S)-1-(1H-indol-3-ylméthyl)-3-propyl-6-(4-phényl-1H-imidazol-2-yl)-2-thioxotétrahydro-4(1H)-pyrimidinone

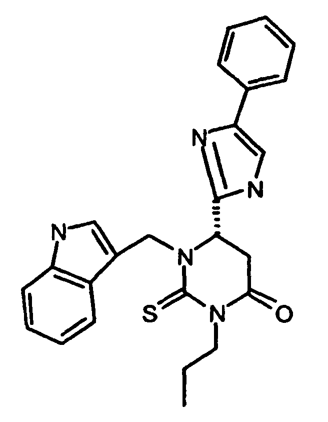

A une solution de (3*S*)-3-[(1*H*-indol-3-ylméthyl)amino]-3-(4-phényl-1*H*-imidazol-2-yl)propanoate de benzyle (90 mg, 1 éq.) dans 2 ml de THF est ajouté du propylisothiocyanate (25 µl, 1,2 éq.). Le mélange est agité pendant 15 heures à une température d'environ 40°C puis dilué avec 2 ml de THF. Une résine aminométhylpolystyrène (acquise auprès de Novabiochem, charge de 3,2 mmol/g, 125 mg, 2 éq.) est ajoutée. Le mélange est agité pendant 5 heures à une température d'environ 20 °C puis filtré sur fritté. Le filtrat est concentré sous pression réduite à 40 °C. Au résidu est ajouté 1 ml de THF et 1 ml de triéthylamine. Le mélange est agité pendant 15 heures à une température d'environ 40 °C puis concentré sous pression réduite. La purification par chromatographie éclair sur gel de silice (éluant : acétate d'éthyle / heptane 8:2) donne le composé attendu (72 mg, rendement de 82%).
RMN (¹H, 400 MHz, CDCl₃) : mélange de 2 atropoisomères : 8,69-6,45 (m, 12H, H arom, NH) ; 6,42, 4,89 (AB, 1H, CH₂, J_{AB} = 14,5 Hz) ; 5,78, 5,42 (AB, 1H, CH₂, J_{AB} = 14,5 Hz) ; 4,99 (m, 1H, CH) ; 4,41-4,36 (m, 1H, CH₂) ; 4,20-4,11 (m, 1H, CH₂) ; 3,49, 2,94 (AB, 1H, CH₂CO, J_{AB} = 16 Hz) ; 3,28, 2,80 (AB, 1H, CH₂CO, J_{AB} = 16 Hz) ; 1,52 (m, 1H, CH₂); 1,40 (m, 1H, CH₂) ; 0,76, 0,62 (2m, 3H, CH₃).
SM/CL : MM calculée = 443,2 ; m/z = 444,2 (M+H).

Les composés suivants (dans leurs deux formes énantiomères) sont préparés de façon analogue à la procédure décrite pour la (6S)-1-(1H-indol-3-ylméthyl)-3-propyl-6-(4-phényl-1H-imidazol-2-yl)-2-thioxotétrahydro-4(1H)-pyrimidinone (hormis la purification finale par chromatographie éclair sur gel de silice qui est facultative) :

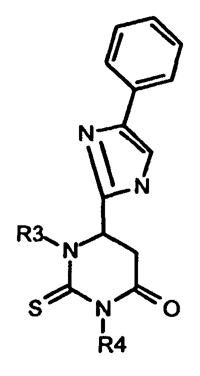

Dans la formule ci-dessus, R3 représente l'un des radicaux ci-après : 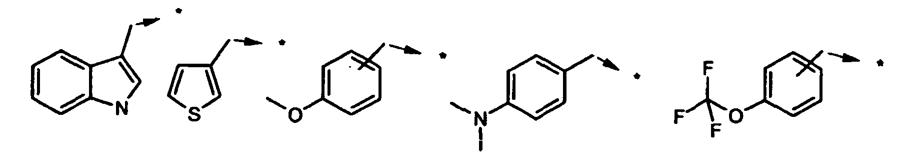 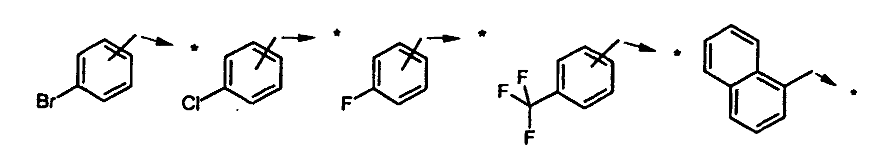 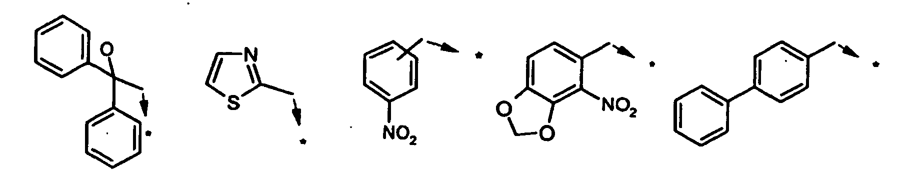 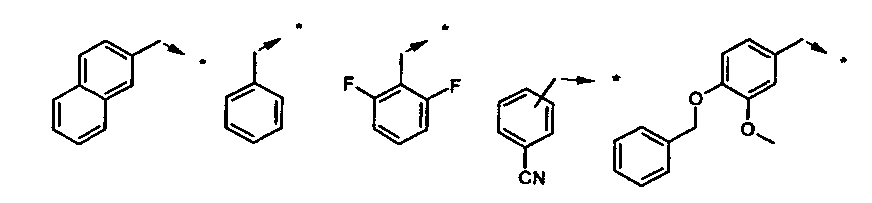 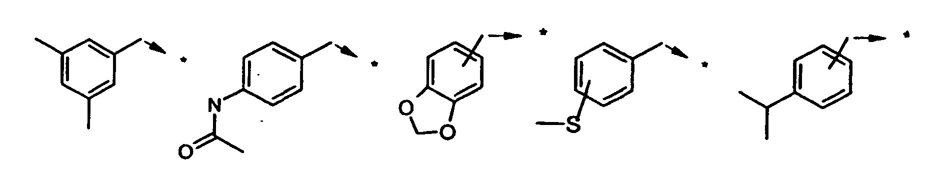 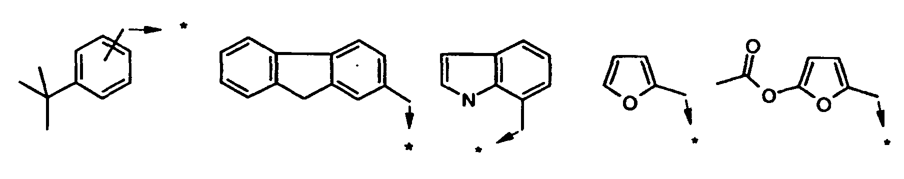 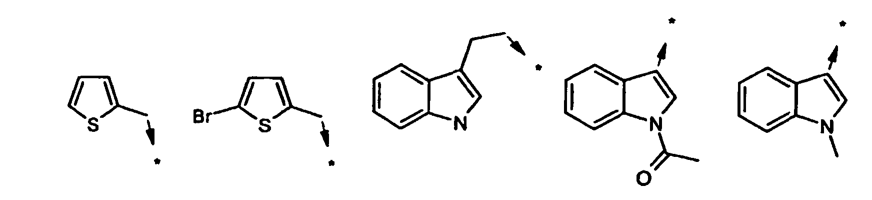 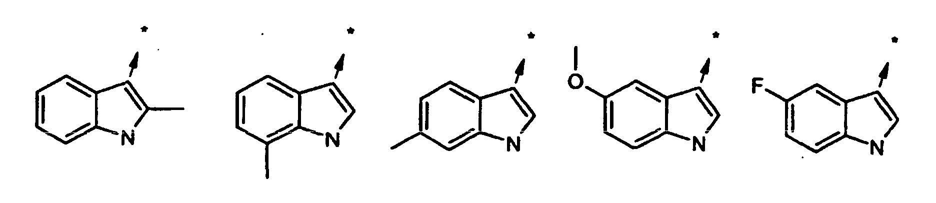 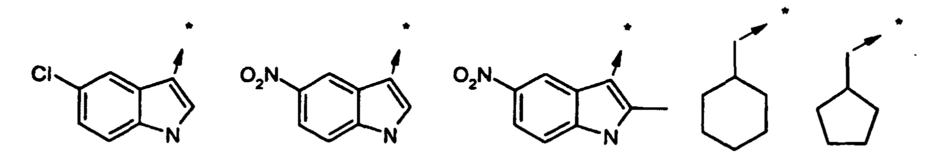 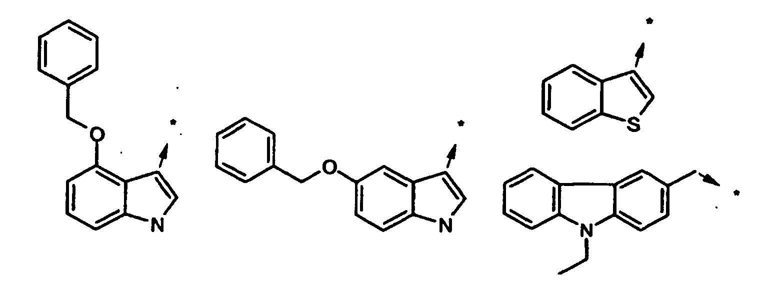 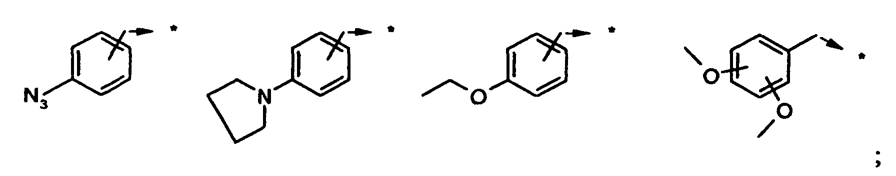 et R4 représente l'un des radicaux ci-après :

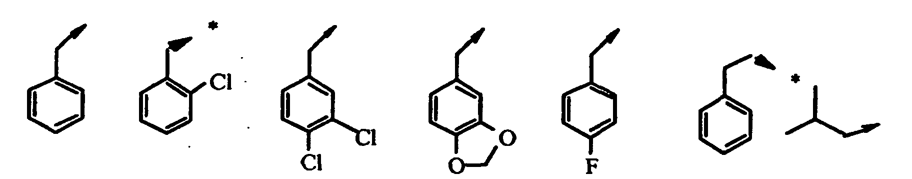

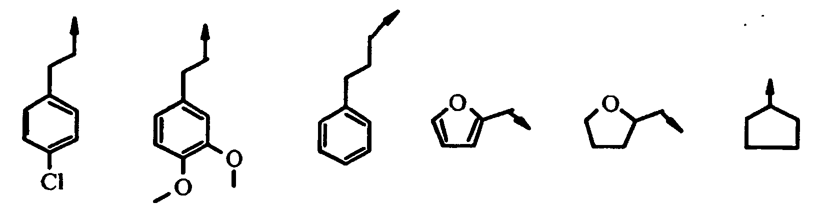

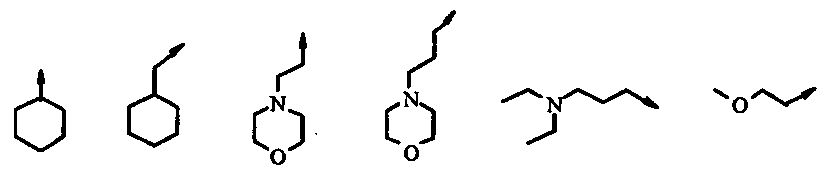

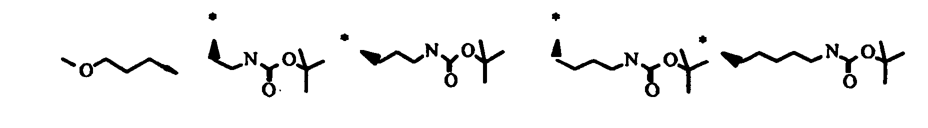

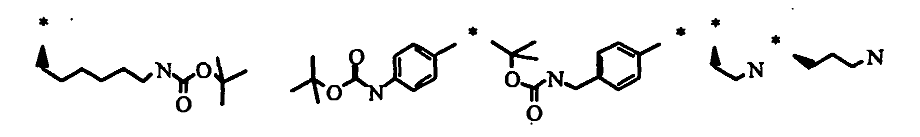

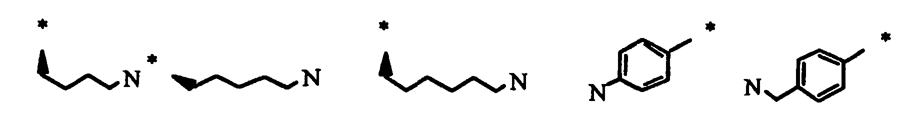

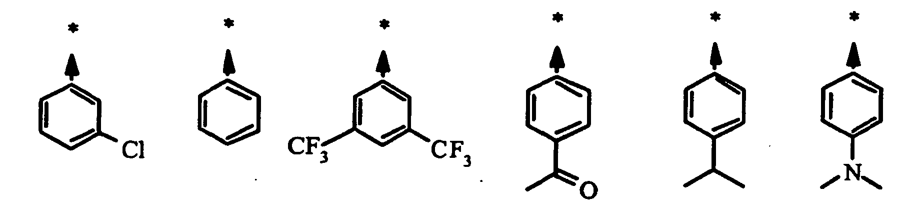

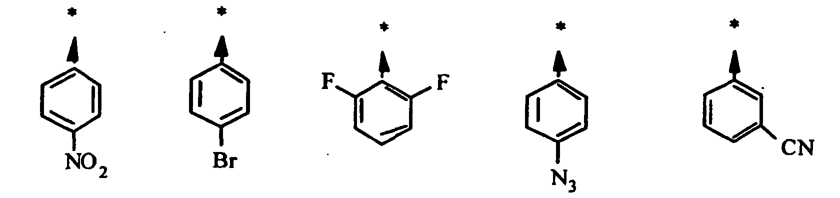

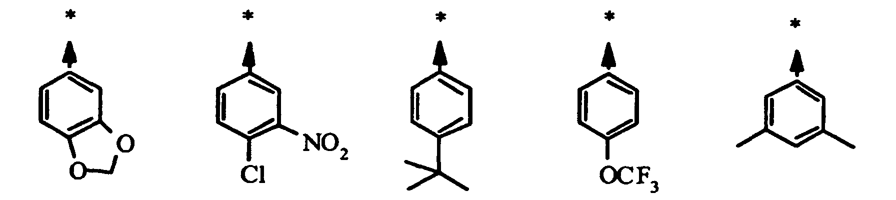

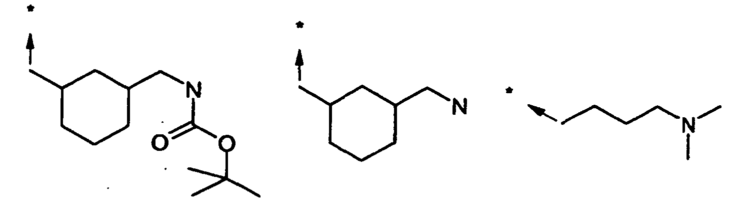

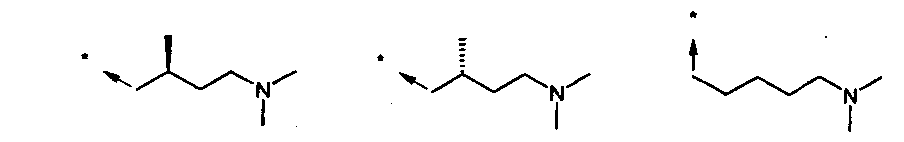

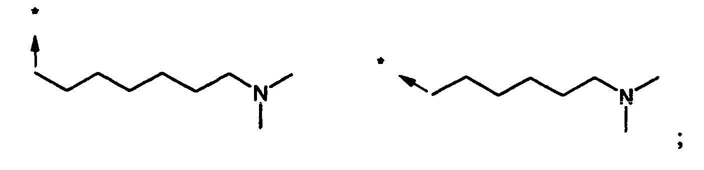

### Préparation de la (6S)-1-(1H-indol-3-ylméthyl)-3-(4-méthoxyphényl)-6-(4-phényl-1H-imidazol-2-yl)dihydro-2,4(1H,3H)-pyrimidinedione

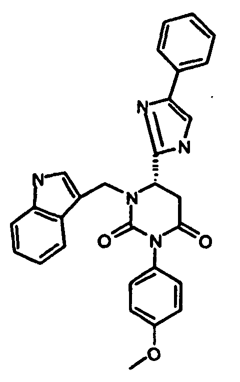

A une solution de (3*S*)-3-[(1*H*-indol-3-ylméthyl)amino]-3-(4-phényl-1*H*-imidazol-2-yl)propanoate de benzyle (100 mg, 1 éq.) dans du THF (2 ml) est ajouté du 4-méthoxyphénylisocyanate (40 µl, 1,2 éq.). Le mélange est agité pendant 5 heures à une température d'environ 20 °C puis dilué avec 2 ml de THF. Une résine aminométhylpolystyrène (acquise auprès de Novabiochem, charge de 3,2 mmol/g, 138 mg, 2 éq.) est ajoutée. Le mélange est agité pendant 3 heures à une température d'environ 20 °C puis filtré sur fritté. Le filtrat est concentré sous pression réduite à 40 °C. Au résidu est ajouté 2 ml de THF et 2 ml de triéthylamine. Le mélange est porté à reflux pendant 24 h puis concentré sous pression réduite. La purification du résidu par chromatographie éclair sur gel de silice (éluant : acétate d'éthyle / heptane 8:2) donne le composé attendu (80 mg, rendement de 74 %).
RMN (¹H, 400 MHz, CDCl₃) : mélange de 2 atropoisomères : 9,67-8,96 (2s, 1H, NH) ; 8,49 (s, 1H, NH) ; 5,15, 4,36 (AB, 1H, CH₂, J_{AB} = 15 Hz) ; 5,08, 4,69 (AB, 1H, CH₂, J_{AB} = 15 Hz); 4,67, 4,57 (2m, 1H, CH); 3,72 (s, 3H, OCH₃); 3,29-2,79 (m, 2H, CH₂CO).
SM/CL : MM calculée = 491,2 ; m/z = 492,3 (M+H).

Les composés suivants (dans leurs deux formes énantiomères) sont préparés de façon analogue à la procédure décrite pour la (6S)-1-(1H-indol-3-ylméthyl)-3-(4-méthoxyphényl)-6-(4-phényl-1H-imidazol-2-yl)dihydro-2,4(1H,3H)-pyrimidinedione (hormis la purification finale par chromatographie éclair sur gel de silice qui est facultative) :

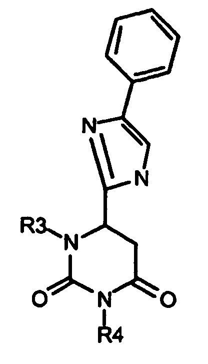

Dans la formule ci-dessus, R3 représente l'un des radicaux ci-après : 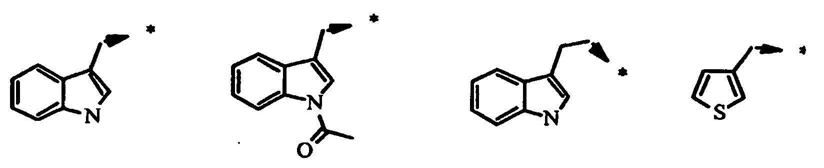 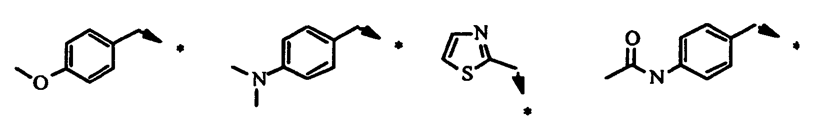 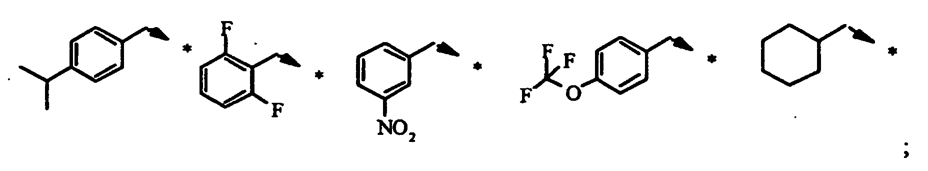 et R4 représente l'un des radicaux ci-après :

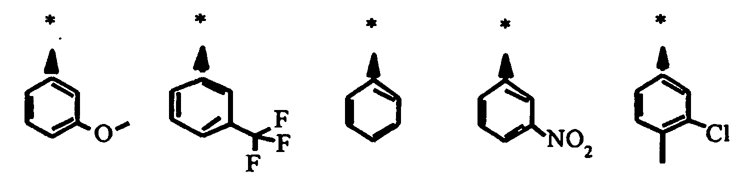

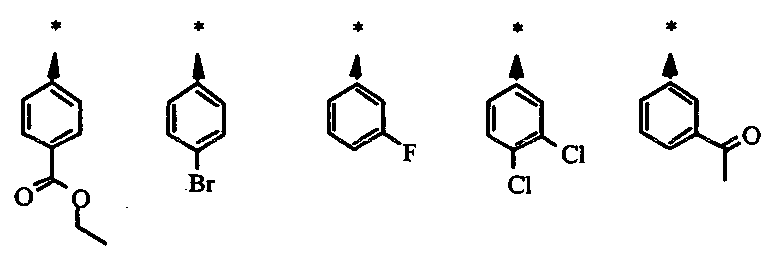

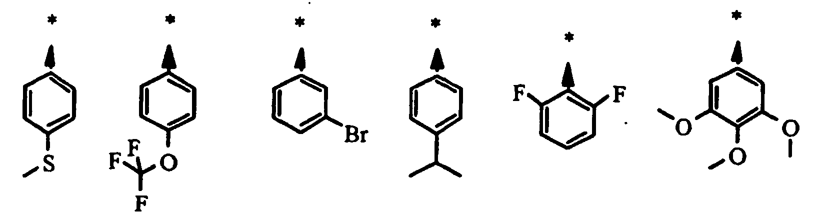

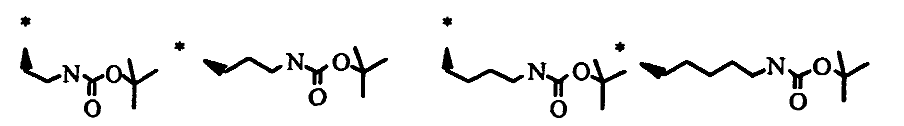

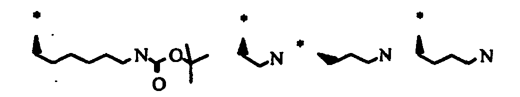

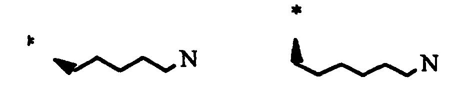

### EXEMPLES

Ci-après sont repris dans des tableaux des exemples préparés selon les méthodes de synthèse décrites ci-dessus. Ces exemples sont présentés pour illustrer les procédures ci-dessus et ne doivent en aucun cas être considérés comme une limite à la portée de l'invention.

### Méthodes analytiques employées pour la caractérisation des composés

Les composés obtenus ont été caractérisés grâce à leur temps de rétention (tr) et à la spectrométrie de masse (MH+).

### α) Spectrométrie de masse

Pour la spectrométrie de masse, un spectromètre de masse simple quadripôle (Micromass, modéle Platform) équipé d'une source *electrospray* est utilisé avec une résolution de 0,8 Da à 50 % de vallée.

Un calibrage est effectué mensuellement entre les masses 80 et 1000 Da à l'aide d'un mélange calibrant d'iodure de sodium et d'iodure de rubidium en solution dans un mélange isopropanol/eau (1/1 Vol.).

### β) Chromatographie liquide à haute performance (HPLC)

Pour la chromatographie liquide, un système HPLC HP1100 (Hewlett-Packard) incluant un dégazeur en ligne, une pompe quaternaire, un four à colonnes et un détecteur UV à barrette de diodes est utilisé.

Différentes conditions d'élution sont employées selon les exemples :
- Conditions (i) :
Eluants :
- **A**: eau + 0,04 % acide trifluoroacétique
- **B**: acétonitrile

| T(min) | A% | B% |
|---|---|---|
| 0 | 100 | 0 |
| 1 | 100 | 0 |
| 8 | 30 | 70 |
| 10 | 30 | 70 |

Débit : 1,1 ml / min
Injection : 5 µl
Colonne : Uptisphere ODS 3µm 33*4,6 mm i.d.
Température : 40 °C
- Conditions (ii) :
Eluants :
- **A**: eau + 0,04 % acide trifluoroacétique
- **B**: acétonitrile

| T(min) | A% | B% |
|---|---|---|
| 0 | 90 | 10 |
| 6 | 15 | 85 |
| 10 | 15 | 85 |

Débit: 1 ml / min
Injection : 5 µl
Colonne : Uptisphere ODS 3µm 50*4,6 mm i.d.
Température : 40 °C

Les conditions d'élution (i) sont employées pour la caractérisation des exemples 1 à 479, 560 à 572 et 733 à 1040. Les conditions (ii) sont employées quant à elles pour les exemples 480 à 559, 573 à 732 et 1041 à 1234. La détection UV se fait à une longueur d'onde de 220 nm pour tous les exemples.

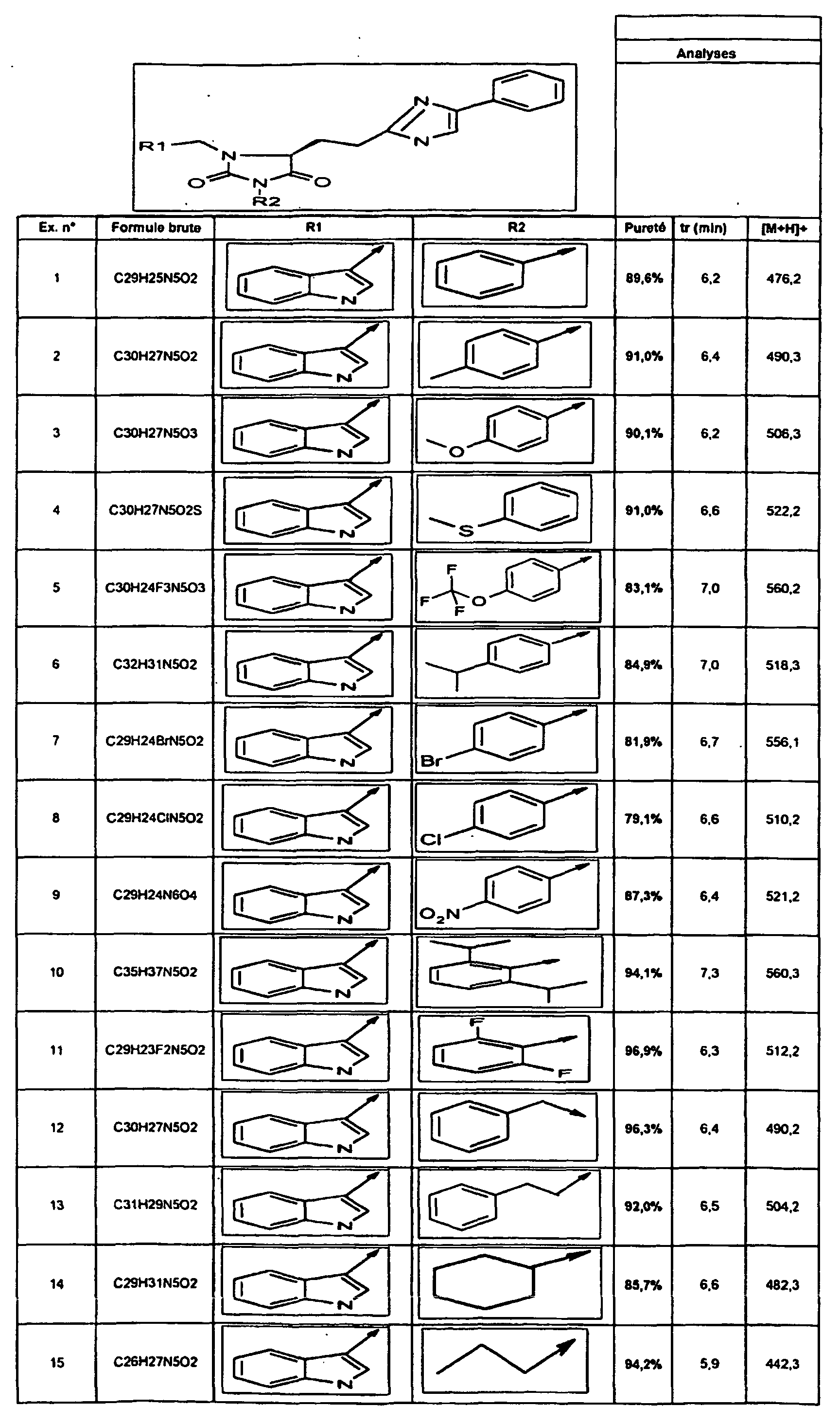

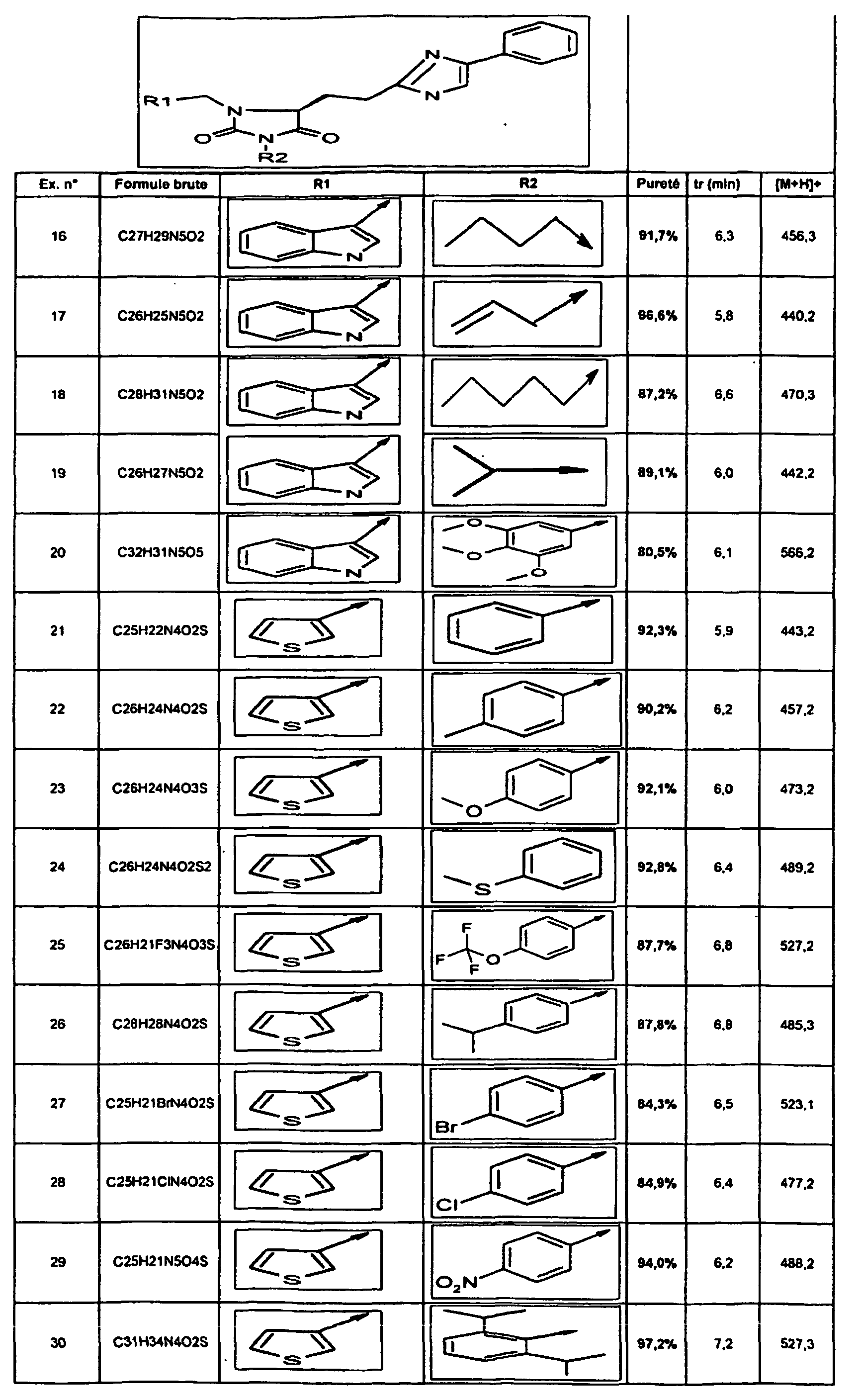

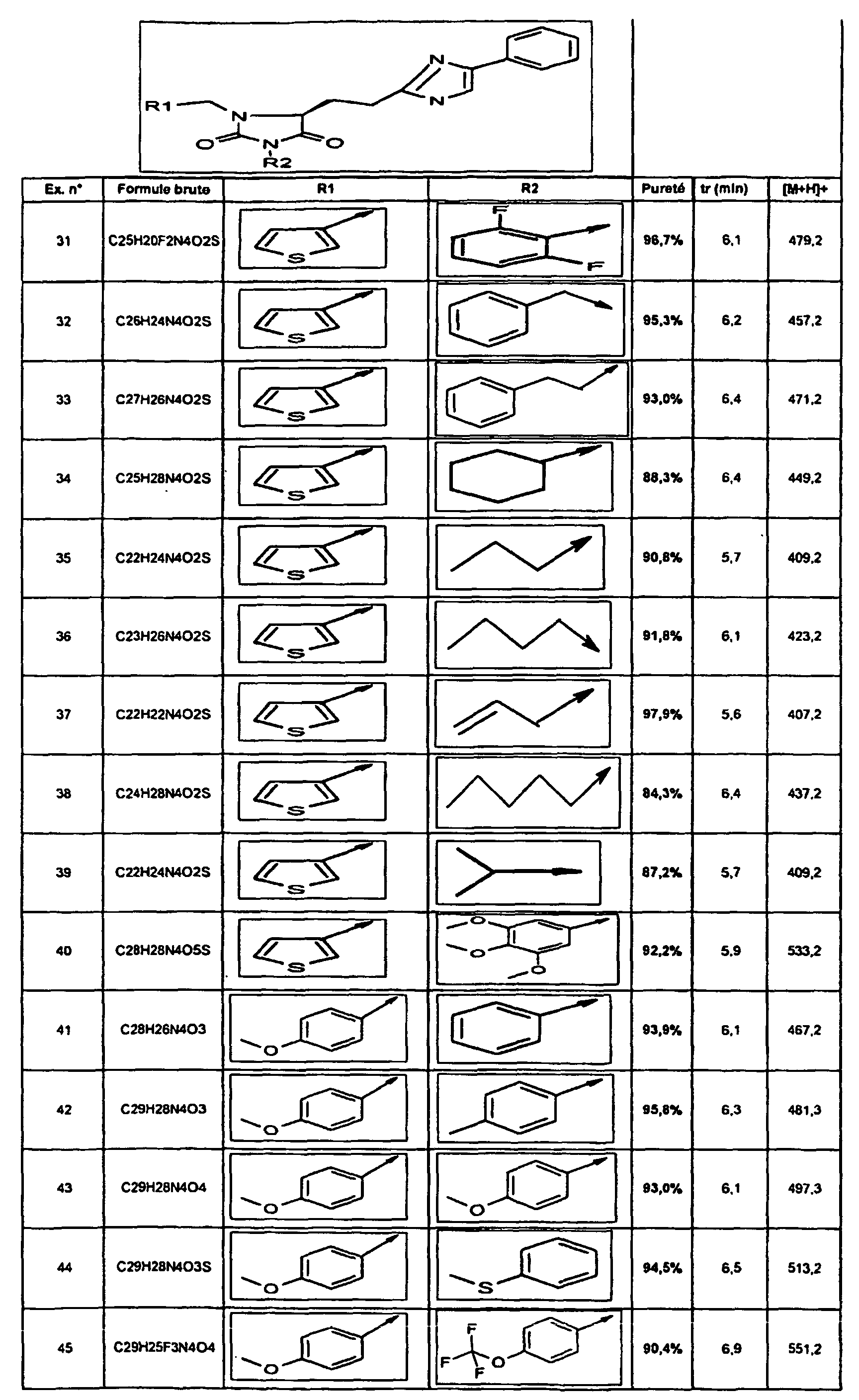

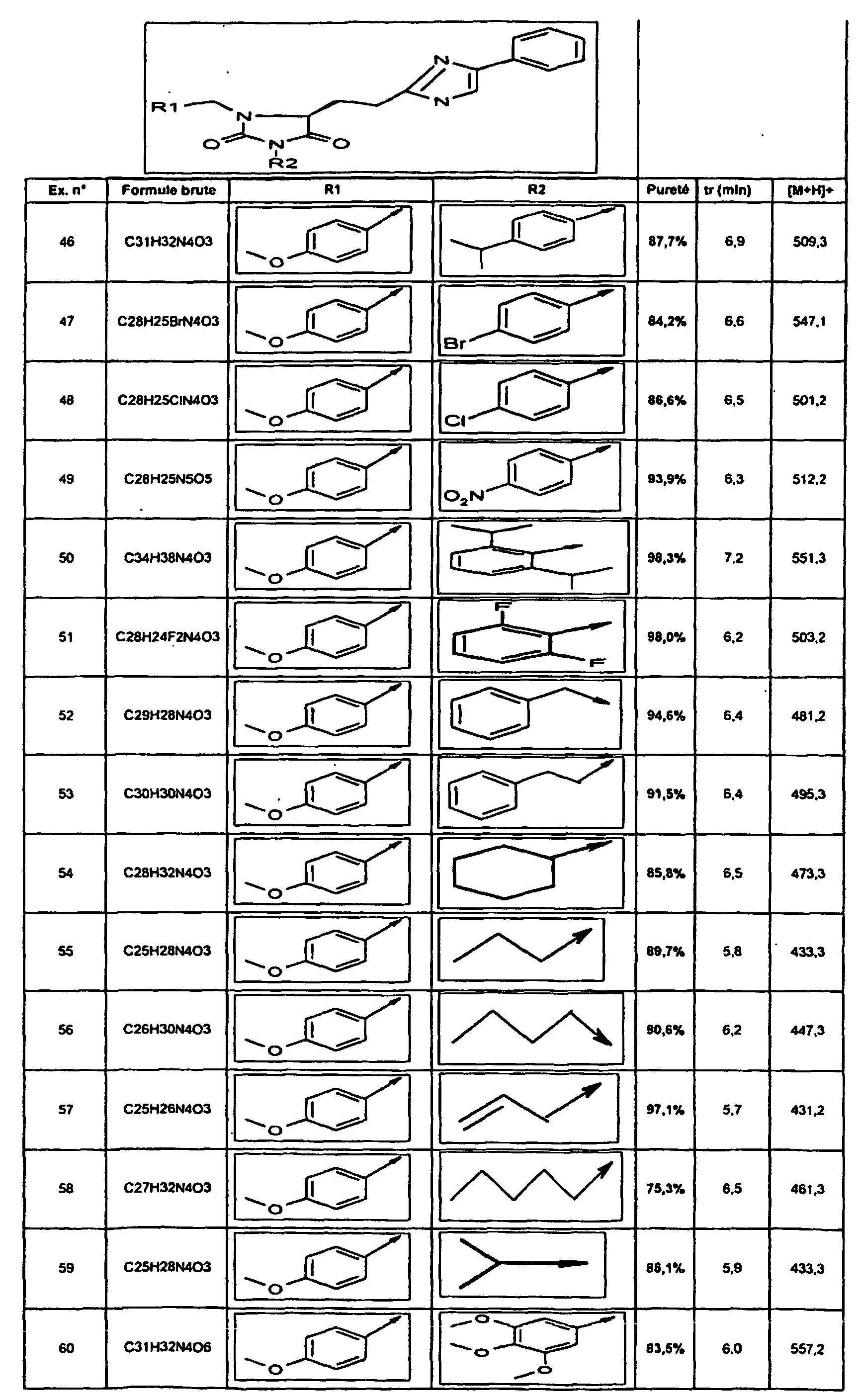

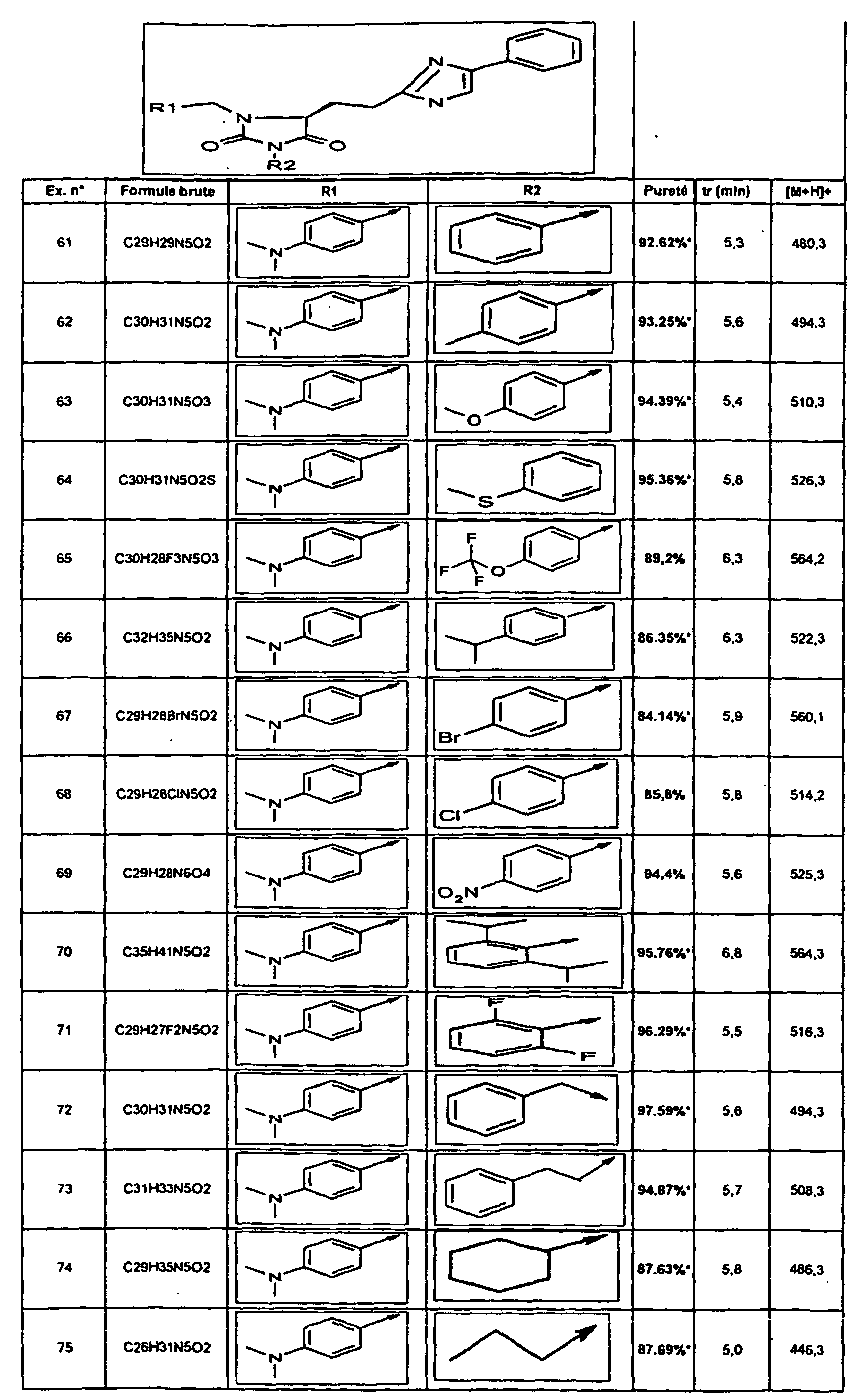

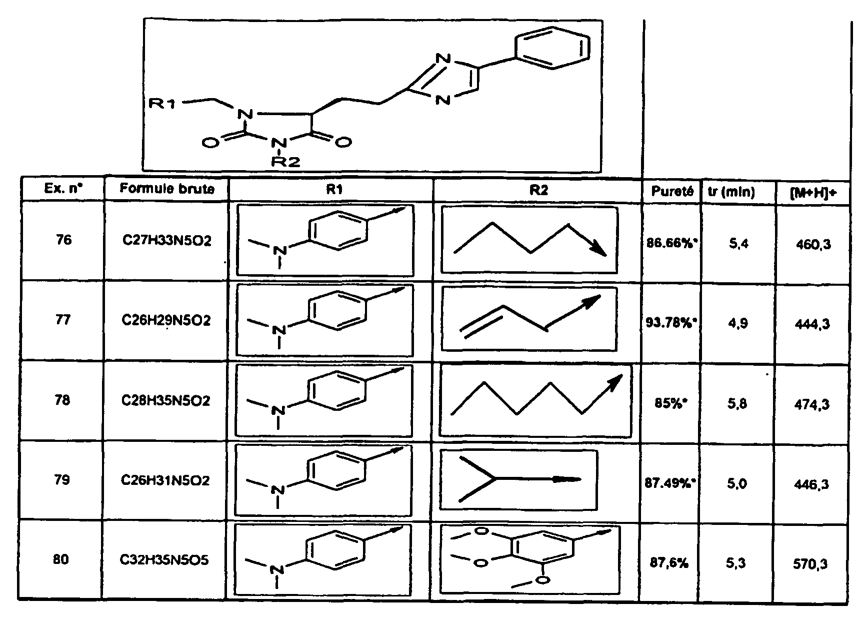

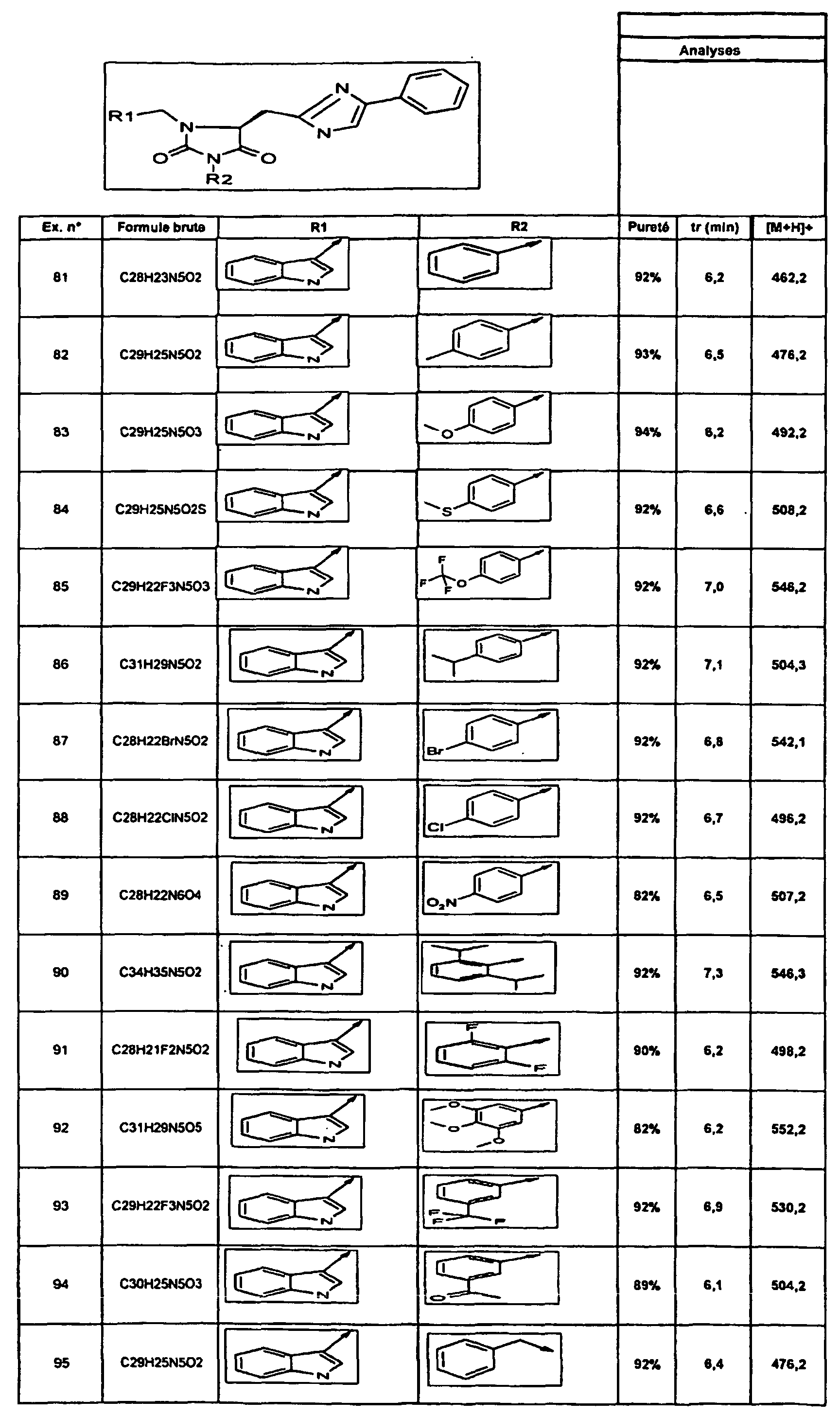

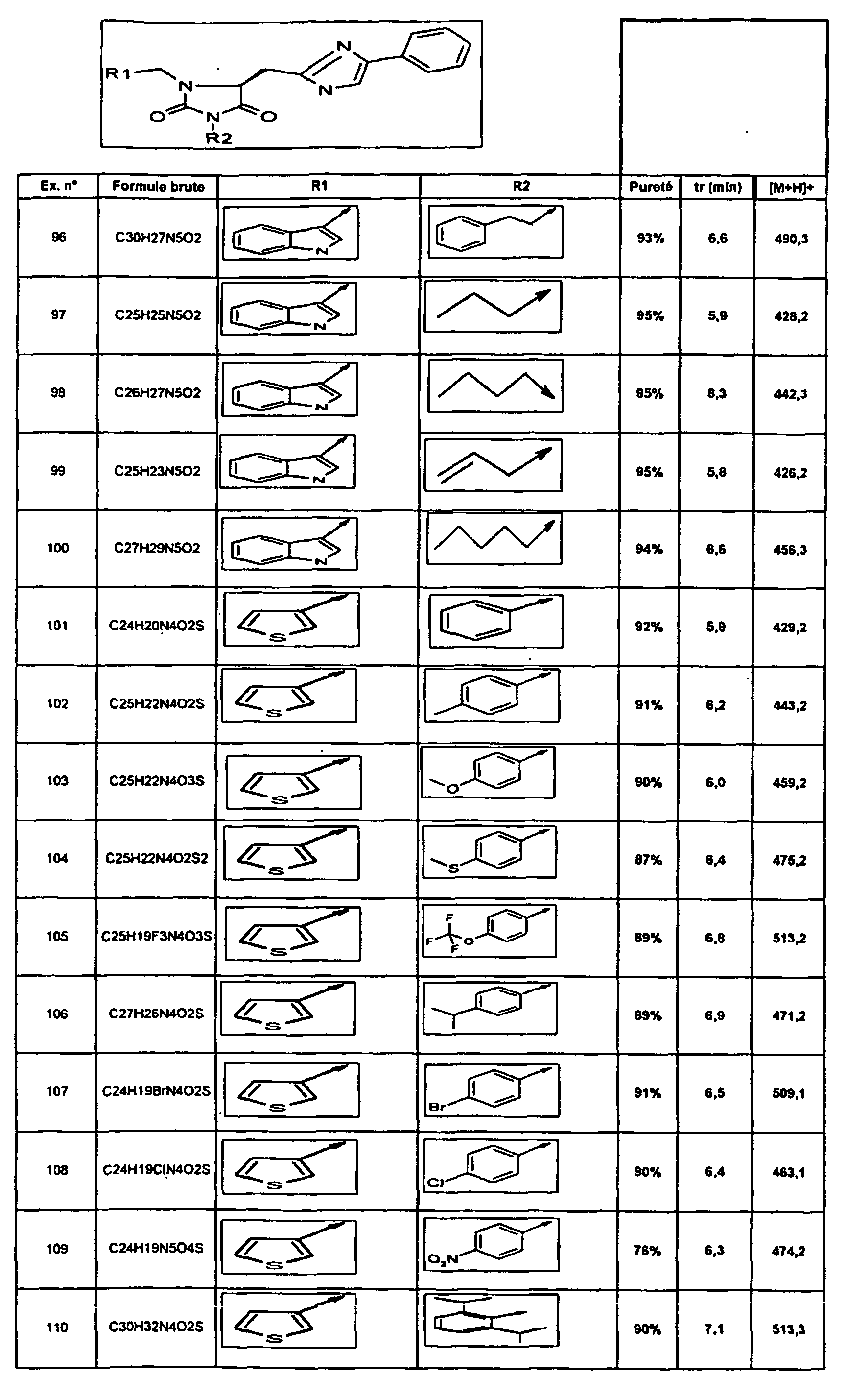

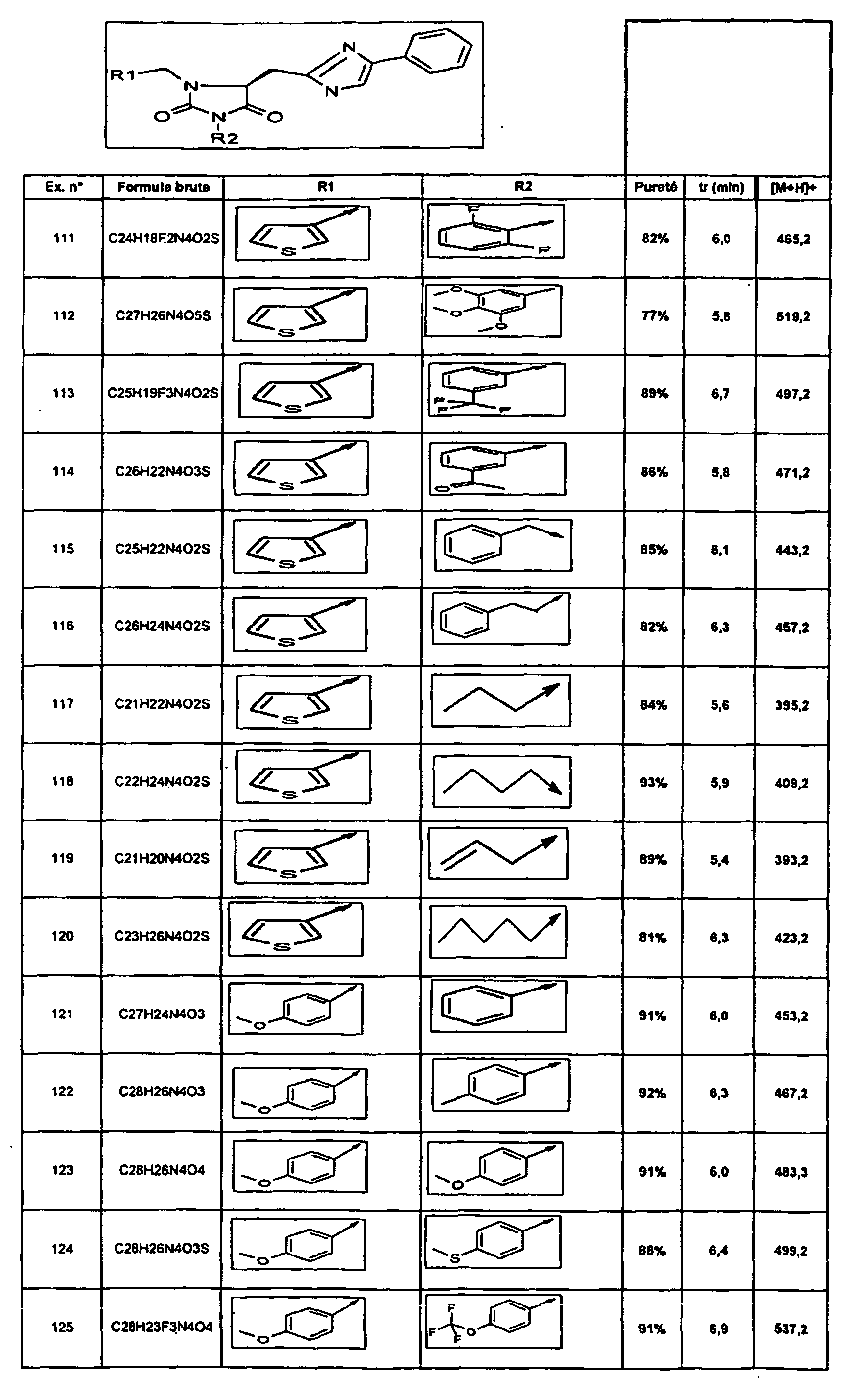

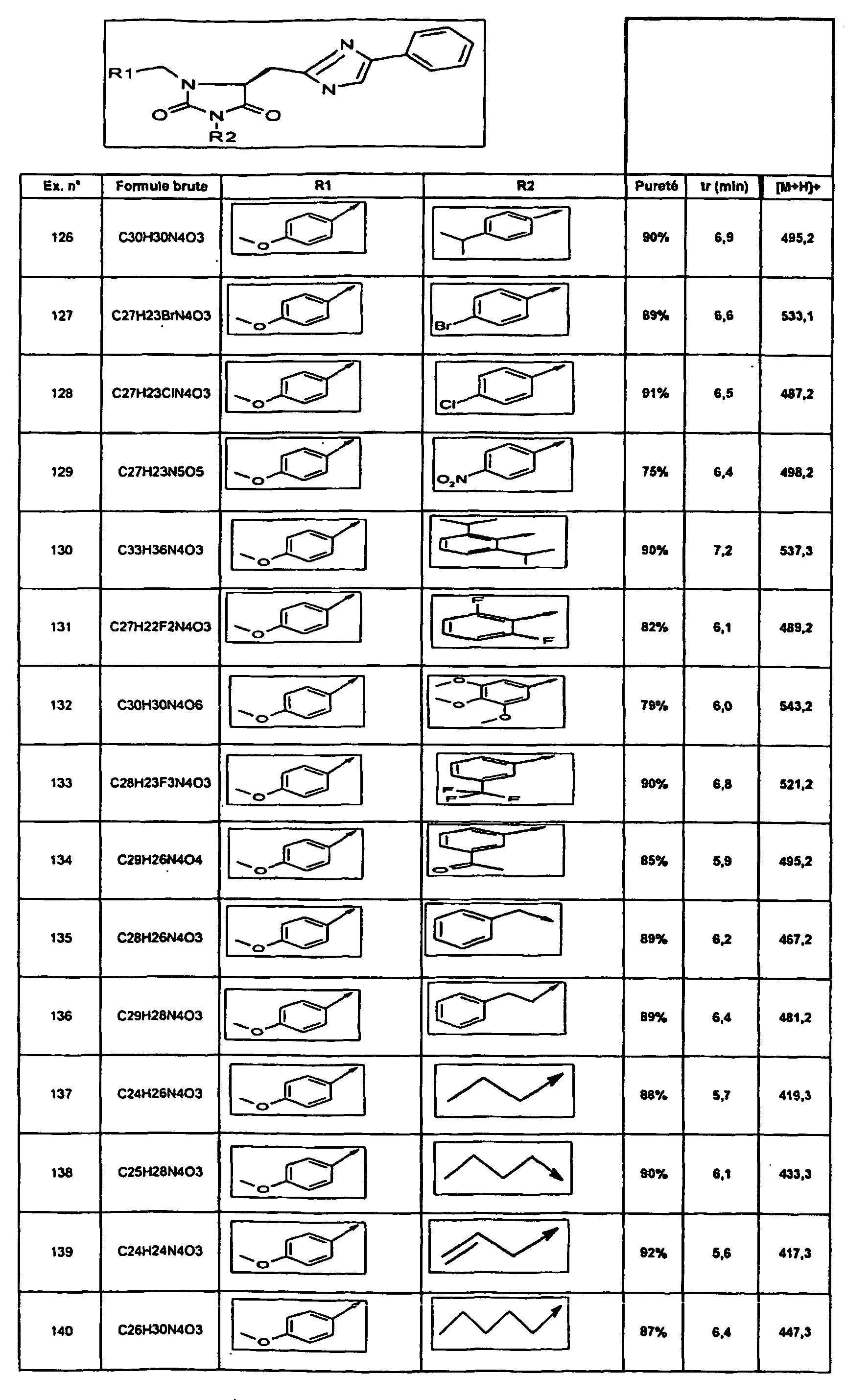

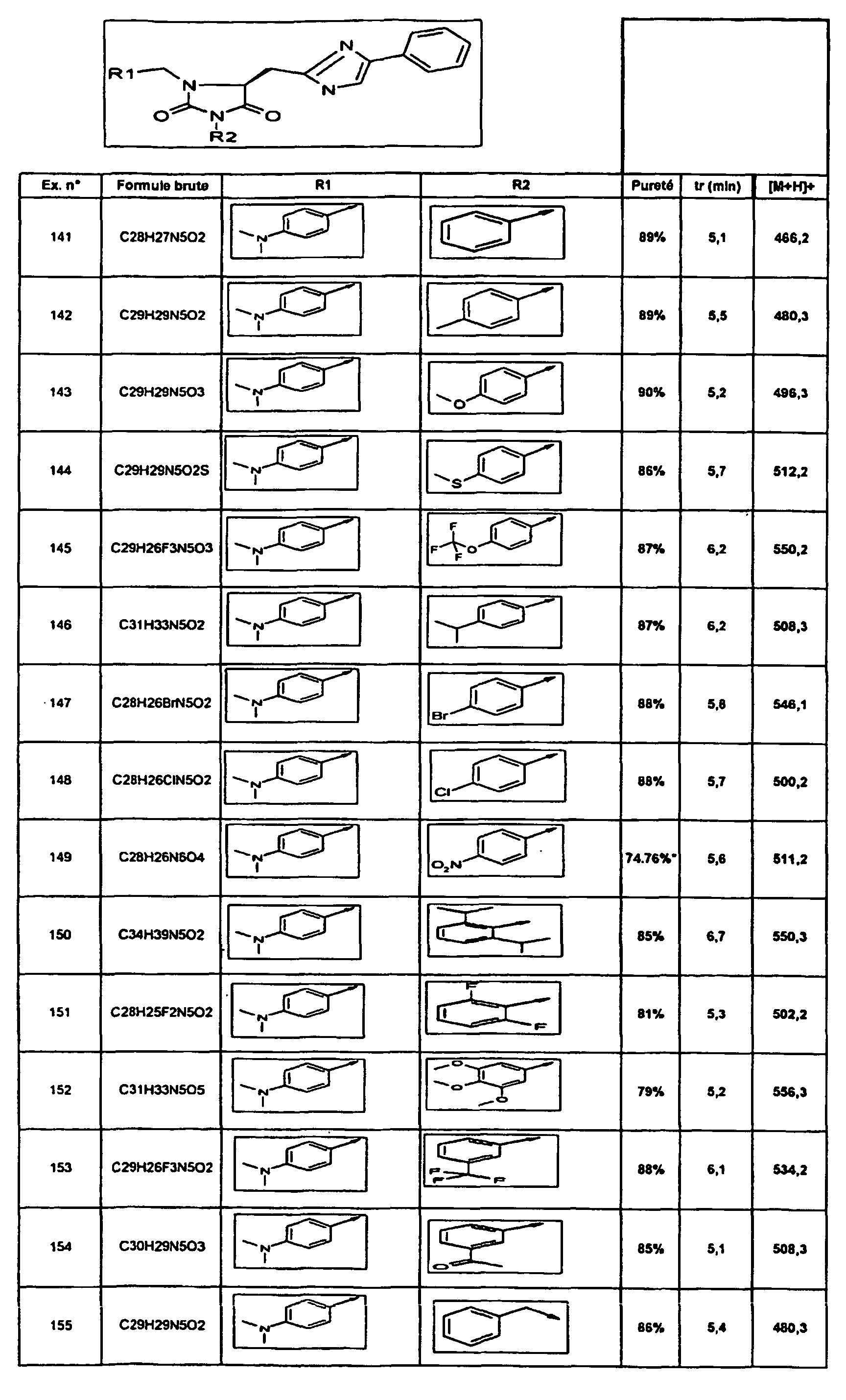

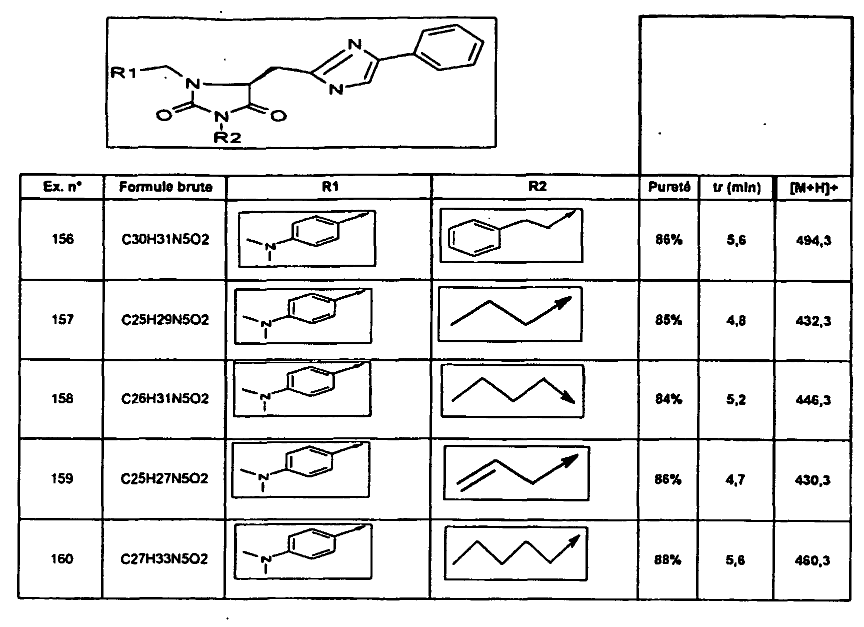

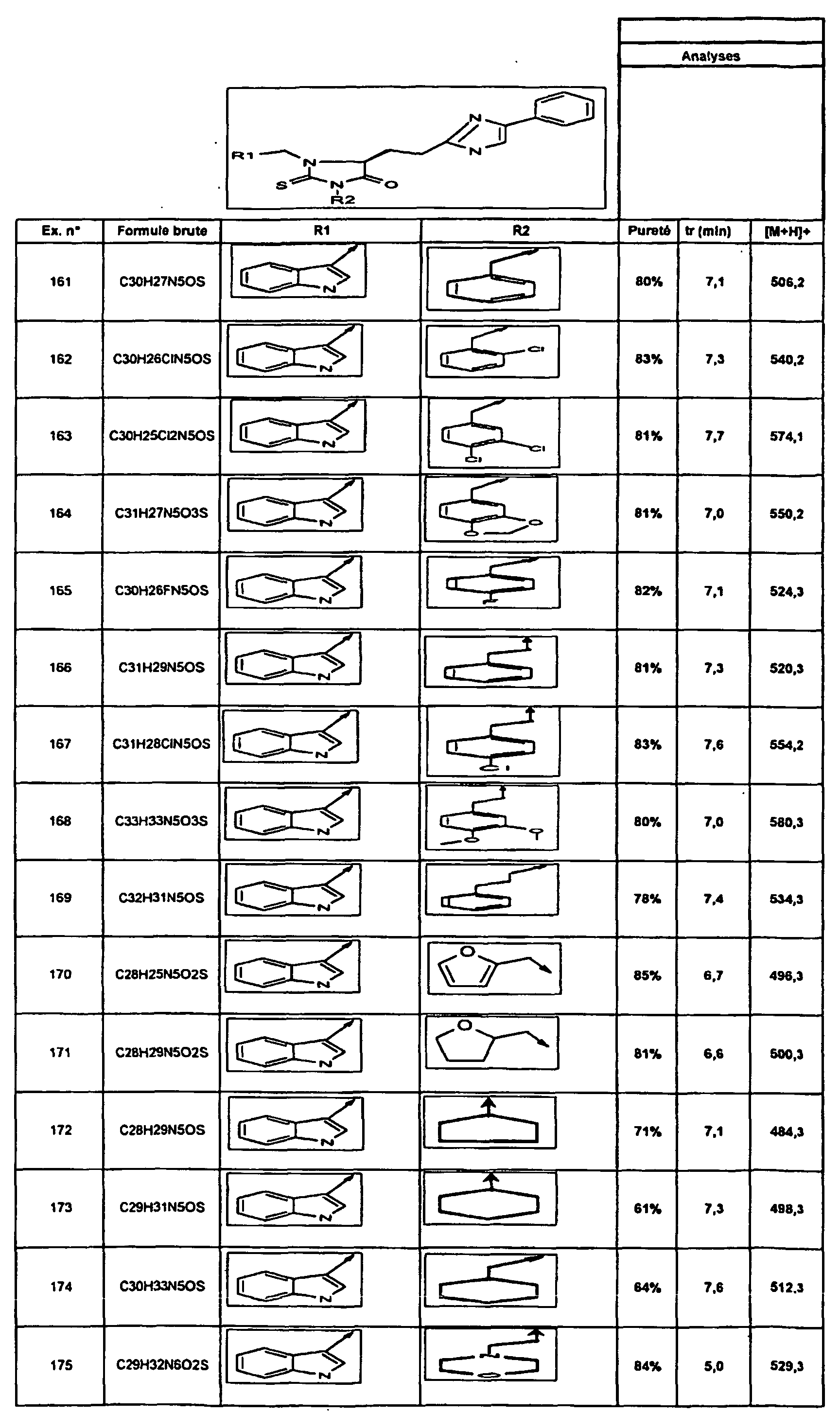

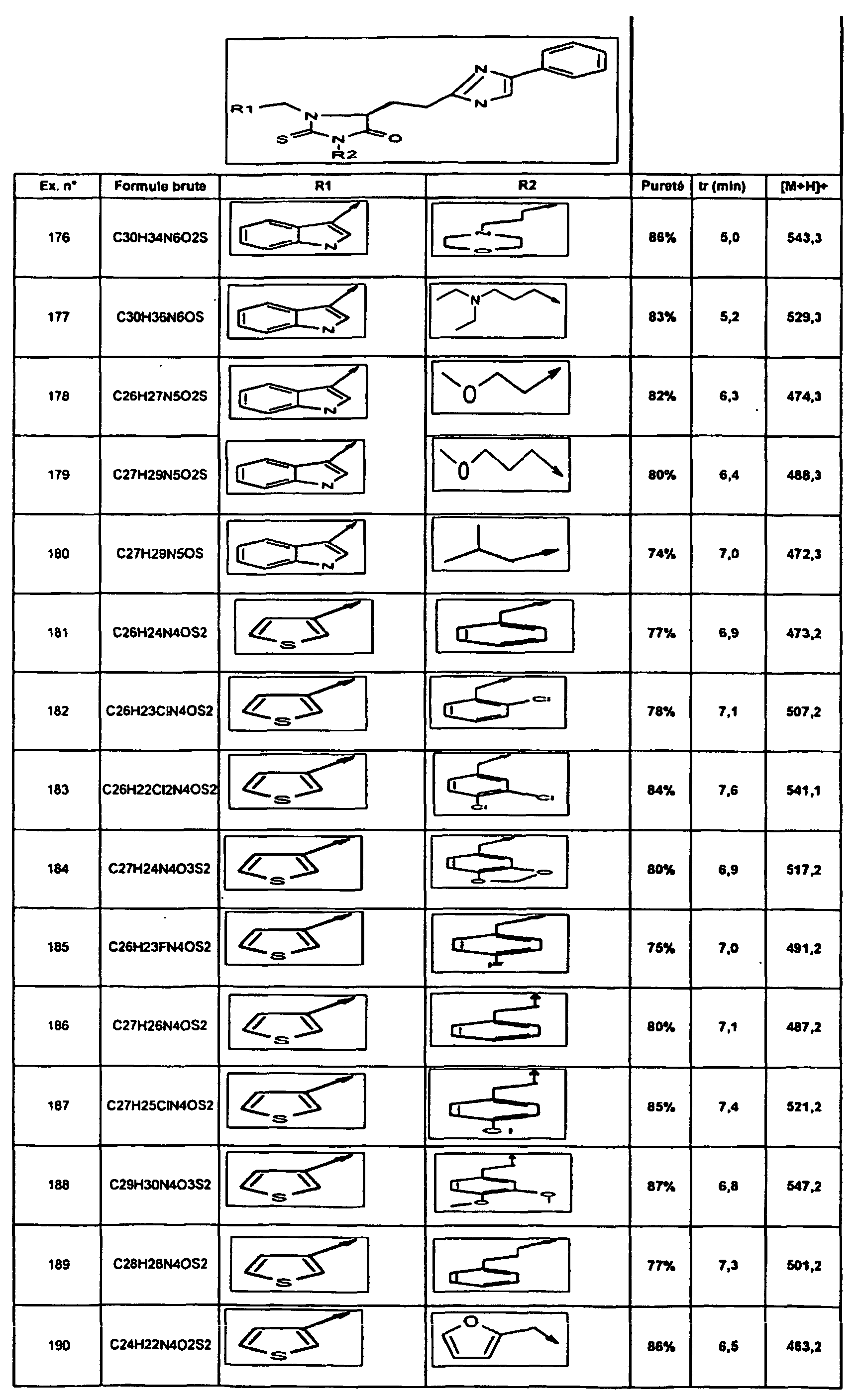

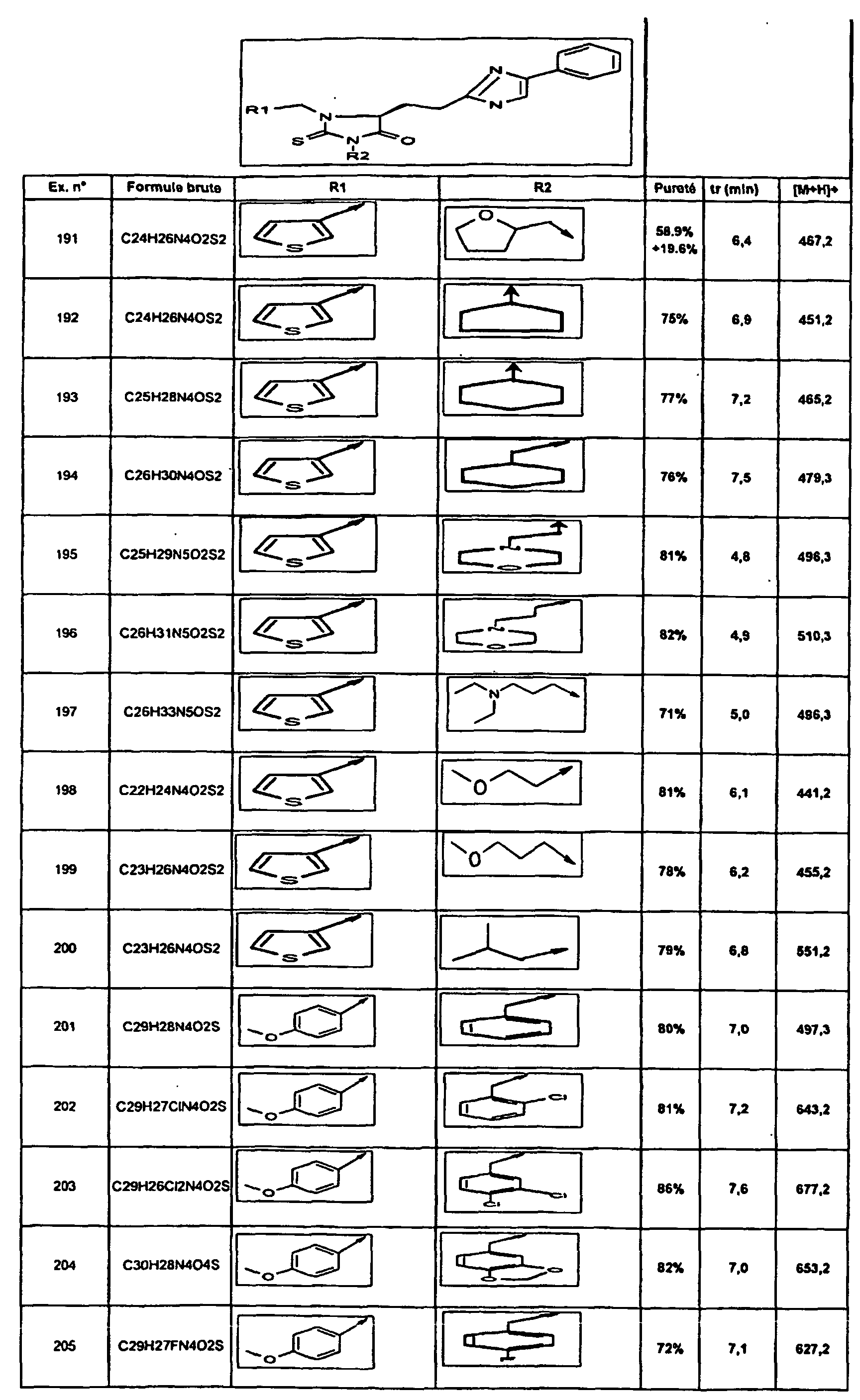

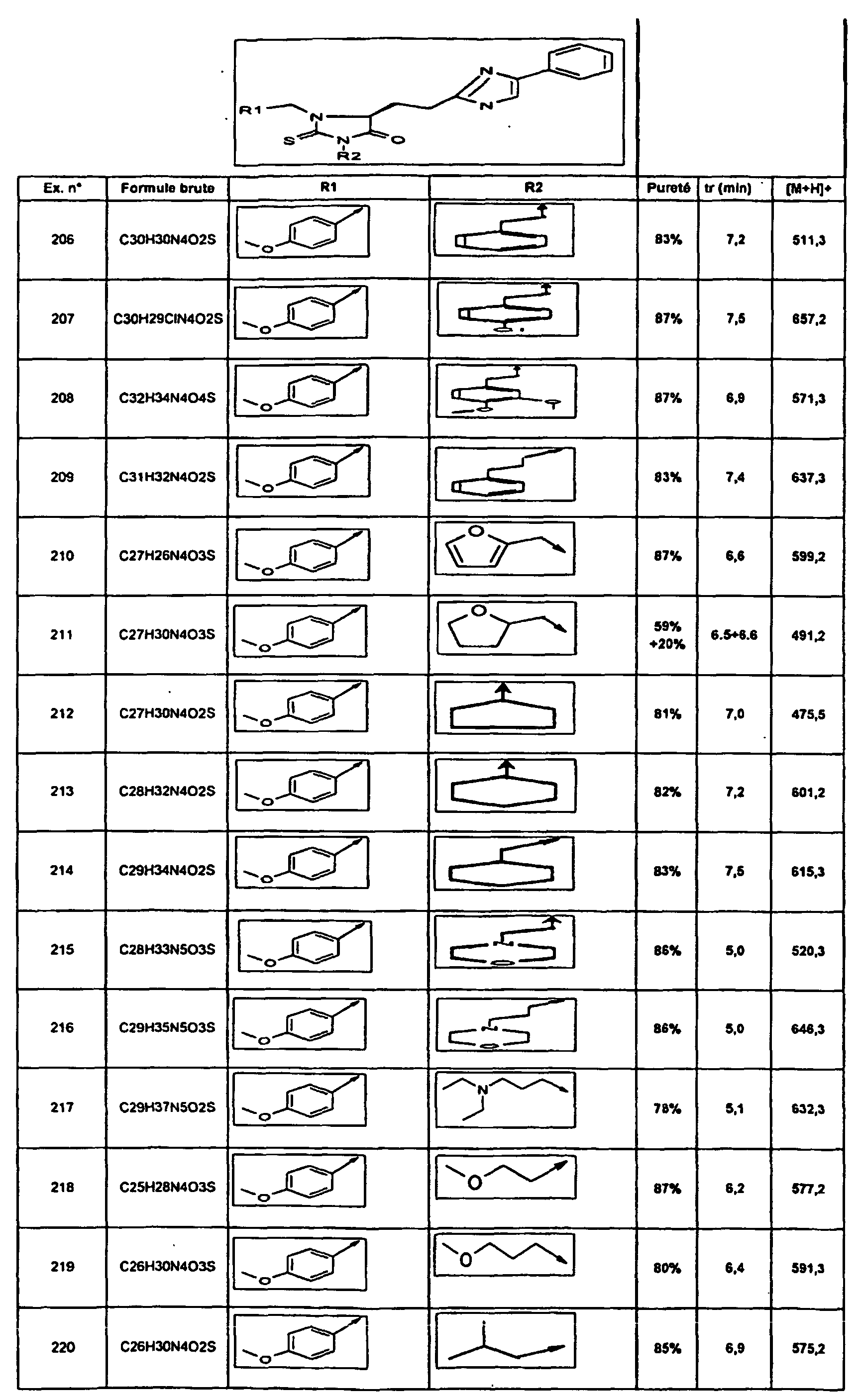

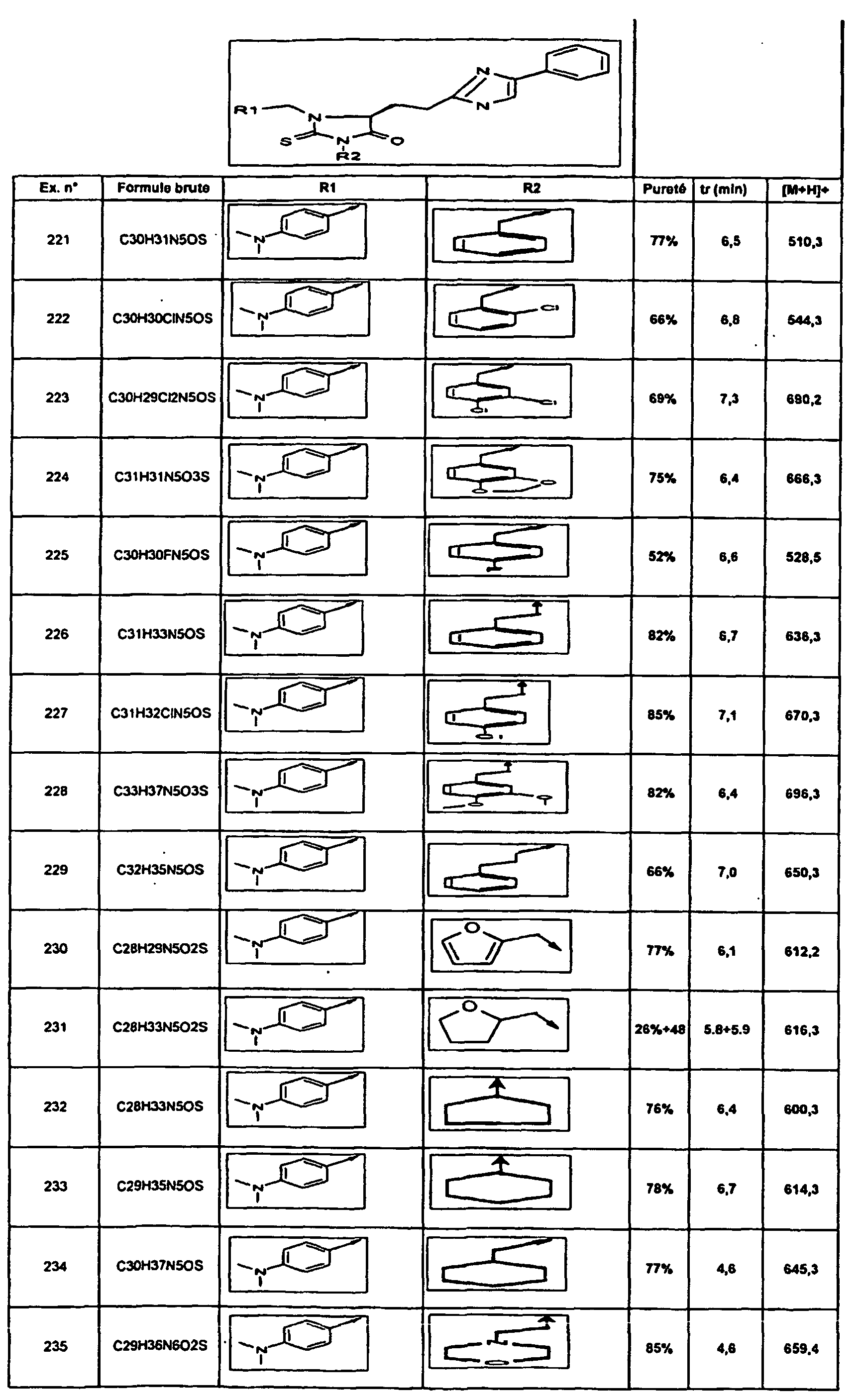

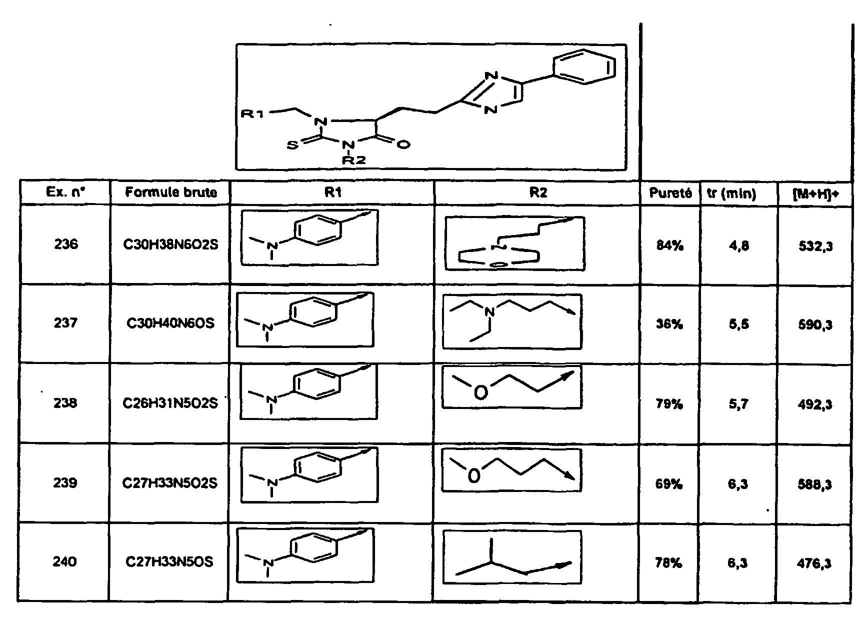

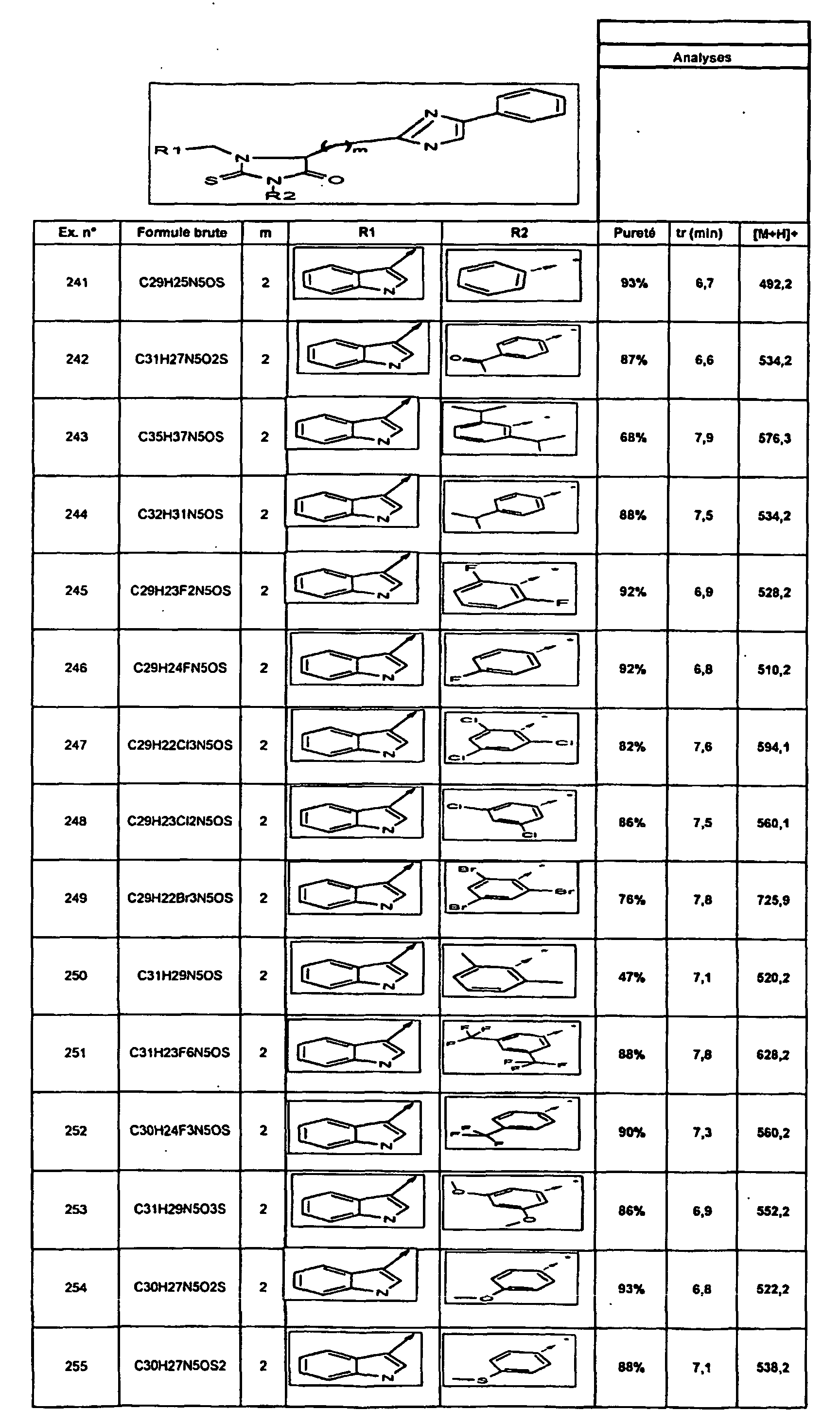

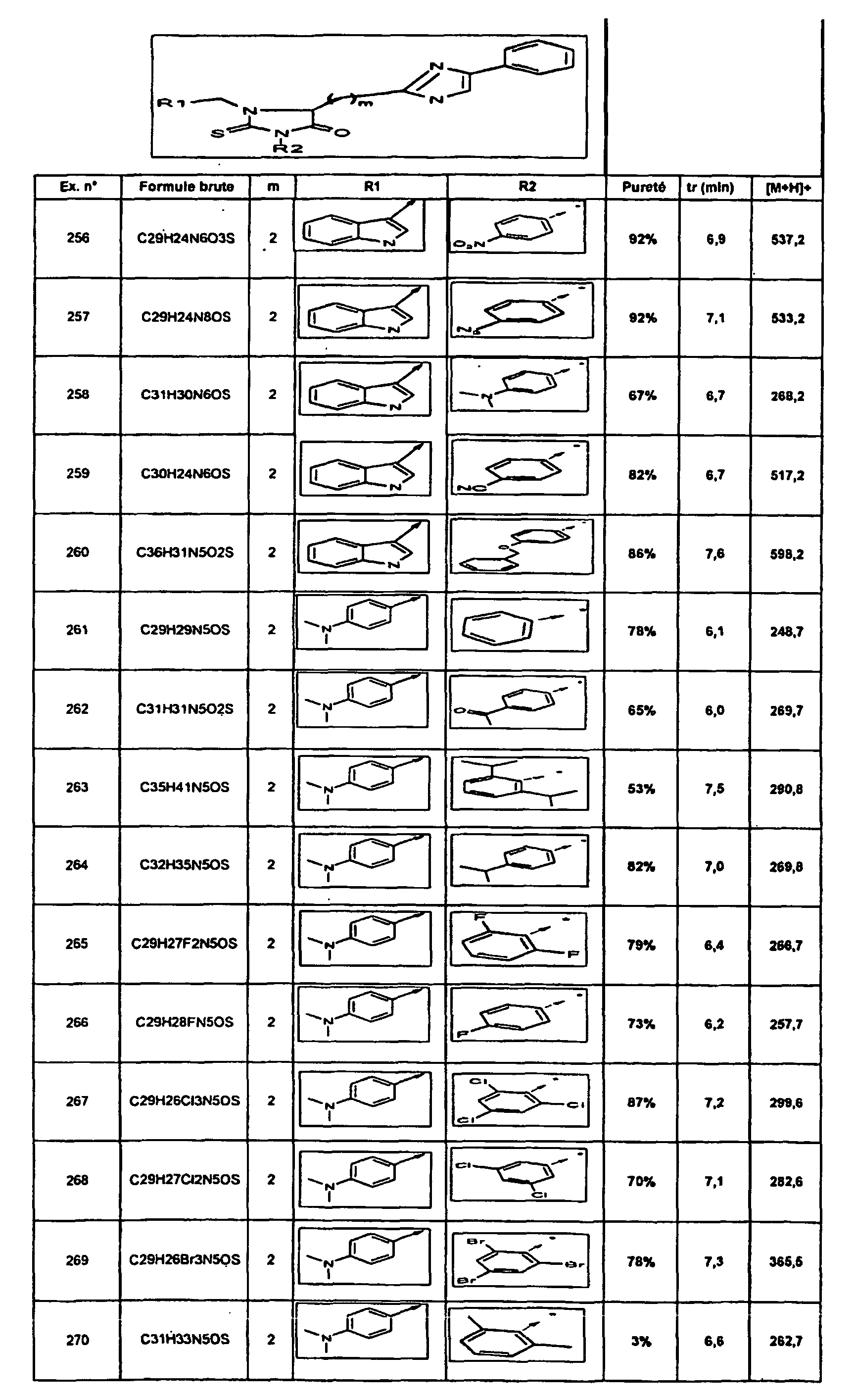

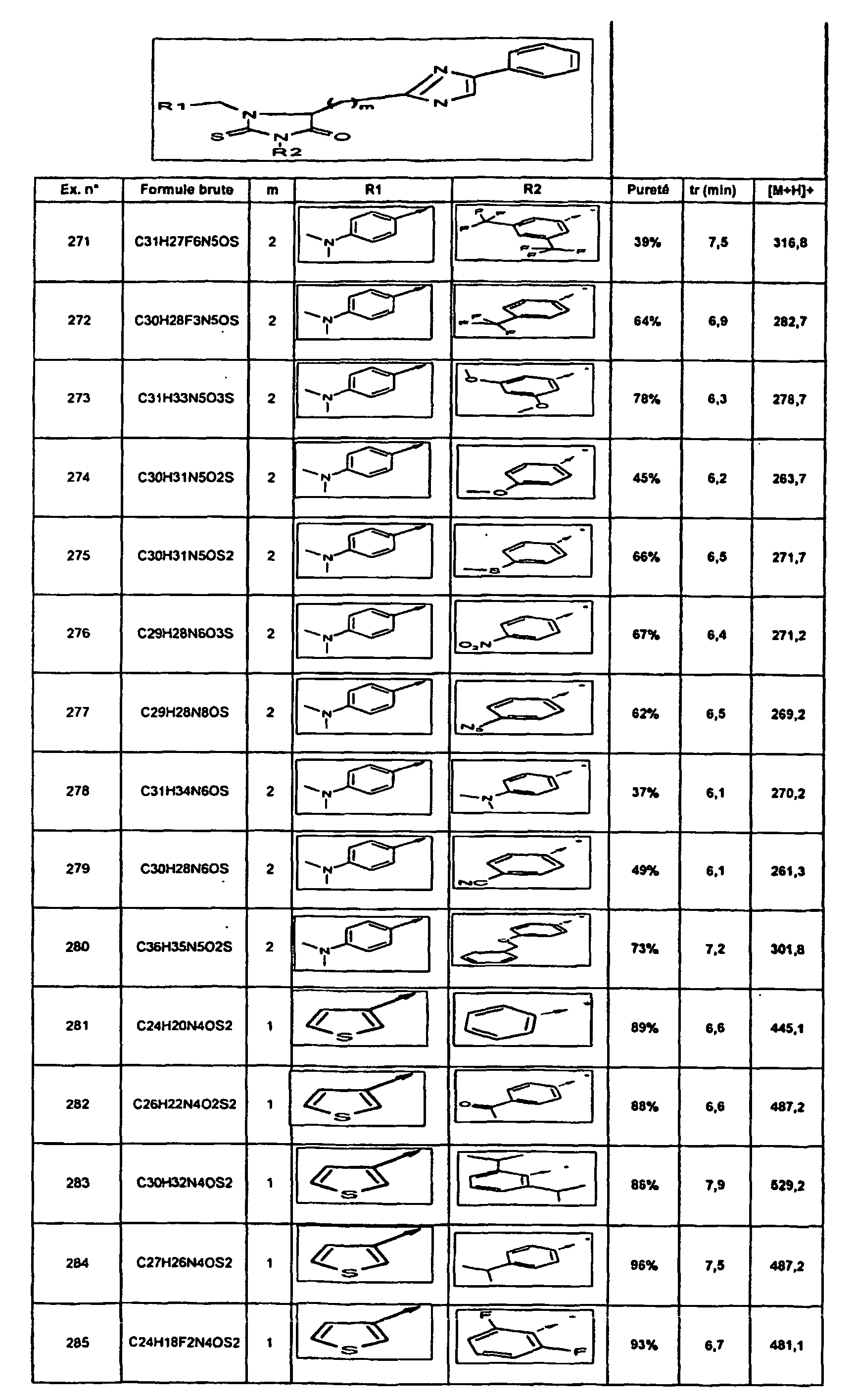

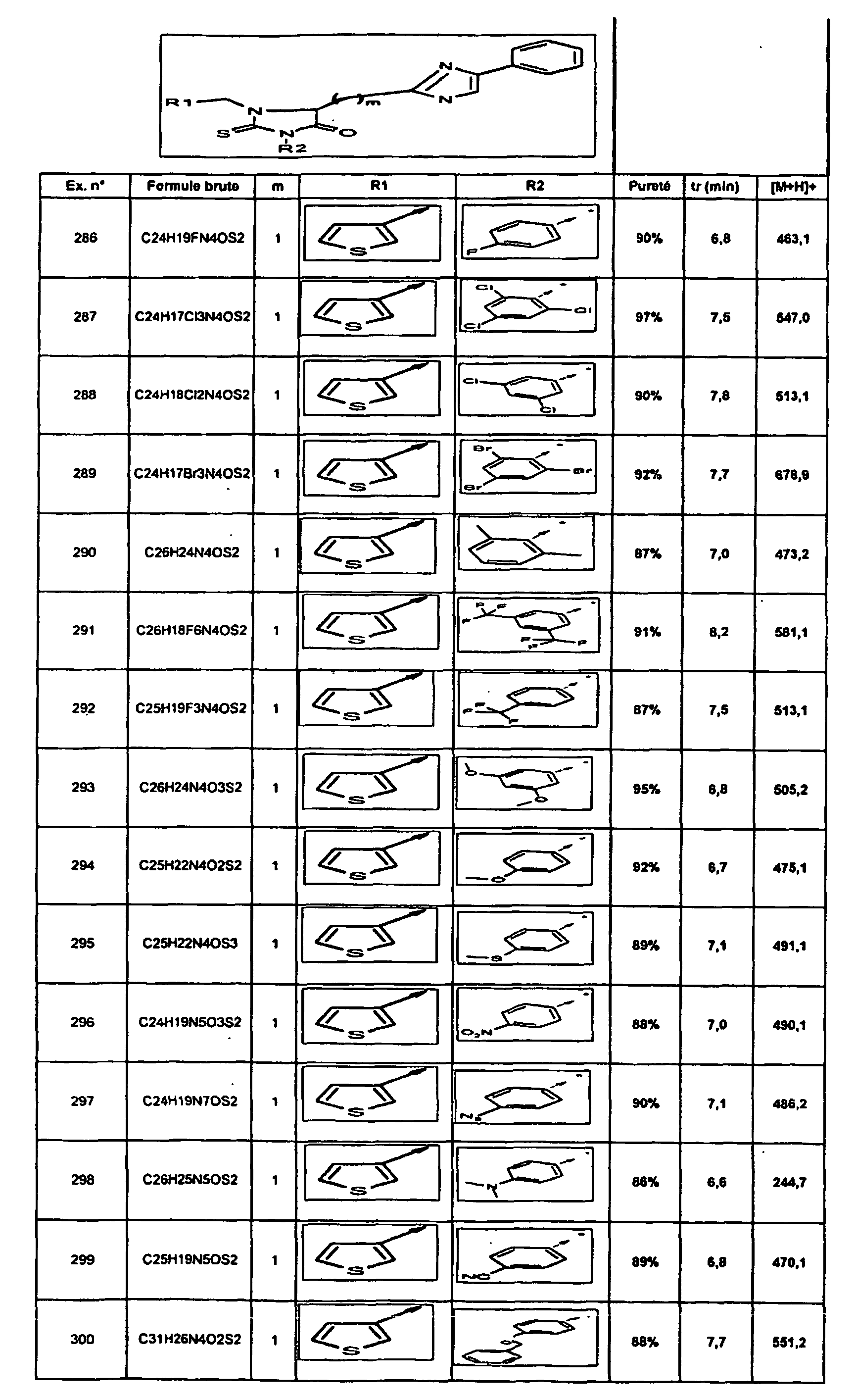

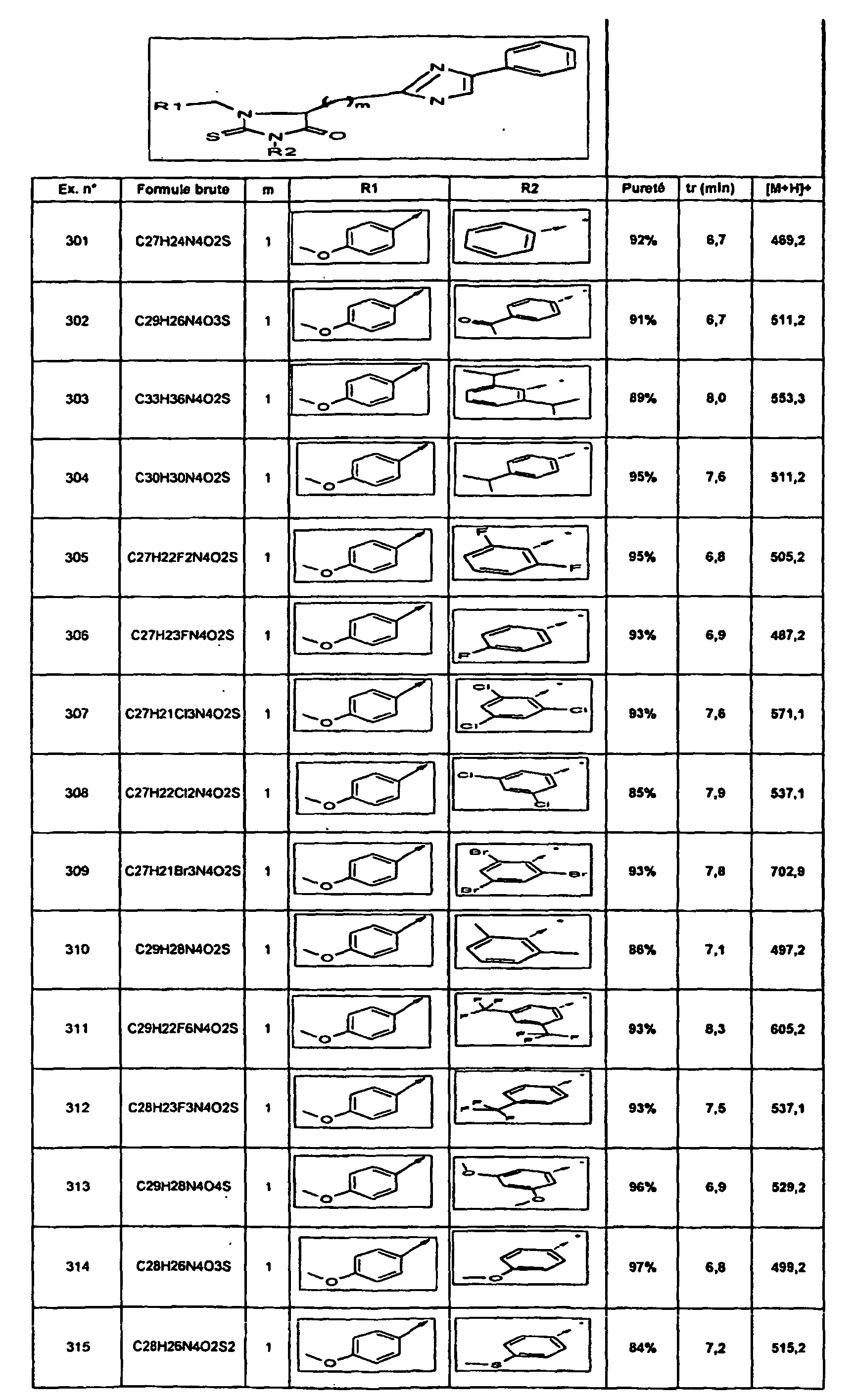

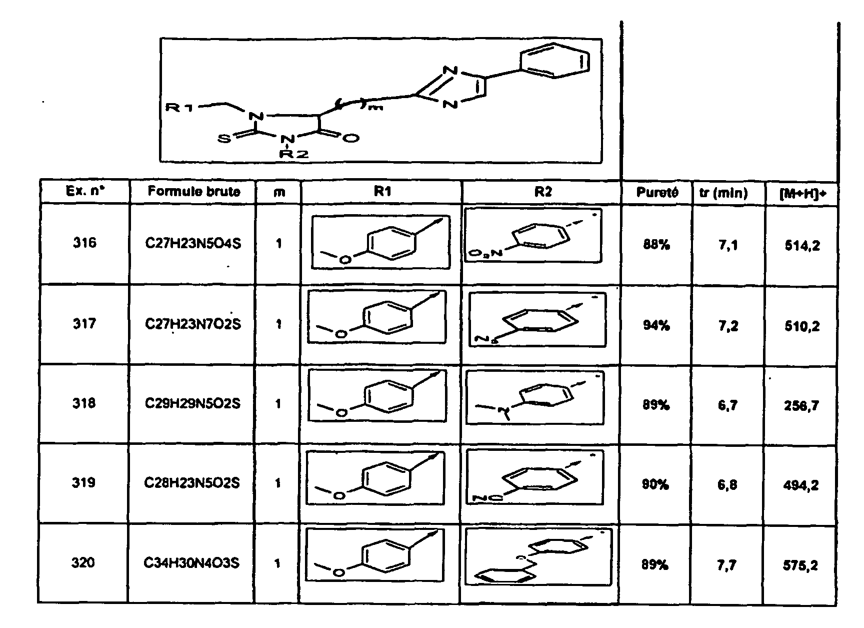

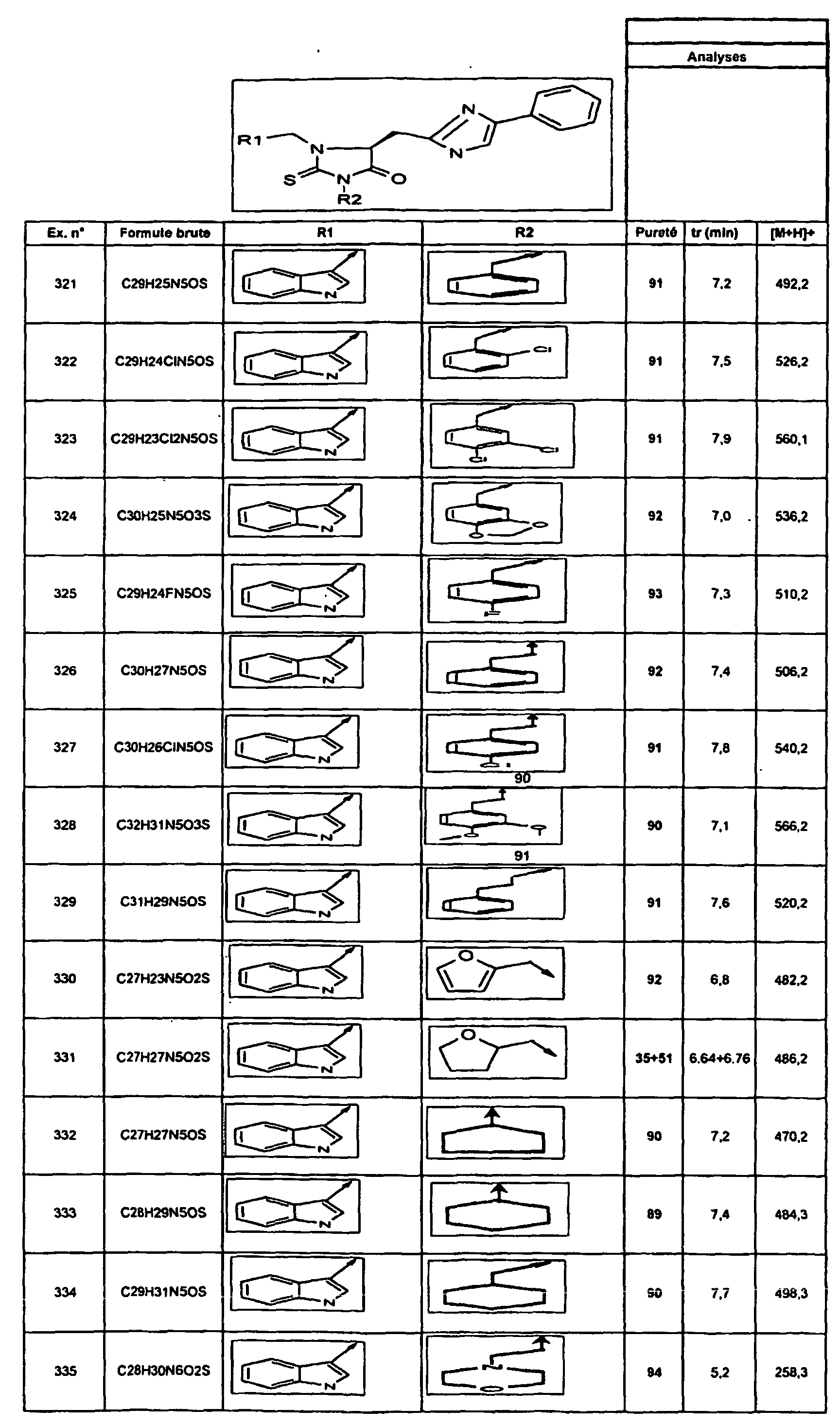

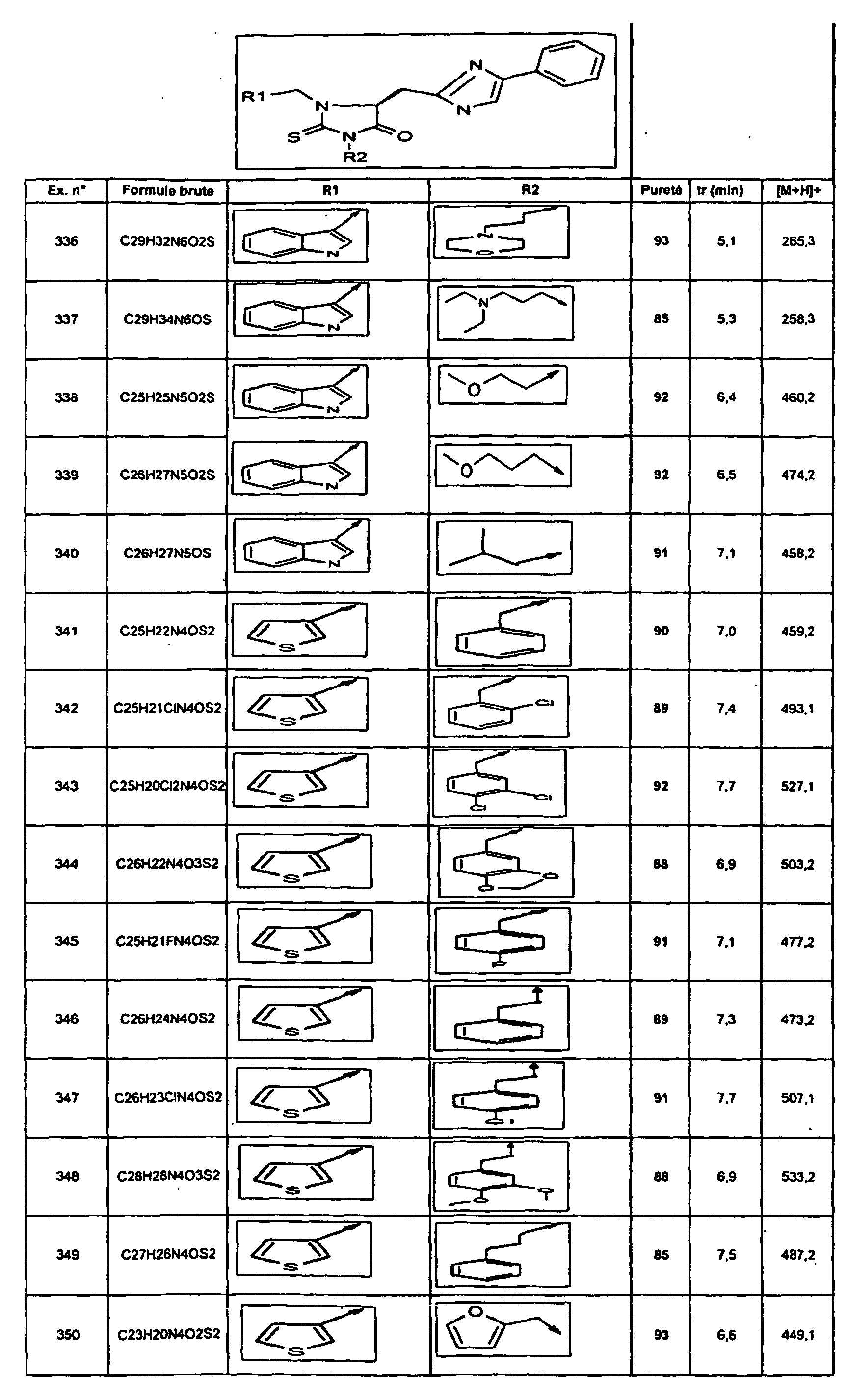

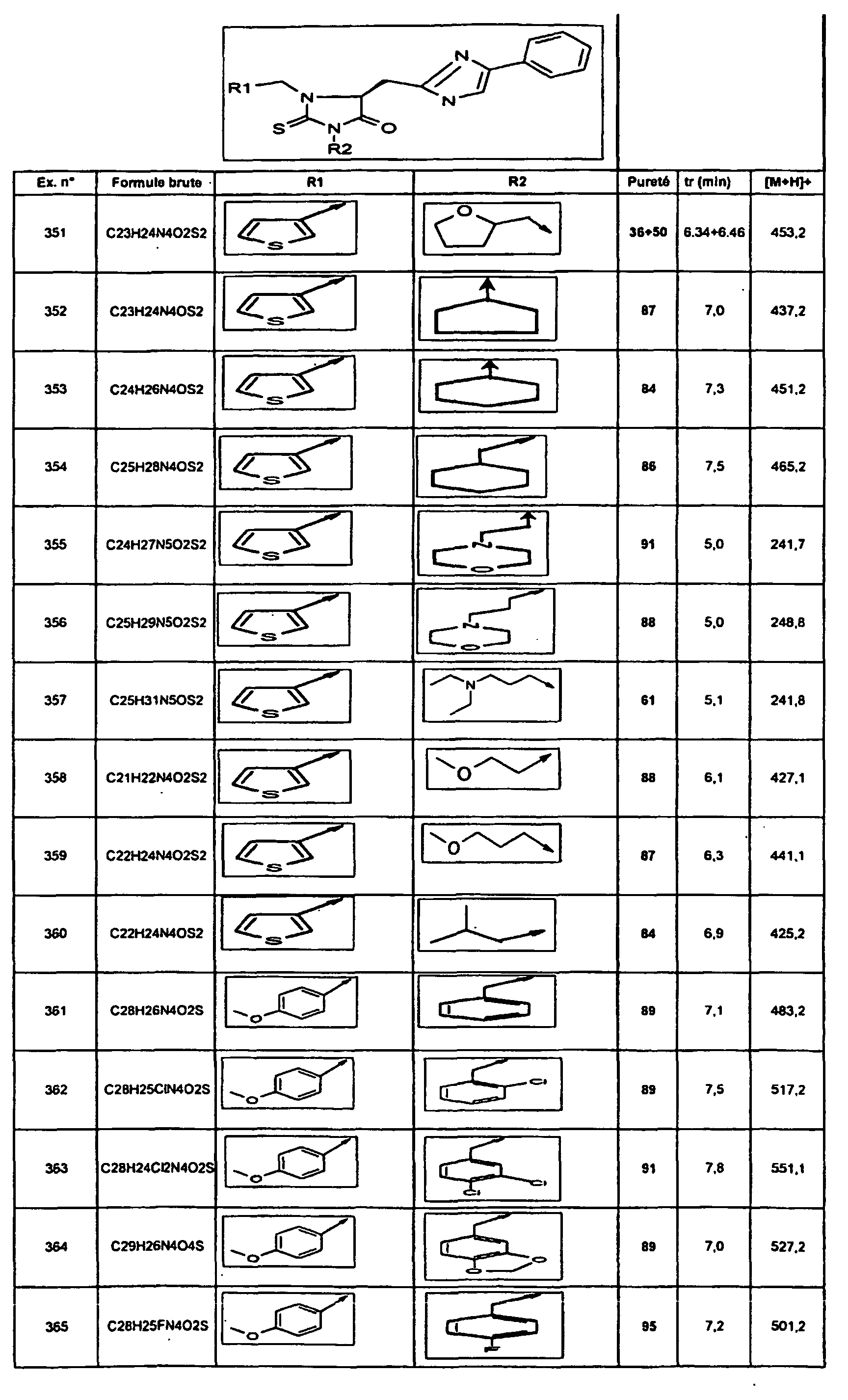

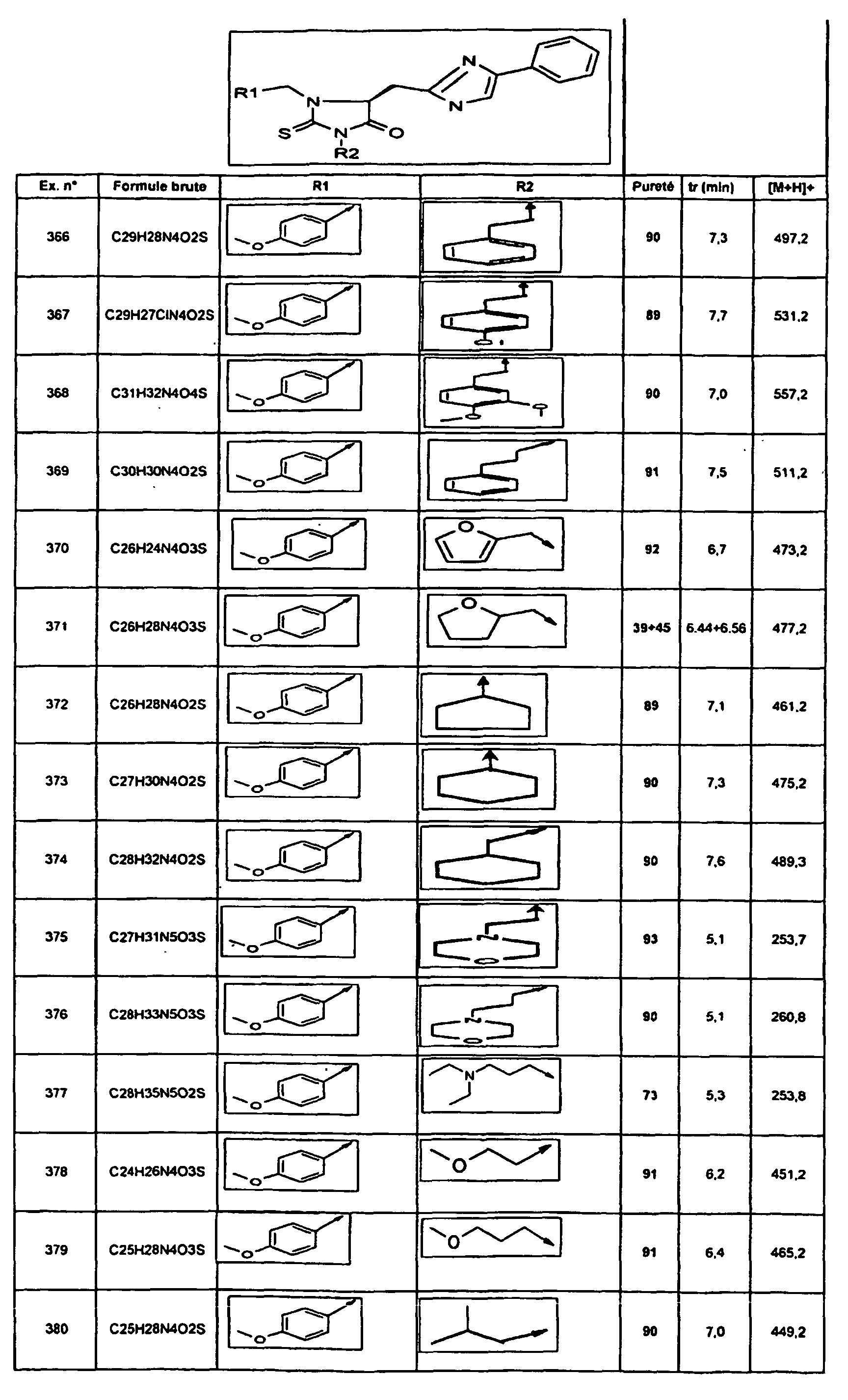

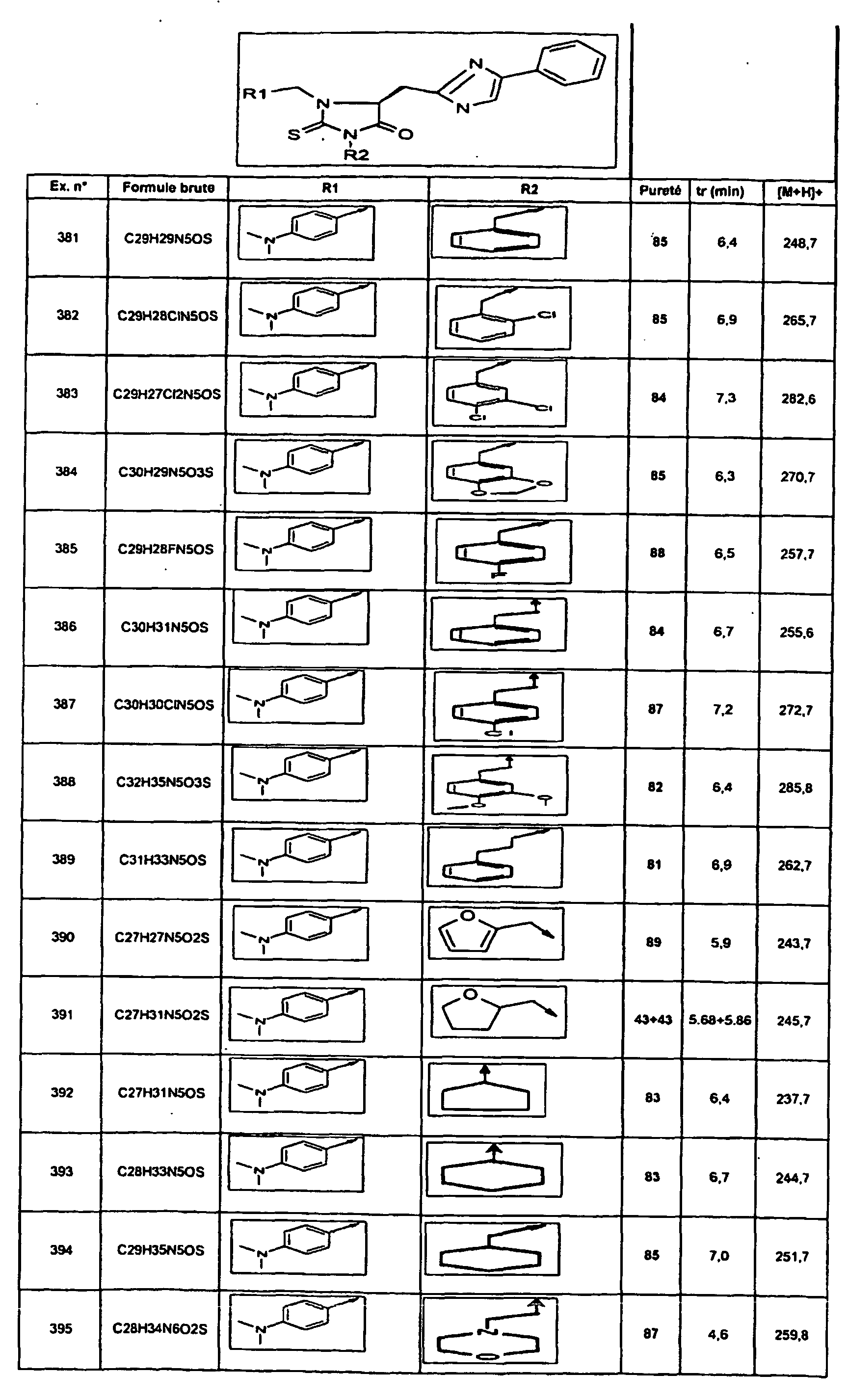

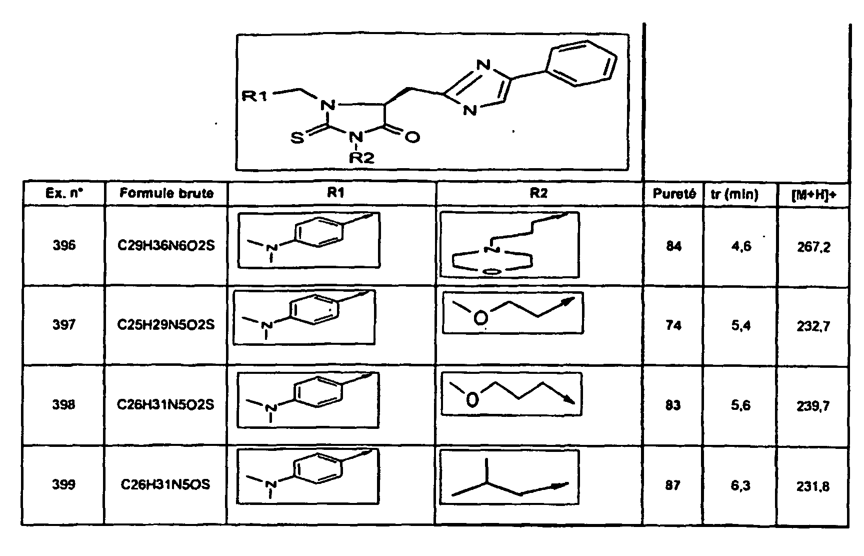

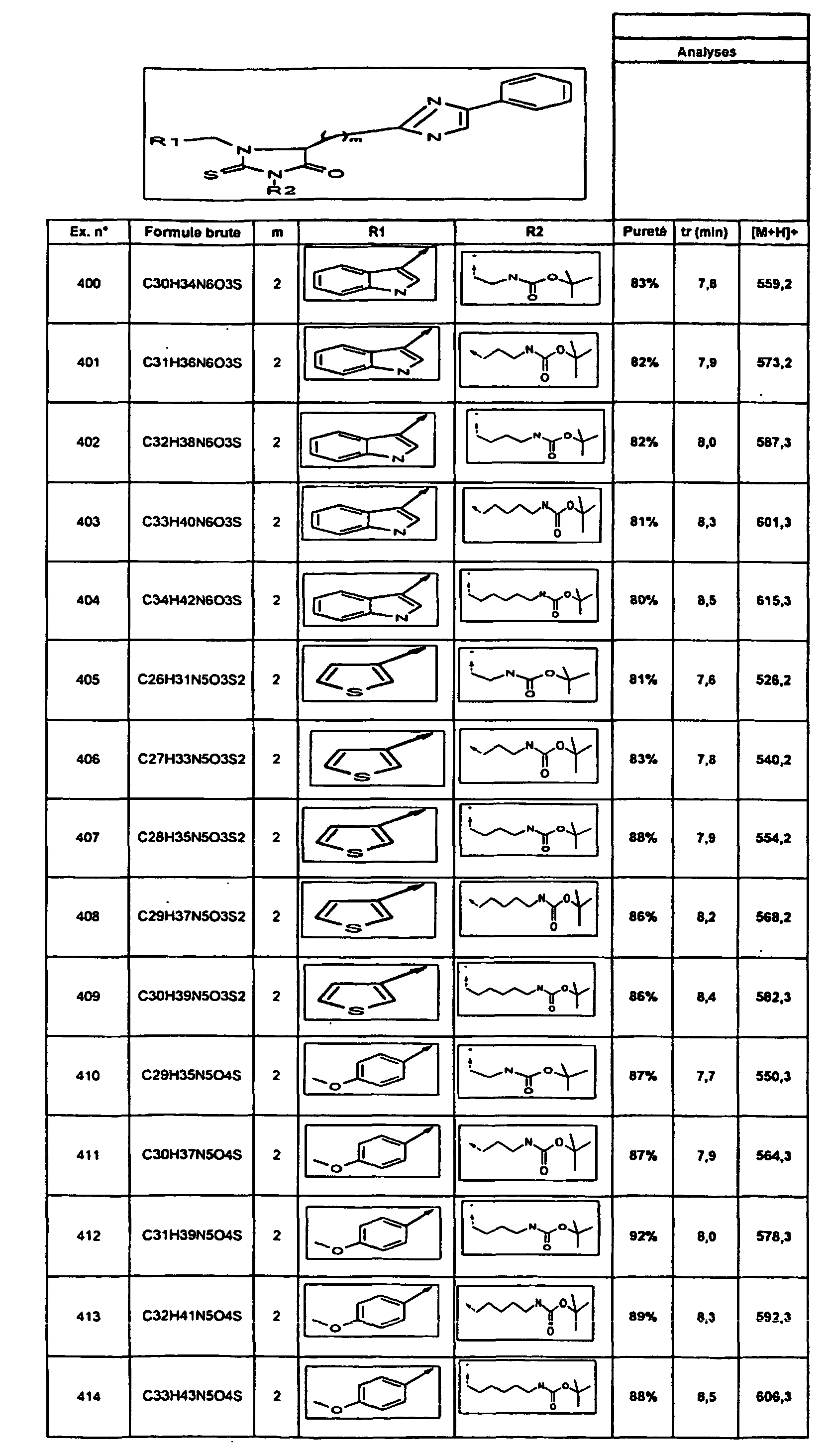

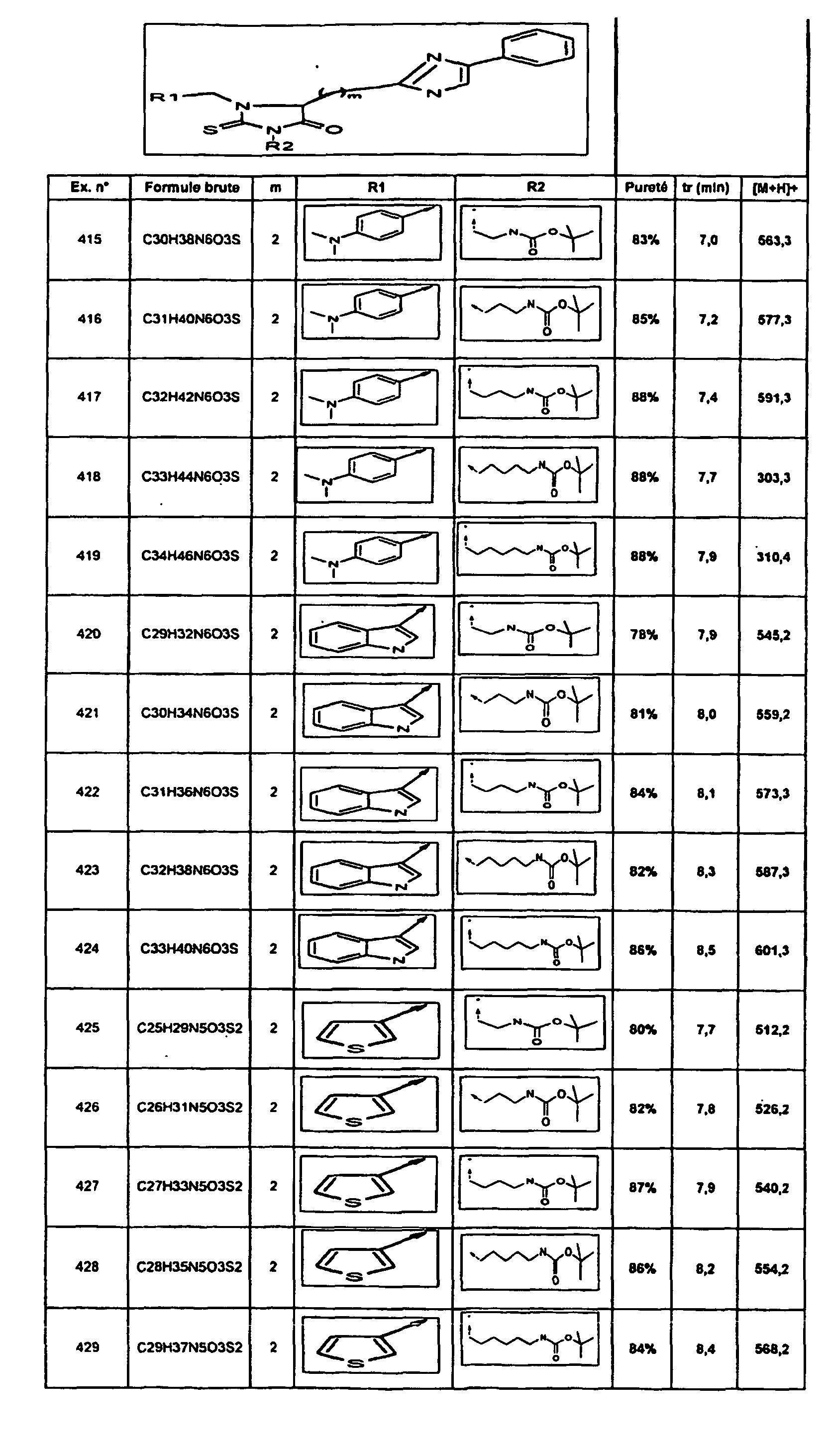

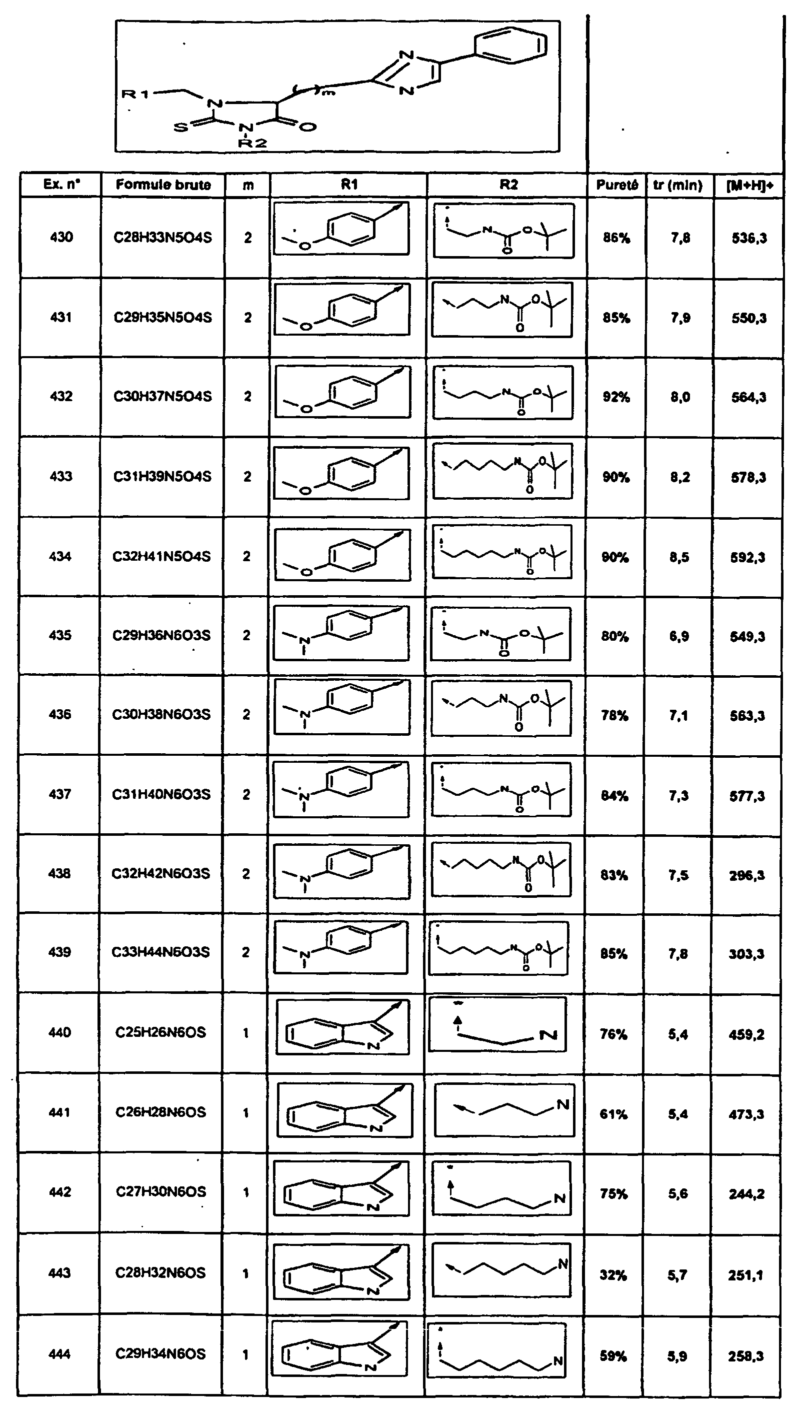

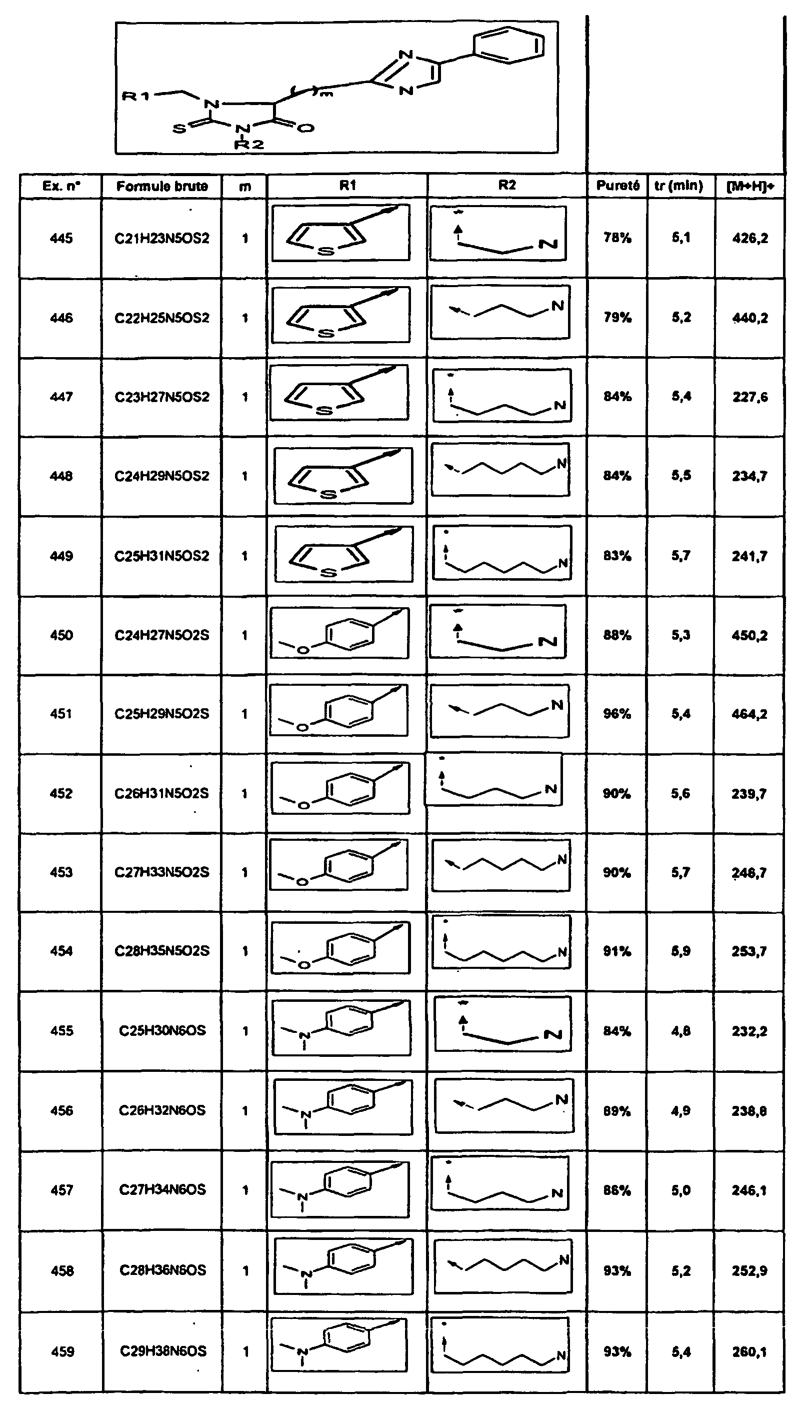

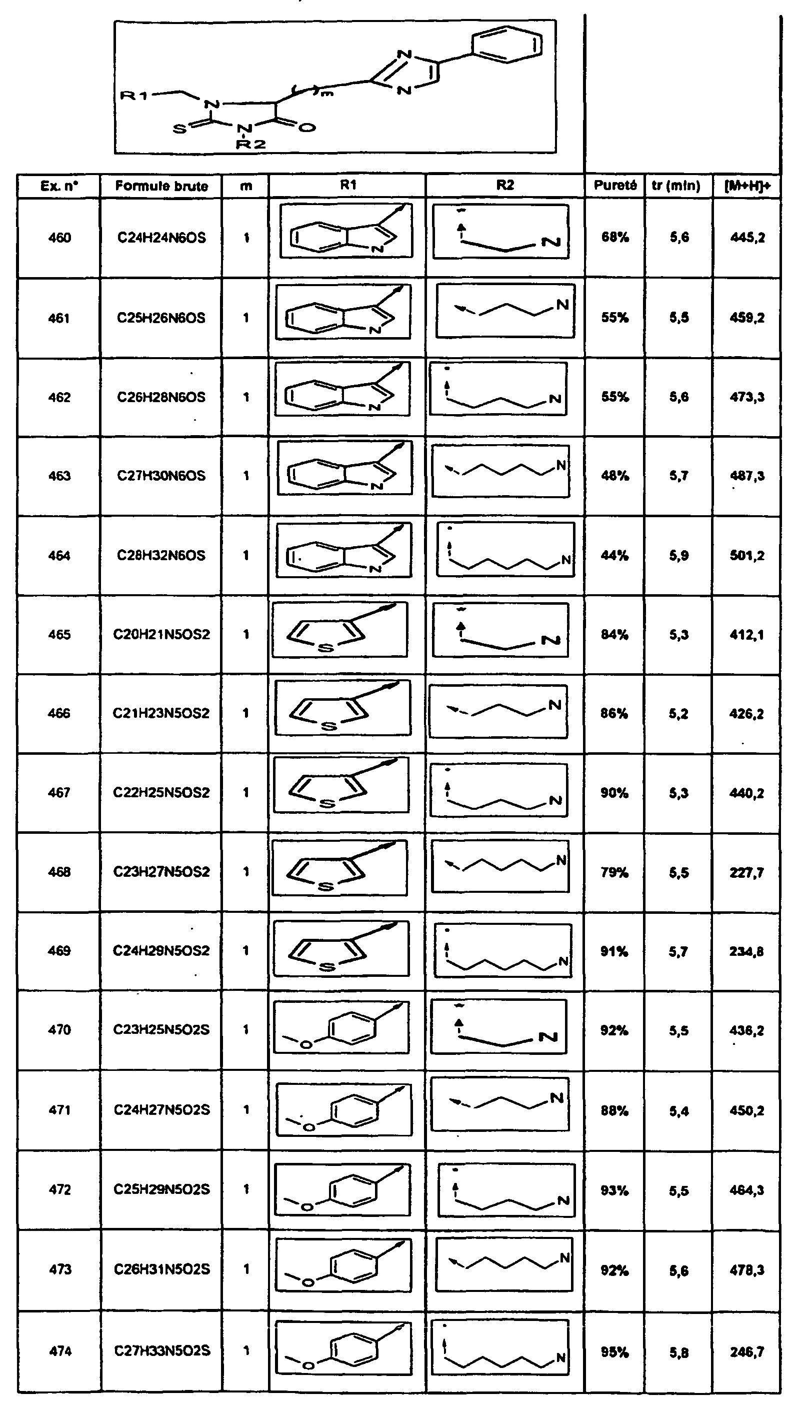

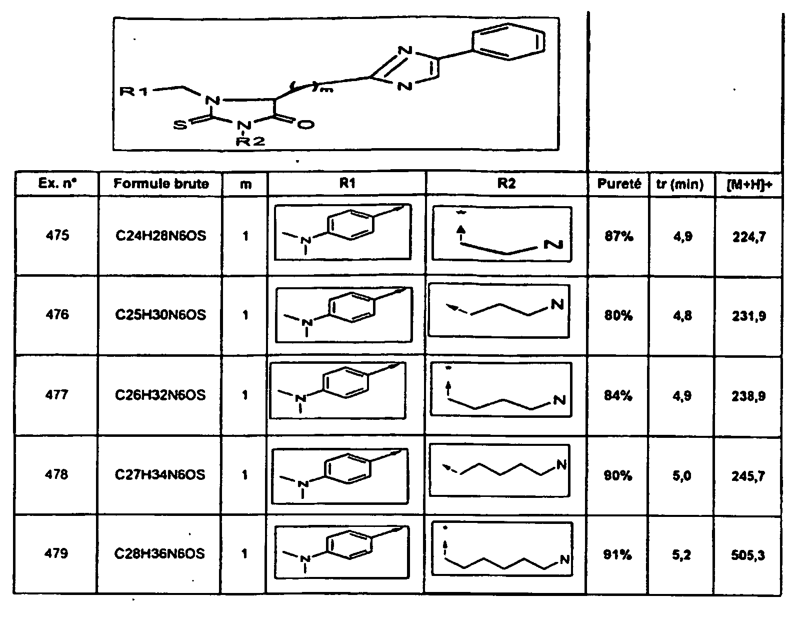

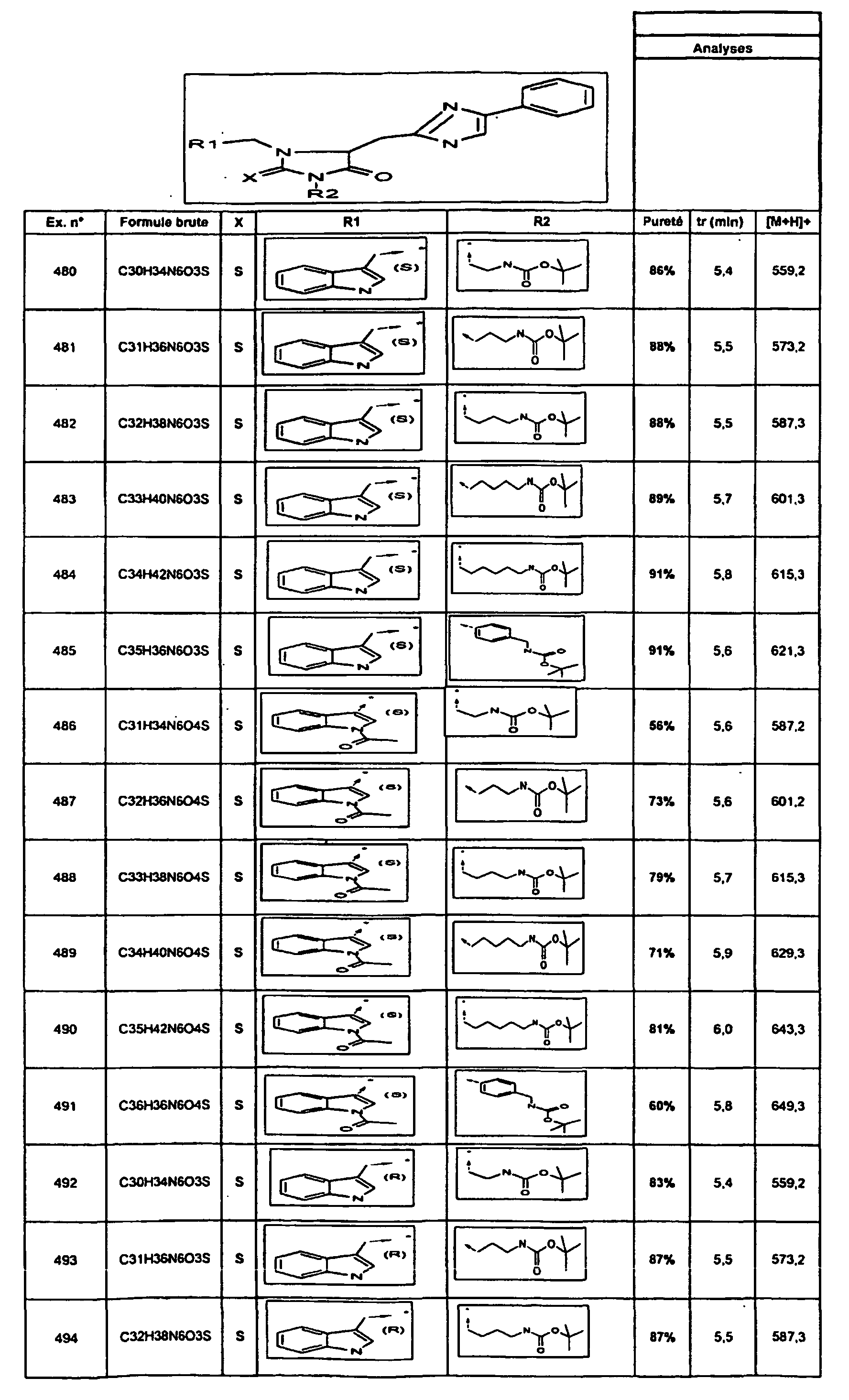

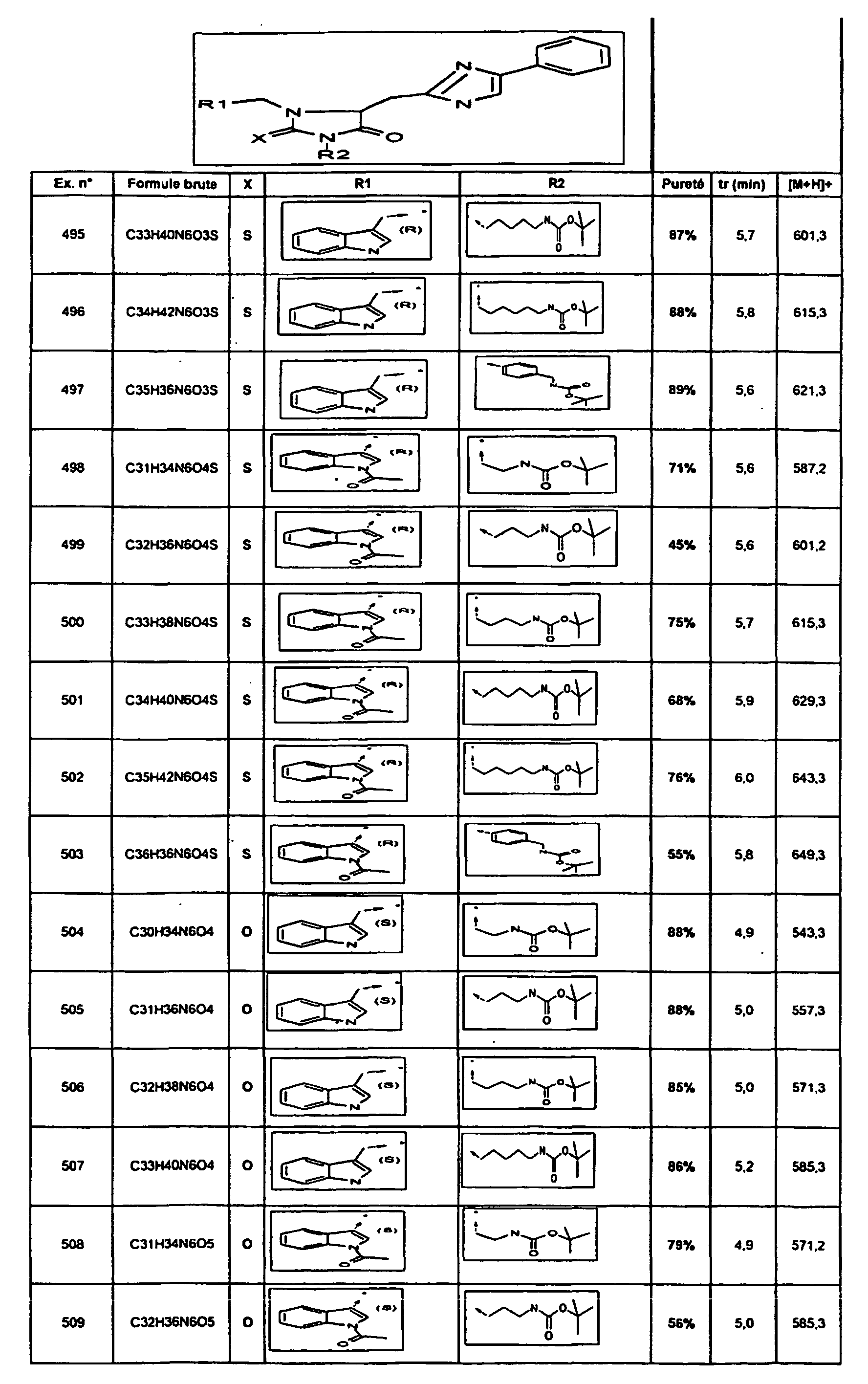

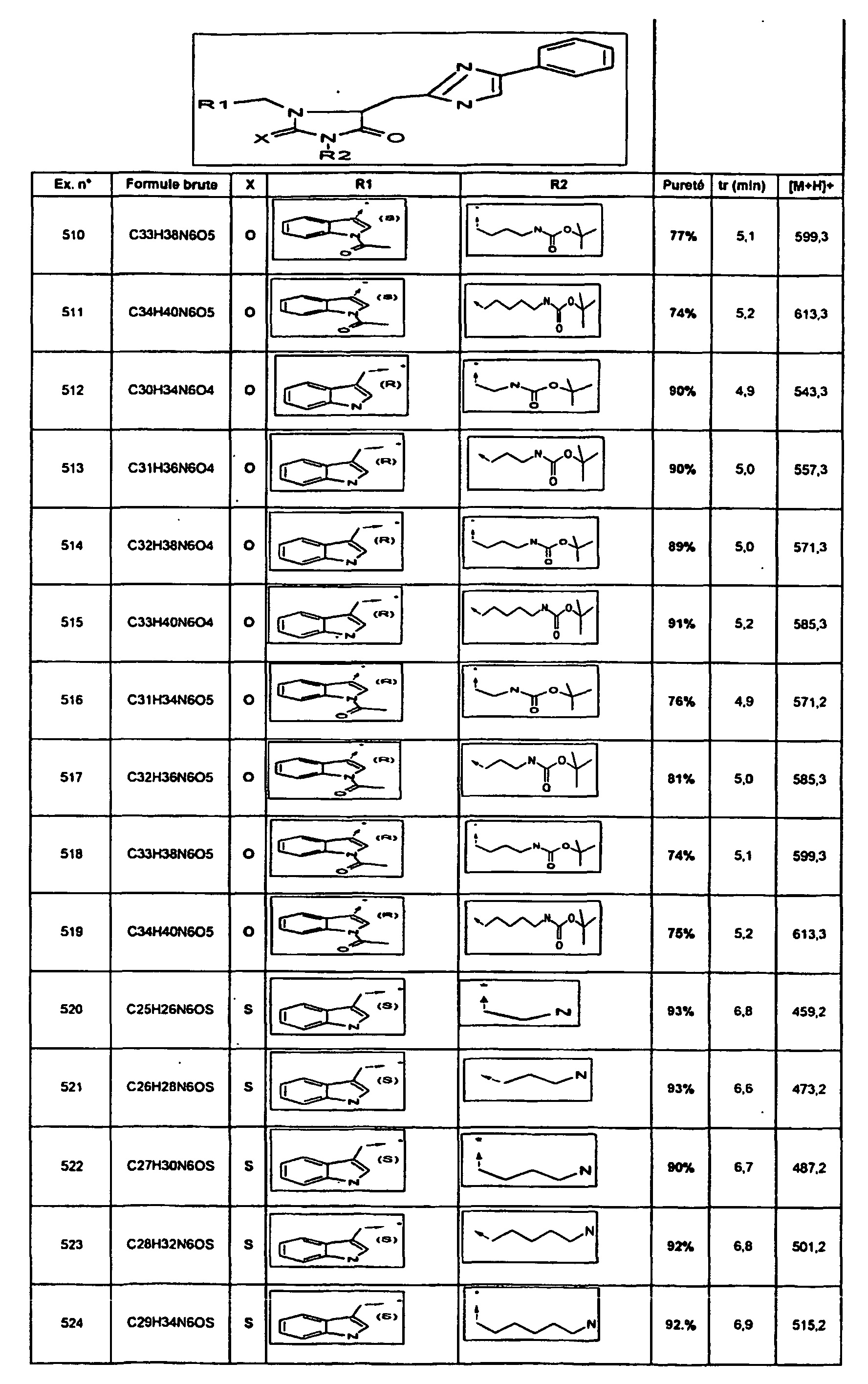

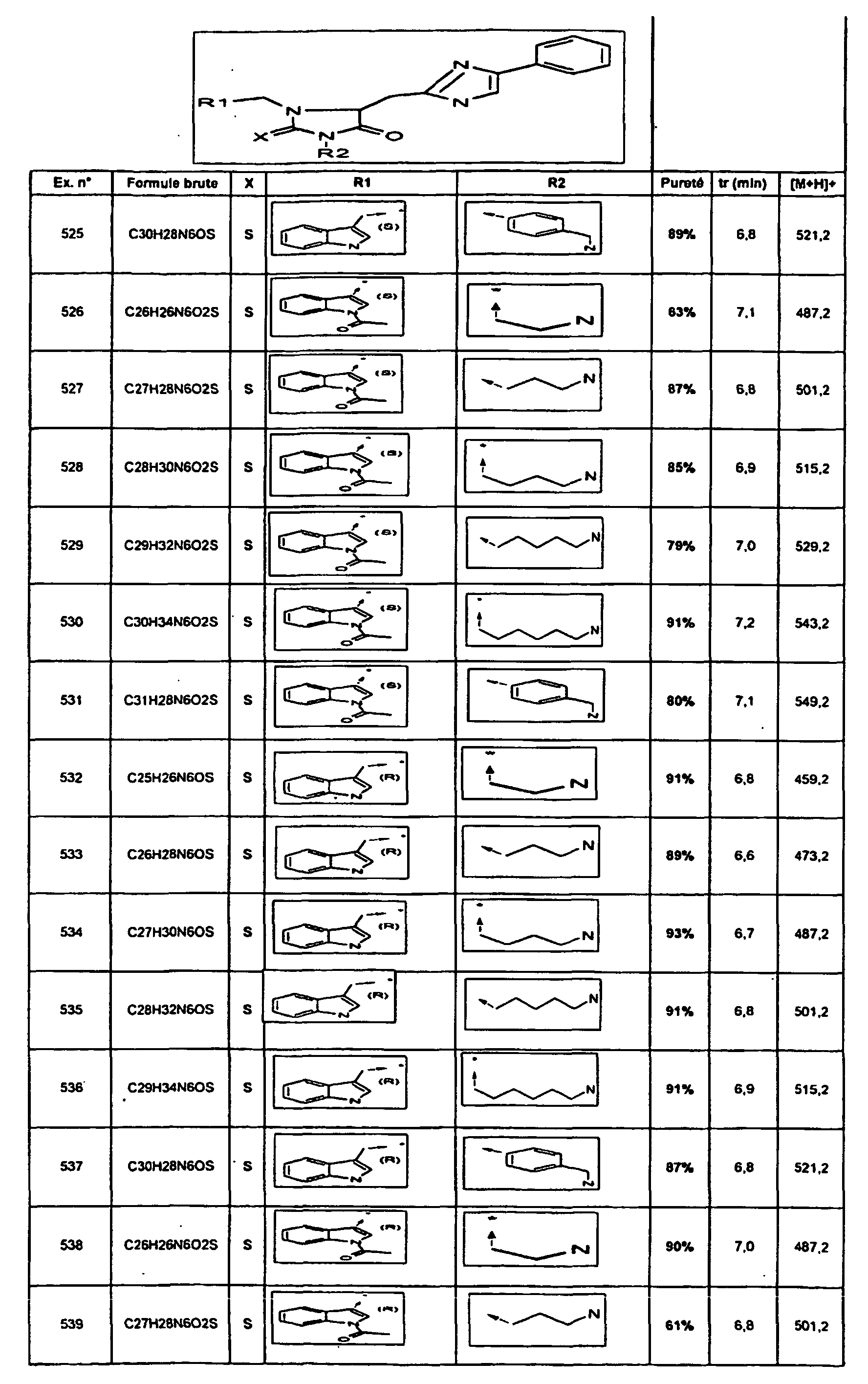

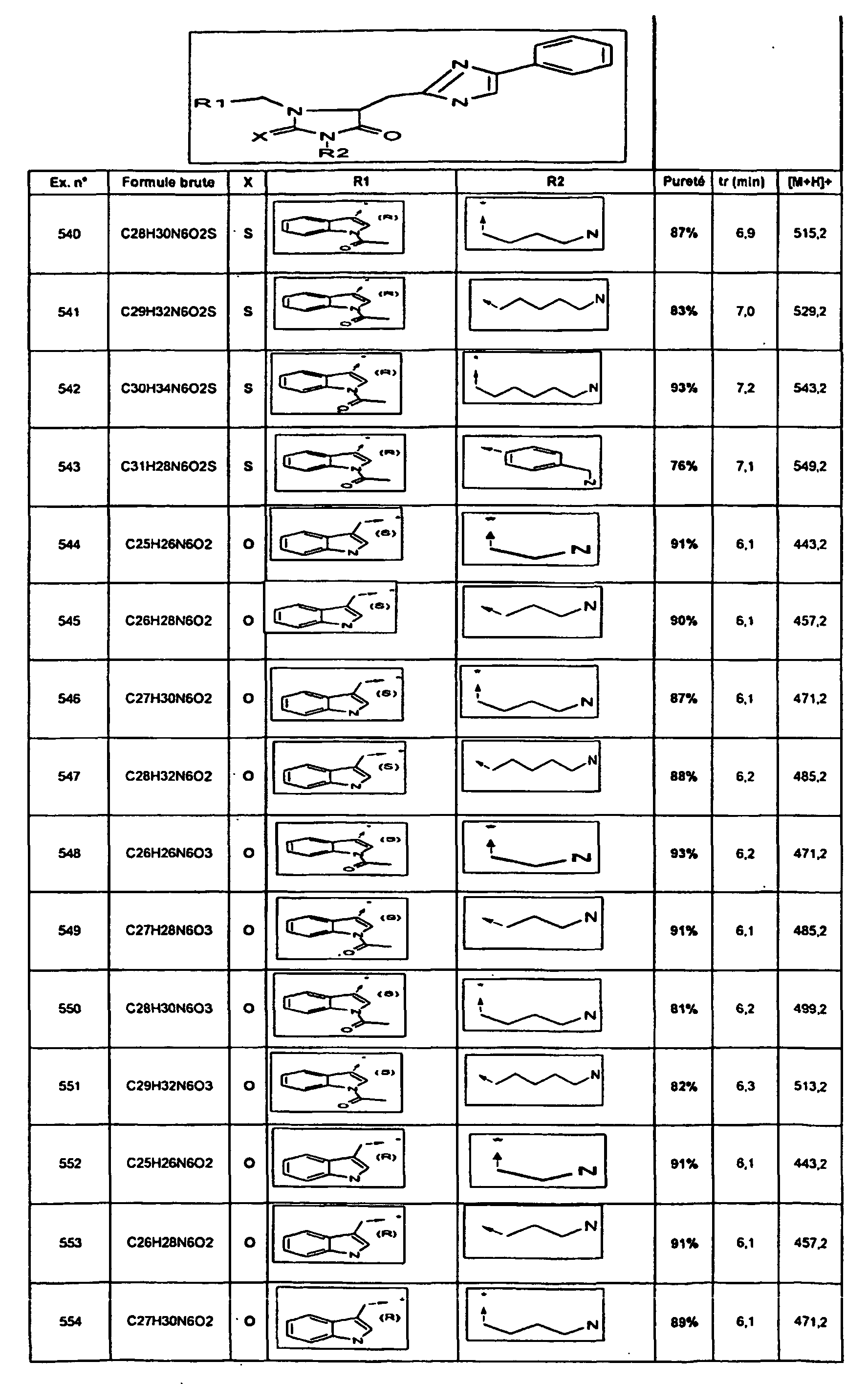

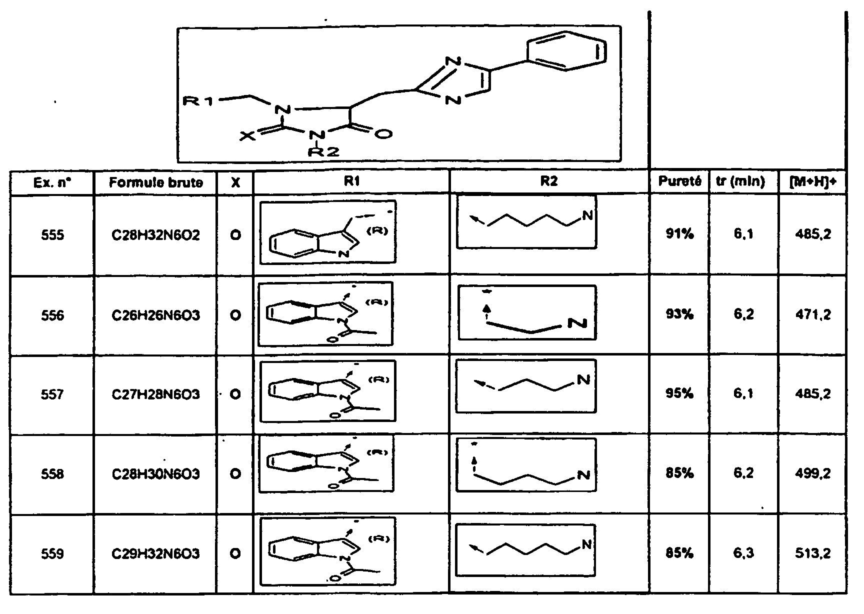

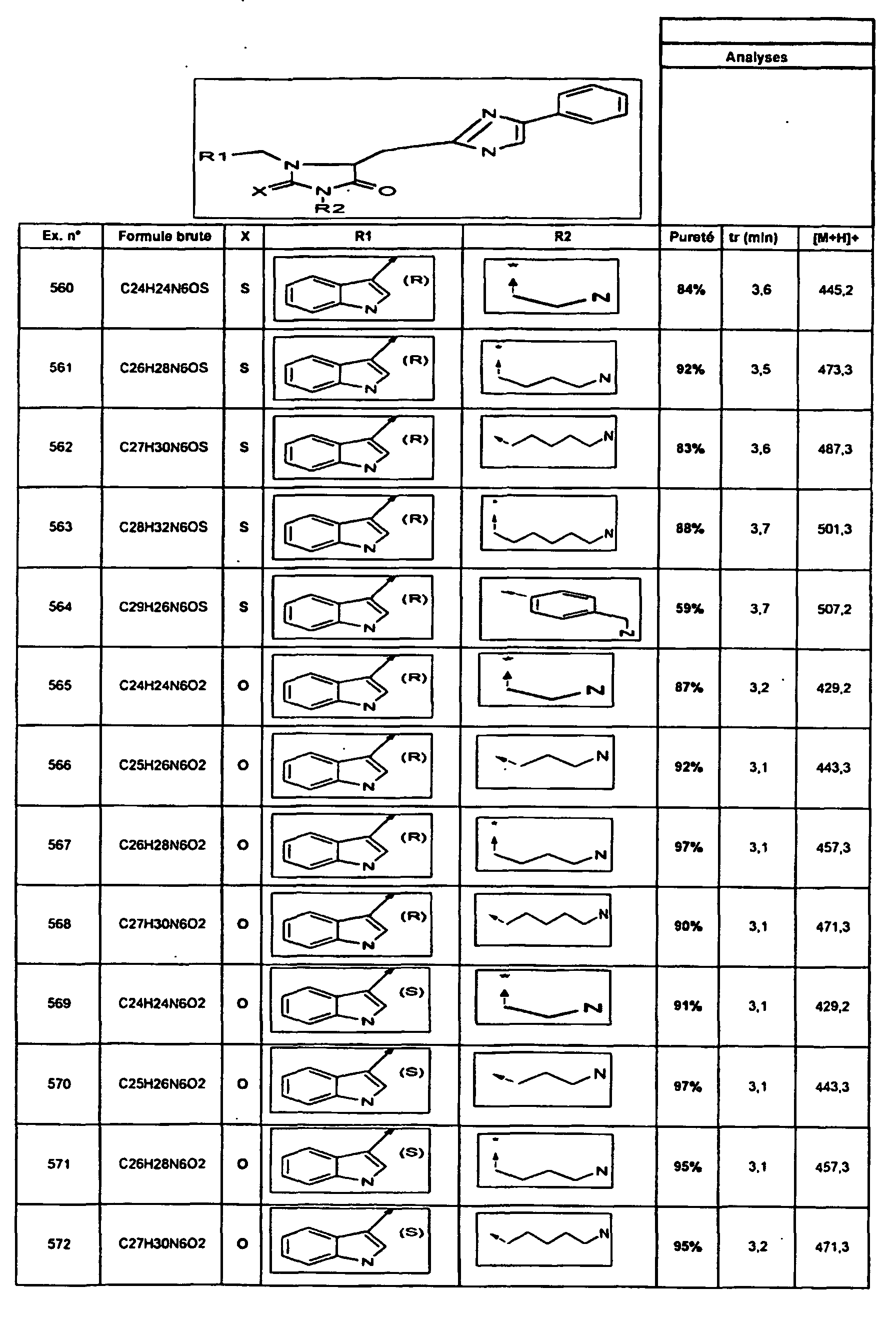

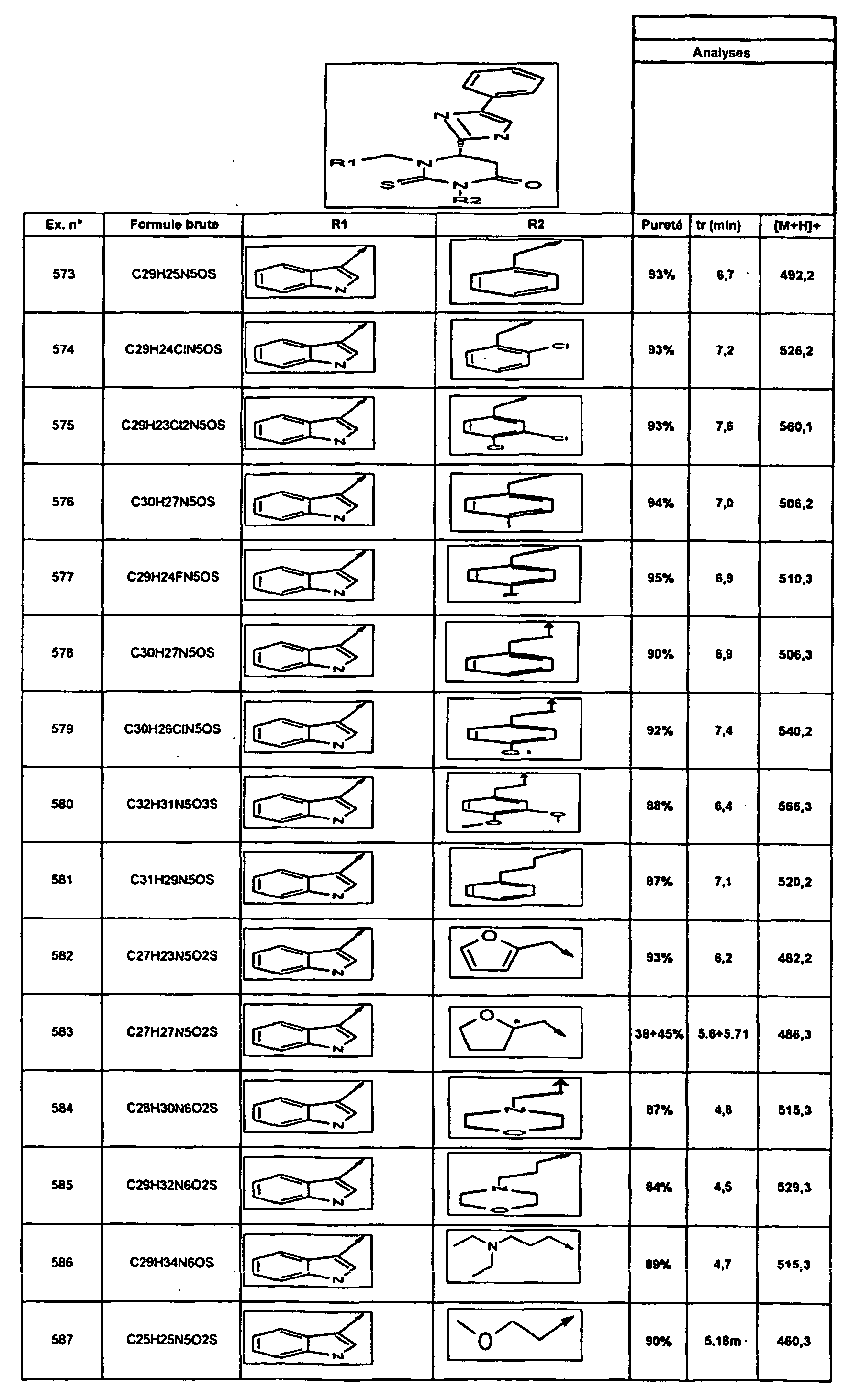

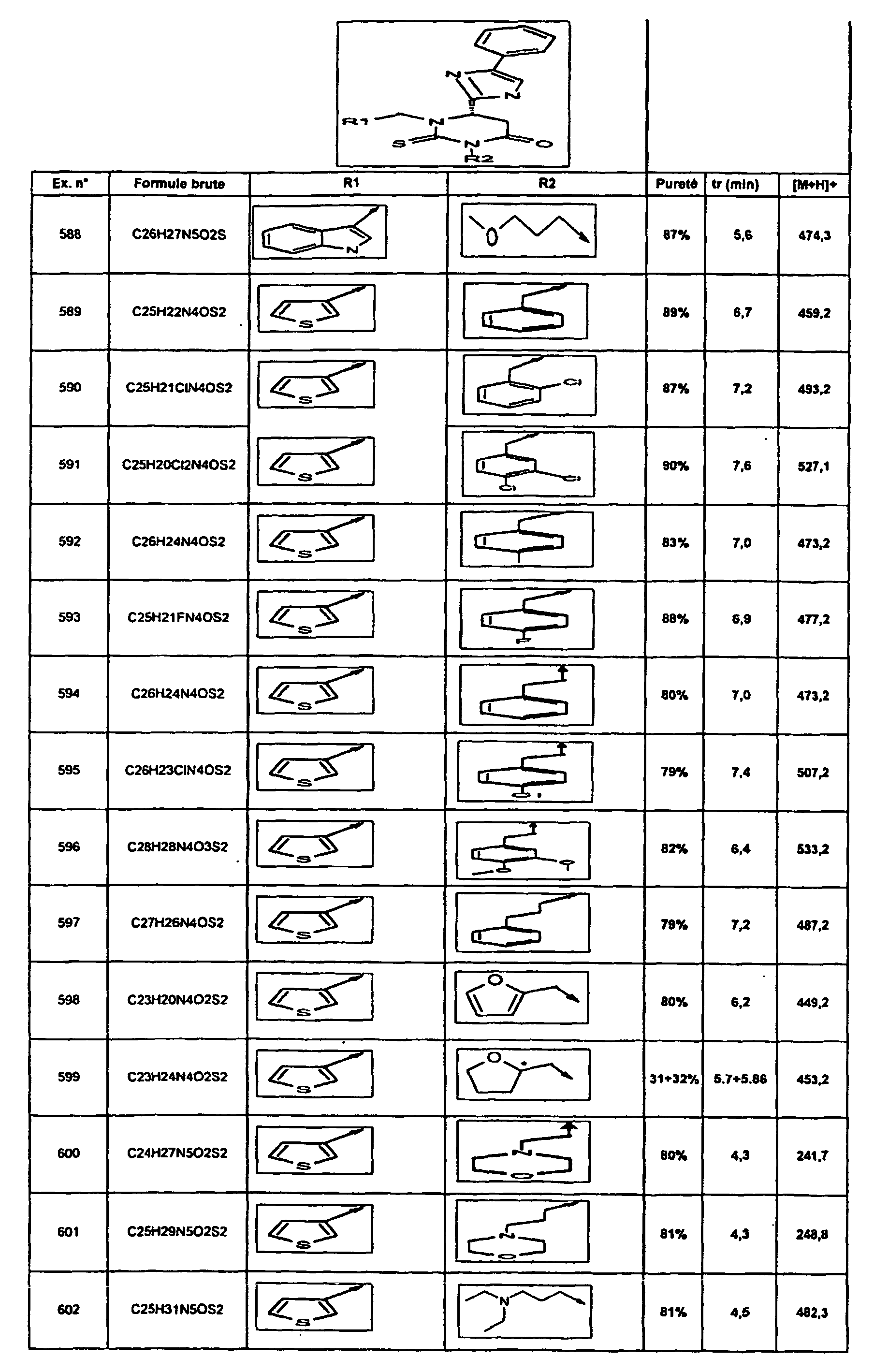

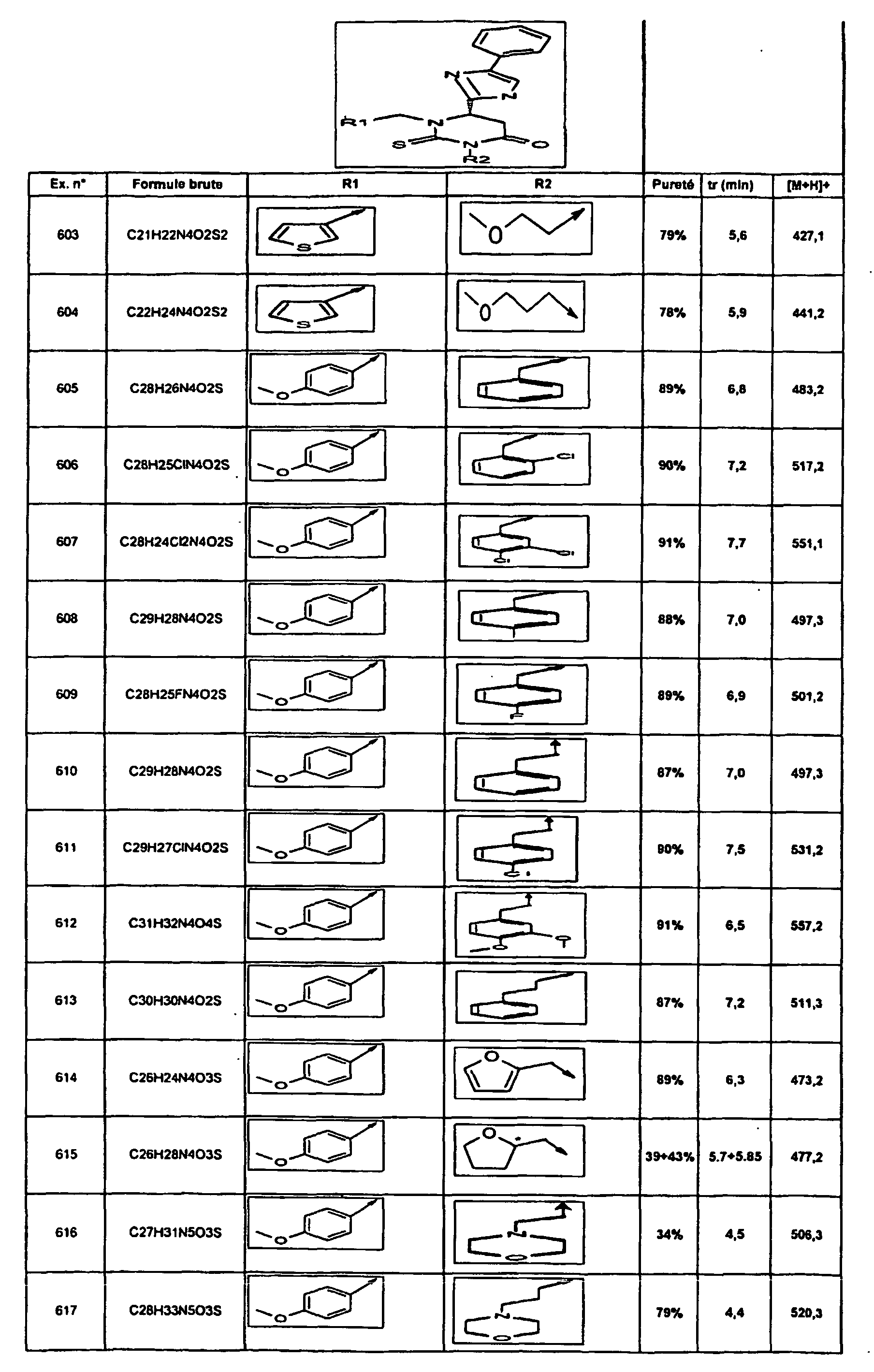

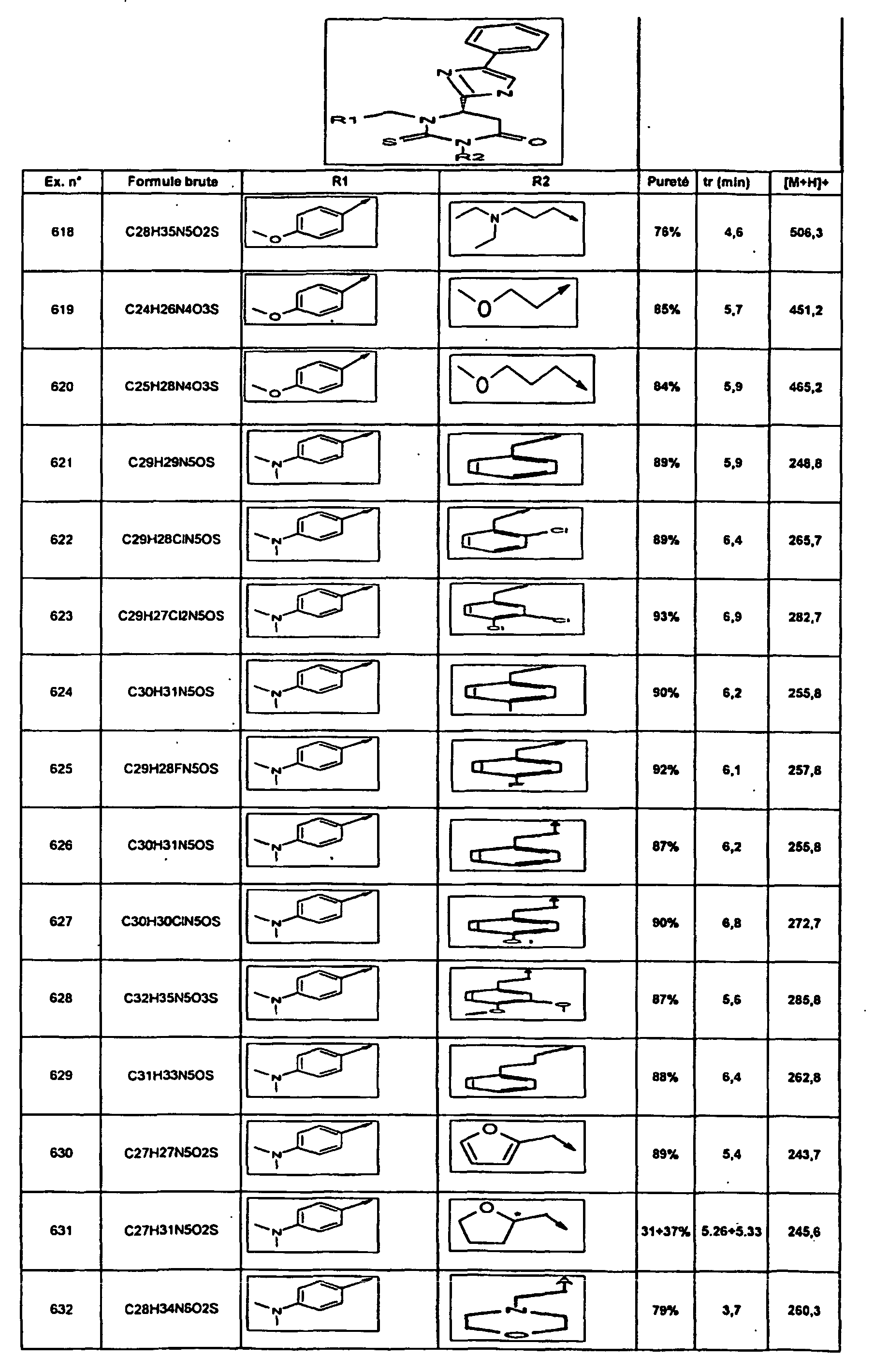

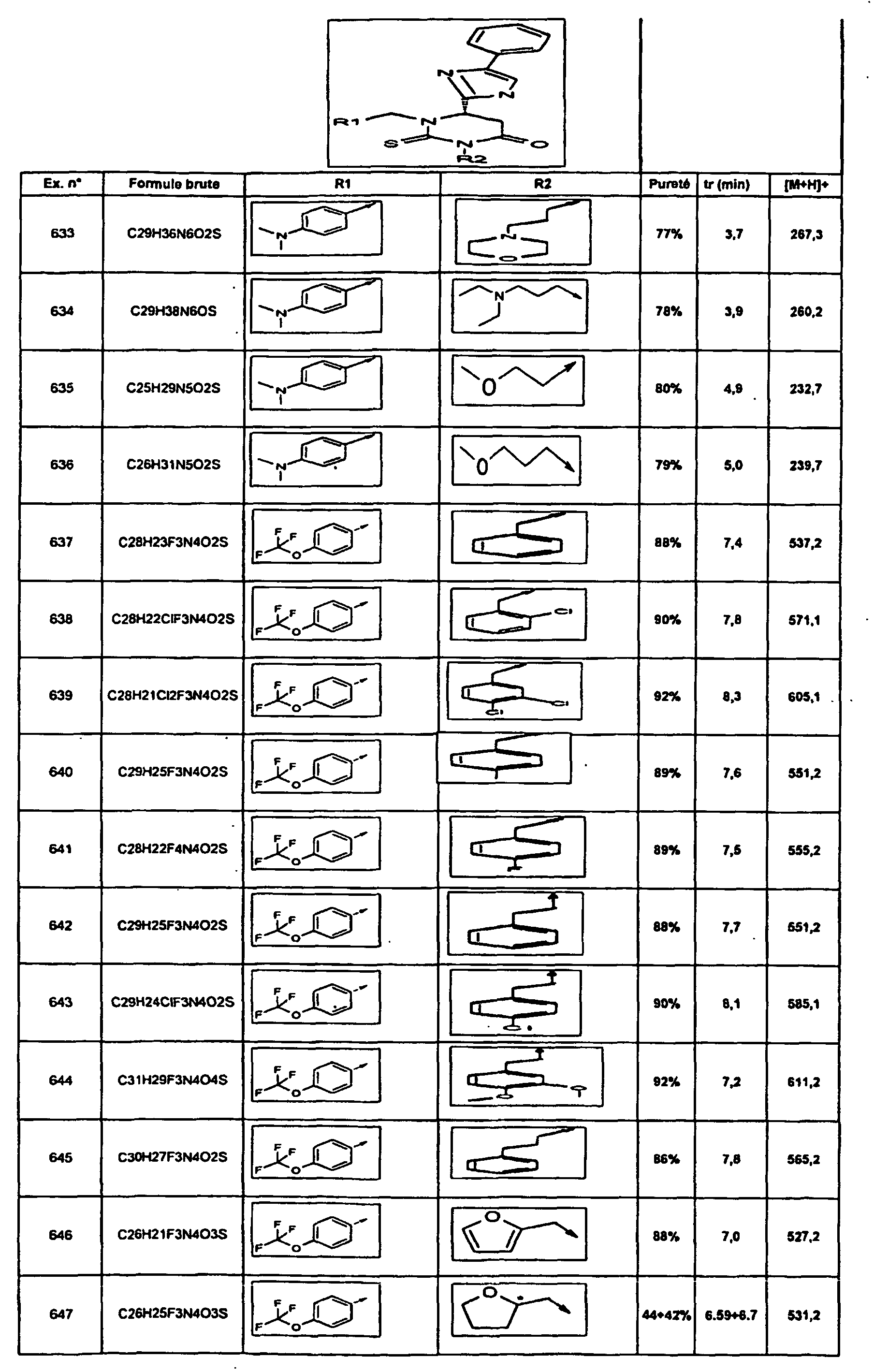

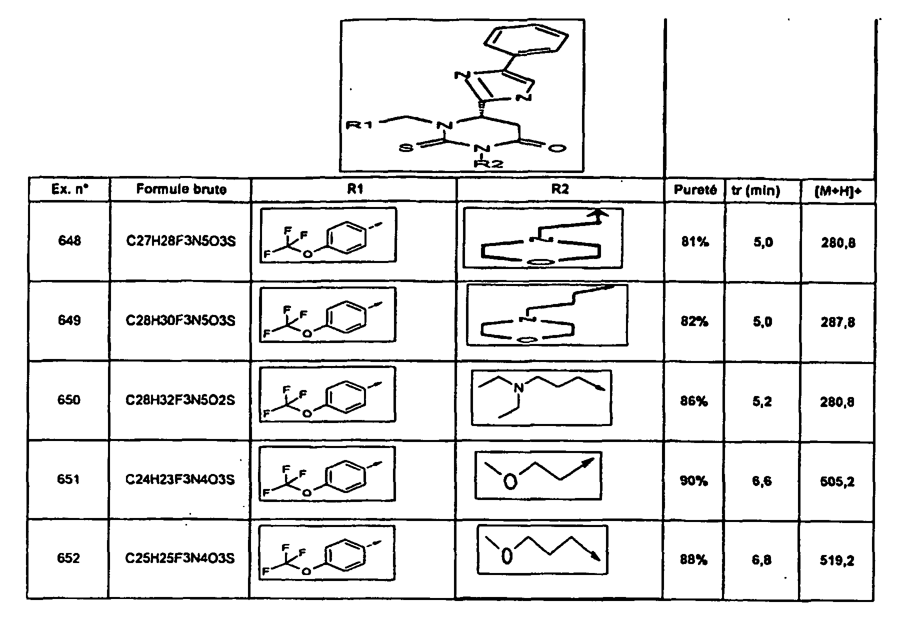

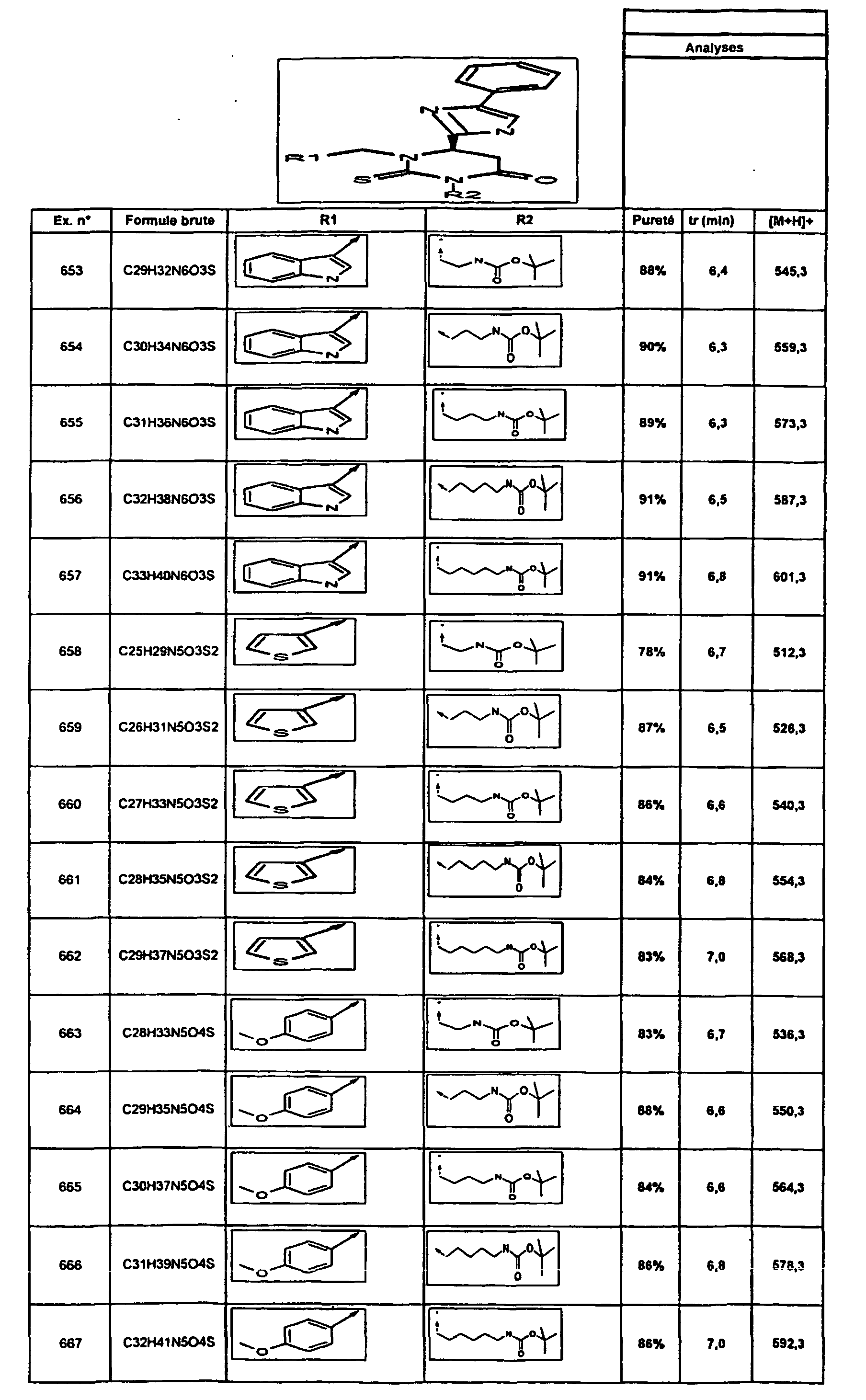

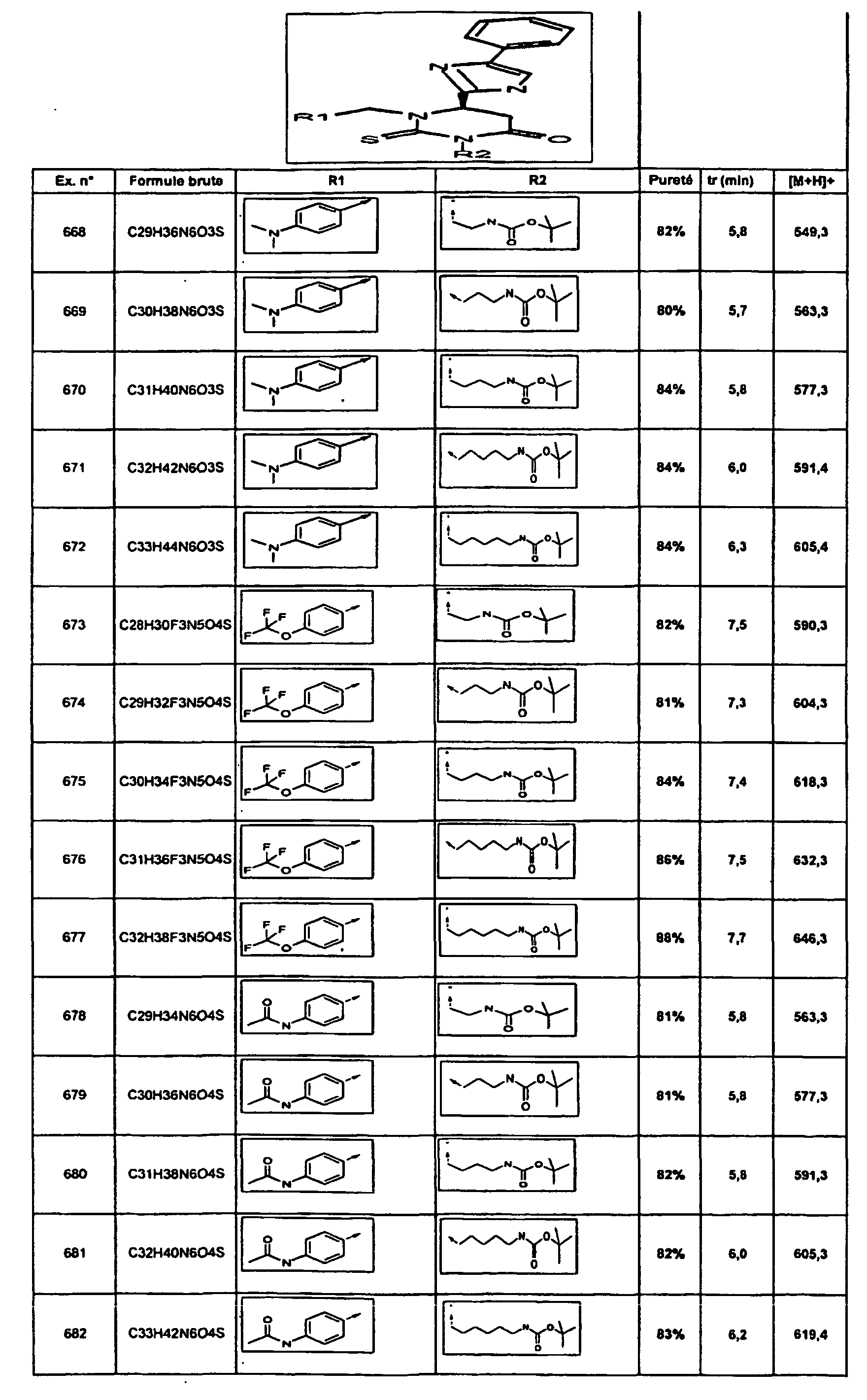

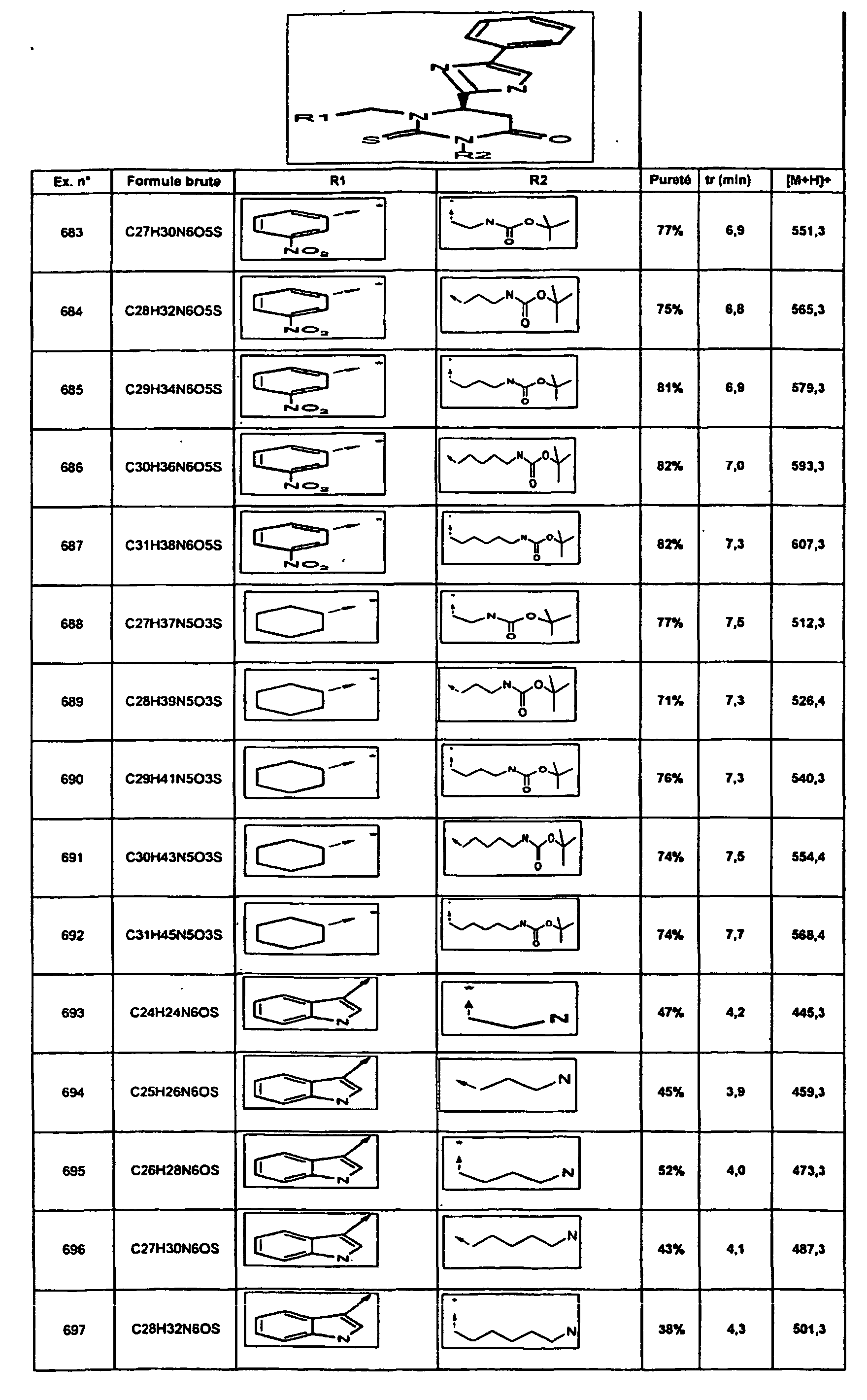

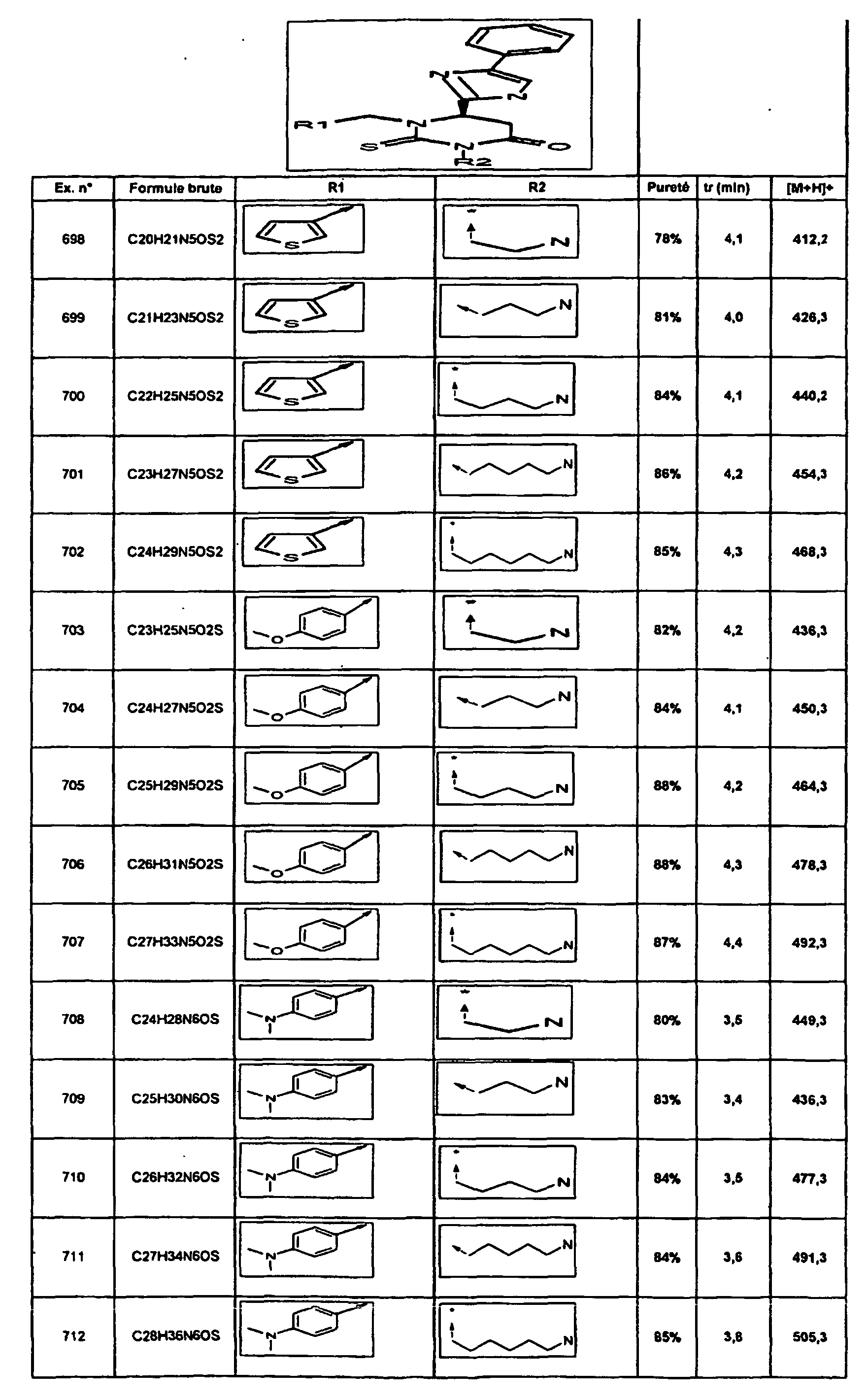

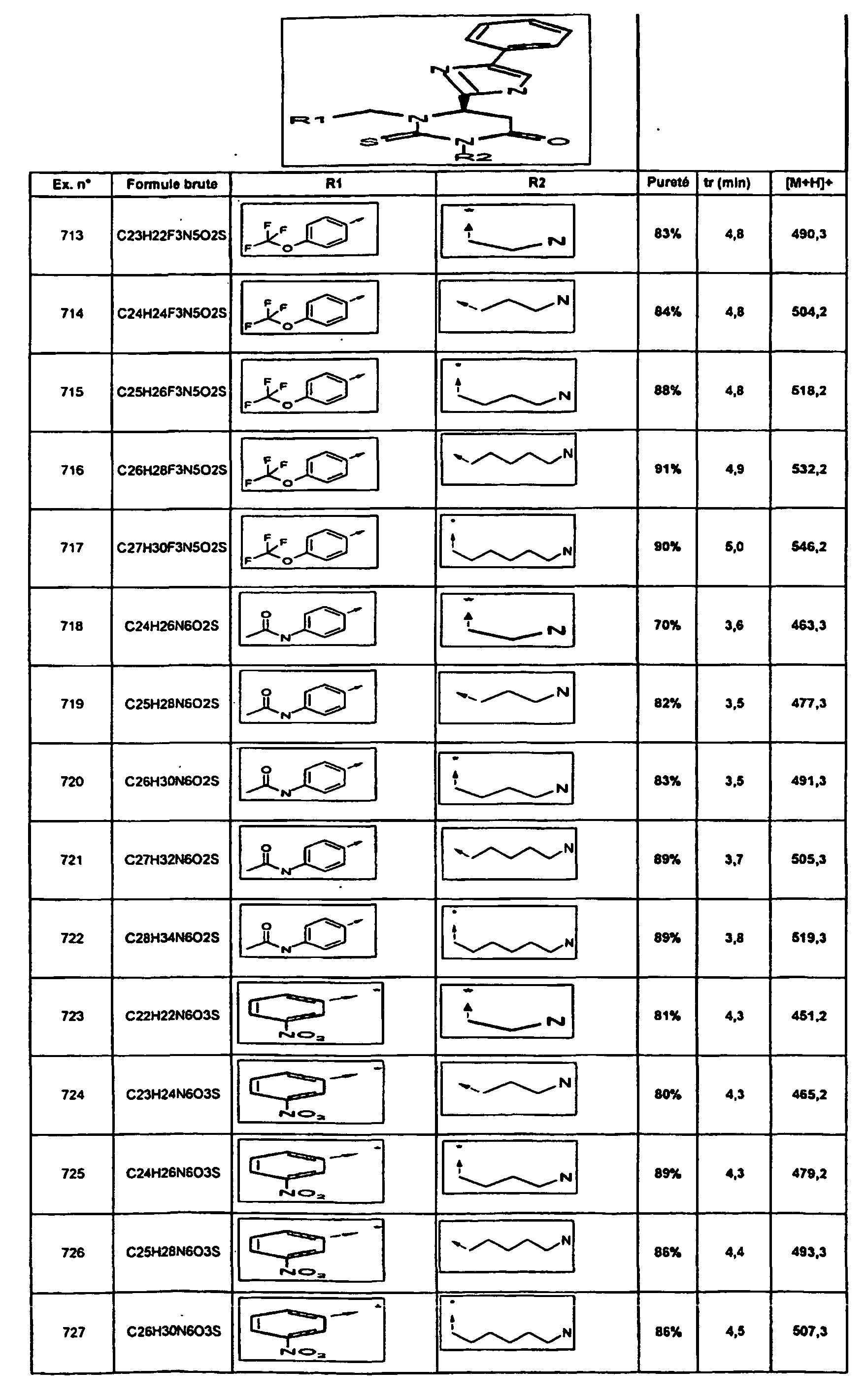

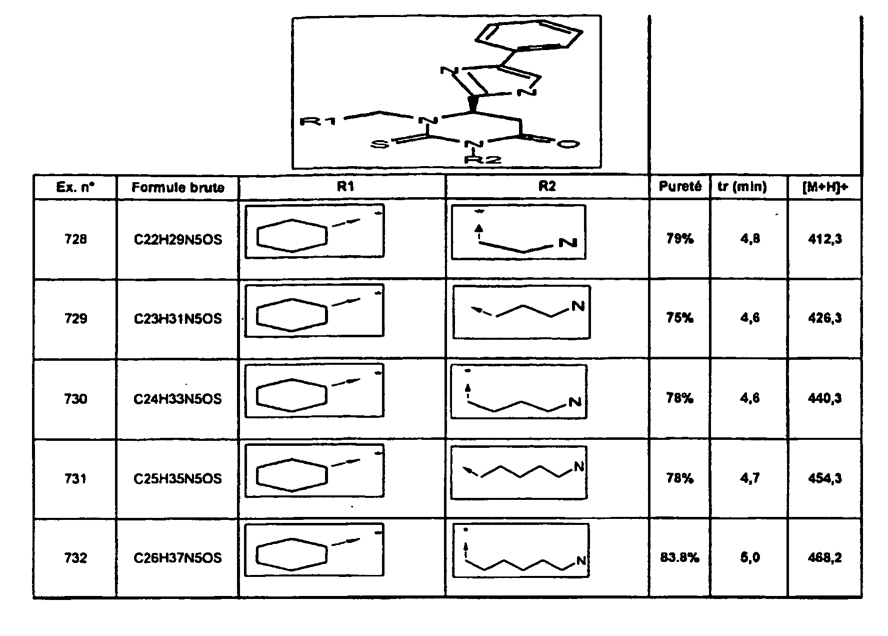

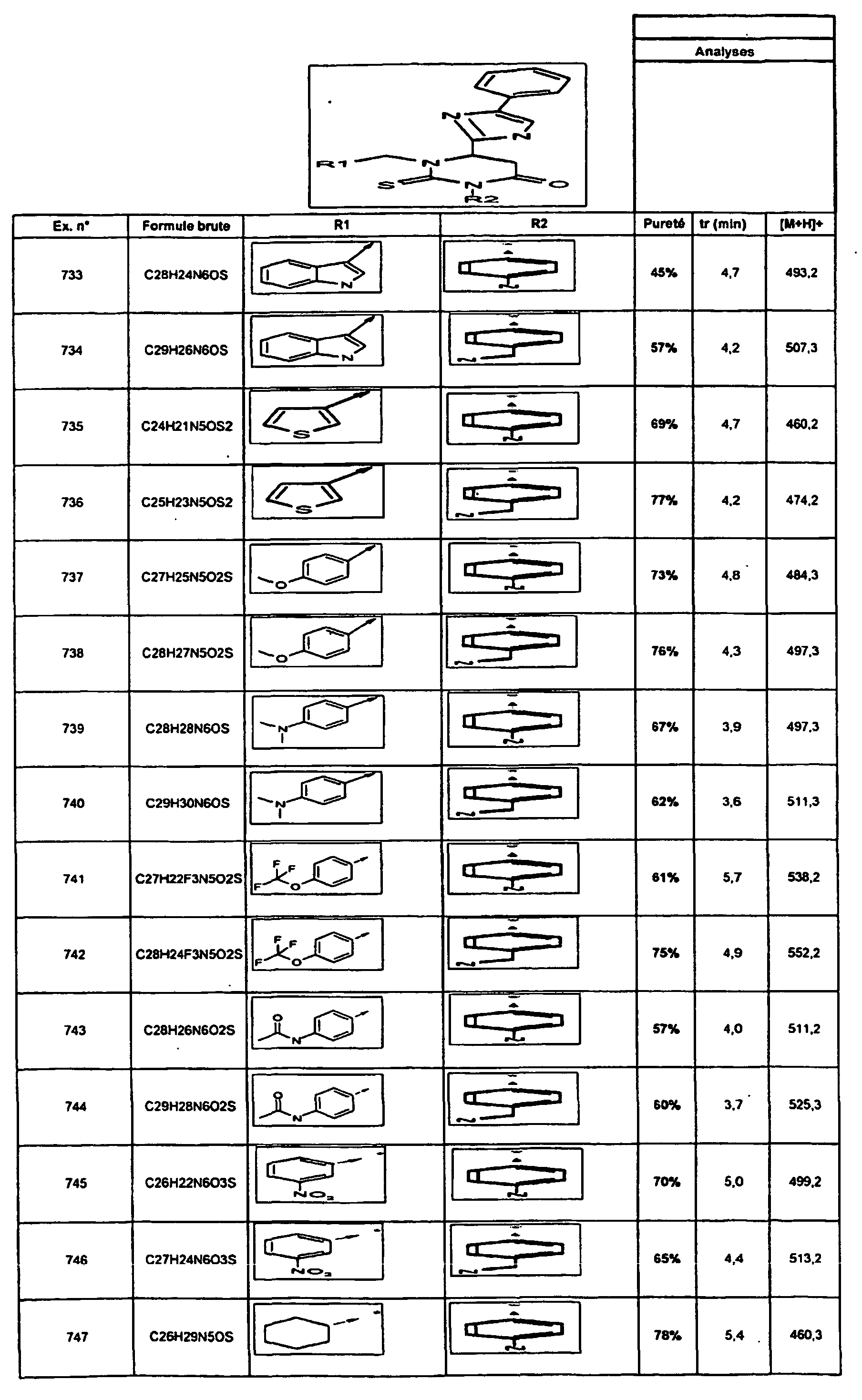

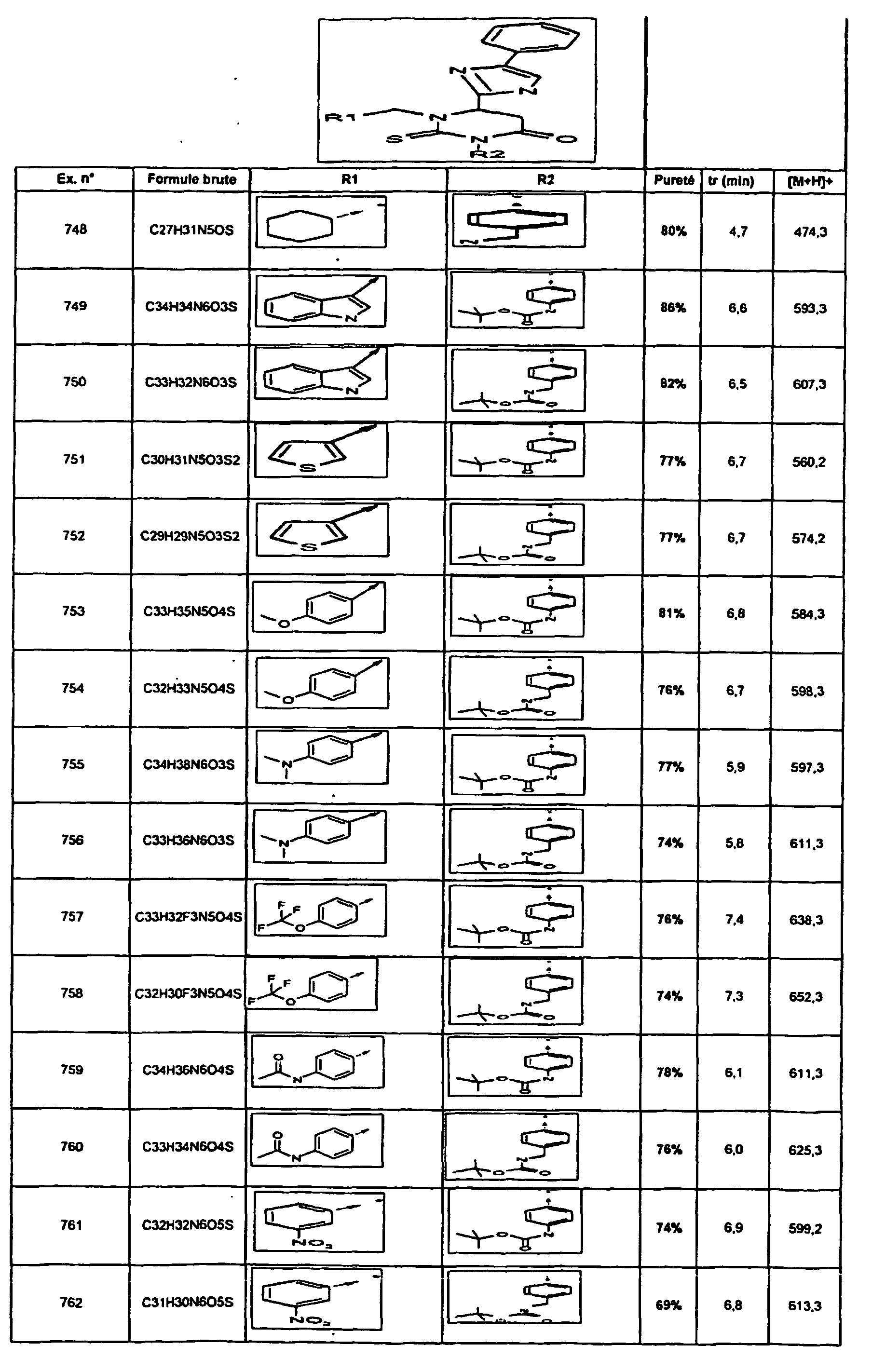

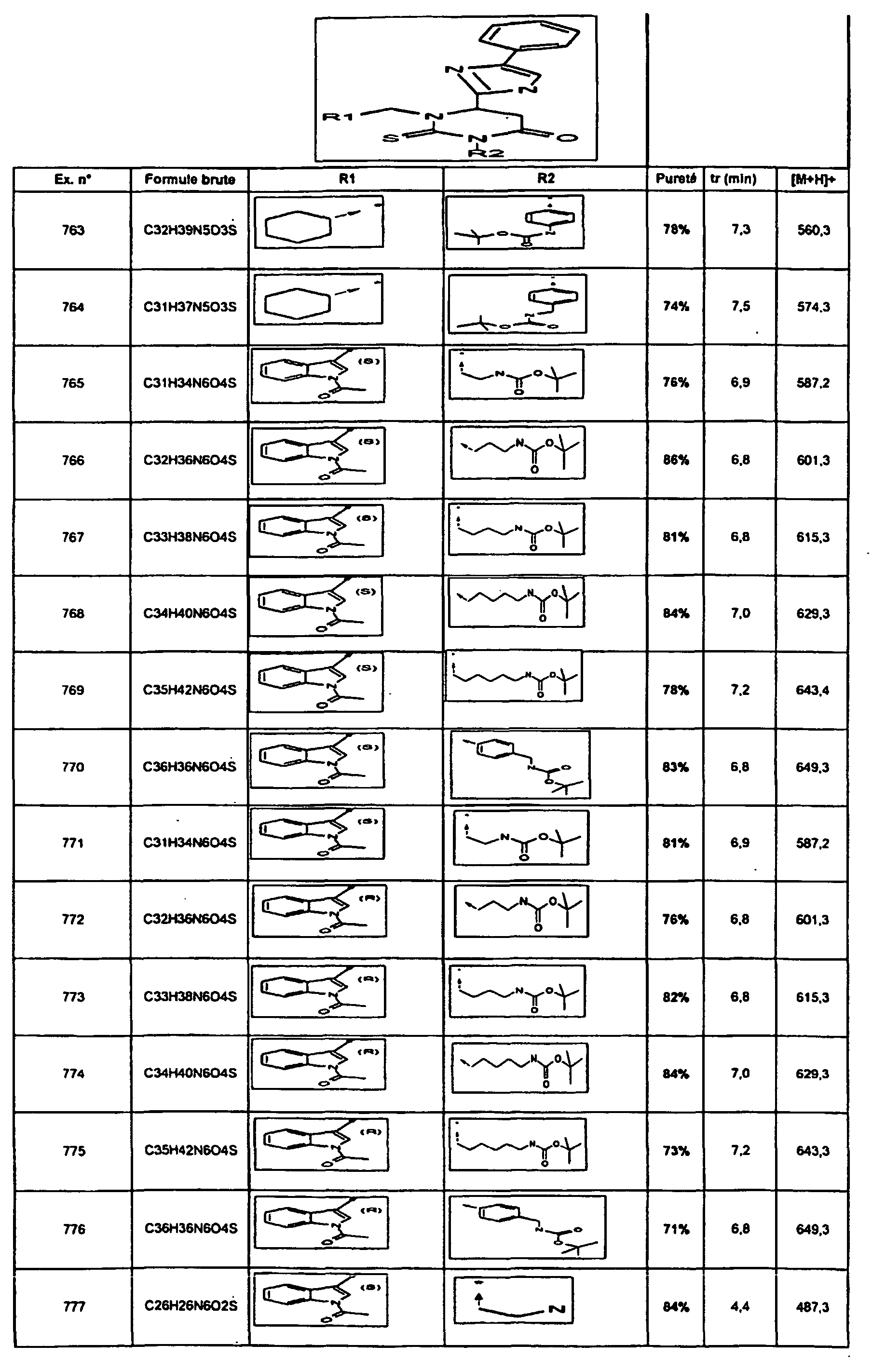

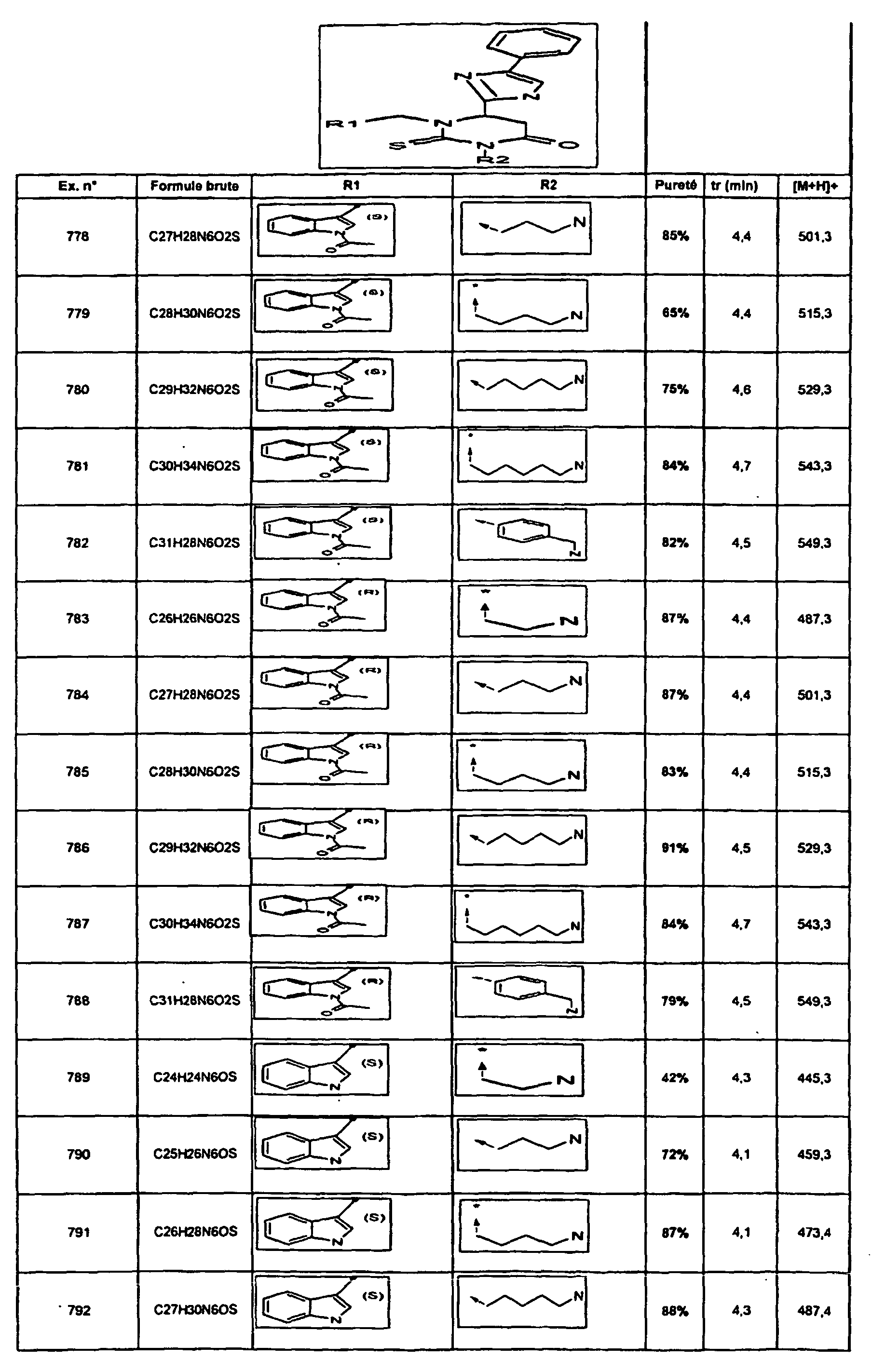

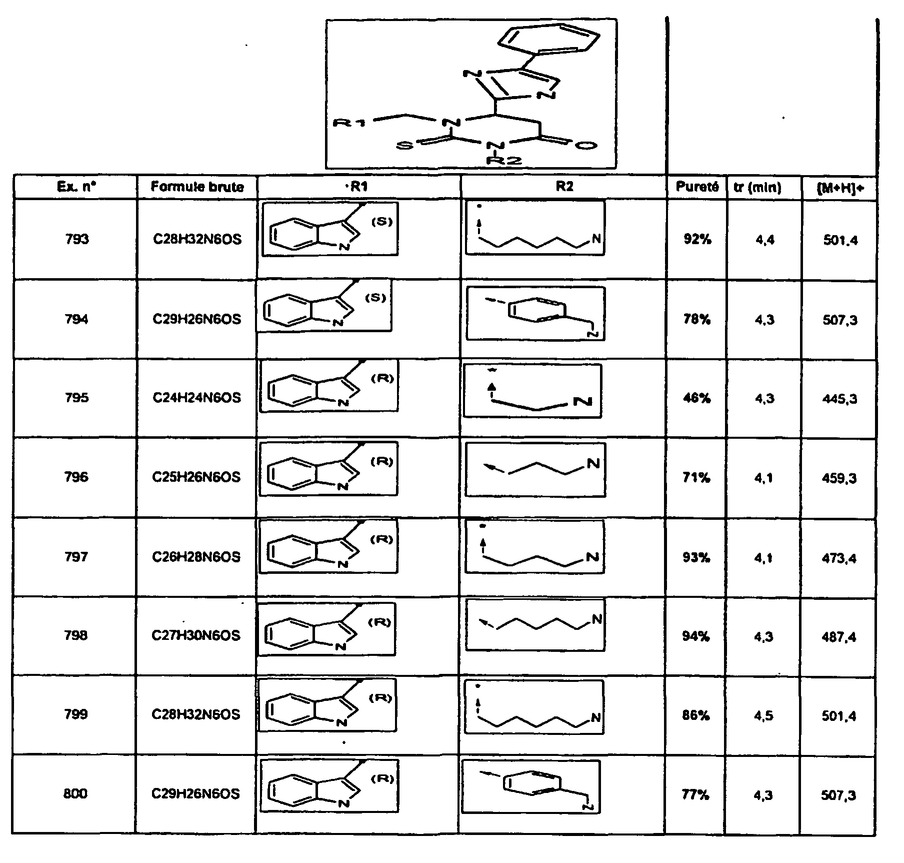

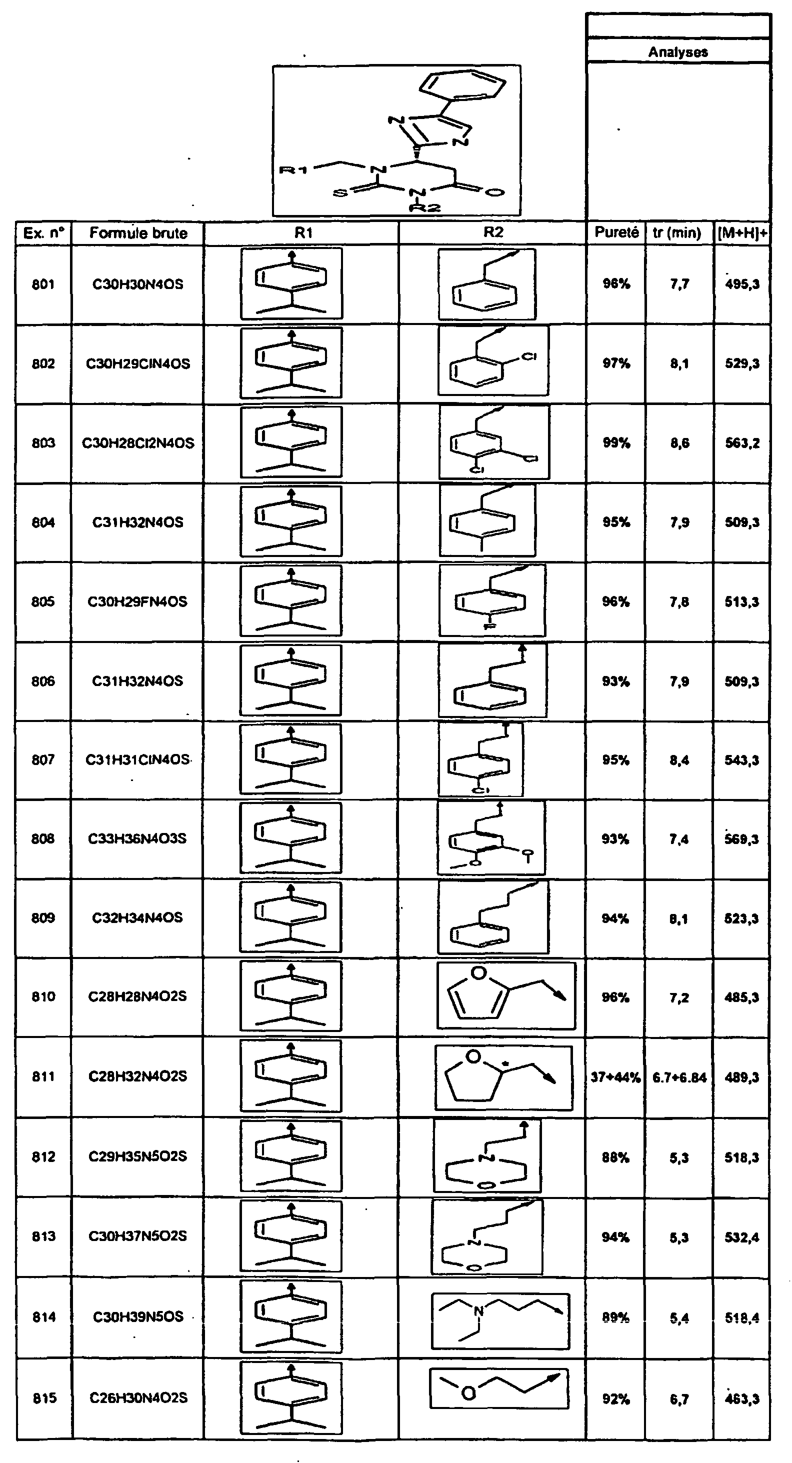

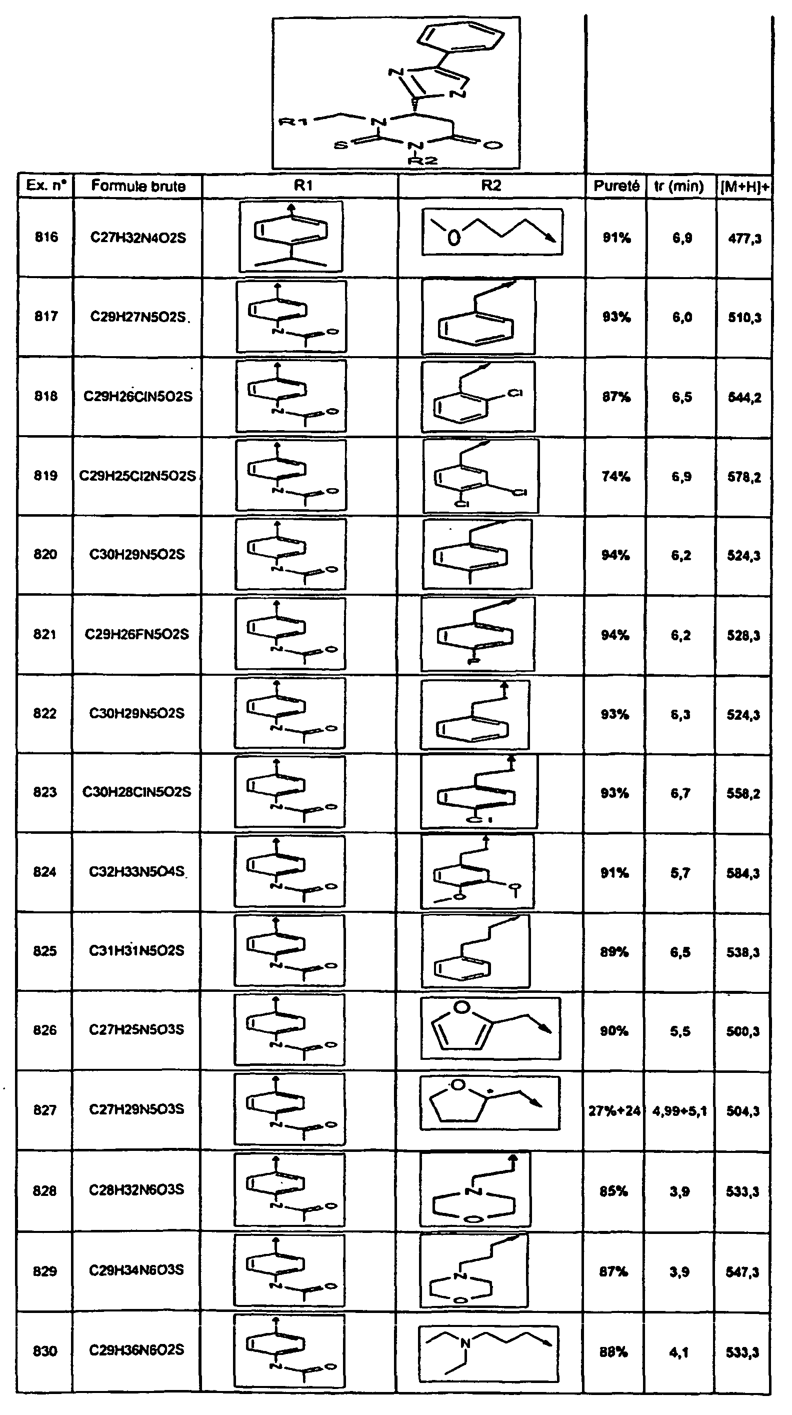

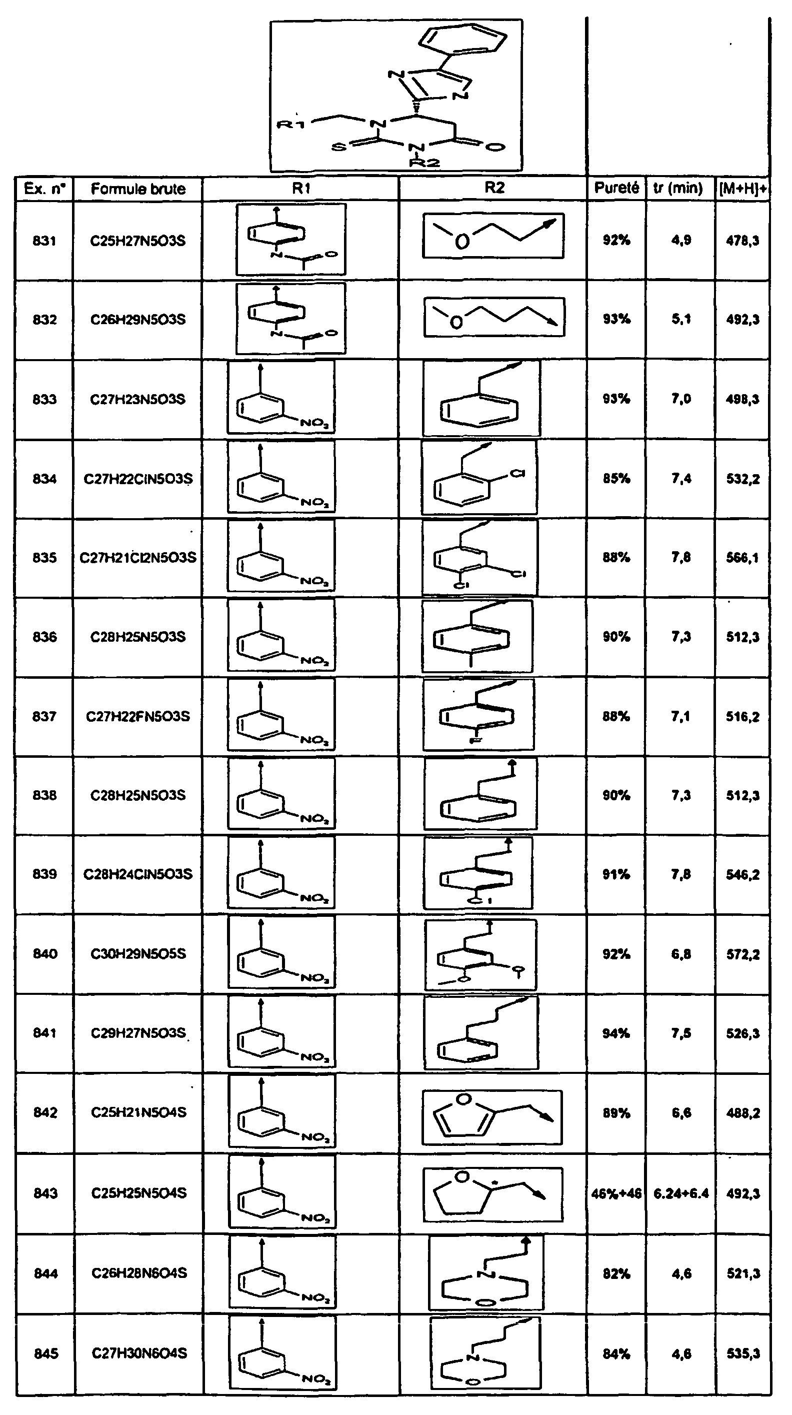

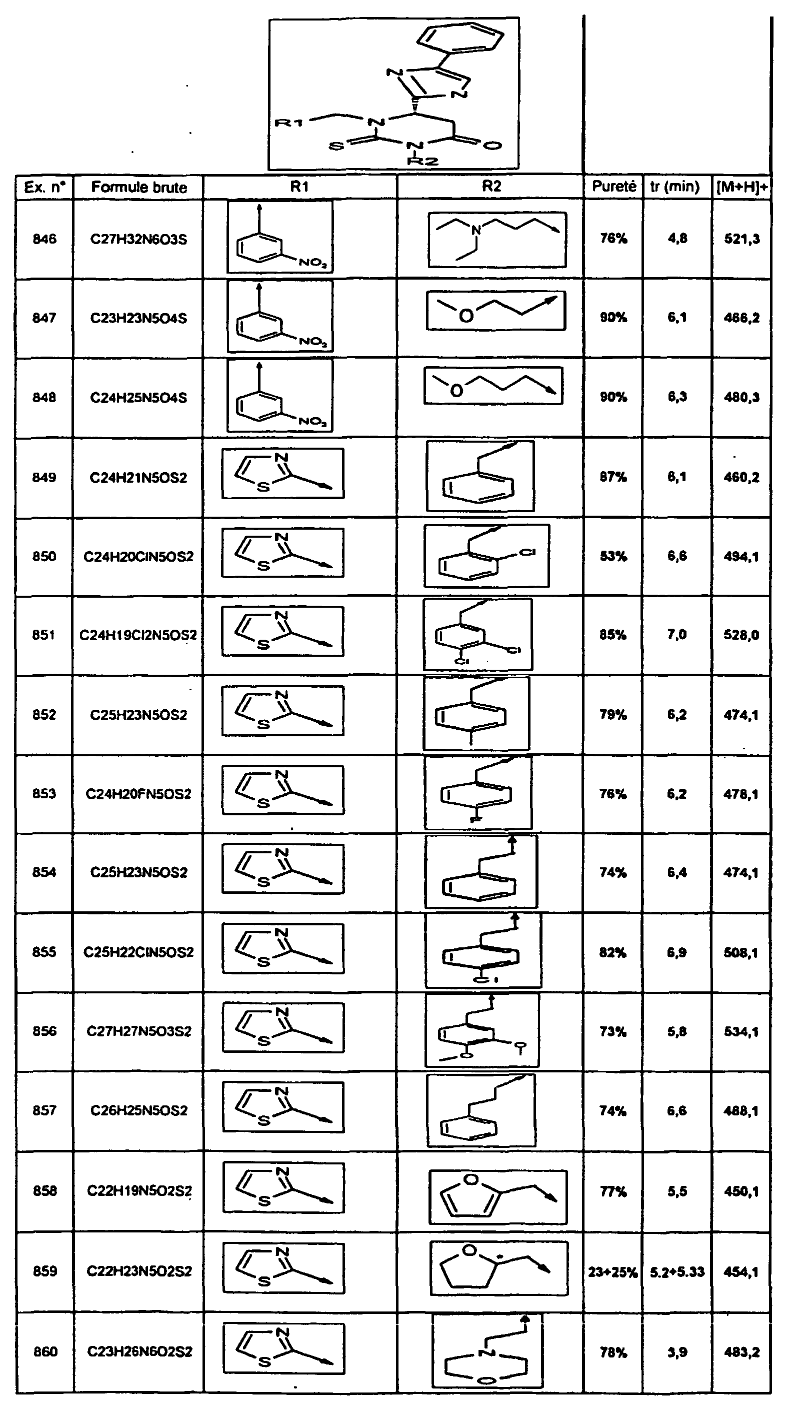

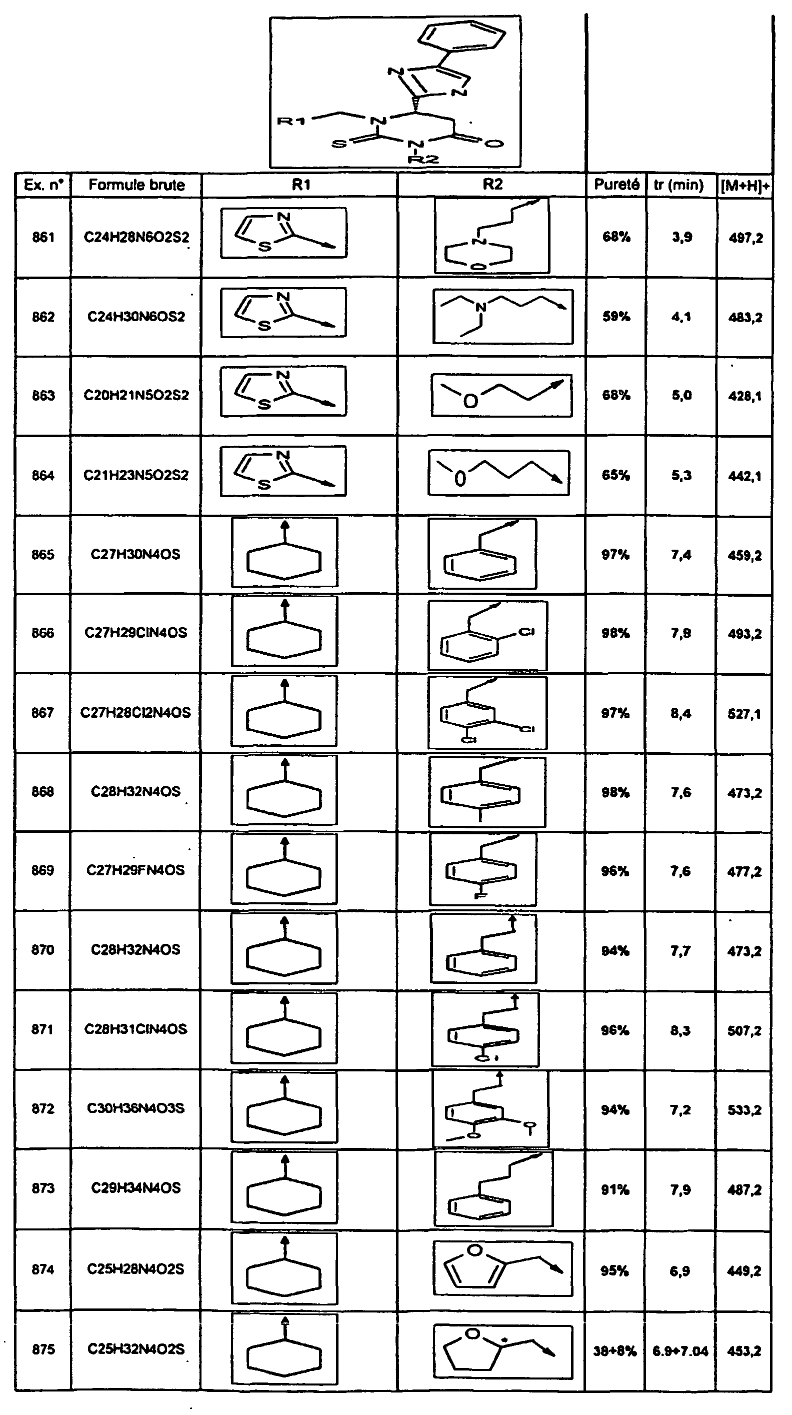

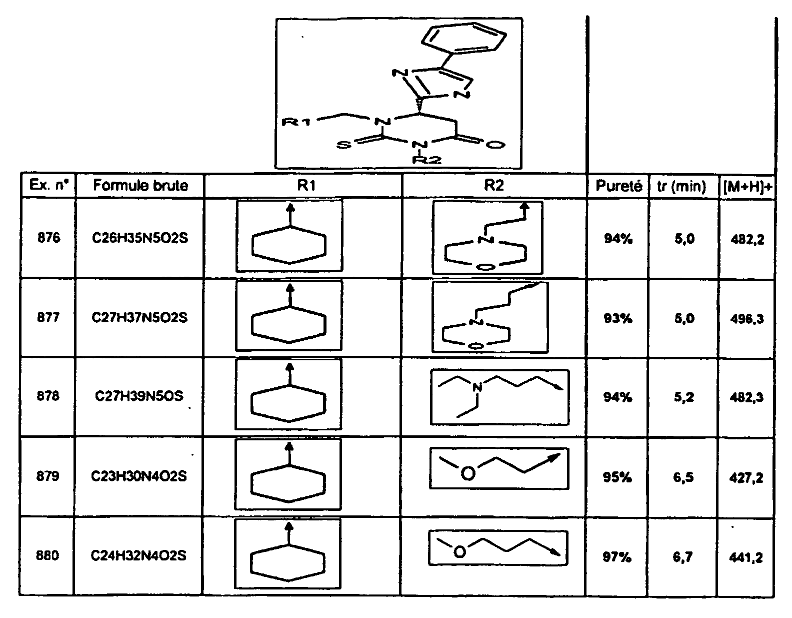

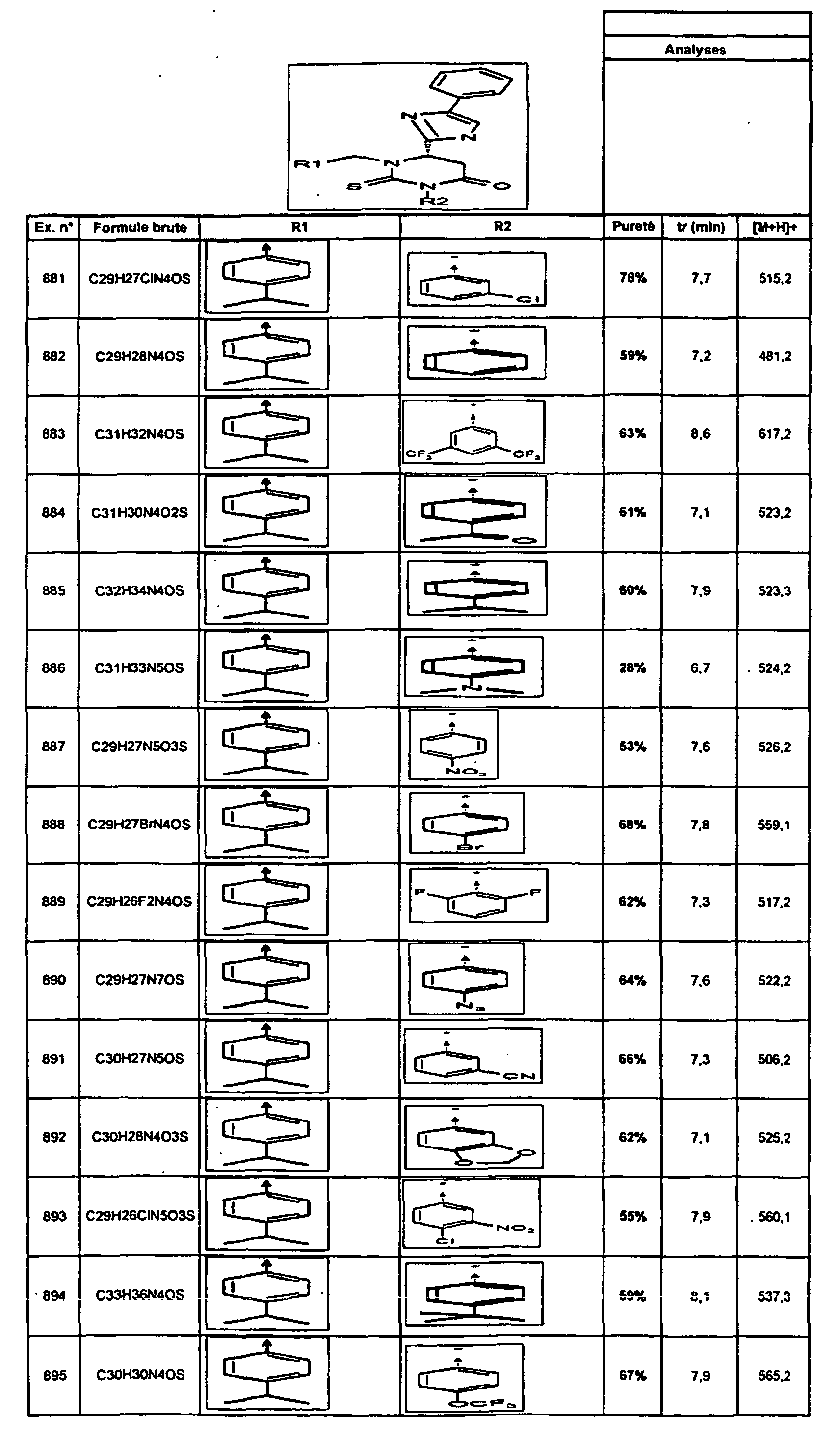

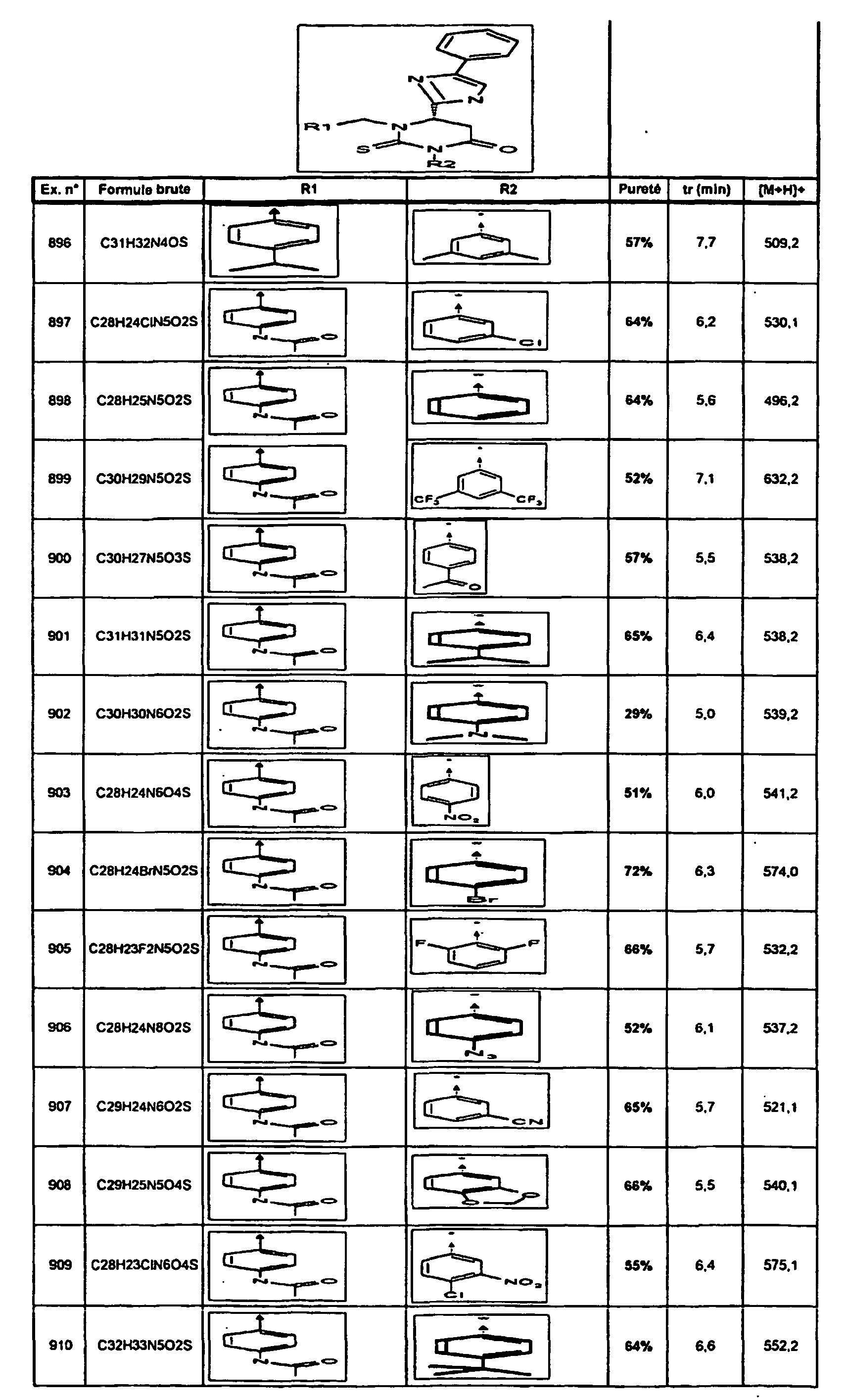

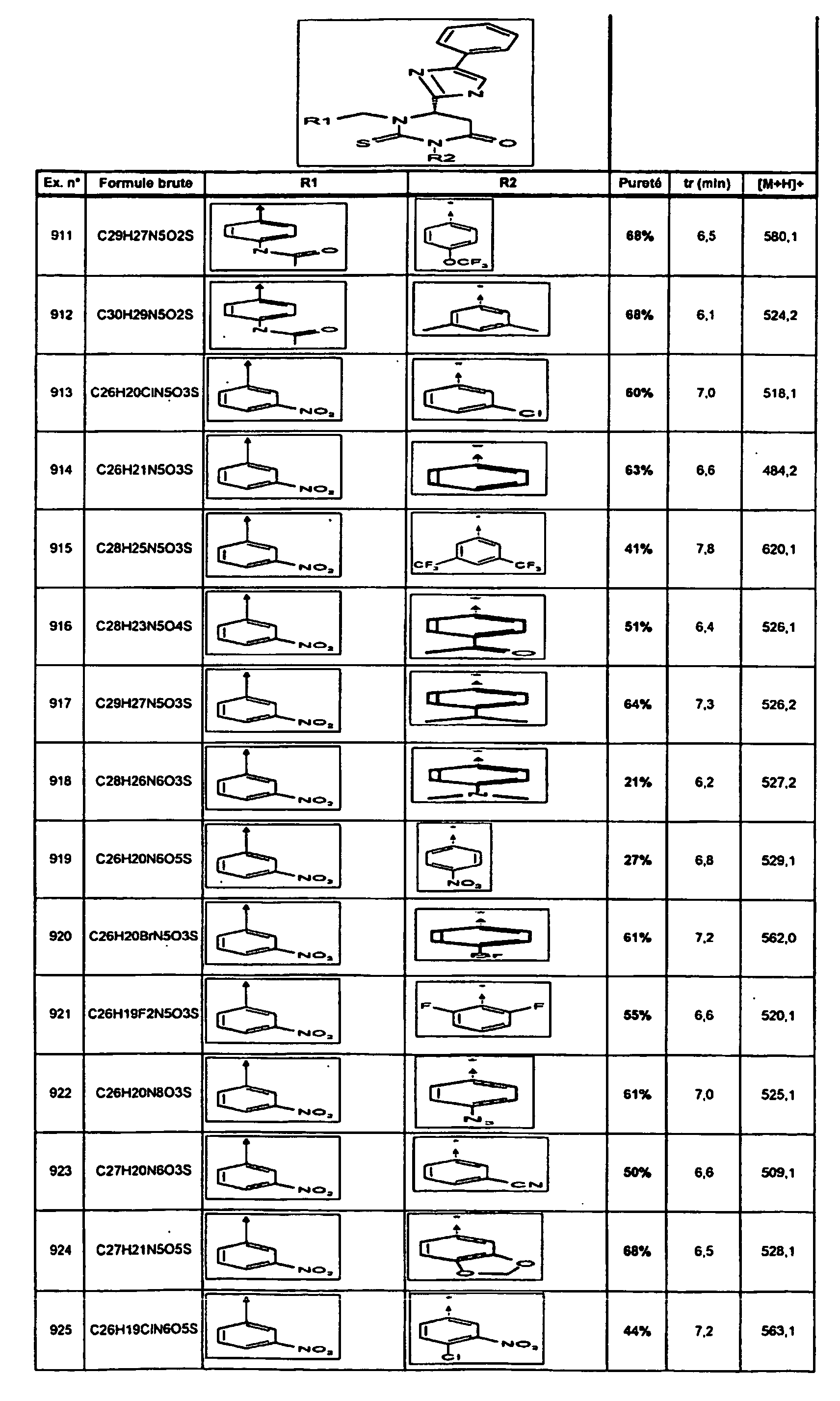

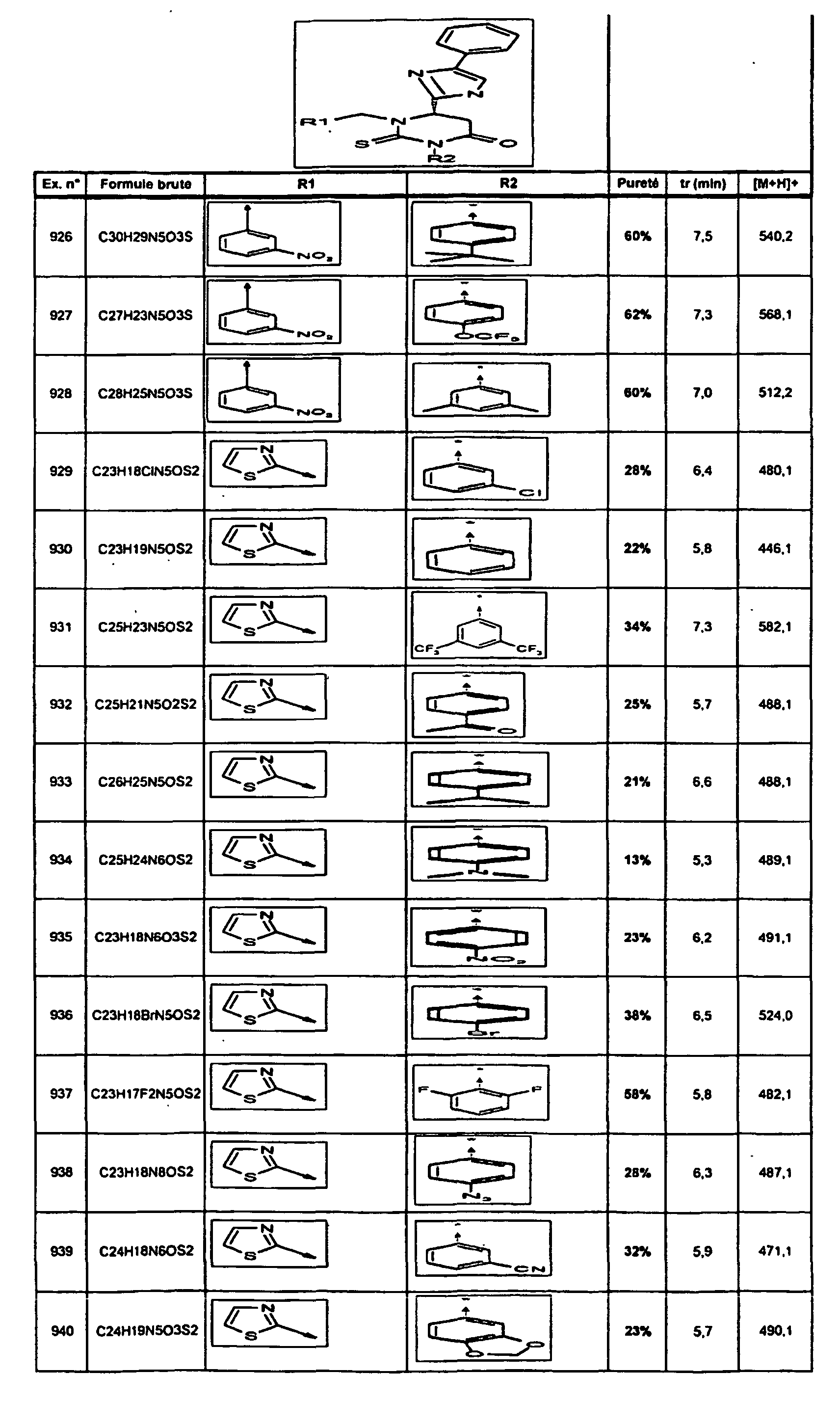

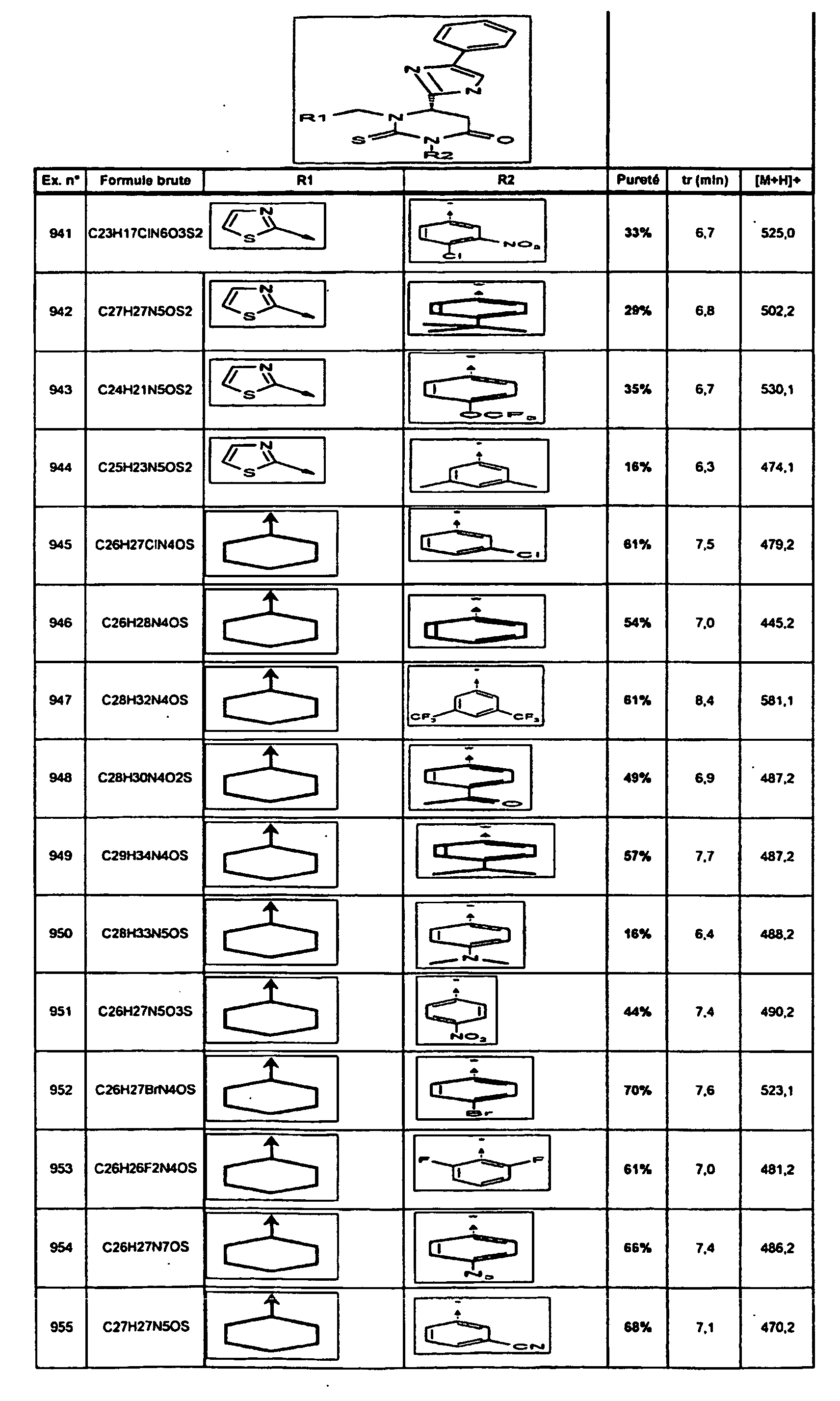

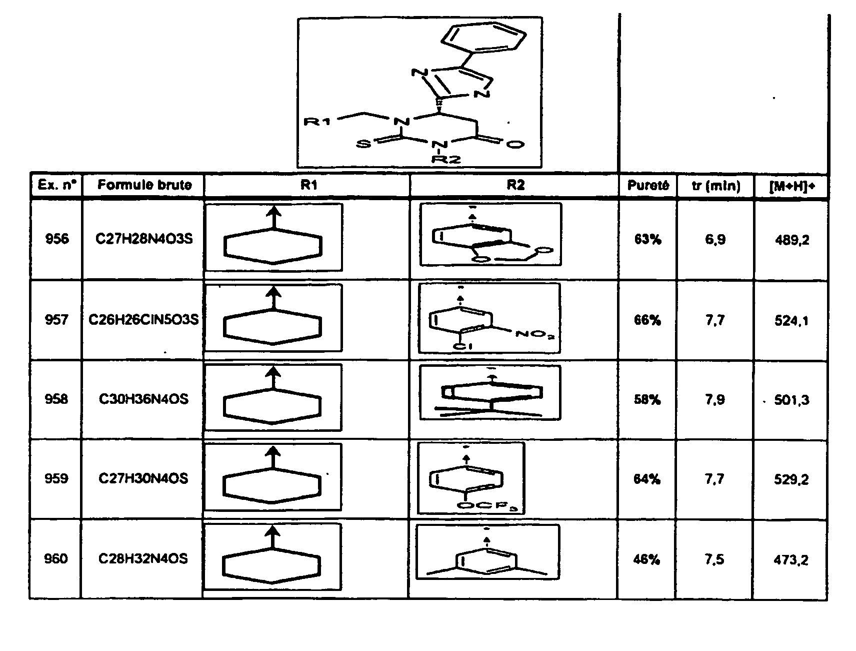

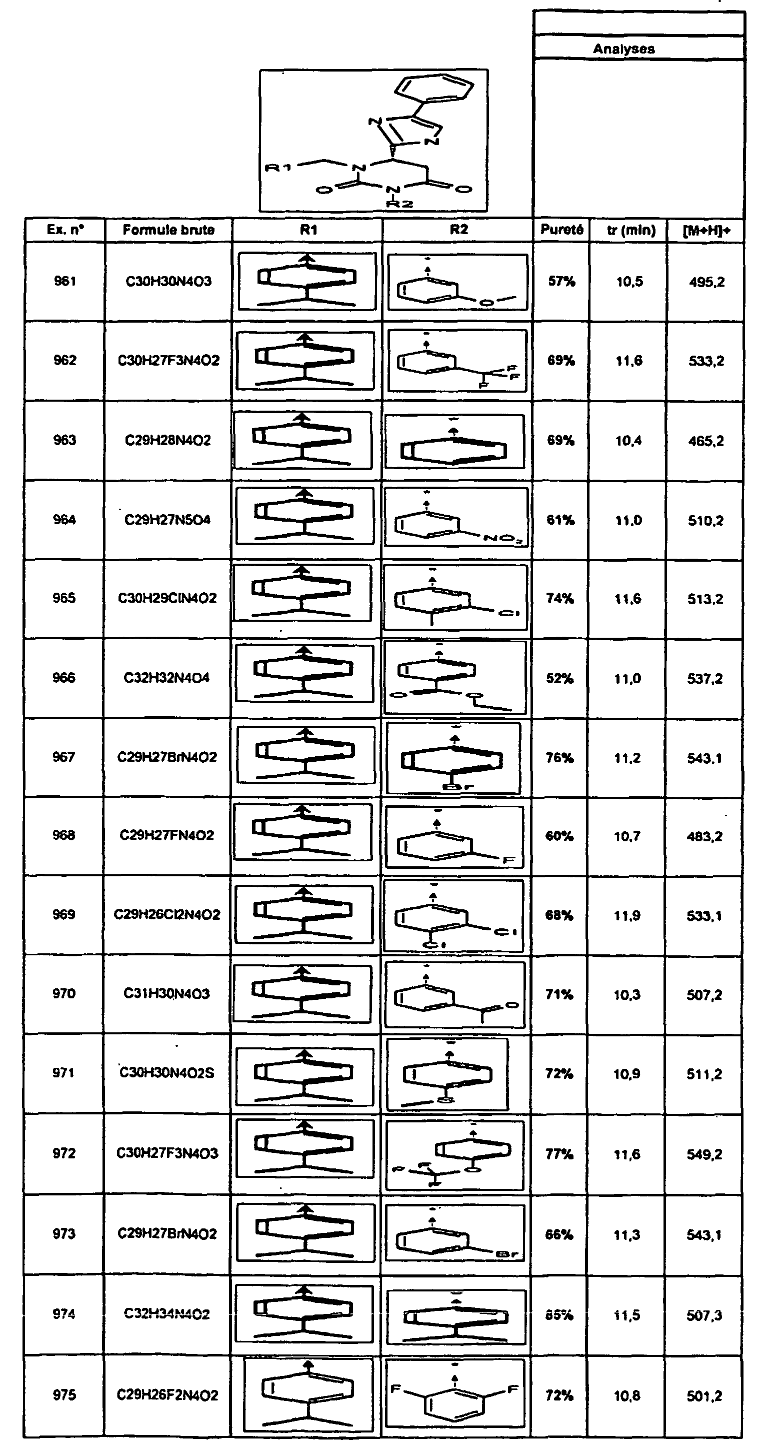

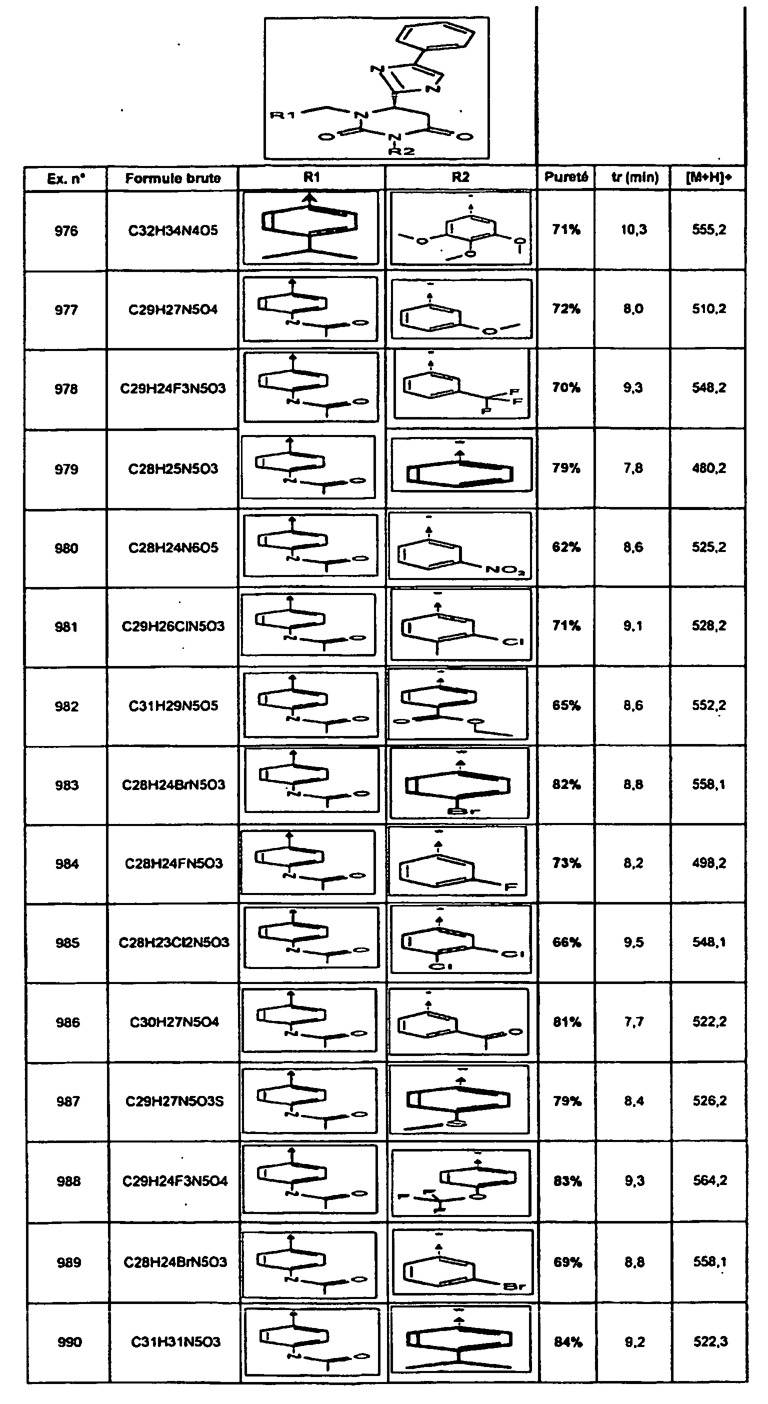

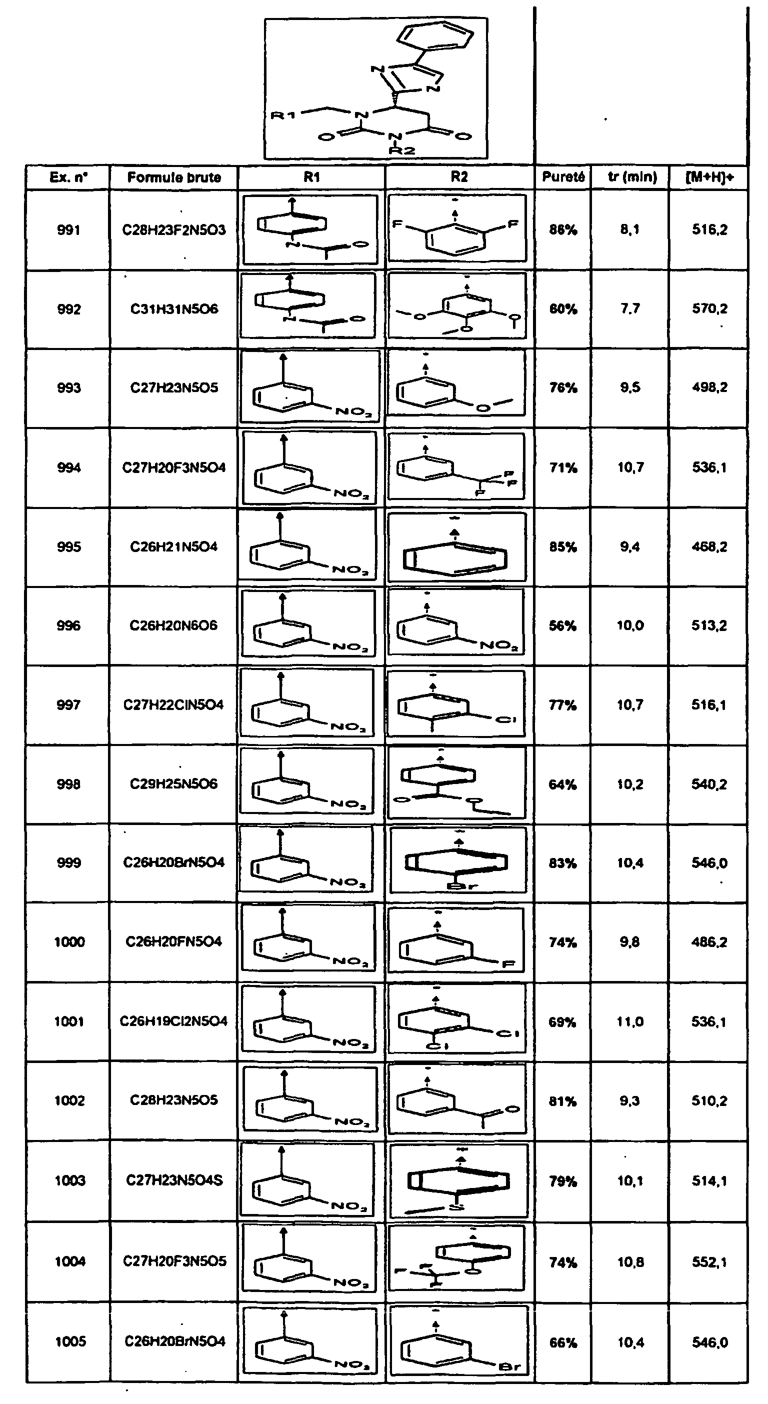

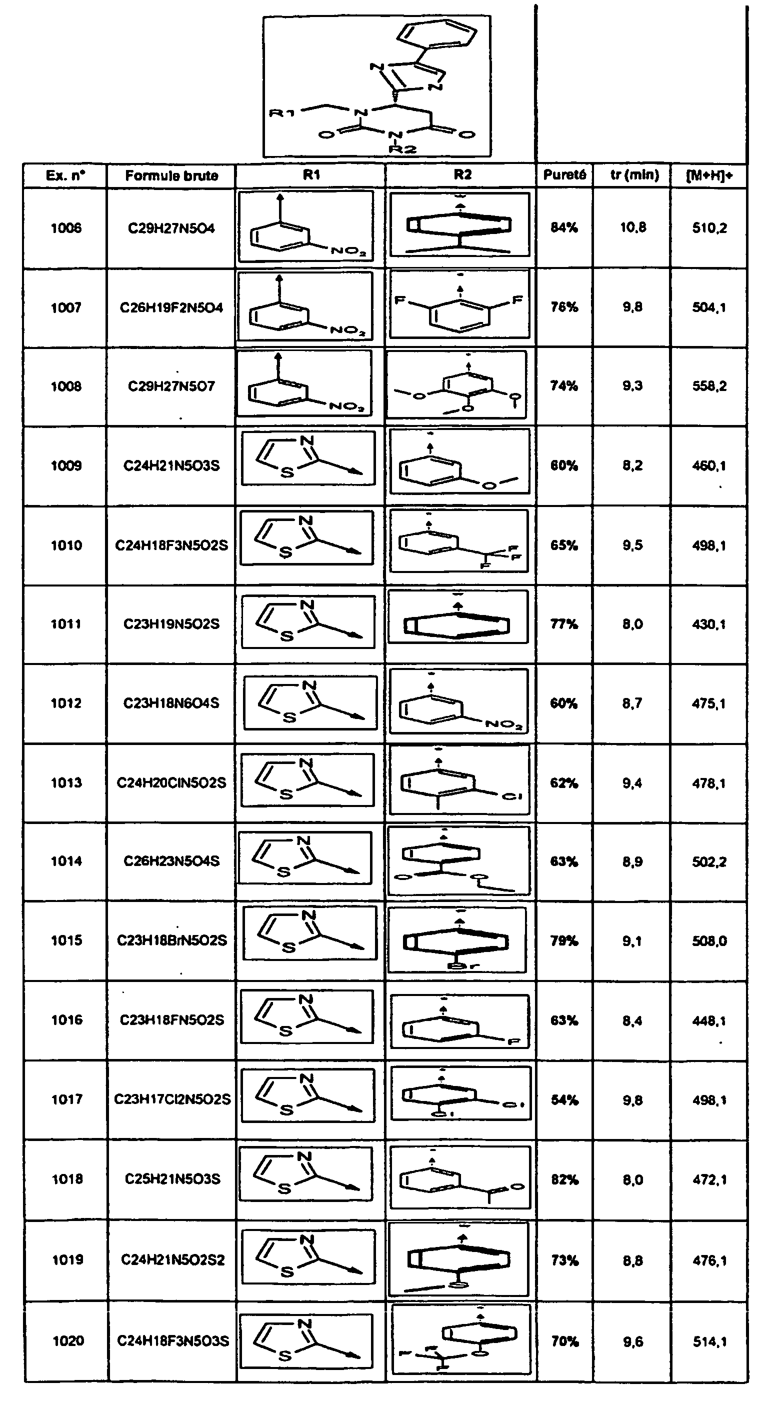

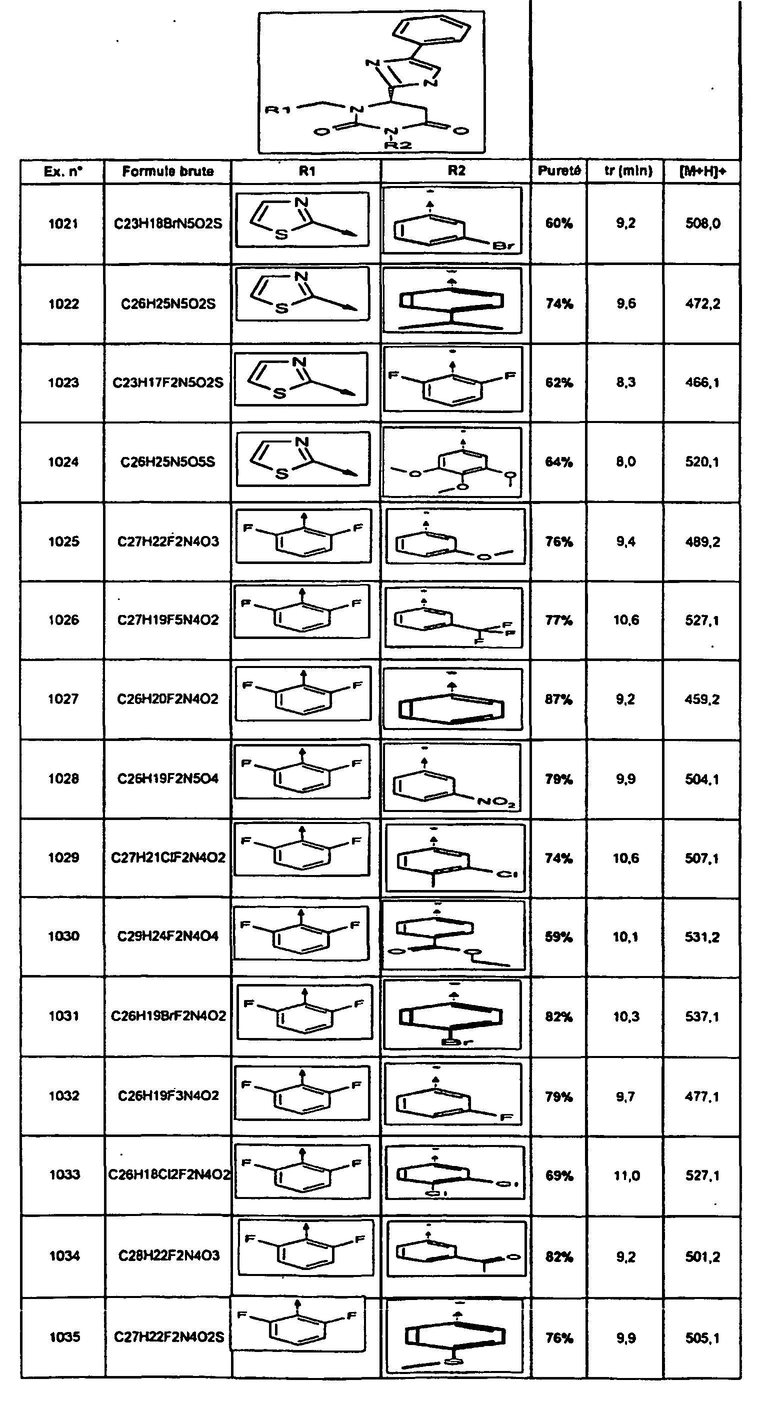

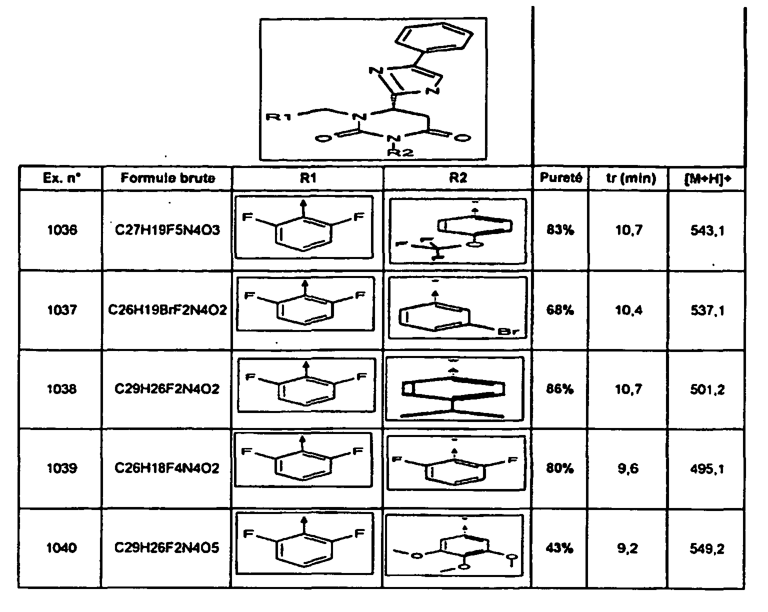

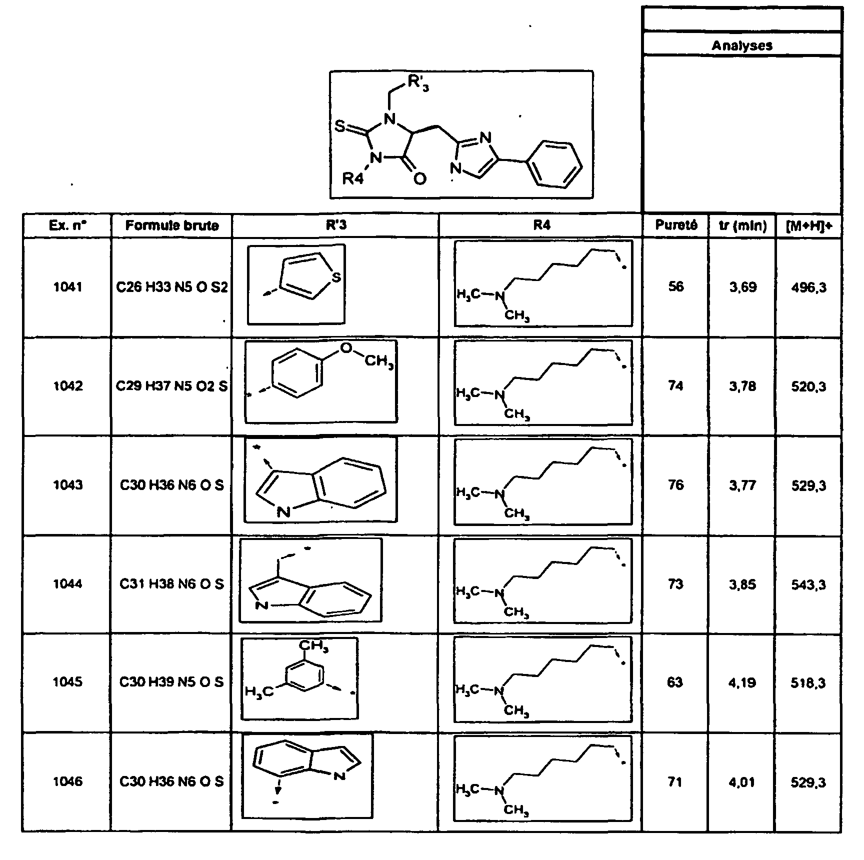

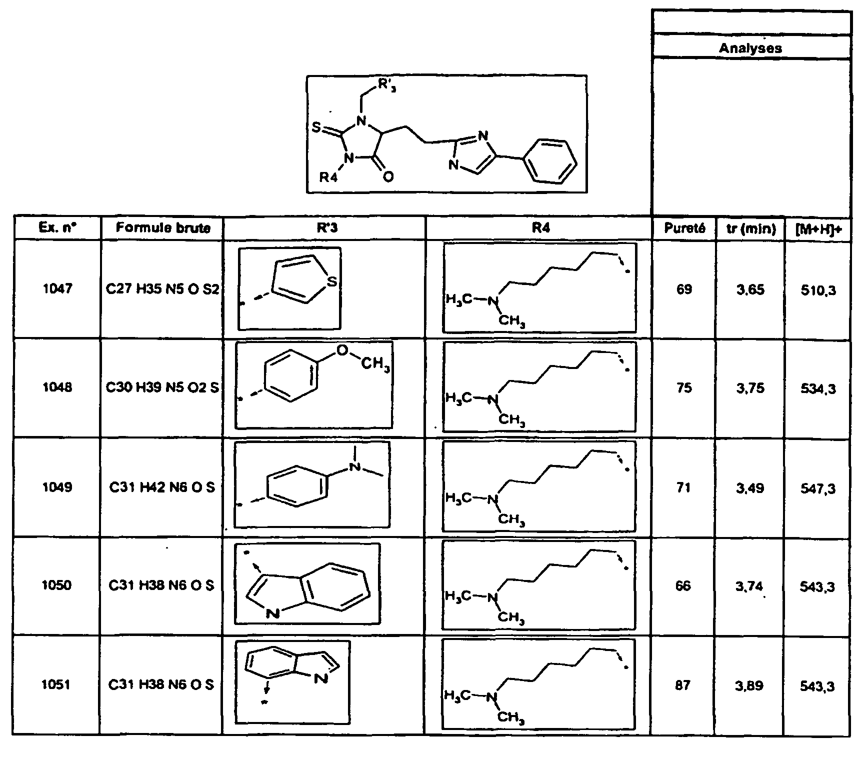

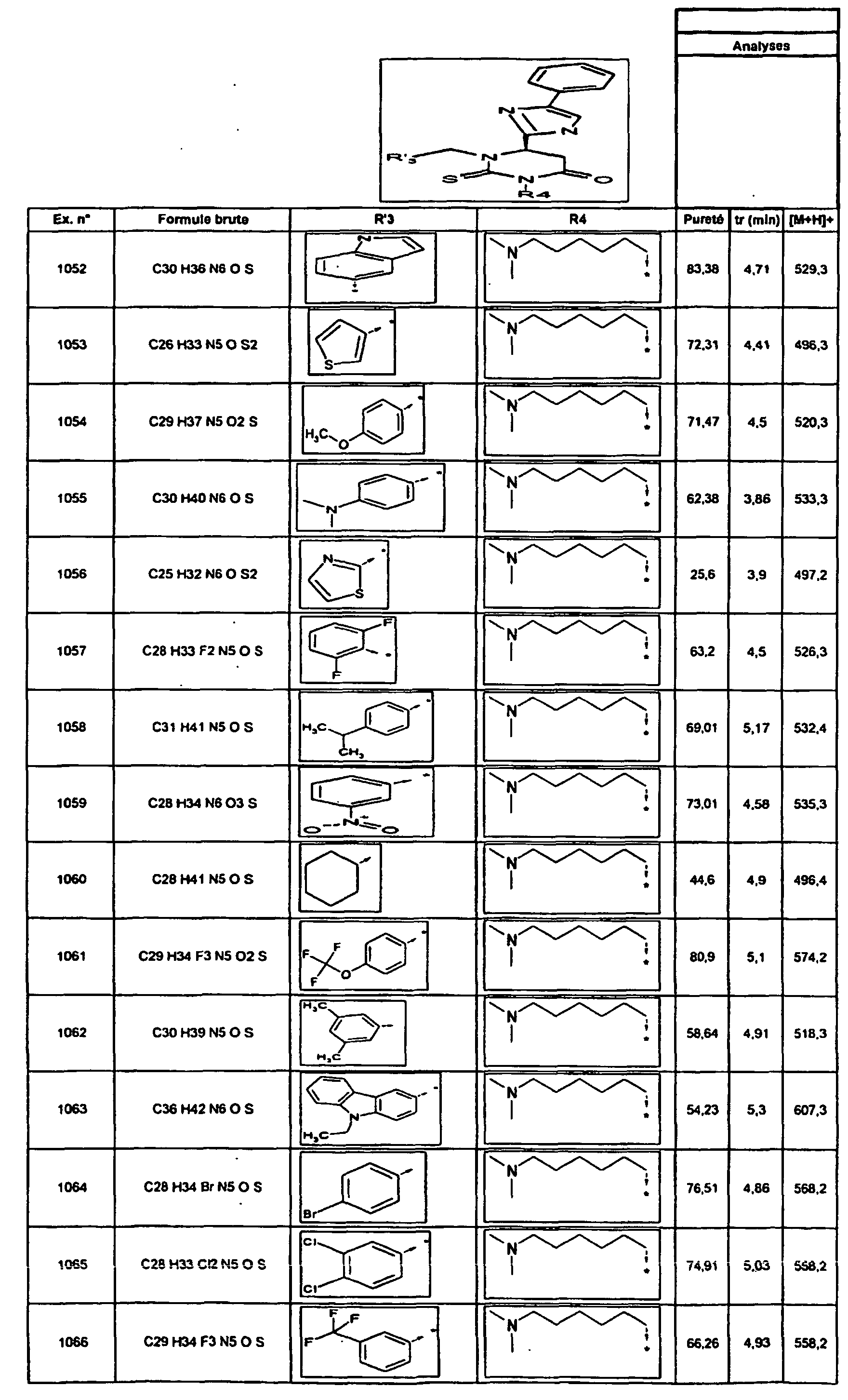

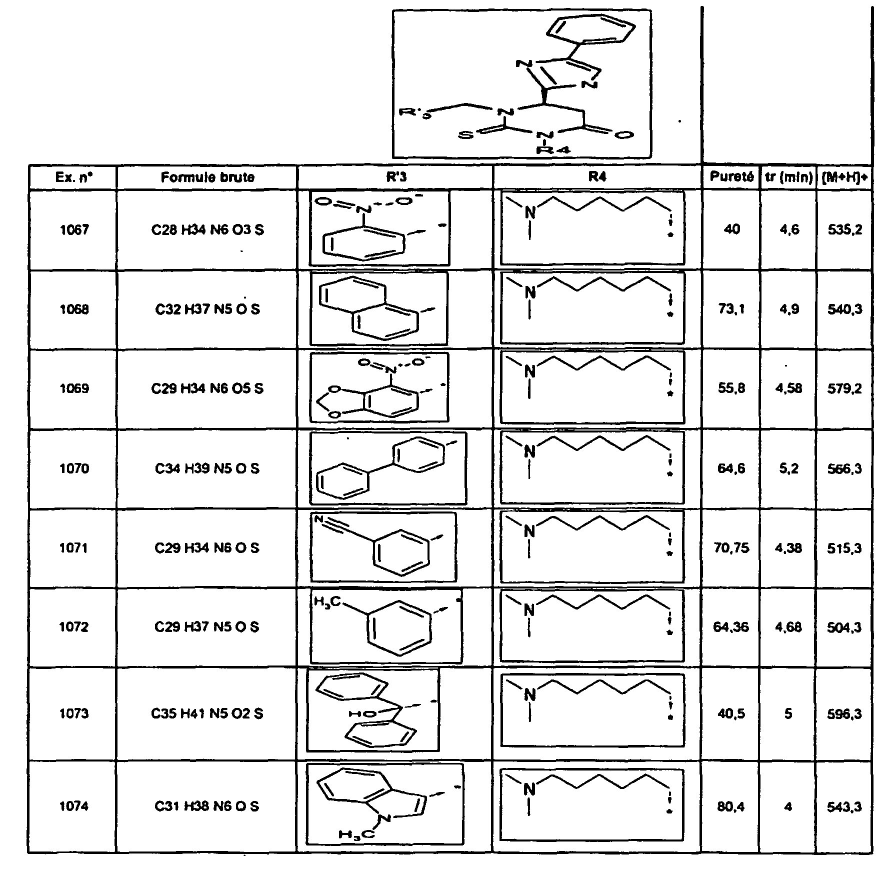

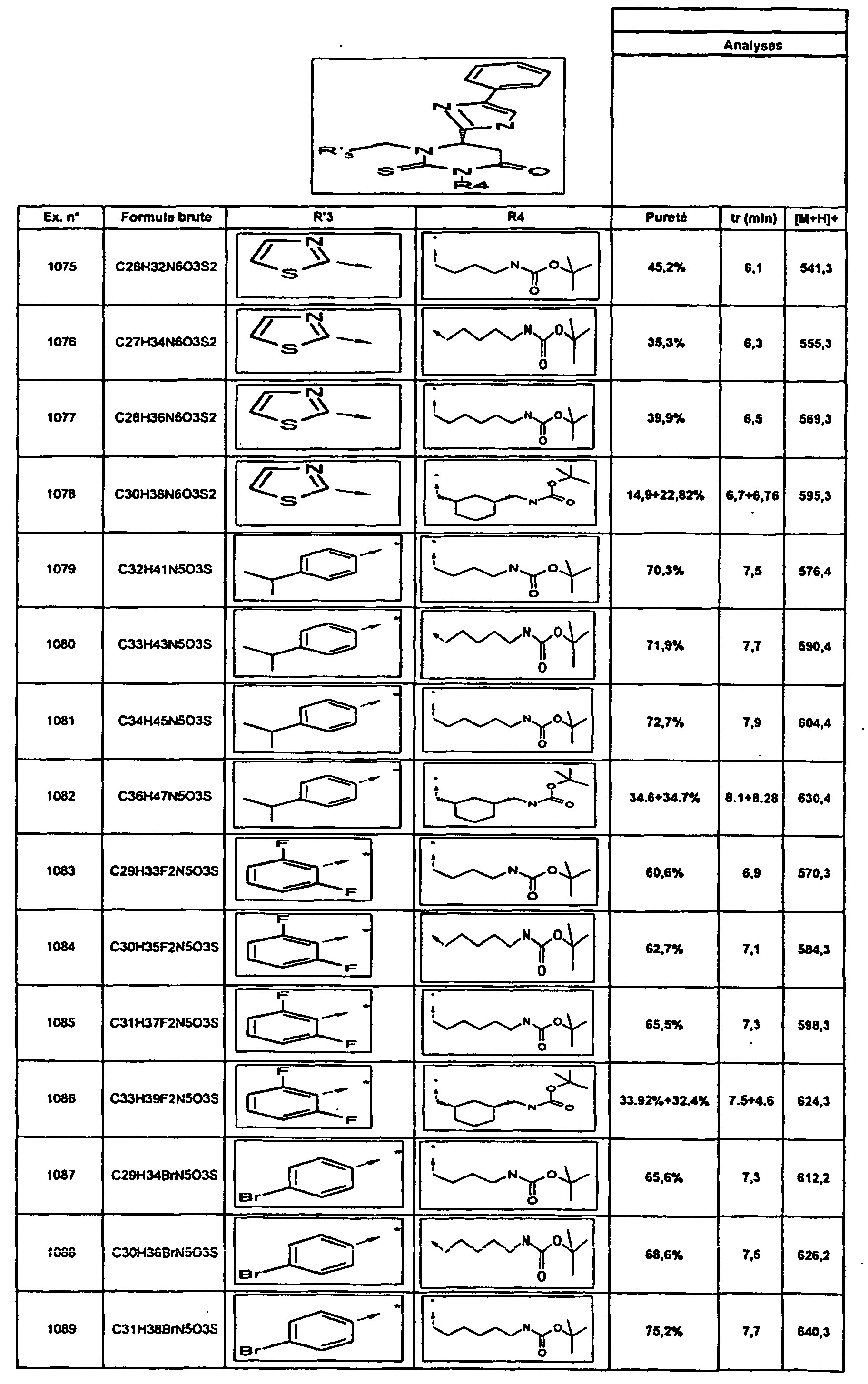

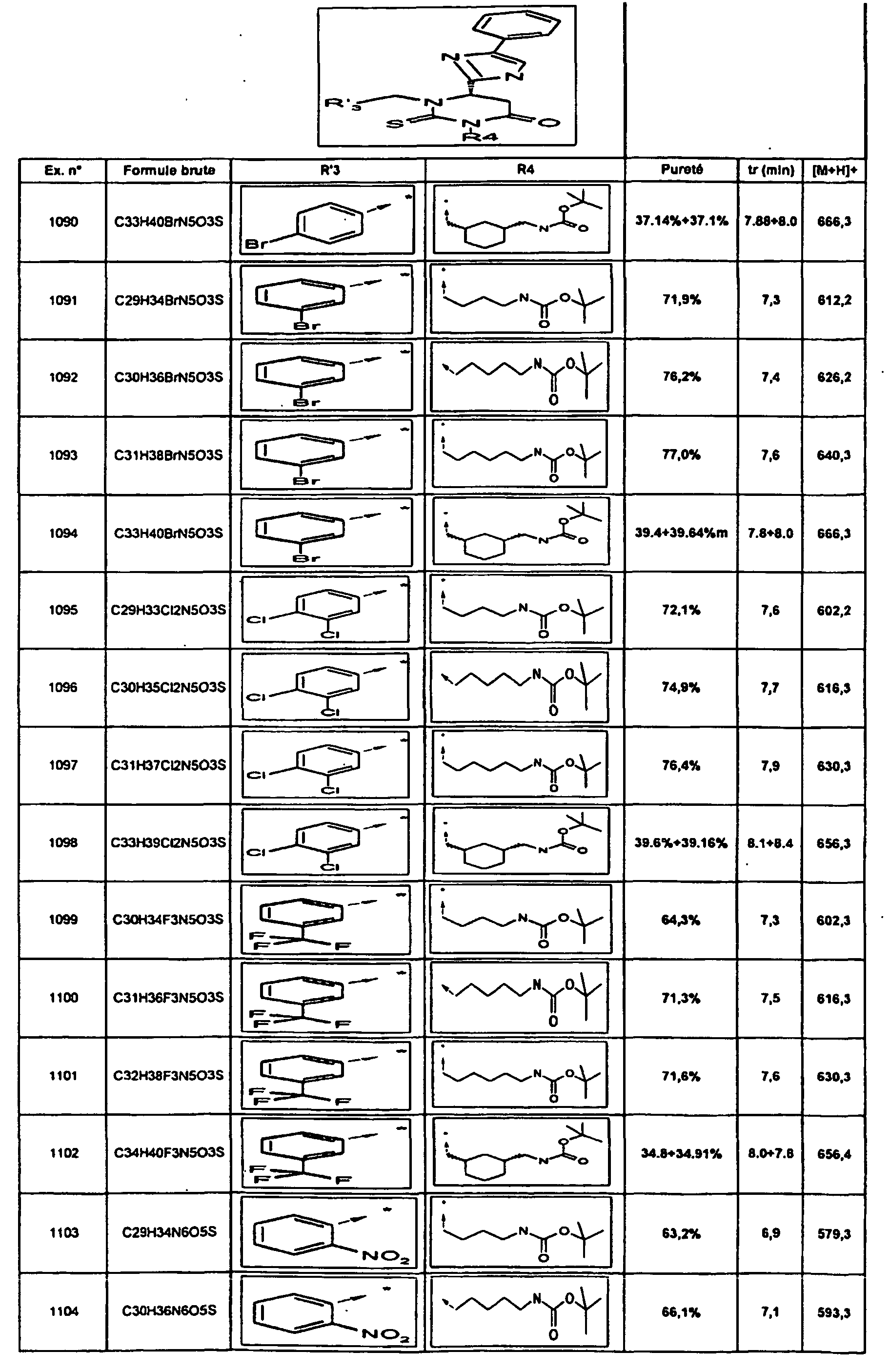

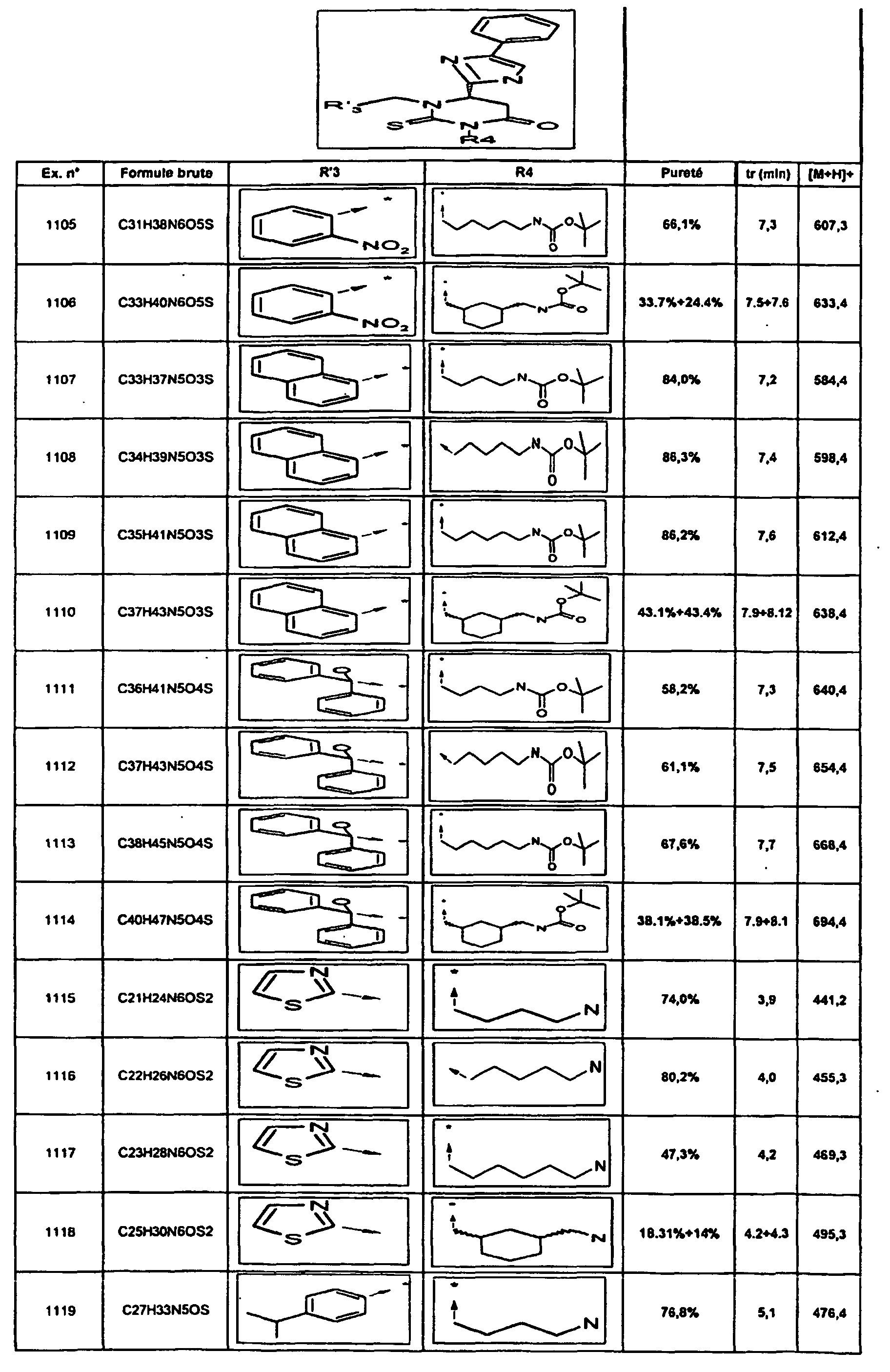

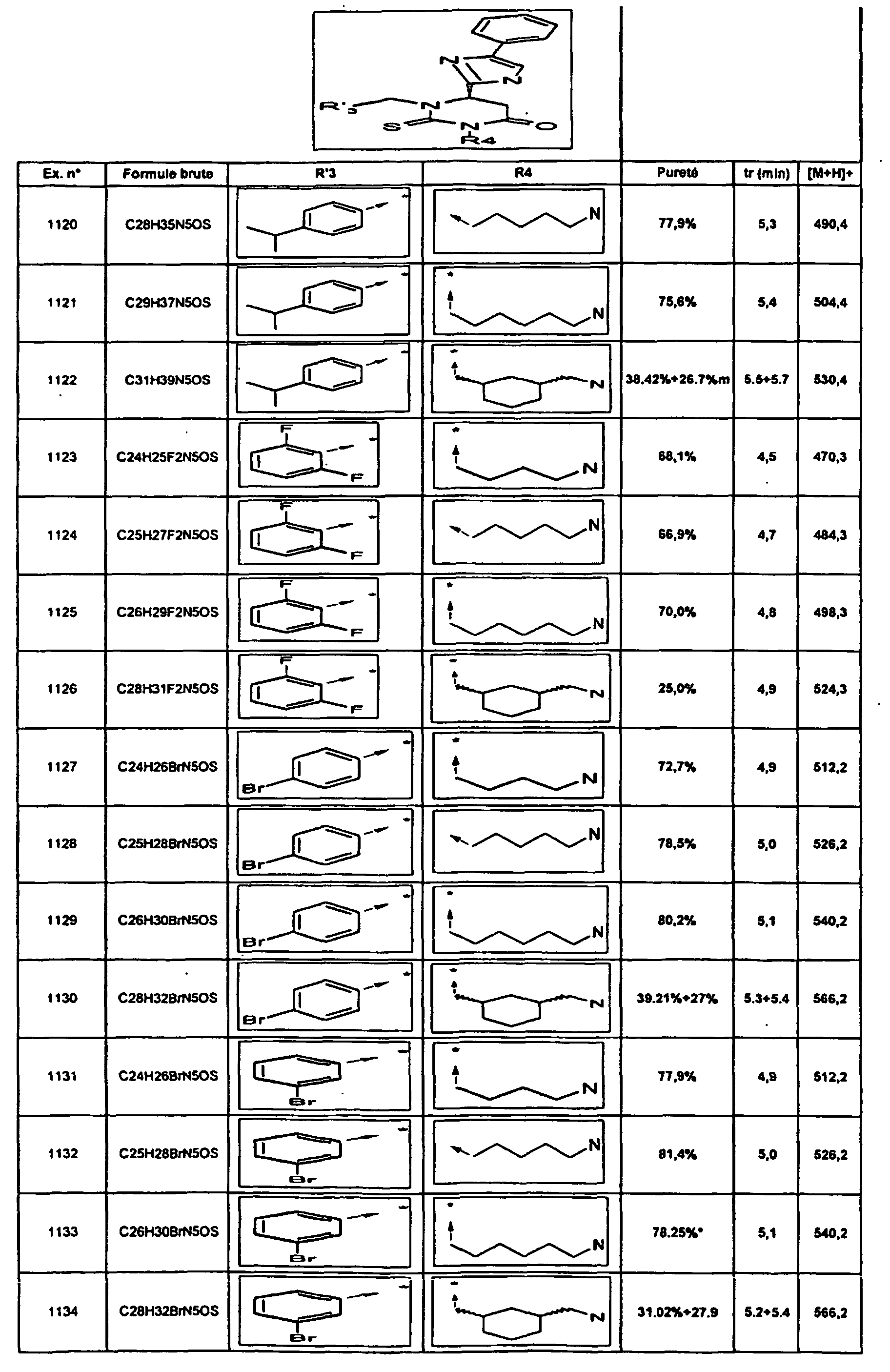

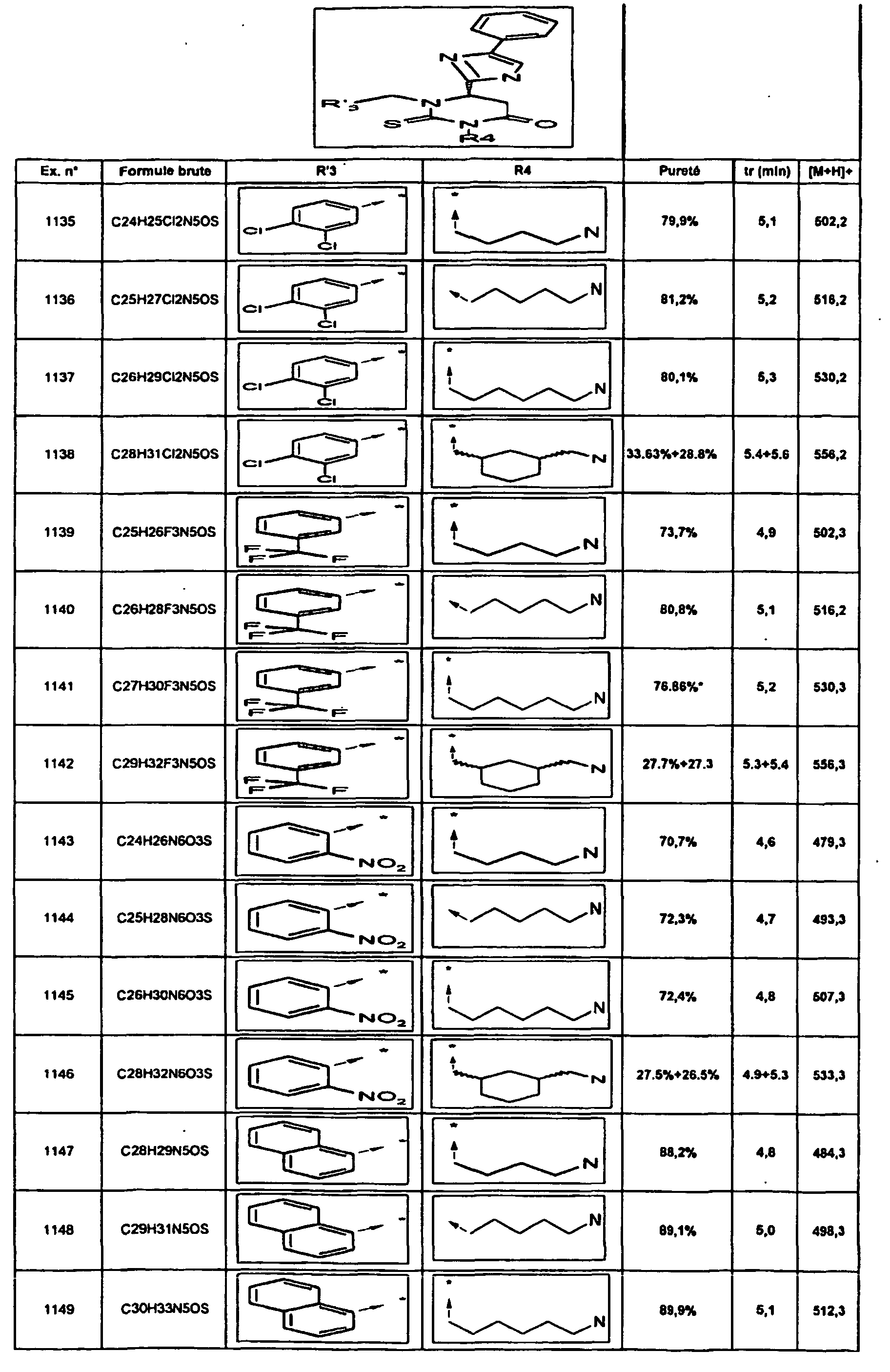

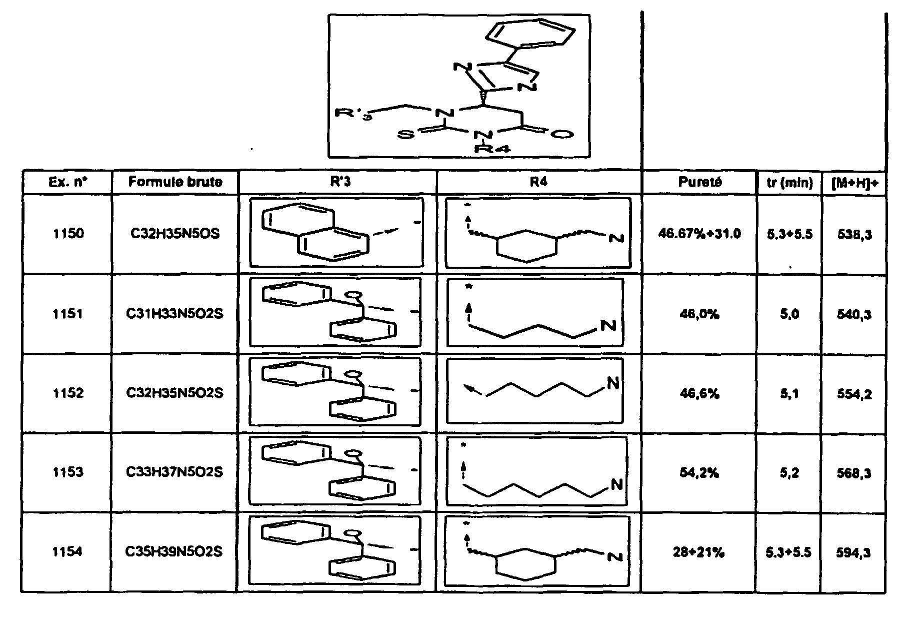

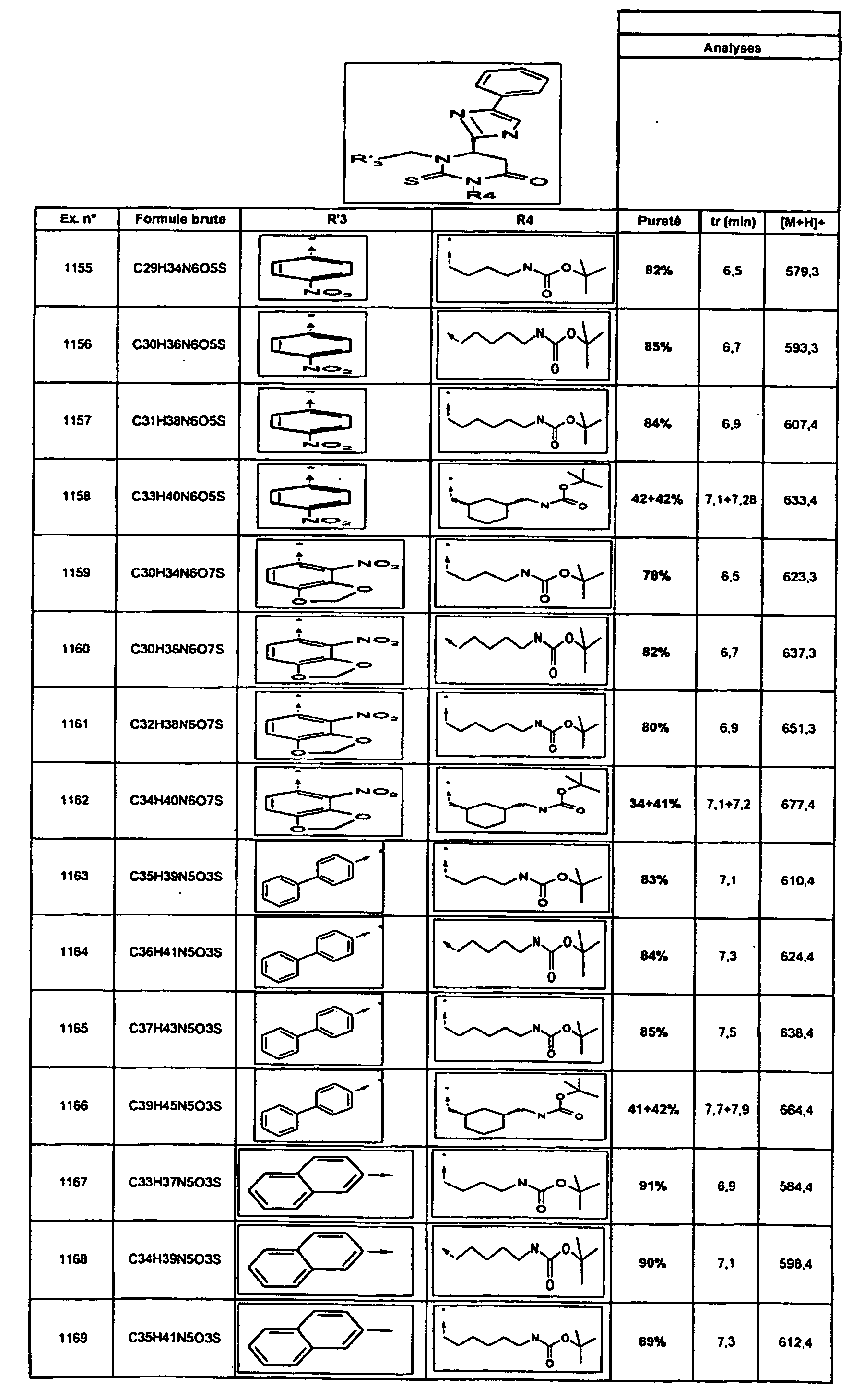

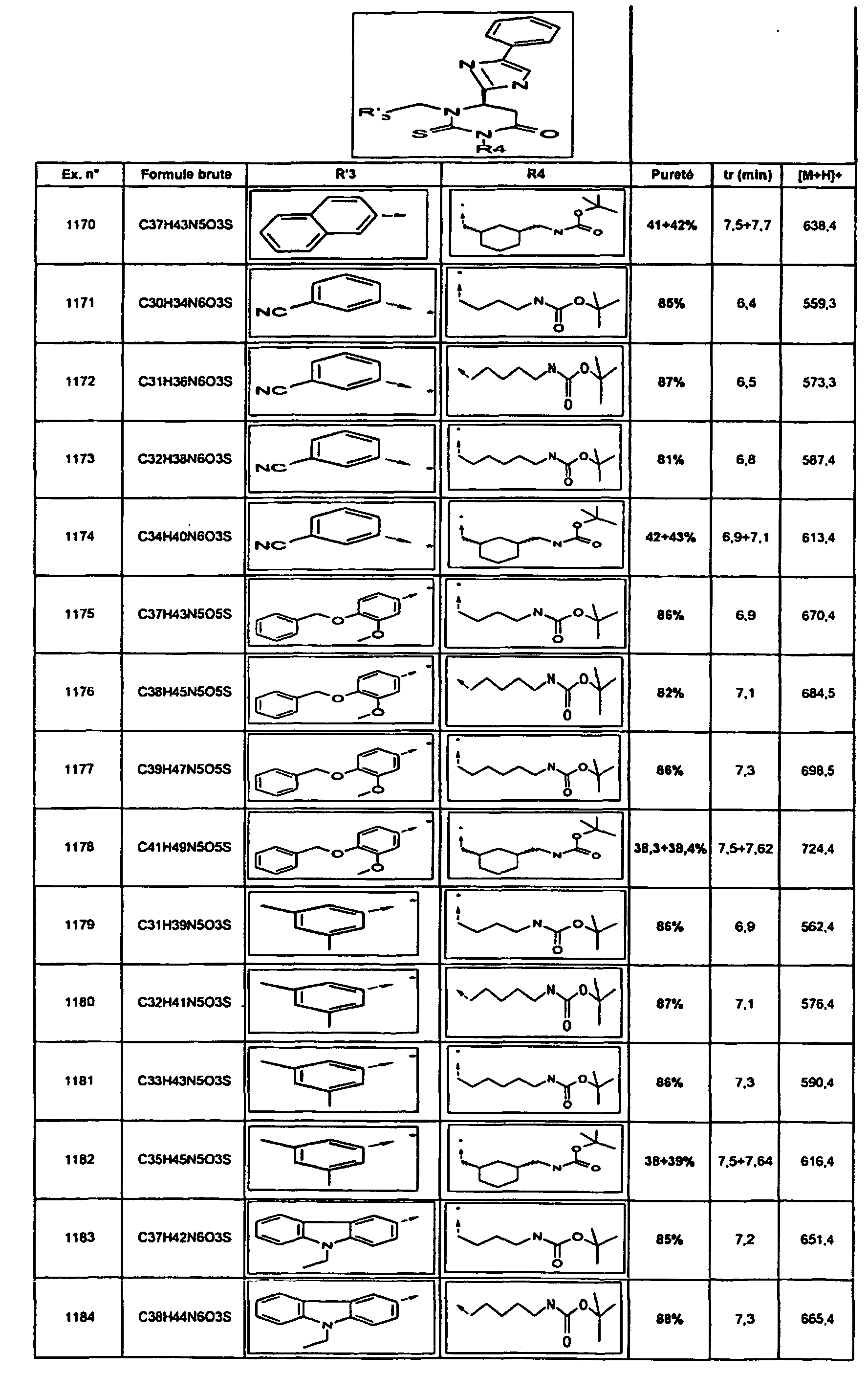

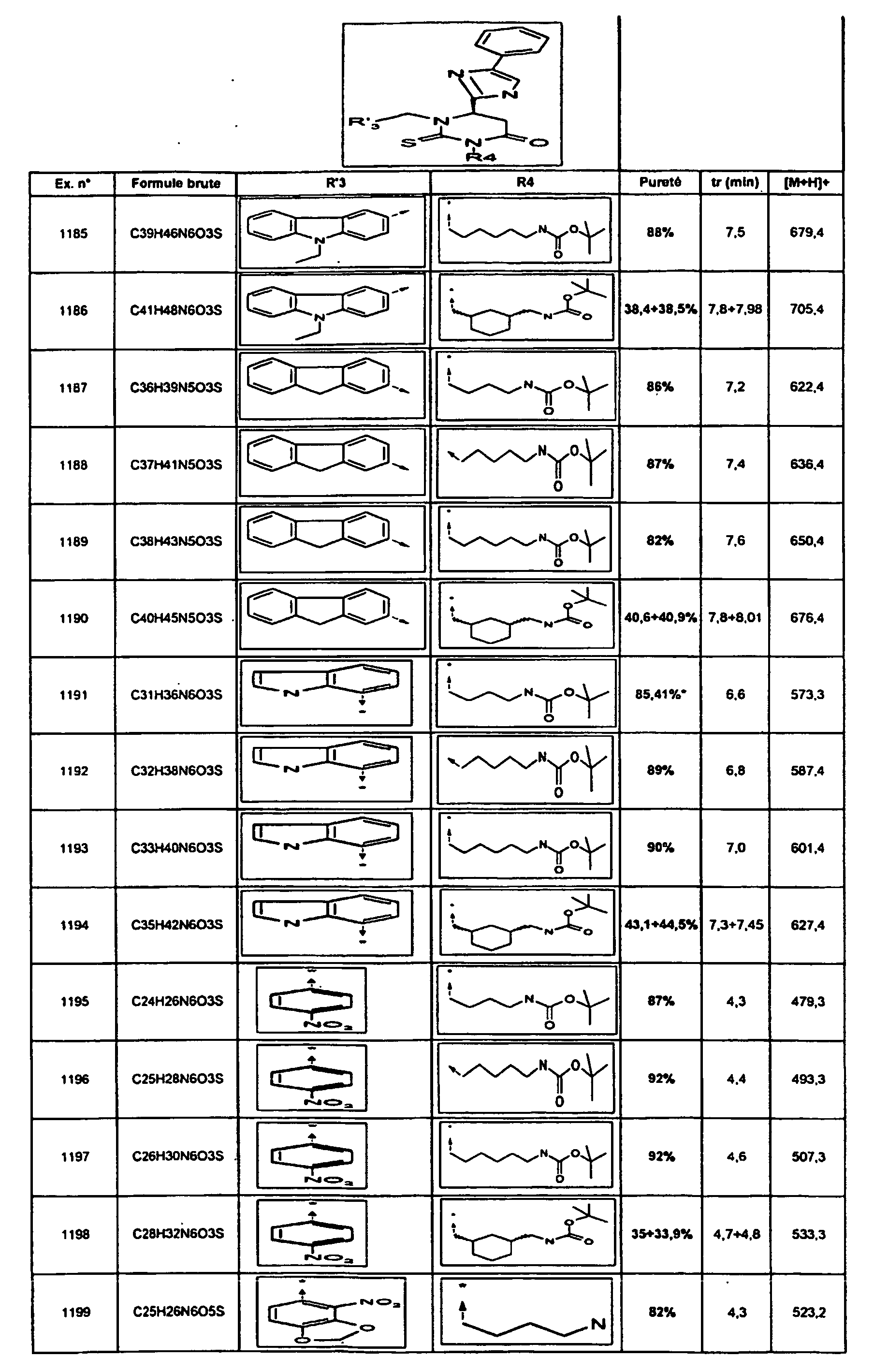

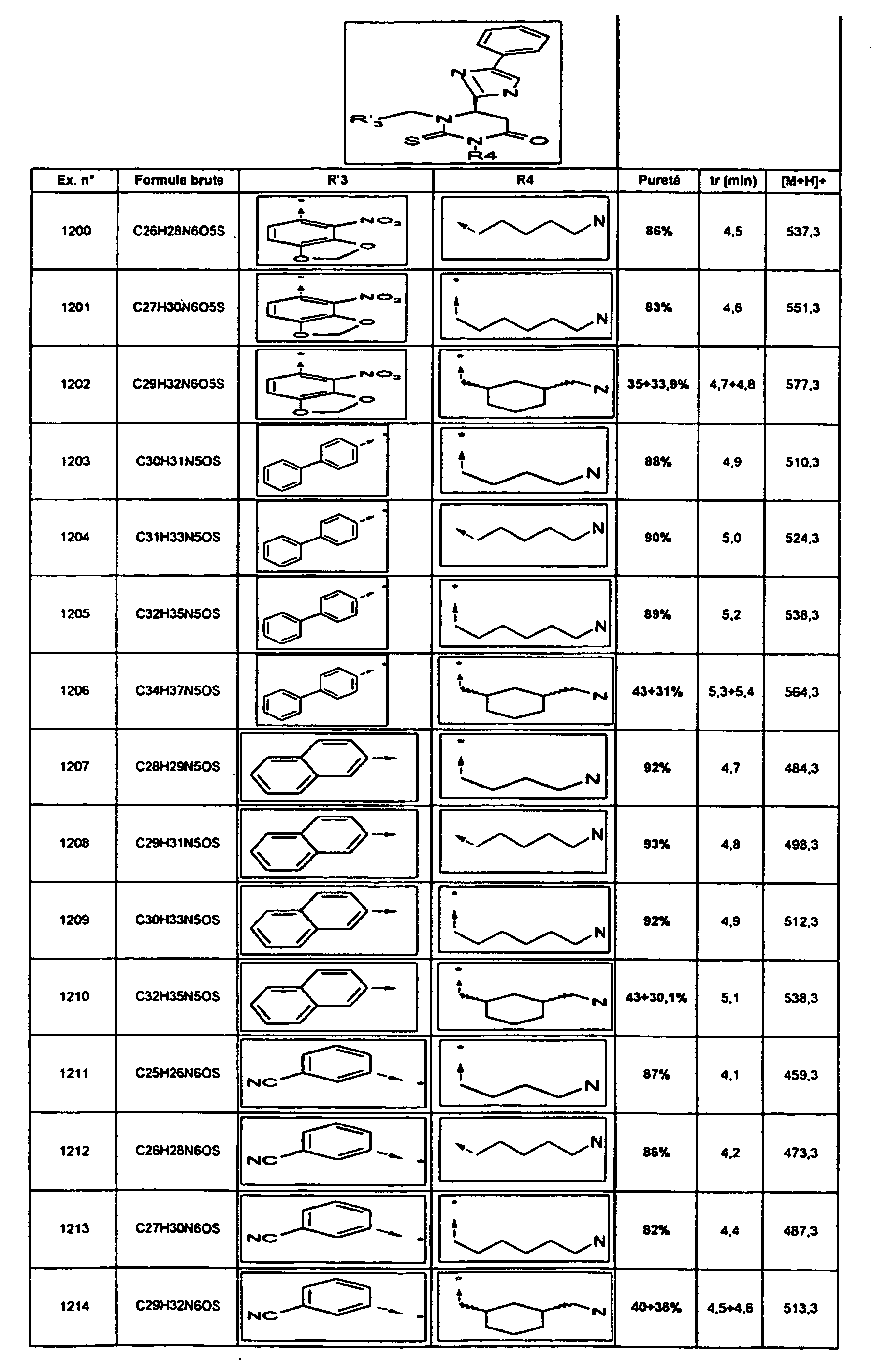

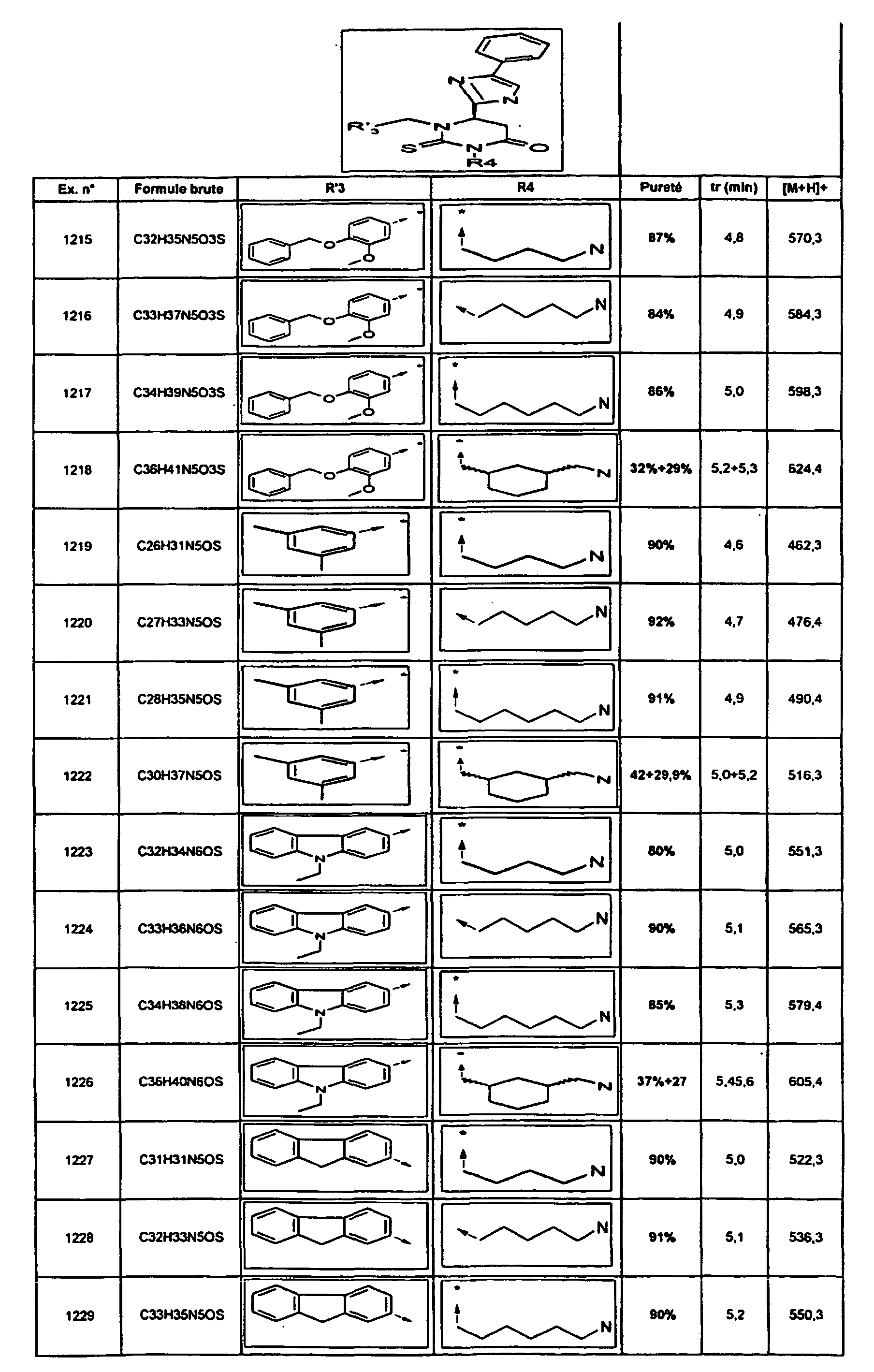

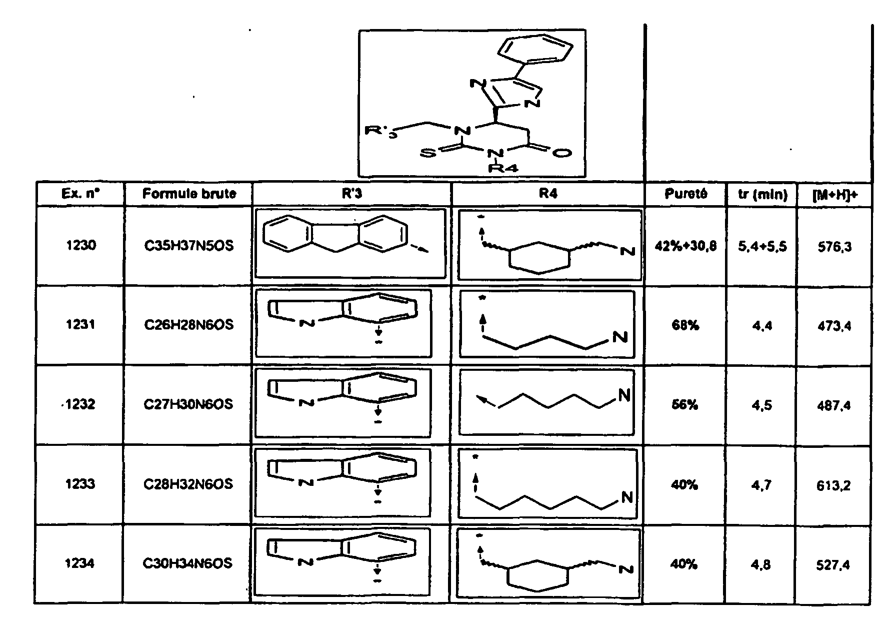

### PROPRIETES PHARMACOLOGIQUES DES COMPOSÉS DE L'INVENTION

Les composés de la présente invention peuvent et ont été testés en ce qui concerne leur affinité pour différents sous-types de récepteurs de la somatostatine selon les procédures décrites ci-après.

### Etude de l'affinité pour les sous-types de récepteurs de la somatostatine humaine :

L'affinité d'un composé de l'invention pour les sous-types de récepteurs de la somatostatine 1 à 5 (sst₁, sst₂, sst₃, sst₄ et sst₅, respectivement) est déterminée par la mesure de l'inhibition de la liaison de [¹²⁵I-Tyr¹¹]SRIF-14 à des cellules transfectées CHO-K1.

Le gène du récepteur sst₁ de la somatostatine humaine a été cloné sous forme d'un fragment génomique. Un segment *Pst*I-*Xmn*I de 1,5 Kb contenant 100 pb de la région 5' non transcrite, 1,17 Kb de la région codante en totalité, et 230 bp de la région 3' non transcrite est modifié par l'addition du linker Bg1II. Le fragment d'ADN résultant est souscloné dans le site *BamH*I d'un pCMV-81 pour donner le plasmide d'expression chez les mammifères (fourni par Dr. Graeme Bell, Univ. Chicago). Une lignée de cellules clonées exprimant de façon stable le récepteur sst₁ est obtenue par transfection dans des cellules CHO-K1 (ATCC) grâce à la méthode de co-précipitation calcium phosphate. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène du récepteur sst₂ de la somatostatine humaine, isolé sous forme d'un fragment génomique d'ADN de 1.7 Kb *Bam*HI-*Hind*III et souscloné dans un vecteur plasmidique pGEM3Z (Promega), a été fourni par le Dr. G. Bell (Univ. of Chicago). Le vecteur d'expression des cellules de mammifères est construit en insérant le fragment *Bam*H1-*Hind*II de 1,7 Kb dans des sites de restriction endonucléase compatibles du plasmide pCMV5. Une lignée de cellules clonées est obtenue par transfection dans des cellules CHO-K1 grâce à la méthode de co-précipitation calcium phosphate. Le plasmide pRSV-neo est inclus comme marqueur de sélection.

Le récepteur sst₃ est isolé comme fragment génomique, et la séquence codante complète est contenue dans un fragment *Bam*HI/*Hind*III de 2,4 Kb. Le plasmide d'expression chez les mammifères, pCMV-h3, est construit par insertion du fragment *Nco*I-*Hind*III de 2,0 Kb dans le site EcoR1 du vecteur pCMV après modification des terminaisons et addition de linkers EcoR1. Une lignée de cellules clonées exprimant de façon stable le récepteur sst₃ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le plasmide d'expression du récepteur sst₄ humain, pCMV-HX, a été fourni par le Dr. Graeme Bell (Univ. Chicago). Ce vecteur contient le fragment génomique codant pour le récepteur sst₄ humain de 1,4 Kb *Nhe*I-*Nhe*I, 456 pb de la région 5' non transcrite, et 200 pb de la région 3' non transcrite, cloné dans les sites *Xba*I/*Eco*R1 de PCMV-HX. Une lignée de cellules clonées exprimant de façon stable le récepteur sst₄ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène correpondant au récepteur sst₅ humain, obtenu par la méthode PCR en utilisant un clone génomique λ comme sonde, a été fourni par le Dr. Graeme Bell (Univ. Chicago). Le fragment PCR résultant de 1,2 Kb contient 21 paires de bases de la région 5' non transcrites, la région codante en totalité, et 55 pb de la région 3' non transcrite. Le clone est inséré dans un site EcoR1 du plasmide pBSSK(+). L'insert est récupéré sous la forme d'un fragment *Hind*III*-Xba*I de 1,2 Kb pour sousclonage dans un vecteur d'expression chez les mammifères, pCVM5. Une lignée de cellules clonées exprimant de façon stable le récepteur sst₅ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Les cellules CHO-K1 exprimant de façon stable l'un des récepteurs sst humain sont cultivées dans un milieu RPMI 1640 contenant 10% de sérum foetal de veau et 0,4 mg/ml de généticine. Les cellules sont collectées avec de l'EDTA 0;5 mM et centrifugées à 500 g pendant environ 5 min à environ 4 °C. Le centrifugat est re-suspendu dans un milieu tampon 50 mM Tris à pH 7,4 et centrifugé deux fois à 500 g pendant environ 5 min à environ 4 °C. Les cellules sont lysées par sonication et centrifugées à 39000 g pendant environ 10 min à 4 °C. Le centrifugat est re-suspendu dans le même milieu tampon et centrifugé at 50000 g pendant 10 min à environ 4 °C et les membranes dans le centrifugat obtenu sont stockées à - 80 °C.

Des tests d'inhibition compétitive de liaison avec [¹²⁵I-Tyr¹¹]SRIF-14 sont effectués en double à l'aide de plaques en polypropylène de 96 puits. Les membranes cellulaires (10 µg protéine/puits) sont incubées avec [¹²⁵I-Tyr¹¹]SRIF-14 (0,05 nM) pendant environ 60 min à environ 37 °C dans un milieu tampon 50 mM HEPES (pH 7,4) comprenant 0,2% BSA, 5 mM de MgCl₂, 200 KIU/ml de Trasylol, 0,02 mg/ml de bacitracine et 0,02 mg/ml de fluorure de phénylméthylsulphonyle.

La [¹²⁵I-Tyr¹¹]SRIF-14 liée est séparée de la [¹²⁵I-Tyr¹¹]SRIF-14 libre par filtration immédiate à travers des plaques filtres en fibre de verre GF/C (Unifilter, Packard) préimprégné avec 0,1 % de polyéthylènimine (P.E.I.), en utilisant un Filtermate 196 (Packard). Les filtres sont lavés avec du tampon 50 mM HEPES à environ 0-4 °C pendant environ 4 secondes et leur radioactivité est déterminée à l'aide d'un compteur (Packard Top Count).

La liaison spécifique est obtenue en soustrayant la liaison non spécifique (déterminée en présence de 0,1 µM SRIF-14) de la liaison totale. Les données relatives à la liaison sont analysées par analyse en régression non-linéaire assistée par ordinateur (MDL) et les valeurs des constantes d'inhibition (Ki) values sont déterminées.

La détermination du caractère agoniste ou antagoniste d'un composé de la présente invention est effectuée à l'aide du test décrit ci-après.

### Test fonctionnel : Inhibition de la production d'AMPc intracellulaire :

Des cellules CHO-K1 exprimant les sous-types de récepteurs de la somatostatine humaine (SRIF-14) sont cultivées dans des plaques à 24 puits dans un milieu RPMI 1640 avec 10% de sérum foetal de veau et 0,4 mg/ml de généticine. Le milieu est changé le jour précédant l'expérience.

Les cellules à raison de 10⁵ cellules/puits sont lavées 2 fois avec 0,5 ml de nouveau milieu RPMI comprenant 0,2 % BSA complété par 0,5 mM de 3-isobutyl-1-méthylxanthine (IBMX) et incubées pendant environ 5 min à environ 37 °C.

La production d'AMP cyclique est stimulée par l'addition de 1 mM de forskoline (FSK) pendant 15-30 minutes à environ 37 °C.

L'effet inhibiteur de la somatostatine d'un composé agoniste est mesuré par l'addition simultanée de FSK (1µM) , SRIF-14 (10⁻¹² M to 10⁻⁶ M) et du composé à tester (10⁻¹⁰ M à 10⁻⁵ M).

L'effet antagoniste d'un composé est mesuré par l'addition simultanée de FSK (1µM), SRIF-14 (1 to 10 nM) et du composé à tester (10⁻¹⁰ M to 10⁻⁵ M).
Le milieu réactionnel est éliminé et 200 ml de HCl 0,1 N sont ajoutés. La quantité d'AMPc est mesurée par un test radioimmunologique (Kit FlashPlate SMP001A, New England Nuclear).

### Résultats :

Les tests effectués selon les protocoles décrits ci-dessus ont permis de montrer que les composés de formule générale **(I)** définie dans la présente demande ont une bonne affinité pour au moins l'un des sous-types de récepteurs de la somatostatine, la constante Kᵢ étant inférieure au micromolaire pour certains des composés exemplifiés, et en particulier pour les composés repris dans les tableaux I et II ci-après.

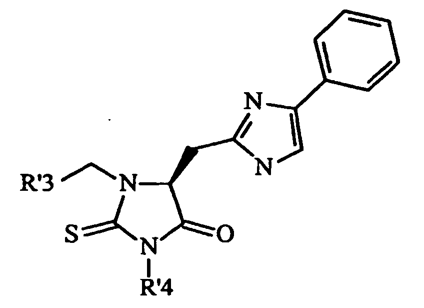

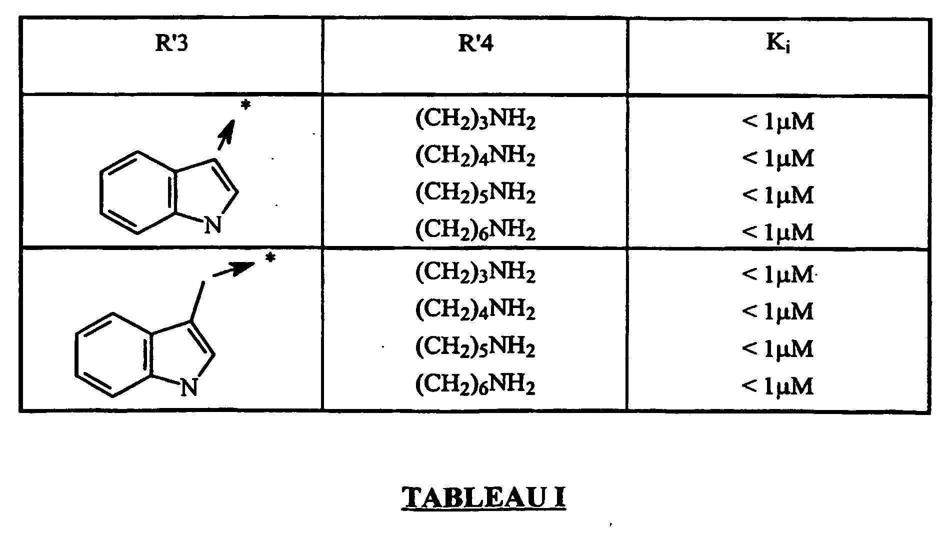

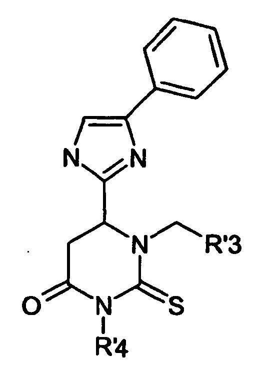

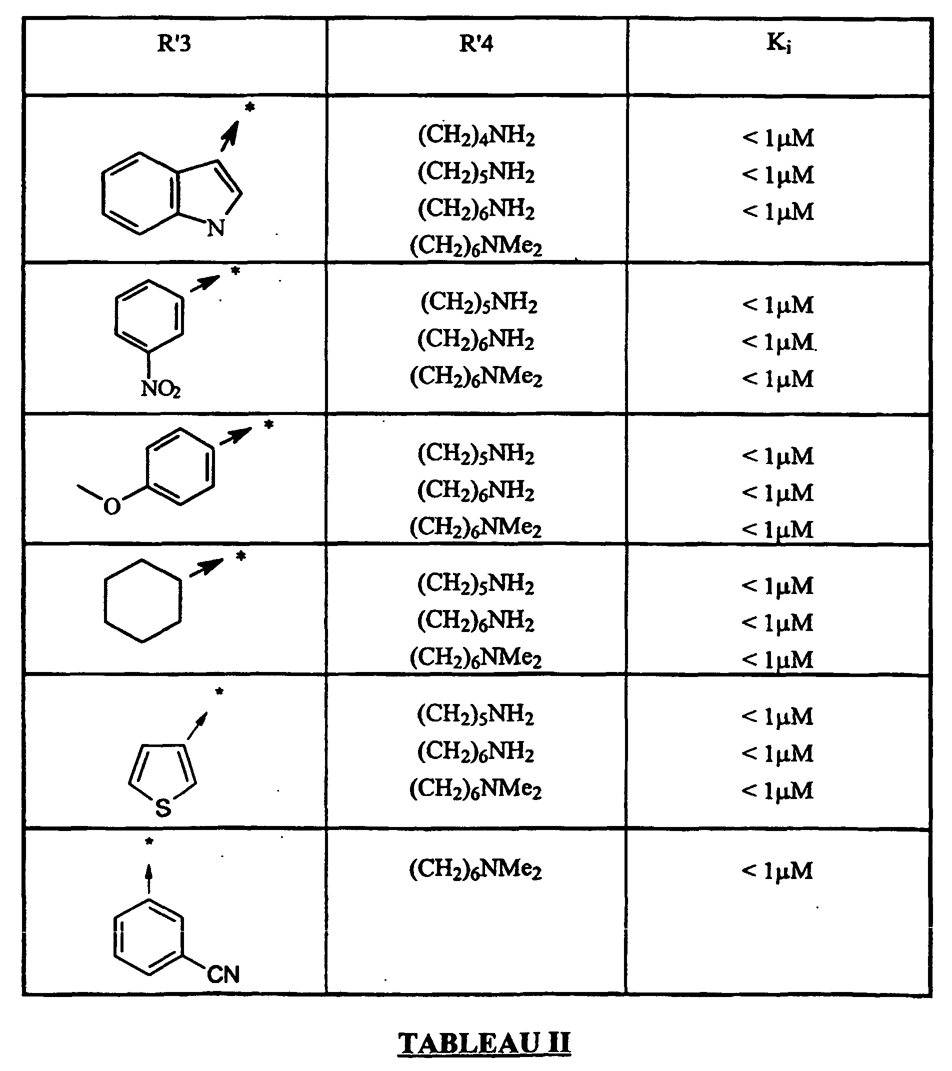

## Revendications

1. Composé de formule générale **(I)** représentée ci-dessous 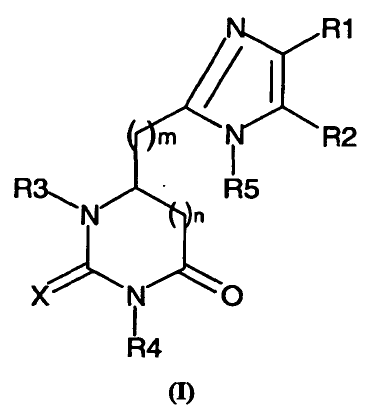 sous forme racémique, d'énantiomère ou toutes combinaisons de ces formes, dans laquelle :
R1 représente un radical phényle éventuellement substitué,
R2 représente H;
R3 représente H ou (CH₂)ₚ-Z3;
Z3 représente (C₁-C₁₂)alkyle, (C₁-C₁₂)alkényle, (C₃-C₈)cycloalkyle, un radical aryle carbocyclique ou hétérocyclique éventuellement substitué, un radical hétérocyclique non aromatique éventuellement substitué, un radical *bis*-arylalkyle ou di-arylalkyle ou encore le radical 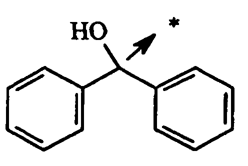
R4 représente (CH₂)ₚ-Z4 ;
Z4 représente amino, (C₁-C₁₂)alkyle, (C₃-C₈)cycloalkyle, (C₁-C₁₂)alkylamino, N,N-di-(C₁-C₁₂)alkylamino, amino(C₃-C₆)cycloalkyle, amino(C₁-C₆)alkyl(C₃-C₆)cycloalkyl(C₁-C₆)alkyle, aminoaryle carbocyclique ou hétérocyclique, (C₁-C₁₂)alkoxy, (C₁-C₁₂)alkényle, -N-C(O)O(C₁-C₆)alkyle, un radical aryle carbocyclique ou hétérocyclique éventuellement substitué, un radical hétérocyclique non aromatique éventuellement substitué, *bis*-arylalkyle, di-arylalkyle ou l'un des radicaux représentés ci-dessous 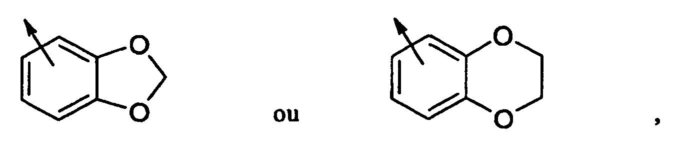
ou encore Z4 représente un radical N(R6)(R7) dans lequel R6 et R7 pris ensemble avec l'atome d'azote qui les porte forment ensemble un hétérocycle de 5 à 7 chaînons ;
R5 représente H ;,
étant entendu qu'un radical éventuellement substitué ou un phényle éventuellement substitué est éventuellement substitué par un ou plus d'un substituant, chacun de préférence choisi indépendamment parmi le groupe constitué des radicaux Cl, F, Br, I, CF₃, NO₂, OH, NH₂, CN, N₃, -OCF₃, (C₁-C₁₂)alkyle, (C₁-C₁₂)alkoxy, -(CH₂)ₚ-phényl-(X1)_{q}, -NH-CO-(C₁-C₆)alkyle, -NH-C(O)O-(C₁-C₆)alkyle, -S-(C₁-C₆)alkyle, -S-phényl-(X1)_{q}, -O-(CH₂)ₚ-phényl-(X1)_{q}, -(CH₂)ₚ-C(O)-O-(C₁-C₆)alkyle, -(CH₂)ₚ-C(O)-(C₁-C₆)alkyle, -O-(CH₂)ₚ-NH₂, -O-(CH₂)ₚ-NH-(C₁-C₆)alkyle, -O-(CH₂)ₚ-N-di-((C₁-C₆)alkyl) et -((C₀-C₁₂))alkyl-(X1)_{q} ;
X1, à chaque fois qu'il intervient, est indépendamment choisi parmi le groupe constitué des radicaux H, Cl, F, Br, I, CF₃, NO₂, OH, NH₂, CN, N₃, -OCF₃, (C₁-C₁₂)alkyle, (C₁-C₁₂)alkoxy, -S-(C₁-C₆)alkyle, -(CH₂)ₚ-amino, -(CH₂)ₚ-NH-(C₁-C₆)alkyle, -(CH₂)ₚ-N-di-((C₁-C₆)alkyl), -(CH₂)ₚ-phényle et -(CH₂)ₚ-NH-(C₃-C₆)cycloalkyle ;
p à chaque fois qu'il intervient est indépendamment 0 ou un entier de 1 à 6 ;
q à chaque fois qu'il intervient est indépendamment un entier de 1 à 5.
X représente O ou S;
n représente 0 ou 1; et enfin
lorsque n représente 0, m représente 1, 2 ou 3, et lorsque n représente 1, m représente 0 ou 1 ;
ou un sel pharmaceutiquement acceptable d'un tel composé.

2. Composé selon la revendication 1, **caractérisé en ce que** :
R1 représente un radical phényle éventuellement substitué ;
R2 représente H ;
R3 représente l'un des radicaux ci-après : 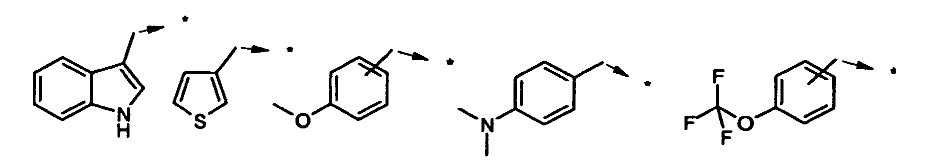 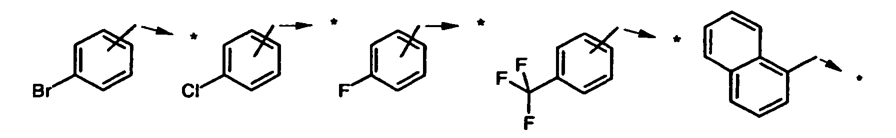 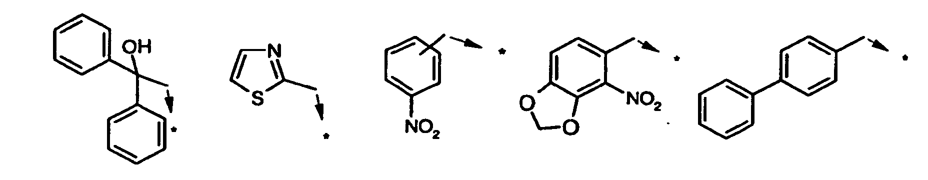 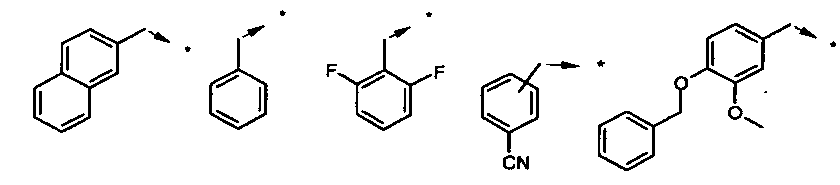 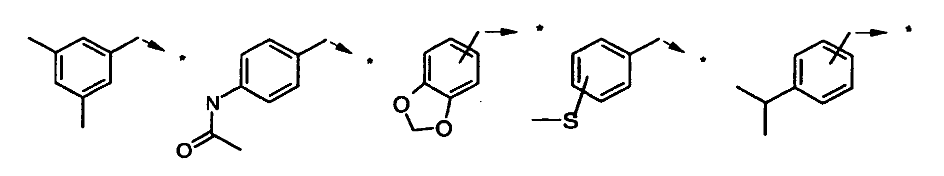 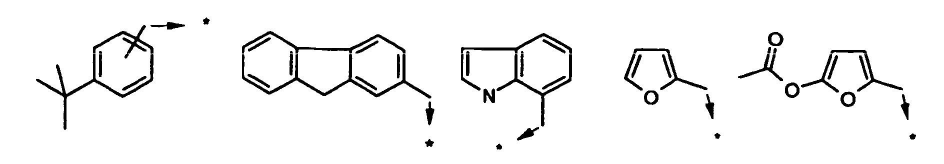 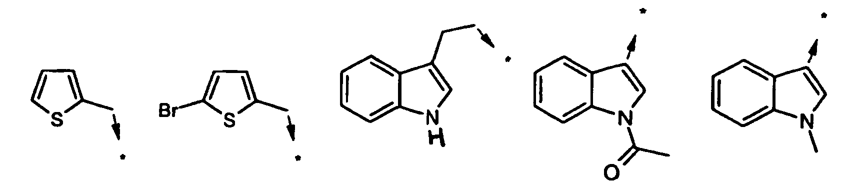 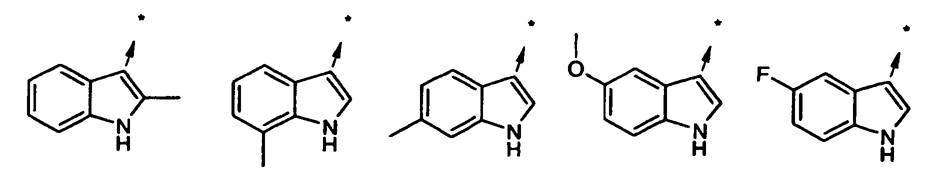 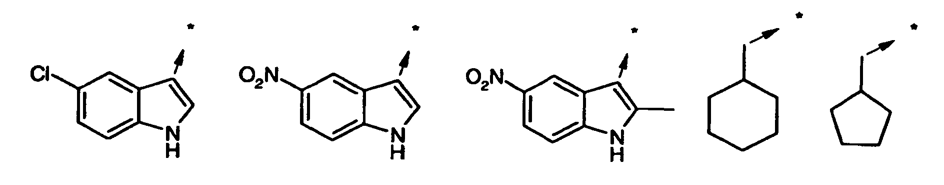 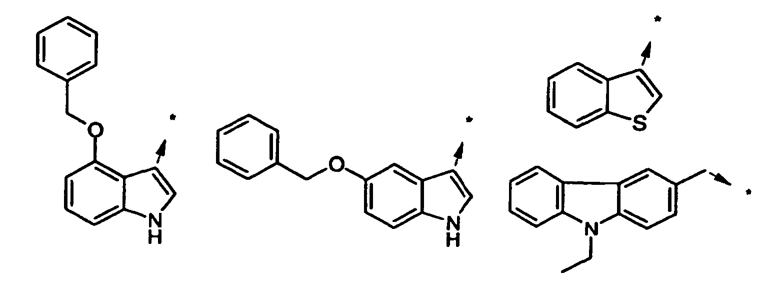 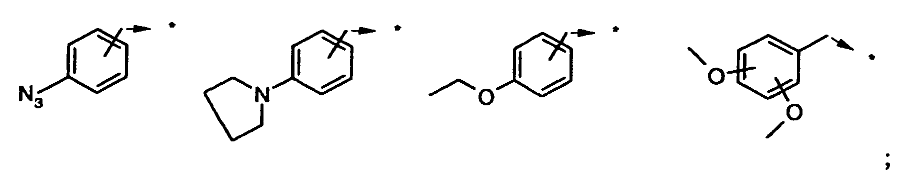
R4 représente l'un des radicaux ci-après : 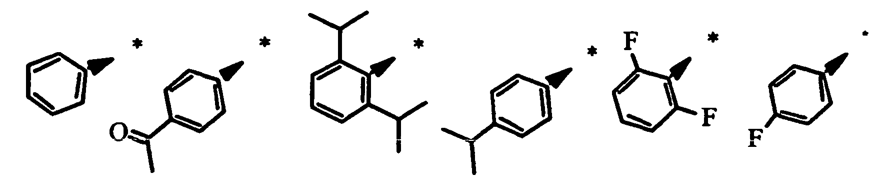 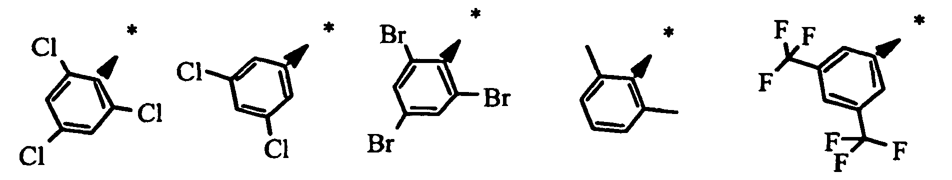 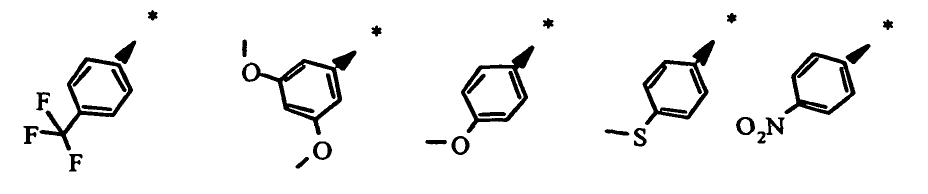 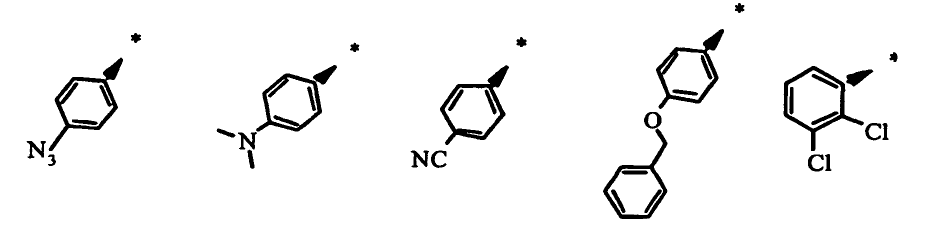 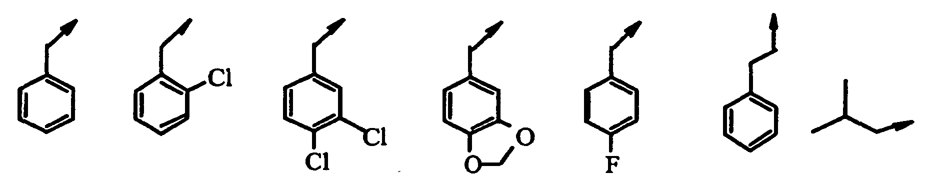 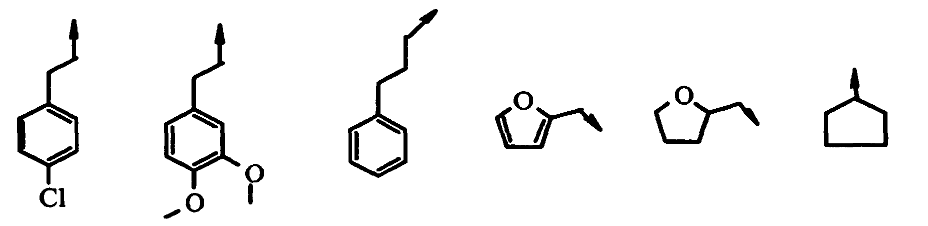 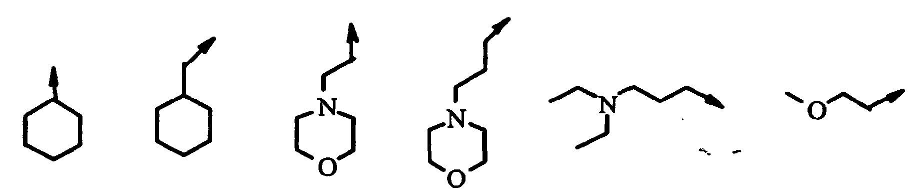 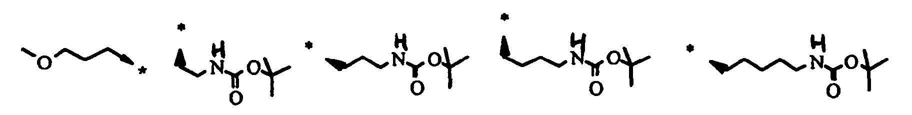 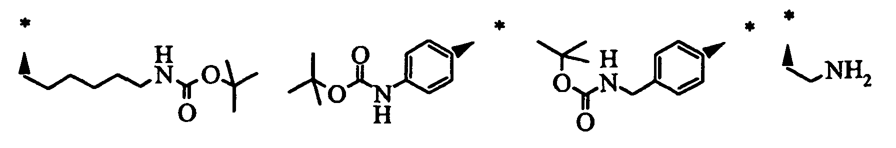 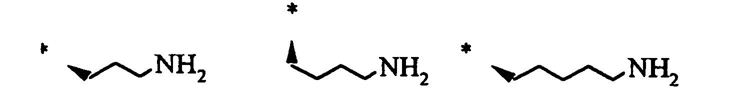 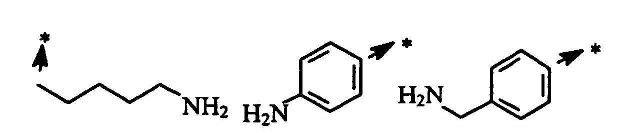 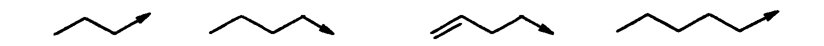 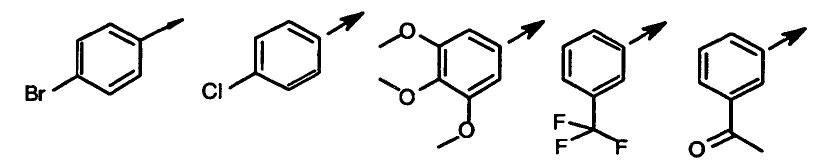 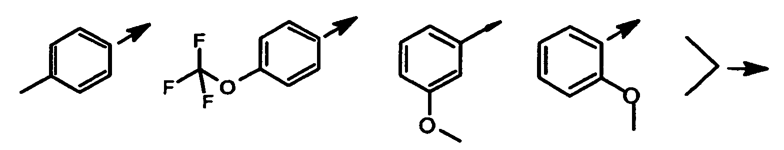 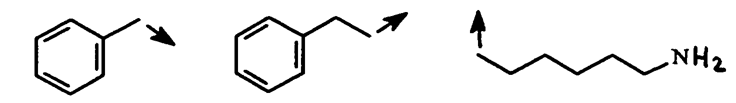 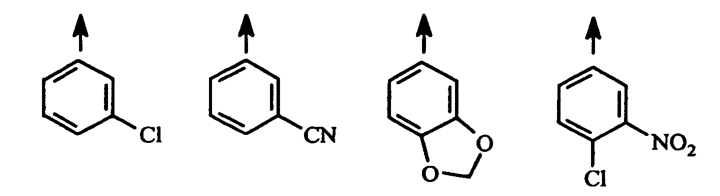 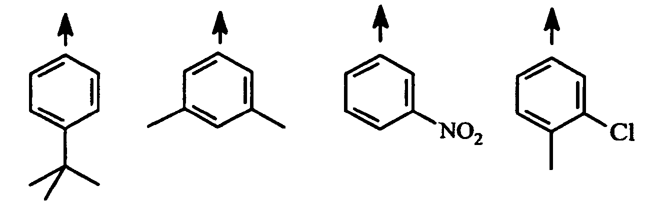 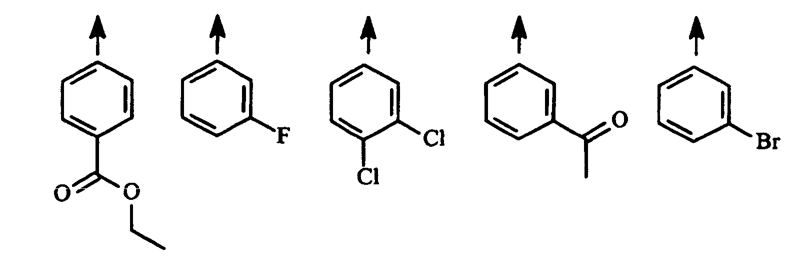 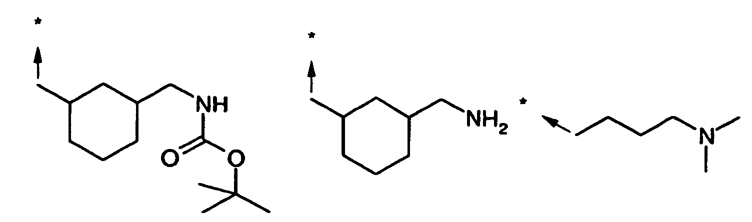 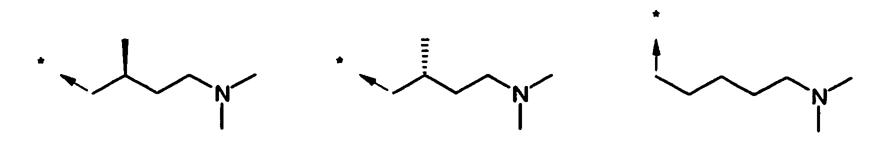 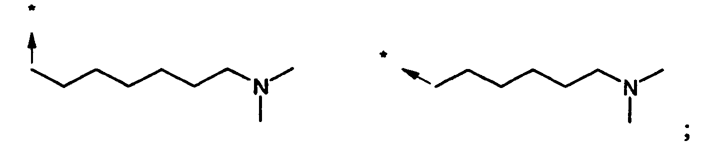
R5 représente H ;
ou un sel pharmaceutiquement acceptable d'un tel composé.

3. Composé selon la revendication 1, **caractérisé en ce que** :
R1 représente le radical phényle éventuellement substitué par un atome halogène ou un radical (C₁-C₁₂)alkyle, (C₁-C₁₂)alkoxy ou nitro ;
R2 et R5 représentent chacun H ;
R3 représente H ou (CH₂)ₚ-Z3;
Z3 représente (C₁-C₁₂)alkyle, (C₃-C₈)cycloalkyle, un radical aryle carbocyclique ou hétérocyclique éventuellement substitué, un radical hétérocyclique non aromatique éventuellement substitué, *bis*-arylalkyle, di-arylalkyle ou l'un des radicaux représentés ci-dessous 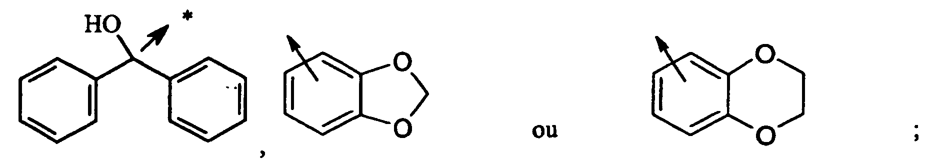
R4 représente (CH₂)ₚ-Z4 ;
Z4 représente amino, (C₃-C₈)cycloalkyle, (C₁-C₁₂)alkylamino, N,N-di-(C₁-C₁₂)alkylamino, amino(C₃-C₆)cycloalkyle, amino(C₁-C₆)alkyl(C₃-C₆)cycloalkyl(C₁-C₆)alkyle, aminoaryle carbocyclique ou hétérocyclique, un radical aryle carbocyclique ou hétérocyclique éventuellement substitué, un radical hétérocyclique non aromatique éventuellement substitué,*bis*-arylalkyle, di-arylalkyle ou l'un des radicaux représentés ci-dessous 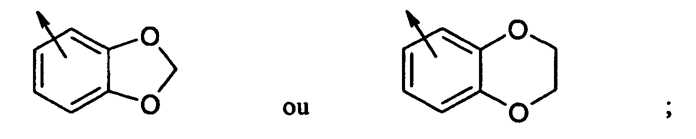 étant entendu qu'un radical éventuellement substitué ou un phényle éventuellement substitué est éventuellement substitué par un ou plus d'un substituant, chacun de préférence choisi indépendamment parmi le groupe constitué des radicaux Cl, F, Br, I, CF₃, NO₂, OH, NH₂, CN, N₃, -OCF₃, (C₁-C₁₂)alkyle, (C₁-C₁₂)alkoxy, -(CH₂)ₚ-phényl-(X1)_{q}, -NH-CO-(C₁-C₆)alkyle, -NH-C(O)O-(C₁-C₆)alkyle, -S-(C₁-C₆)alkyle, -S-phényl-(X1)_{q}, -O-(CH₂)ₚ-phényl-(X1)_{q}, -(CH₂)ₚ-C(O)-O-(C₁-C₆)alkyle, -(CH₂)ₚ-C(O)-(C₁-C₆)alkyle, -O-(CH₂)ₚ-NH₂, -O-(CH₂)ₚ-NH-(C₁-C₆)alkyle, -O-(CH₂)ₚ-N-di-((C₁-C₆)alkyl) et -((C₀-C₁₂))alkyl-(X1)_{q} ;
X1, à chaque fois qu'il intervient, est indépendamment choisi parmi le groupe constitué des radicaux H, Cl, F, Br, I, CF₃, NO₂, OH, NH₂, CN, N₃, -OCF₃, (C₁-C₁₂)alkyle, (C₁-C₁₂)alkoxy, -S-(C₁-C₆)alkyle, -(CH₂)ₚ-amino, -(CH₂)ₚ-NH-(C₁-C₆)alkyle, -(CH₂)ₚ-N-di-((C₁-C₆)alkyl), -(CH₂)ₚ-phényle et -(CH₂)ₚ-NH-(C₃-C₆)cycloalkyle ;
p à chaque fois qu'il intervient est indépendamment 0 ou un entier de 1 à 6 ; et
q à chaque fois qu'il intervient est indépendamment un entier de 1 à 5 ;
ou un sel pharmaceutiquement acceptable d'un tel composé.

4. Composé selon la revendication 3, **caractérisé en ce que** :
R1 représente le radical phényle éventuellement substitué par un atome halogène ou un radical (C₁-C₁₂)alkyle, (C₁-C₁₂)alkoxy ou nitro ;
R2 et R5 représentent chacun H ;
R3 représente (CH₂)ₚ -Z3,
Z3 représentant un radical (C₃-C₈)cycloalkyle ou un radical éventuellement substitué choisi parmi les radicaux phényle, naphtyle, furannyle, thiophène, indolyle, pyrrolyle et benzothiophène ;
R4 représente (CH₂)ₚ-Z4 ;
Z4 représentant amino, (C₁-C₁₂)alkylamino, N,N-di-(C₁-C₁₂)alkylamino ou amino(C₁-C₆)alkyl(C₃-C₆)cycloalkyl-(C₁-C₆)alkyle ;
X représente S ;
p à chaque fois qu'il intervient est indépendamment 0 ou un entier de 1 à 6 ;
m représente 0, 1 ou 2 ; et enfin
n représente 0 ou 1;
ou un sel pharmaceutiquement acceptable d'un tel composé.

5. Composé selon la revendication 4, **caractérisé en ce qu'**il s'agit d'un composé :
- de sous-formule générale **(I)a** représentée ci-dessous : 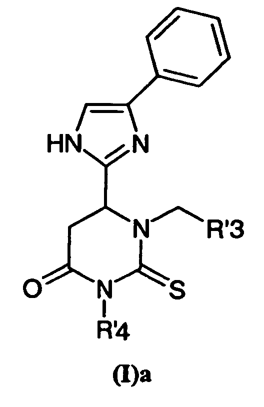 dans laquelle :
R'3 représente l'un des radicaux représentés ci-dessous : 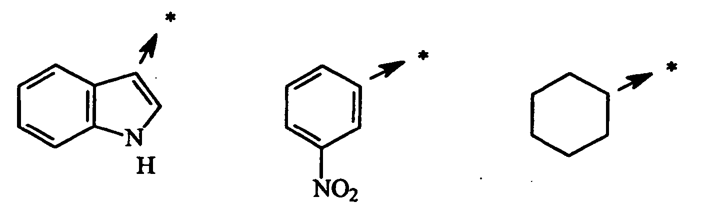 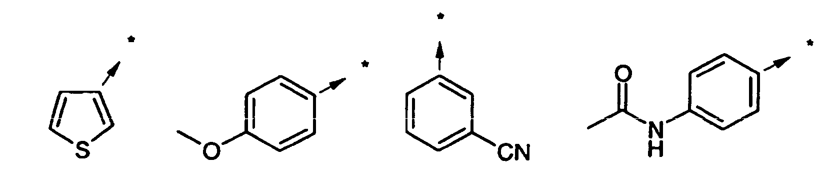
et R'4 représente l'un des radicaux représentés ci-dessous : 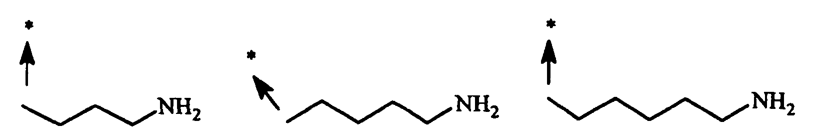 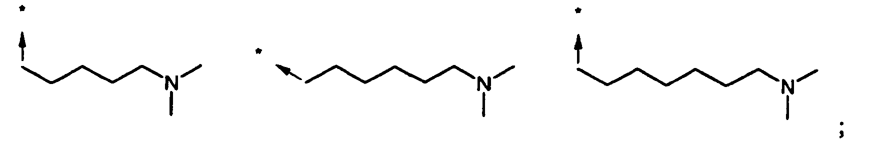
- de sous-formule générale **(I)b** représentée ci-dessous : 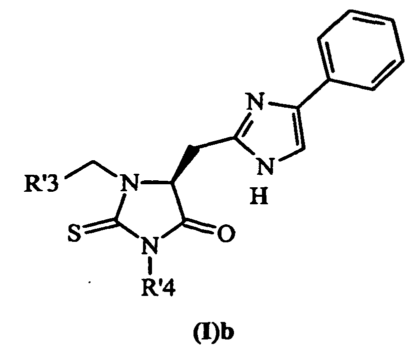 dans laquelle :
R'3 représente l'un des radicaux représentés ci-dessous : 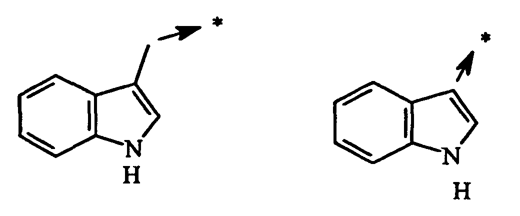
et R'4 représente l'un des radicaux représentés ci-dessous : 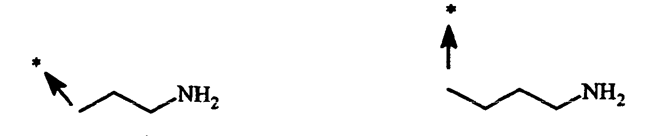 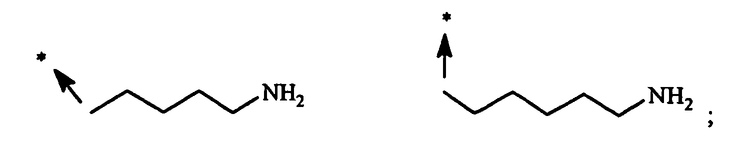
- de sous-formule générale **(I)c** représentée ci-dessous : 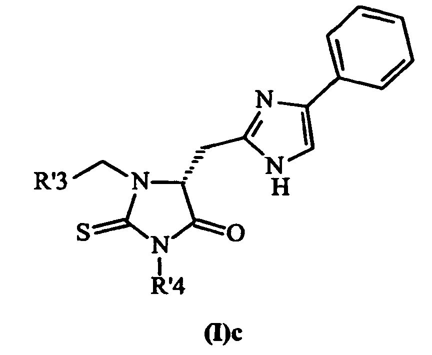 dans laquelle :
R'3 représente l'un des radicaux représentés ci-dessous : 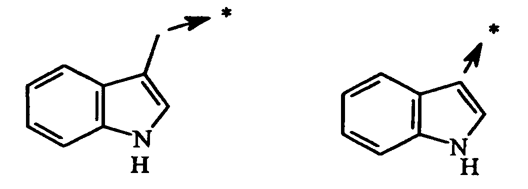
et R'4 représente l'un des radicaux représentés ci-dessous : 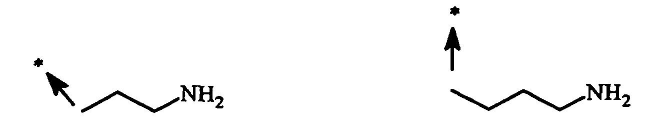 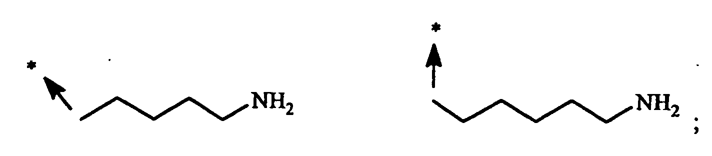
ou un sel pharmaceutiquement acceptable d'un tel composé.

6. Procédé de préparation d'un composé de formule générale **(I)** selon la revendication 1 dans lequel n représente 0, **caractérisé en ce que** l'on traite dans un solvant aprotique le composé de formule générale **(II)** 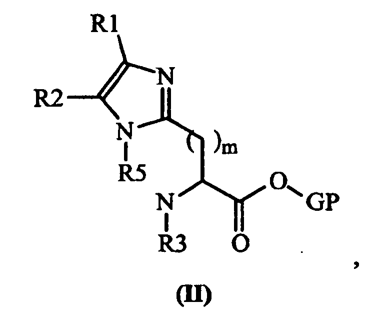 dans laquelle m, R1, R2, R3 et R5 ont la même signification que dans la formule générale **(I)** de la revendication 1, et le radical O-GP est un groupe protecteur partant dérivé d'un alcool et en particulier benzyloxy, méthoxy ou tert-butoxy,
avec un isocyanate ou isothiocyanate de formule générale **(III)**
R4-N=C=X **(III)**
dans laquelle R4 et X ont la même signification que dans la formule générale **(I)** de la revendication 1,
de préférence en présence d'une base tertiaire pendant une durée d'environ 1 à 24 heures et à une température de préférence comprise entre 20 et 70 °C.

7. Procédé de préparation d'un composé de formule générale **(I)** selon la revendication 1 dans lequel n représente 1, **caractérisé en ce que** l'on traite dans un solvant aprotique le composé de formule générale **(IV)** 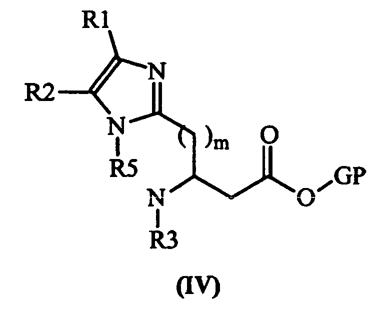 dans laquelle m, R1, R2, R3 et R5 ont la même signification que dans la formule générale **(I)** de la revendication 1, et le radical O-GP est un groupe protecteur partant dérivé d'un alcool et en particulier benzyloxy, méthoxy ou tert-butoxy,
avec un isocyanate ou isothiocyanate de formule générale **(III)**
R4-N=C=X **(III)**
dans laquelle R4 et X ont la même signification que dans la formule générale **(I)** de la revendication 1,
de préférence en présence d'une base tertiaire pendant une durée d'environ 1 à 48 heures et à une température de préférence comprise entre 20 et 70 °C.

8. A titre de composé industriel nouveau et d'intermédiaire dans le procédé de synthèse des composés de formule générale **(I)** selon la revendication 1, un composé de formule générale **(V)**, 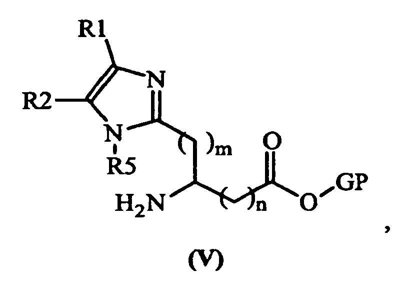 dans laquelle :
R1, R2, R5, m et n ont la même signification que dans la formule générale **(I)** ;
et le radical O-GP est choisi parmi les groupes benzyloxy, méthoxy ou tert-butoxy.

9. Composé selon la revendication 8, **caractérisé en ce qu'**il s'agit :
- du (2*S*)-2-amino-3-[(4-phényl)-1H-imidazol-2-yl]propanoate de benzyle ;
- du (2*R*)-2-amino-3-[(4-phényl)-1H-imidazol-2-yl]propanoate de benzyle ;
- du (2*S*)-2-amino-4-[(4-phényl)-1H-imidazol-2-yl]butanoate de benzyle ; ou
- du (2*R*)-2-amino-4-[(4-phényl)-1H-imidazol-2-yl]butanoate de benzyle.

10. A titre de médicament, un composé selon l'une des revendications 1 à 5 ou un sel pharmaceutiquement acceptable dudit composé.

11. Composition pharmaceutique comprenant à titre de principe actif un composé selon l'une des revendications 1 à 5 ou un sel pharmaceutiquement acceptable dudit composé.

12. Utilisation d'un composé selon l'une des revendications 1 à 5 ou d'un sel pharmaceutiquement acceptable dudit composé pour préparer un médicament destiné à traiter les états pathologiques ou les maladies dans lesquels un (ou plusieurs) des récepteurs de la somatostatine est (sont) impliqué(s).

13. Utilisation selon la revendication 12, **caractérisée en ce que** les états pathologiques ou les maladies à traiter sont choisis parmi le groupe composé des états pathologiques ou des maladies qui suivent : l'acromégalie, les adénomes hypophysaires, la maladie de Cushing, les gonadotrophinomes et les prolactinomes, les effets secondaires cataboliques des glucocorticoïdes, le diabète insulinodépendant, la rétinopathie diabétique, la néphropathie diabétique, le syndrome X, le phénomène de Dawn, l'angiopathie, l'angioplastie, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, les ulcères, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, les diarrhées induites par la chimiothérapie, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, le saignement des vaisseaux greffés, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et, dans d'autres domaines thérapeutiques, les céphalées y compris les céphalées associées aux tumeurs hypophysaires, les douleurs, les désordres inflammatoires comme l'arthrite, les accès de panique, la chimiothérapie, la cicatrisation des plaies, l'insuffisance rénale résultant d'un retard de croissance, l'hyperlipidémie, l'obésité et le retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis, le rejet chronique des allogreffes ainsi que la maladie d'Alzheimer et enfin l'ostéoporose.

14. Utilisation selon la revendication 13, **caractérisée en ce que** les états pathologiques ou les maladies à traiter sont choisis parmi le groupe composé des états pathologiques ou des maladies qui suivent : l'acromégalie, les adénomes hypophysaires ou les tumeurs gastroentéropancréatiques endocriniennes dont le syndrome carcinoïde, et les saignements gastro-intestinaux.

## Patentansprüche

1. Verbindung der nachstehenden allgemeinen Formel (I) 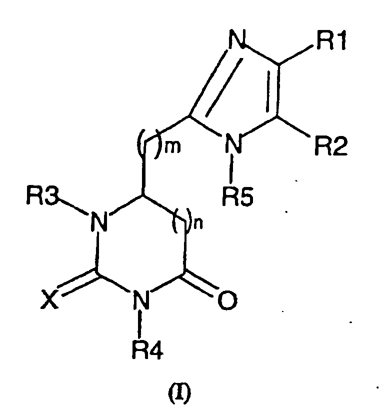 in racemischer Form, in Enantiomerform oder in allen Kombinationen dieser Formen, in der:
R1 einen ggf. substituierten Phenylrest darstellt,
R2 H darstellt;
R3 H oder (CH₂)ₚ-Z3 darstellt;
wobei Z3 (C₁-C₁₂)Alkyl, (C₁-C₁₂)Alkenyl, (C₃-C₈)Cycloalkyl, einen ggf. substituierten carbocyclischen oder heterocyclischen Arylrest, einen ggf. substituierten nicht aromatischen heterocyclischen Rest, einen *bis*-Arylalkyl- oder Di-Arylalkylrest oder den Rest 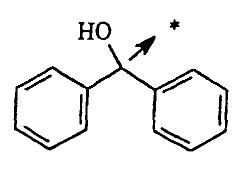 darstellt;
R4 (CH₂)ₚ-Z4 darstellt;
wobei Z4 Amino, (C₁-C₁₂)Alkyl, (C₃-C₈)Cycloalkyl, (C₁-C₁₂)Alkylamino, N,N-Di-(C₁-C₁₂)alkylamino, Amino(C₃-C₆)cycloalkyl, Amino(C₁-C₆)alkyl(C₃-C₆)cycloalkyl(C₁-C₆)alkyl, carbocyclisches oder heterocyclisches Aminoaryl, (C₁-C₁₂)Alkoxy, C₁-C₁₂)Alkenyl, -N-C(O)O(C₁-C₆)Alkyl, einen ggf. substituierten carbocyclischen oder heterocyclischen Arylrest, einen ggf. substituierten nicht aromatischen heterocyclischen Rest, bis-Arylalkyl, Di-arylalkyl oder einen der nachstehenden Reste 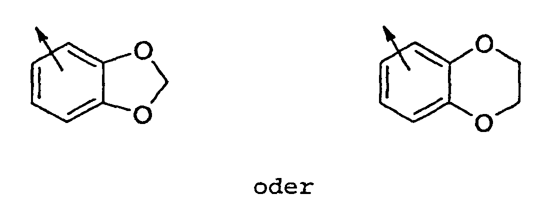 darstellt
oder Z4 einen Rest N(R6)(R7) darstellt, in dem R6 und R7 zusammen mit dem sie tragenden Stickstoff zusammen einen Heterocyclus mit 5 bis 7 Gliedern bilden;
R5 H darstellt;
wobei gilt, dass ein ggf. substituierter Rest oder ein ggf. substituiertes Phenyl ggf. durch einen oder mehr als einen Substituenten substituiert ist, wobei jeder vorzugsweise unabhängig voneinander aus der Gruppe ausgewählt ist, die aus den Resten Cl, F, Br, I, CF₃, NO₂, OH, NH₂, CN, N₃, -OCF₃, (C₁-C₁₂)Alkyl, (C₁-C₁₂)Alkoxy, -(CH₂)ₚ-Phenyl-(X1)_{q}, -NH-CO-(C₁-C₆)Alkyl, -NH-C(O)O-(C₁-C₆)Alkyl, -S-(C₁-C₆)Alkyl, -S-Phenyl-(X1)_{q}, -O-(CH₂)ₚ-Phenyl-(X1)_{q}, -(CH₂)ₚ-C(O)-O-(C₁-C₆)Alkyl, -(CH₂)ₚ-C(O)-(C₁-C₆)Alkyl, -O-(CH₂)ₚ-NH₂, -O-(CH₂)ₚ-NH-(C₁-C₆)Alkyl, -O-(CH₂)ₚ-N-Di-((C₁-C₆)alkyl) und -((C₀-C₁₂))Alkyl-(X1)_{q} besteht;
X1, jedes Mal wenn es auftritt, unabhängig voneinander aus der Gruppe ausgewählt ist, die aus den Resten H, Cl, F, Br, I, CF₃, NO₂, OH, NH₂, CN, N₃, -OCF₃, (C₁-C₁₂)Alkyl, (C₁-C₁₂)Alkoxy, -S-(C₁-C₆)Alkyl, -(CH₂)ₚ-Amino, -(CH₂)ₚ-NH-(C₁-C₆)Alkyl, -(CH₂)ₚ-N-Di-((C₁-C₆)alkyl), -(CH₂)ₚ-Phenyl und - (CH₂)ₚ-NH-(C₃-C₆)Cycloalkyl besteht;
P, jedes Mal wenn es auftritt, unabhängig voneinander 0 oder eine ganze Zahl von 1 bis 6 ist;
Q, jedes Mal wenn es auftritt, unabhängig voneinander eine ganze Zahl von 1 bis 5 ist;
X O oder S darstellt;
n 0 oder 1 darstellt; und schließlich
wenn n 0 darstellt, stellt m 1, 2 oder 3 dar, und wenn n 1 darstellt, stellt m 0 oder 1 dar;
oder ein pharmazeutisch annehmbares Salz einer solchen Verbindung.

2. Verbindung nach Anspruch 1, **dadurch gekennzeichnet, dass**
R1 einen ggf. substituierten Phenylrest darstellt;
R2 H darstellt;
R3 einen der nachstehenden Reste darstellt: 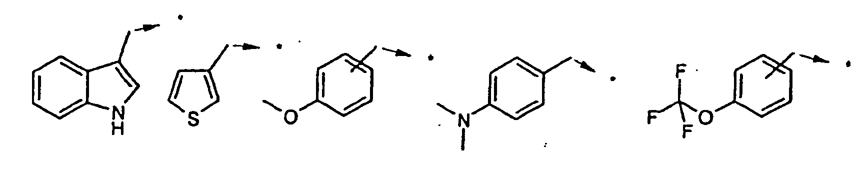 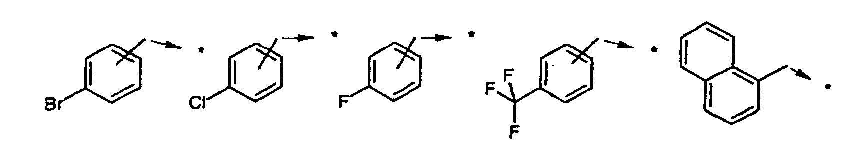 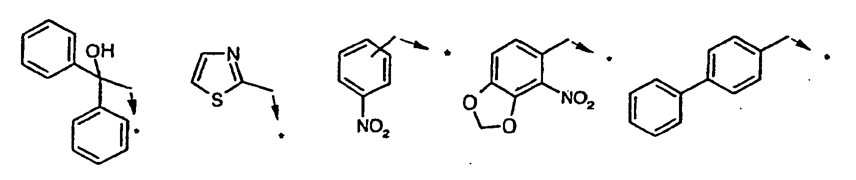 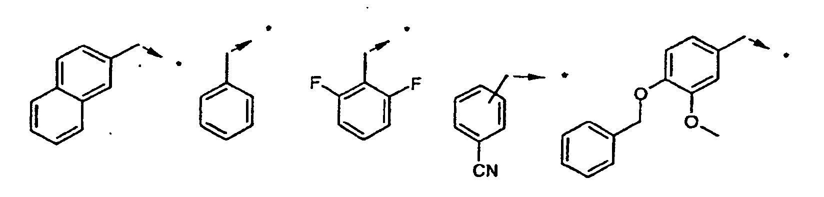 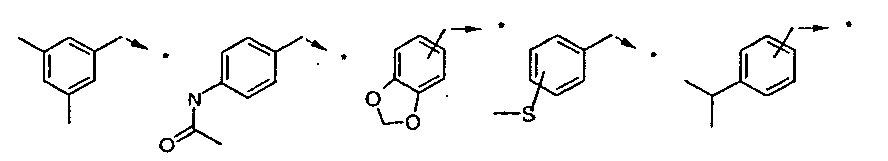 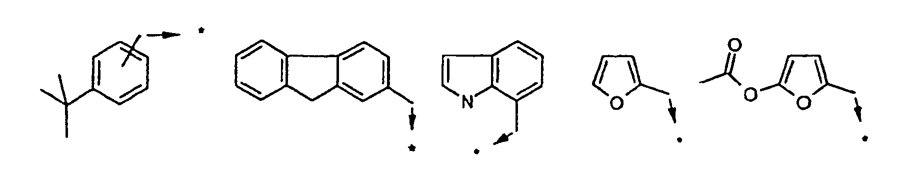 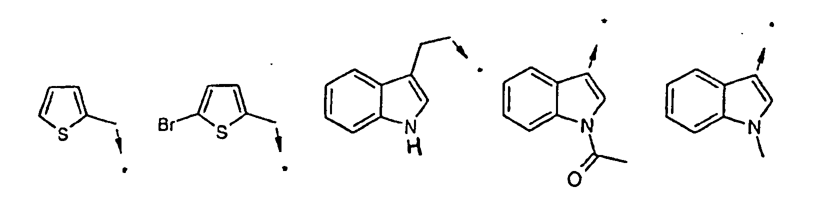 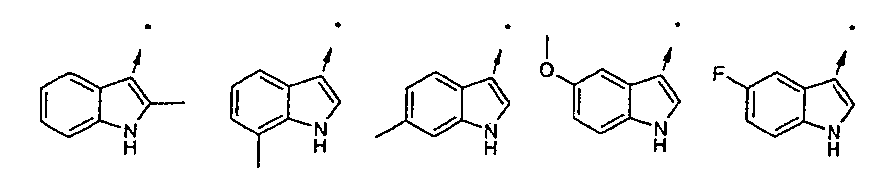 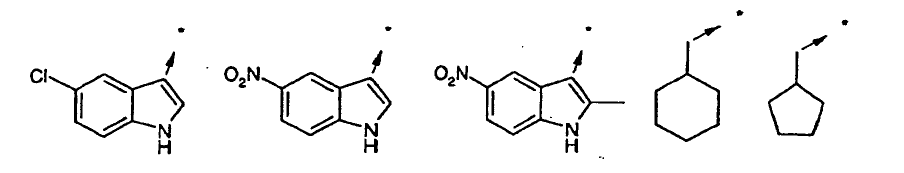 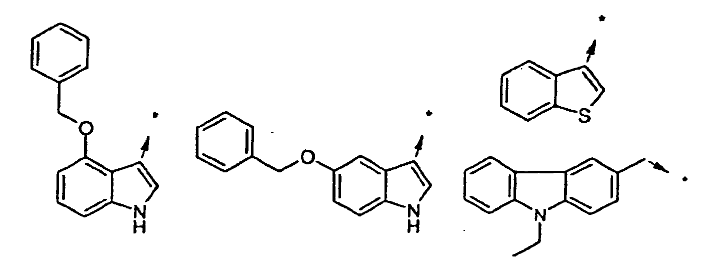 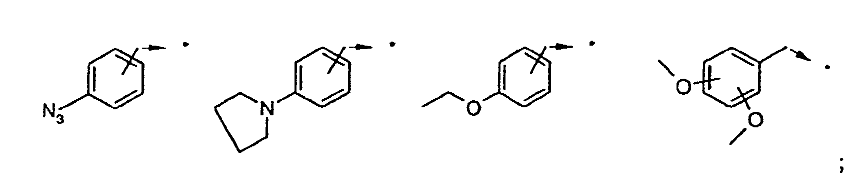 R4 einen der nachstehenden Reste darstellt: 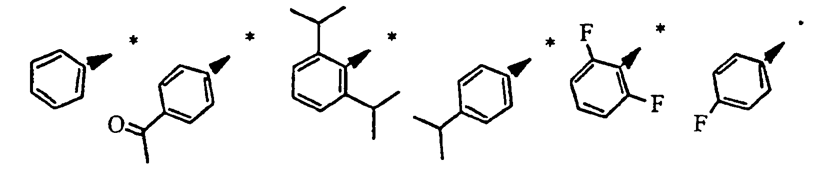 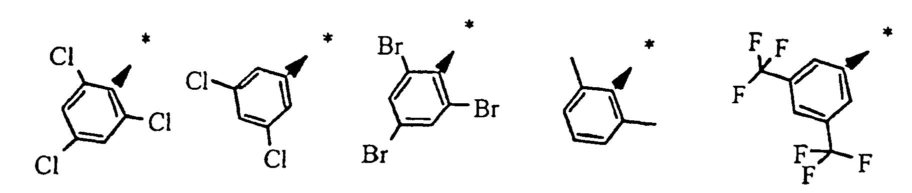 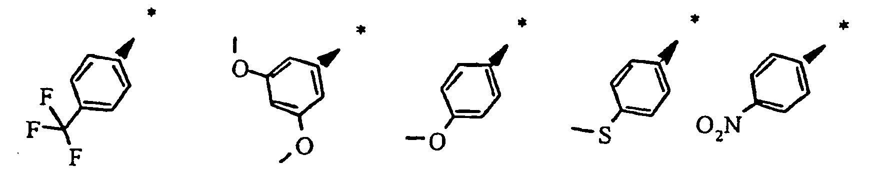 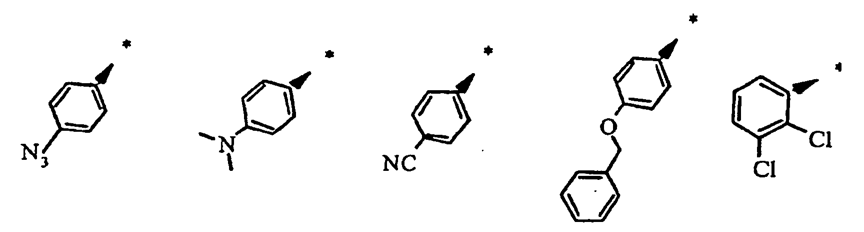 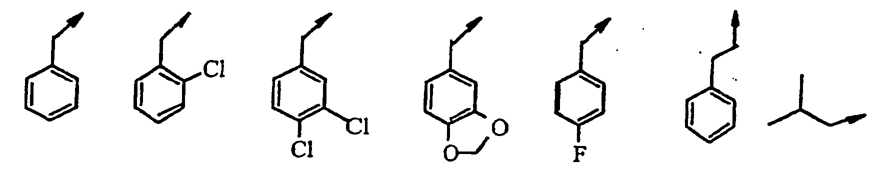 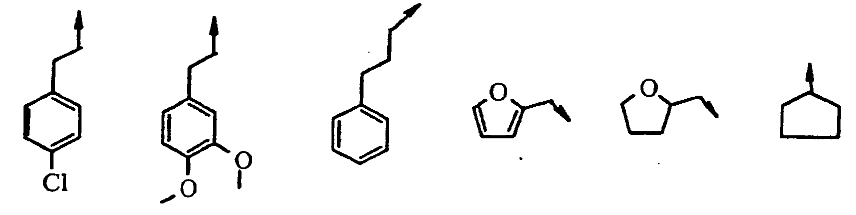 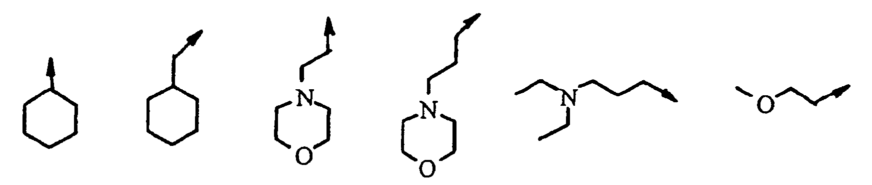 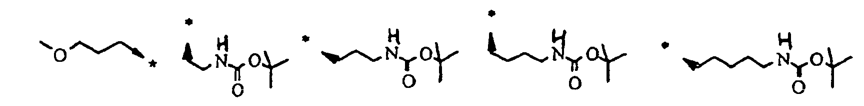 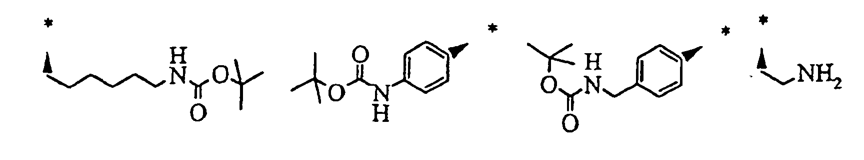 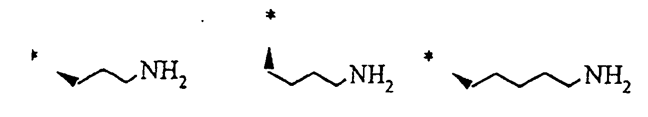 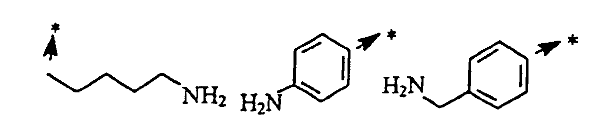 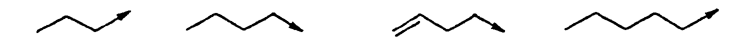 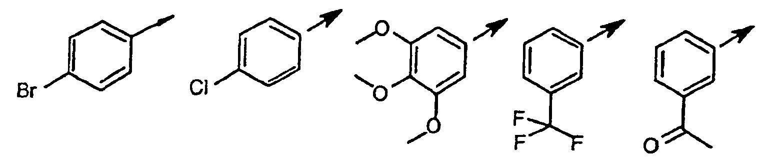 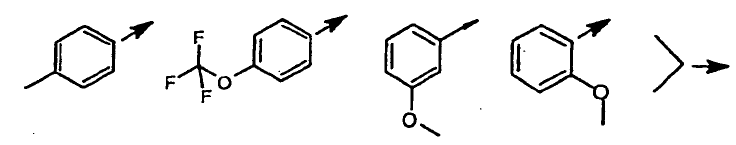 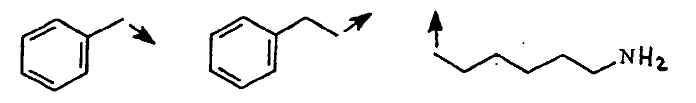 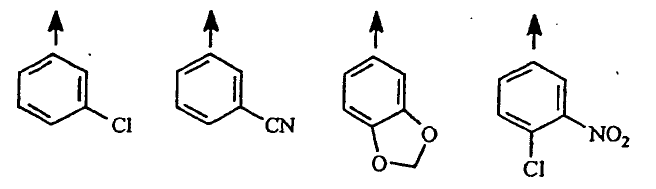 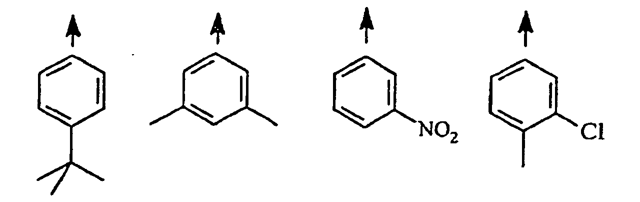 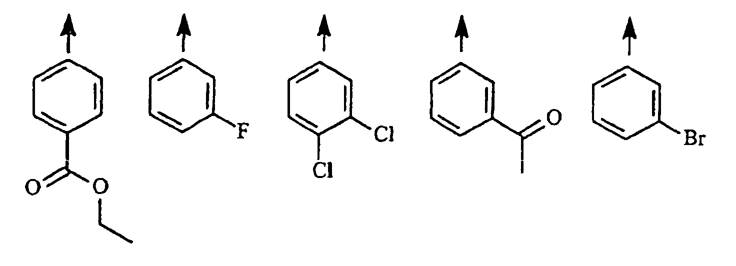 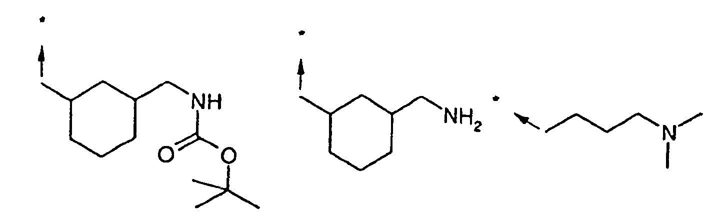 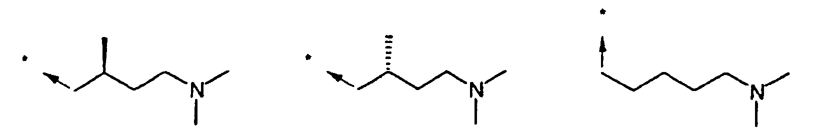 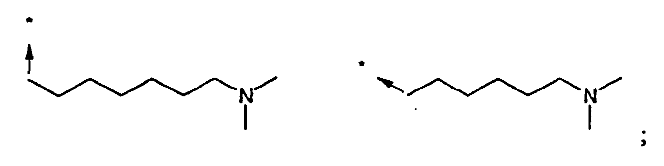 R5 H darstellt;
oder ein pharmazeutisch annehmbares Salz einer solchen Verbindung.

3. Verbindung nach Anspruch 1, **dadurch gekennzeichnet, dass**
R1 einen ggf. durch ein Halogenatom substituierten Phenylrest oder einen Rest (C₁-C₁₂)Alkyl, (C₁-C₁₂)Alkoxy oder Nitro darstellt;
R2 und R5 jeweils H darstellen;
R3 H oder (CH₂)ₚ-Z3 darstellt;
wobei Z3 (C₁-C₁₂)Alkyl, (C₁-C₈)Cycloalkyl, einen ggf. substituierten carbocyclischen oder heterocyclischen Arylrest, einen ggf. substituierten nicht aromatischen heterocyclischen Rest, *bis*-Arylalkyl, Di-arylalkyl oder einen der nachstehenden Reste 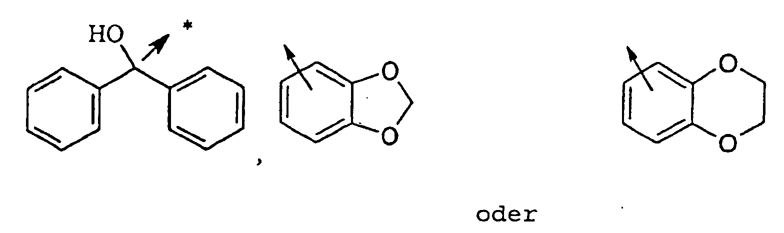 darstellt;
R4 (CH₂)ₚ-Z4 darstellt;
wobei Z4 Amino, (C₃-C₈)Cycloalkyl, (C₁-C₁₂)Alkylamino, N,N-Di-(C₁-C₁₂)alkylamino, Amino(C₃-C₈)cycloalkyl, Amino(C₁-C₆)alkyl(C₃-C₆)cycloalkyl(C₁-C₆)alkyl, carbocyclisches oder heterocyclisches Aminoaryl, einen ggf. substituierten carbocyclischen oder heterocyclischen Arylrest, einen ggf. substituierten nicht aromatischen heterocyclischen Rest, *bis*-Arylalkyl, Di-arylalkyl oder einen der nachstehenden Reste 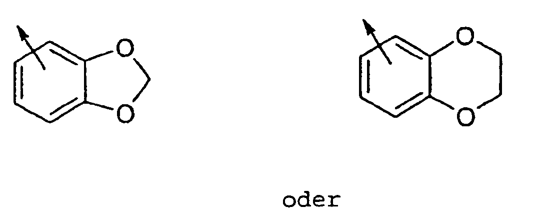 darstellt;
wobei gilt, dass ein ggf. substituierter Rest oder ein ggf. substituiertes Phenyl ggf. durch einen oder mehr als einen Substituenten substituiert ist, der jeweils unabhängig voneinander aus der Gruppe ausgewählt ist, die aus den Resten Cl, F, Br, I, CF₃, NO₂, OH, NH₂, CN, N₃, -OCF₃, (C₁-C₁₂)Alkyl, (C₁-C₁₂)Alkoxy, -(CH₂)ₚ-Phenyl-(X1)_{q}, -NH-CO-(C₁-C₆)Alkyl, -NH-C(O)O-(C₁-C₆)Alkyl, -S-(C₁-C₆)Alkyl, -S-Phenyl-(X1)_{q}, -O-(CH₂)ₚ-Phenyl-(X1)_{q}, -(CH₂)ₚ-C(O)-O-(C₁-C₆)Alkyl, -(CH₂)ₚ-C(O)-(C₁-C₆)Alkyl, -O-(CH₂)ₚ-NH₂, -O-(CH₂)ₚ-NH-(C₁-C₆)Alkyl, -O-(CH₂)ₚ-N-Di-((C₁-C₆)alkyl), und -((C₀-C₁₂))Alkyl-(X1)_{q} besteht;
X1, jedes Mal wenn es auftritt, unabhängig voneinander aus der Gruppe ausgewählt ist, die aus den Resten H, Cl, F, Br, I, CF₃, NO₂, OH, NH₂, CN, N₃, -OCF₃, (C₁-C₁₂)Alkyl, (C₁-C₁₂)Alkoxy, -S-(C₁-C₆)Alkyl, -(CH₂)ₚ-Amino, -(CH₂)ₚ-NH-(C₁-C₆)Alkyl, -(CH₂)ₚ-N-Di-((C₁-C₆)Alkyl), -(CH₂)ₚ-Phenyl und -(CH₂)ₚ-NH-(C₃-C₆)Cycloalkyl besteht;
P, jedes Mal wenn es auftritt, unabhängig voneinander 0 oder eine ganze Zahl von 1 bis 6 ist; und
Q, jedes Mal wenn es auftritt, unabhängig voneinander eine ganze Zahl von 1 bis 5 ist;
oder ein pharmazeutisch annehmbares Salz einer solchen Verbindung.

4. Verbindung nach Anspruch 3, **dadurch gekennzeichnet, dass**
R1 einen ggf. durch ein Halogenatom substituierten Phenylrest oder einen (C₁-C₁₂)Alkyl-, (C₁-C₁₂)Alkoxy- oder Nitrorest darstellt;
R2 und R5 jeweils H darstellen;
R3 (CH₂)ₚ-Z3 darstellt,
wobei Z3 einen Rest (C₃-C₈)Cycloalkyl oder einen ggf. substituierten Rest darstellt, der aus den Resten Phenyl, Naphthyl, Furanyl, Thiophen, Indolyl, Pyrrolyl und Benzothiophen ausgewählt ist;
R4 (CH₂)ₚ-Z4 darstellt;
wobei Z4 Amino, (C₁-C₁₂)Alkylamino, N,N-Di-(C₁-C₁₂)Alkylamino oder Amino (C₁-C₆)alkyl(C₃-C₆)cycloalkyl-(C₁-C₆)alkyl darstellt;
X S darstellt;
P, jedes Mal wenn es auftritt, unabhängig voneinander 0 oder eine ganze Zahl von 1 bis 6 darstellt;
m 0, 1 oder 2 darstellt; und
n 0 oder 1 darstellt;
oder ein pharmazeutisch annehmbares Salz einer solchen Verbindung.

5. Verbindung nach Anspruch 4, **dadurch gekennzeichnet, dass** es sich um eine der folgenden Verbindungen handelt:
- eine Verbindung der nachstehenden allgemeinen Unterformel (I)a: 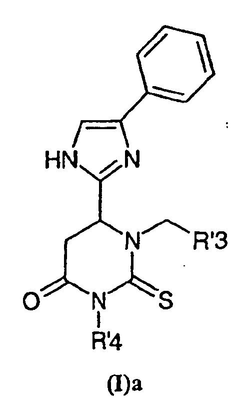 in der:
R'3 einen der nachstehenden Reste darstellt: 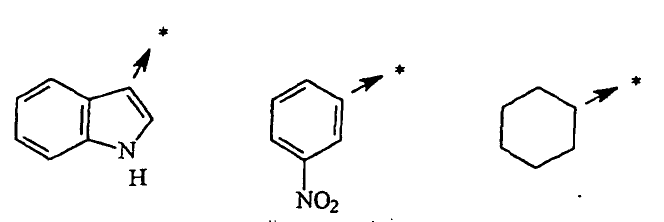 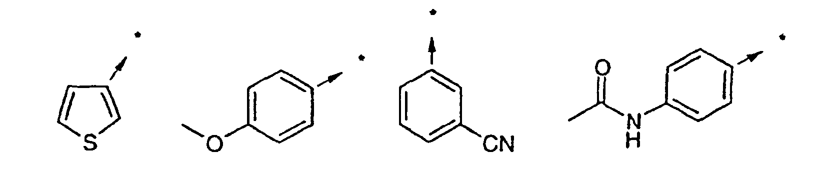
und R'4 einen der nachstehenden Reste darstellt: 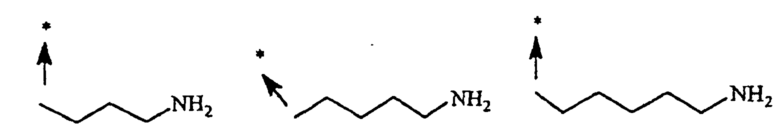 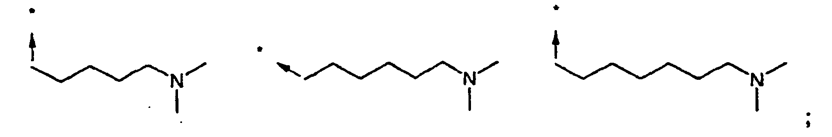
- eine Verbindung der nachstehenden allgemeinen Unterformel (I)b: 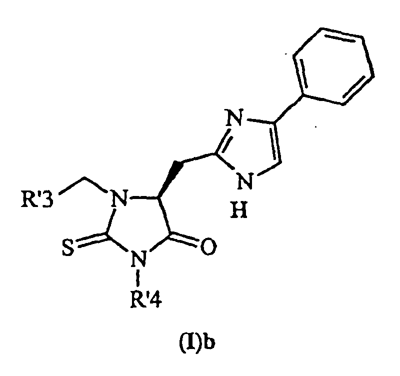 in der:
R'3 einen der nachstehenden Reste darstellt: 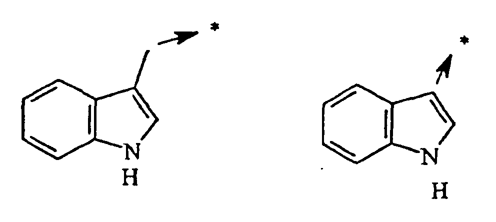
und R'4 einen der nachstehenden Reste darstellt: 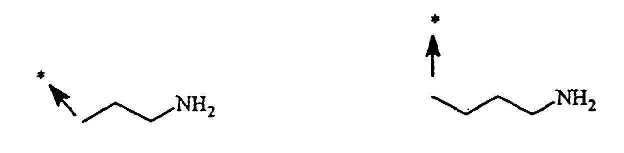 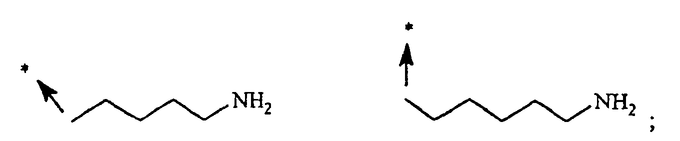
- eine Verbindung der nachstehenden allgemeinen Unterformel (I)c: 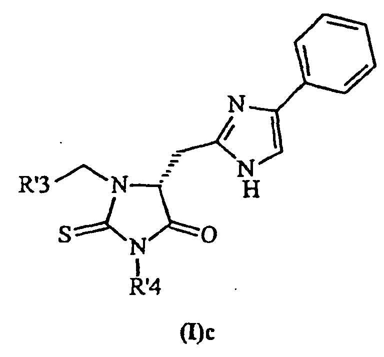 in der:
R'3 einen der nachstehenden Reste darstellt: 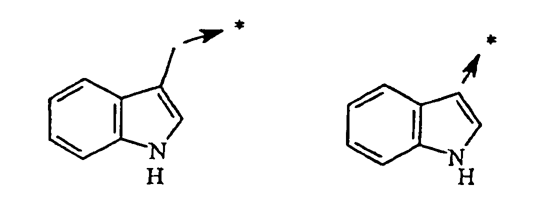
und R'4 einen der unten dargestellten Reste darstellt: 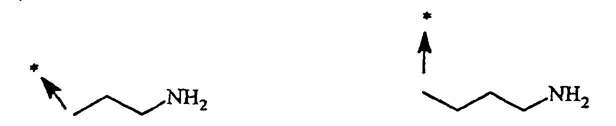 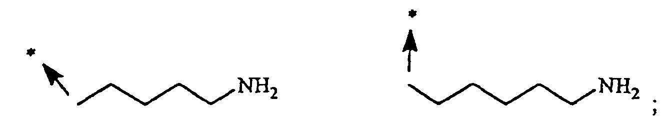
oder ein pharmezeutisch annehmbares Salz einer solchen Verbindung.

6. Verfahren zur Herstellung einer Verbindung der allgemeinen Formel (I) nach Anspruch 1, in der n 0 darstellt, **dadurch gekennzeichnet, dass** man in einem aprotischen Lösungsmittel die Verbindung der allgemeinen Formel (II), 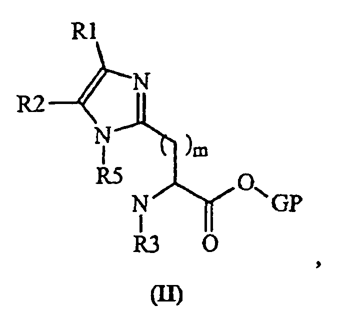 in der m, R1, R2, R3 und R5 dieselbe Bedeutung wie in der allgemeinen Formel (I) des Anspruchs 1 haben und der Rest O-GP eine von einem Alkohol abgeleitete Abgangsschutzgruppe und insbesondere Benzyloxy, Methoxy oder tert-Butoxy ist, mit einem Isocyanat oder Isothiocyanat der allgemeinen Formel (III)
R4-N=C=X , (III)
in der R4 und X dieselbe Bedeutung wie in der allgemeinen Formel (I) des Anspruchs 1 haben,
bevorzugt in Gegenwart einer tertiären Base während einer Dauer von etwa 1 bis 24 Stunden und bei einer bevorzugten Temperatur von 20 bis 70°C behandelt.

7. Verfahren zur Herstellung einer Verbindung der allgemeinen Formel (I) nach Anspruch 1, in der n 1 darstellt, **dadurch gekennzeichnet, dass** man in einem aprotischen Lösungsmittel die Verbindung der allgemeinen Formel (IV), 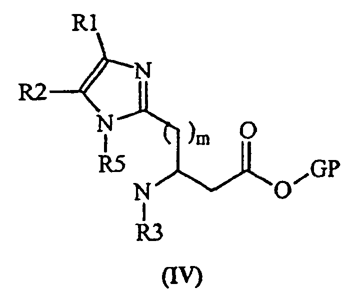 in der m, R1, R2, R3 und R5 dieselbe Bedeutung wie in der allgemeinen Formel (I) des Anspruchs 1 haben und der Rest O-GP eine von einem Alkohol abgeleitete Abgangsschutzgruppe und insbesondere Benzyloxy, Methoxy oder tert-Butoxy ist,
mit einem Isocyanat oder Isothiocyanat der allgemeinen Formel (III)
R4-N=C=X , (III)
in der R4 und X dieselbe Bedeutung wie in der allgemeinen Formel (I) des Anspruchs 1 haben,
bevorzugt in Gegenwart einer tertiären Base während einer Zeit von etwa 1 bis 48 Stunden und bei einer bevorzugten Temperatur von 20 bis 70°C behandelt.

8. Eine Verbindung der allgemeinen Formel (V), 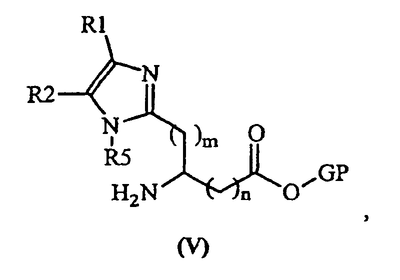 in der:
R1, R2, R5, m und n dieselbe Bedeutung wie in der allgemeinen Formel (I) haben und der Rest O-GP aus den Gruppen Benzyloxy, Methoxy oder tert-Butoxy ausgewählt ist,
in Form einer neuen Industrieverbindung und eines Zwischenprodukts in dem Syntheseverfahren der Verbindungen der allgemeinen Formel (I) gemäß Anspruch 1.

9. Verbindung nach Anspruch 8, **dadurch gekennzeichnet, dass** es sich um eine der folgenden Verbindungen handelt:
- (2*S*)-2-Amino-3-[(4-phenyl)-1H-imidazol-2-yl]benzylpropanoat;
- (2*R*)-2-Amino-3-[(4-phenyl)-1H-imidazol-2-yl]benzylpropanoat;
- (2*S*)-2-Amino-4-[(4-phenyl)-1H-imidazol-2-yl]benzylbutanoat; oder
- (2*R*)-2-Amino-4-[(4-phenyl)-1H-imidazol-2-yl]benzylbutanoat.

10. Verbindung nach einem der Ansprüche 1 bis 5 oder ein pharmazeutisch annehmbares Salz dieser Verbindung in Form eines Medikaments.

11. Pharmazeutische Zusammensetzung, die als Wirkstoff eine Verbindung nach einem der Ansprüche 1 bis 5 oder ein pharmazeutisch annehmbares Salz dieser Verbindung umfasst.

12. Verwendung einer Verbindung nach einem der Ansprüche 1 bis 5 oder eines pharmazeutisch annehmbaren Salzes dieser Verbindung für die Herstellung eines Medikaments, das für die Behandlung von pathologischen Zuständen oder Krankheiten bestimmt ist, an denen ein oder mehrere Somatostatinrezeptoren beteiligt sind.

13. Verwendung nach Anspruch 12, **dadurch gekennzeichnet, dass** die zu behandelnden pathologischen Zustände oder Krankheiten aus der Gruppe ausgewählt sind, die aus den folgenden pathologischen Zuständen oder Krankheiten besteht: Akromegalie, Hypophysenadenome, Cushing-Syndrom, Gonadotrophinome und Prolaktinome, katabolische Nebenwirkungen der Glukokortikoide, insulinabhängiger Diabetes, diabetische Retinopathie, diabetische Nephropathie, X-Syndrom, Dawn-Syndrom, Angiopathie, Angioplastie, Hyperthyroidie, Gigantismus, endokrine gastroenteropankreatische Tumore, darunter das Karzinoidsyndrom, VIPom, Insulinom, Nesidioblastose, Hyperinsulinämie, Glukagonom, Gastrinom und Zollinger-Ellison-Syndrom, GFR-Syndrom sowie akute Blutung der Ösophagusvarizen, Geschwüre, gastroösophagealer Reflux, gastroduodenaler Reflux, Pankreatitis, enterokutane und pankreatische Fisteln, aber auch Diarrhoen, refraktäre Diarrhoen des erworbenen Immundepressionssyndroms, chronische sekretorische Diarrhoe, mit Reizdarmsyndrom verbundene Diarrhoe, mit Chemotherapie induzierte Diarrhoen, mit dem Gastrin-freisetzenden Peptid verbundene Störungen, durch Darmtransplantationen bedingte sekundäre Krankheiten, portale Hypertonie sowie Hämorrhagien der Varizen bei Zirrhose-Patienten, gastrointestinale Hämorrhagie, Hämorrhagie des Gastroduodenalgeschwürs, Blutung bei implantierten Gefäßen, Crohn-Krankheit, systemische Sklerosen, Dumping-Syndrom, Dünndarmsyndrom, Hypotonie, Sklerodermie und medulläres Thyroidkarzinom, mit einer Zellenhyperproliferation verbundene Krankheiten wie Krebs und insbesondere Brustkrebs, Prostatakrebs, Thyroidkrebs sowie Pankreaskrebs und Kolorektalkrebs, Fibrosen und insbesondere Nierenfibrose, Leberfibrose, Lungenfibrose, Hautfibrose, auch Fibrose des Zentralnervensystems sowie der Nase und mit Chemotherapie induzierte Fibrose und, in anderen therapeutischen Bereichen, Kopfschmerzen einschließlich mit Hypophysentumoren verbundene Kopfschmerzen, Schmerzen, entzündliche Störungen wie Arthritis, Panikattacken, Chemotherapie, Wundvernarbung, Niereninsuffizienz infolge einer Wachstumsverzögerung, Hyperlipidämie, Fettleibigkeit und Wachstumsverzögerung infolge Fettleibigkeit, intrauterinäre Wachstumsverzögerung, Skelettdysplasie, Noonan-Syndrom, Atemstillstand während des Schlafs, Graves-Krankheit, polyzystisches Ovarialsyndrom, pankreatische Pseudozysten und Asziten, Leukämie, Meningiom, krebsbedingte Kachexie, H.pylori-Hemmung, Psoriasis, chronische Abstoßung von Alloimplantaten sowie Alzheimer-Krankheit und schließlich Osteoporose.

14. Verwendung nach Anspruch 13, **dadurch gekennzeichnet, dass** die zu behandelnden pathologischen Zustände oder Krankheiten aus der Gruppe ausgewählt sind, die aus den folgenden pathologischen Zuständen und Krankheiten besteht: Akromegalie, Hypophysenadenome oder endokrine gastroenteropankreatische Tumore, darunter das Karzinoidsyndrom, und Magendarmblutungen.

## Claims

1. Compound of general formula **(I)** represented below 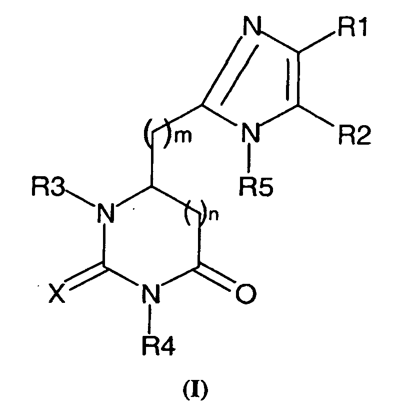 in racemic, enantiomeric form or all combinations of these forms, in which:
R1 represents an optionally substituted phenyl radical;
R2 represents H;
R3 represents H or (CH₂)ₚ-Z3;
Z3 represents (C₁-C₁₂)alkyl, (C₁-C₁₂)alkenyl, (C₃-C₈)cycloalkyl, an optionally substituted carbocyclic or heterocyclic aryl radical, an optionally substituted non-aromatic heterocyclic radical, a *bis*-arylalkyl or di-arylalkyl radical or also the 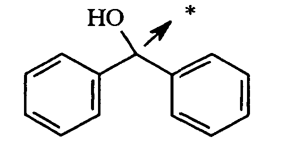 radical;
R4 represents (CH₂)ₚ-Z4;
Z4 represents amino, (C₁-C₁₂)alkyl, (C₃-C₈)cycloalkyl, (C₁-C₁₂)alkylamino, N,N-di-(C₁-C₁₂)alkylamino, amino(C₃-C₆)cycloalkyl, amino(C₁-C₆)alkyl(C₃-C₆)cycloalkyl(C₁-C₆)alkyl, carbocyclic or heterocyclic aminoaryl, (C₁-C₁₂)alkoxy, (C₁-C₁₂)alkenyl, N-C(O)O(C₁-C₆)alkyl, an optionally substituted carbocyclic or heterocyclic aryl radical, an optionally substituted non-aromatic heterocyclic radical, *bis*-arylalkyl, di-arylalkyl or one of the radicals represented below 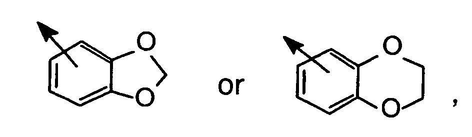
or also Z4 represents an N(R6)(R7) radical in which R6 and R7 taken together with the nitrogen atom which carries them form together a heterocycle with 5 to 7 members;
R5 represents H;
it being understood that an optionally substituted radical or an optionally substituted phenyl is optionally substituted by one or more substituents, each preferably chosen independently from the group constituted by the Cl, F, Br, I, CF₃, NO₂, OH, NH₂, CN, N₃, -OCF₃, (C₁-C₁₂)alkyl, (C₁-C₁₂)alkoxy, -(CH₂)ₚ-phenyl-(X1)_{q}, -NH-CO-(C₁-C₆)alkyl, -NH-C(O)O-(C₁-C₆)alkyl, -S-(C₁-C₆)alkyl, -S-phenyl-(X1)_{q}, -O-(CH₂)ₚ-phenyl-(X1)_{q}, -(CH₂)ₚ-C(O)-O-(C₁-C₆)alkyl, -(CH₂)ₚ-C(O)-(C₁-C₆)alkyl, -O-(CH₂)ₚ-NH₂, -O-(CH₂)ₚ-NH-(C₁-C₆)alkyl, -O-(CH₂)ₚ-N-di-((C₁-C₆)alkyl) and -((C₀-C₁₂))alkyl-(X1)_{q} radicals;
X1, each time that it occurs, is independently chosen from the group constituted by the H, Cl, F, Br, I, CF₃, NO₂, OH, NH₂, CN, N₃, -OCF₃, (C₁-C₁₂)alkyl, (C₁-C₁₂)alkoxy, -S-(C₁-C₆)alkyl, -(CH₂)ₚ-amino, -(CH₂)ₚ-NH-(C₁-C₆)alkyl, -(CH₂)ₚ-N-di-((C₁-C₆)alkyl), -(CH₂)ₚ-phenyl and -(CH₂)ₚ-NH-(C₃-C₆)cycloalkyl radicals;
p each time that it occurs is independently 0 or an integer from 1 to 6;
q each time that it occurs is independently an integer from 1 to 5.
X represents O or S;
n represents 0 or 1; and finally
when n represents 0, m represents 1, 2 or 3, and when n represents 1, m represents 0 or 1.
or a pharmaceutically acceptable salt of such a compound.

2. Compound according to claim 1, **characterized in that**:
R1 represents an optionally substituted phenyl radical;
R2 represents H;
R3 represents one of the following radicals: 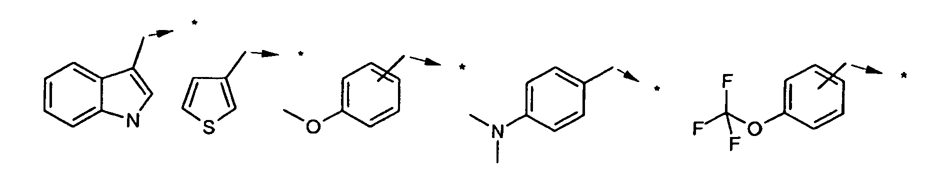 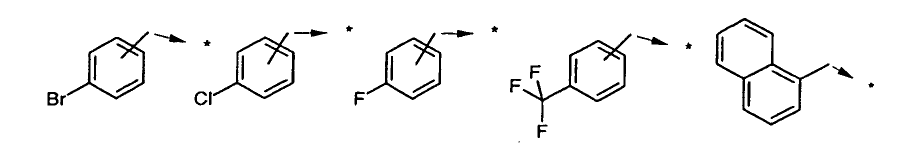 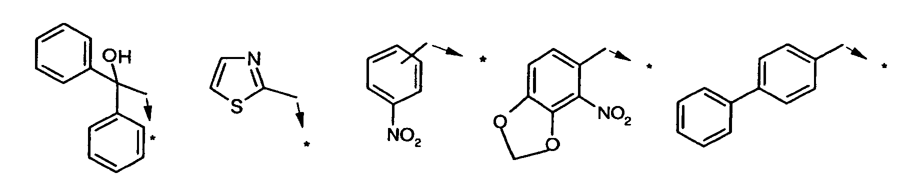 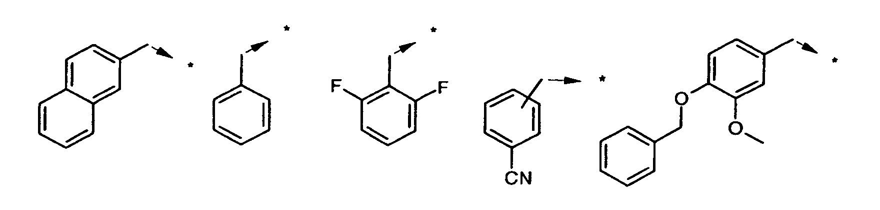 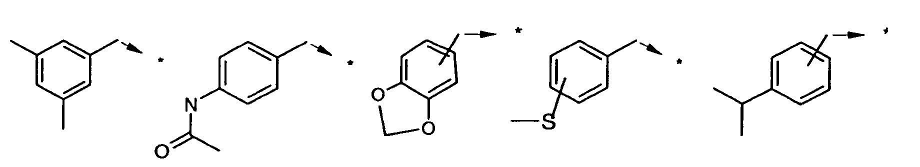 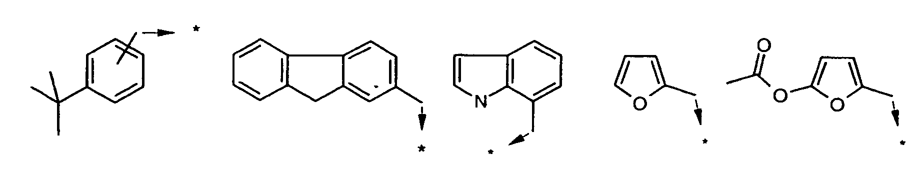 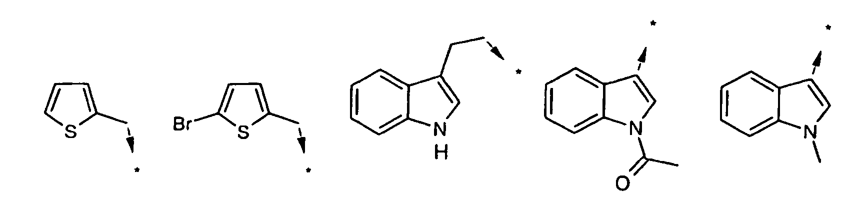 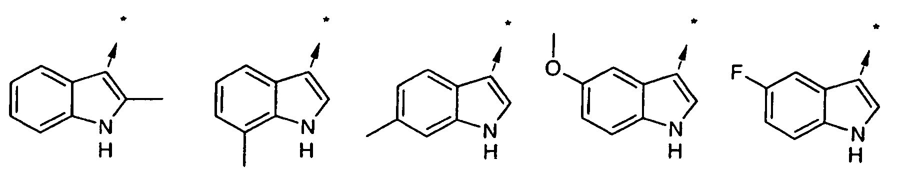 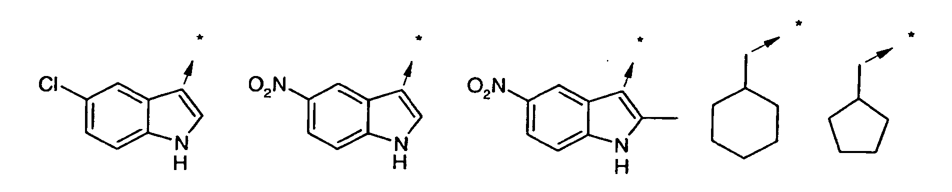 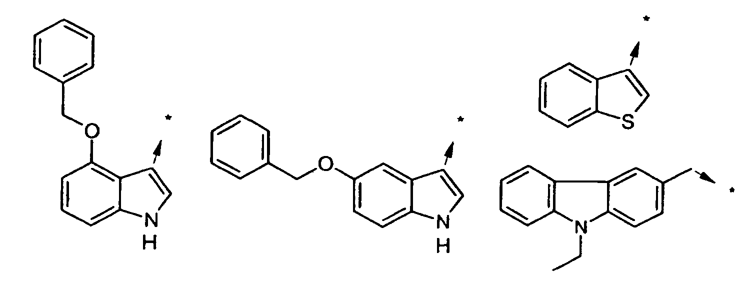 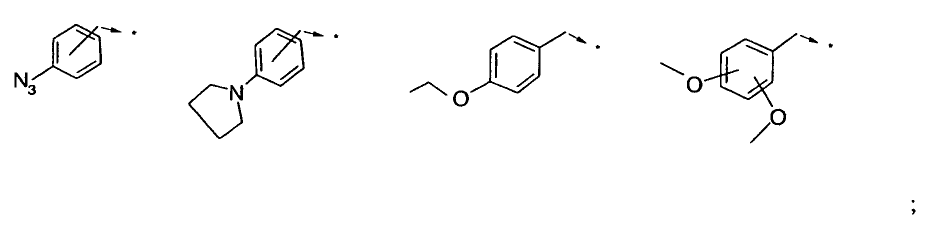
R4 represents one of the following radicals: 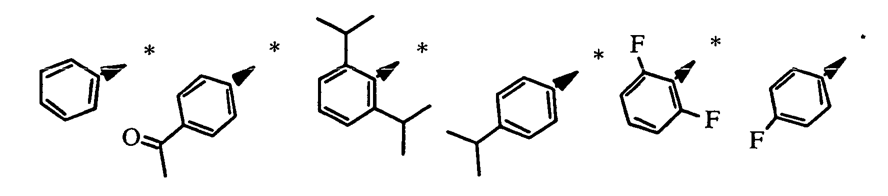 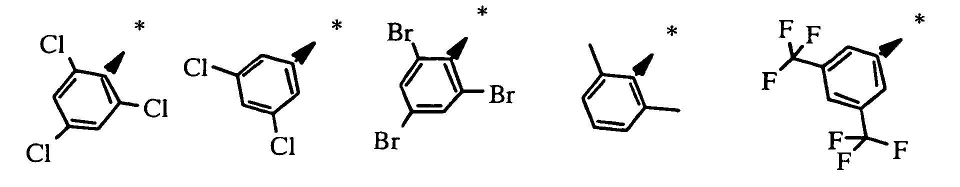 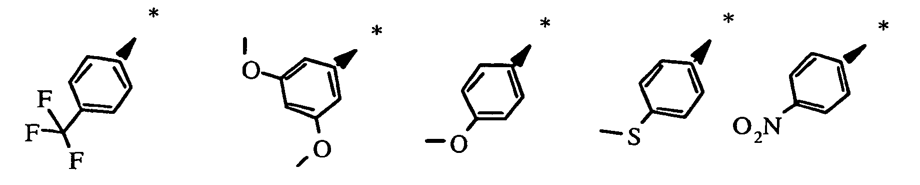 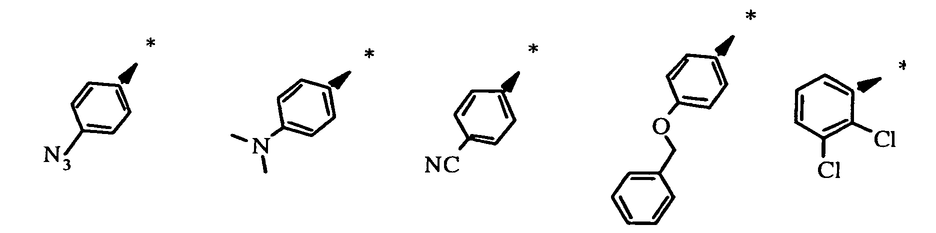 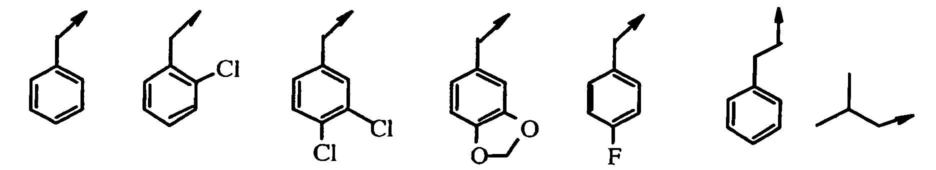 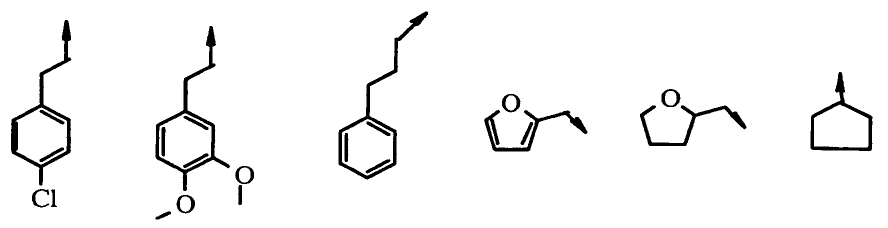 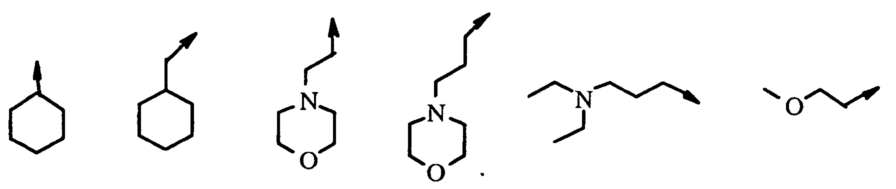 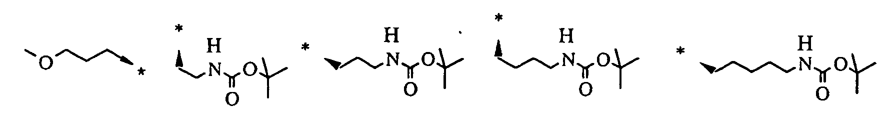 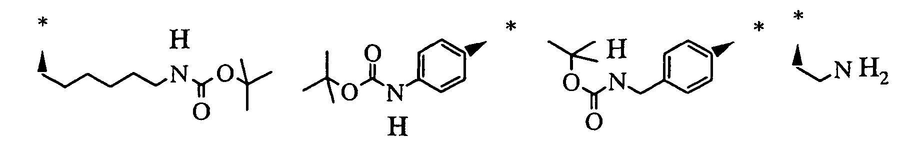 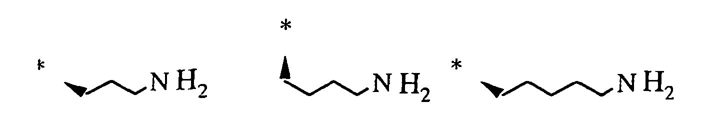 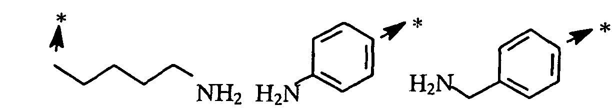 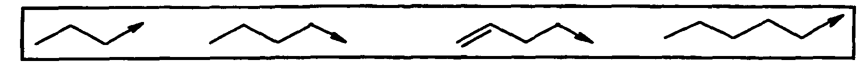 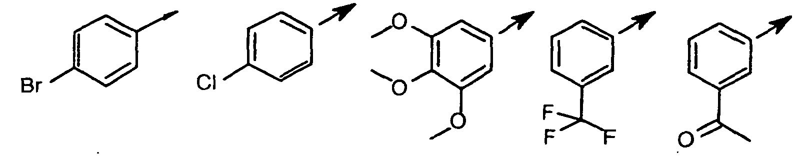 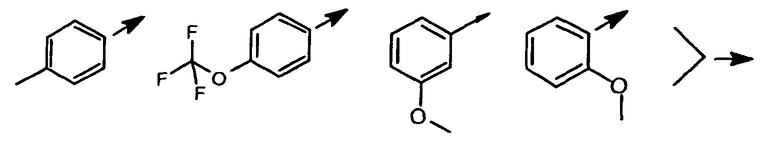 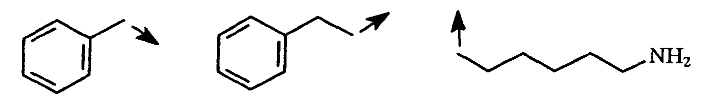 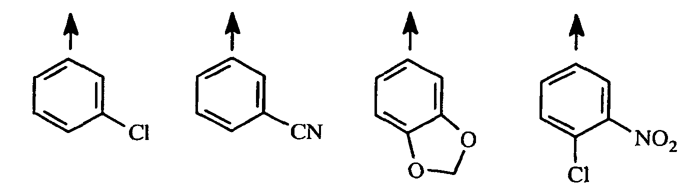 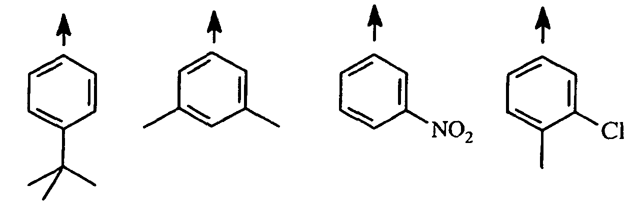 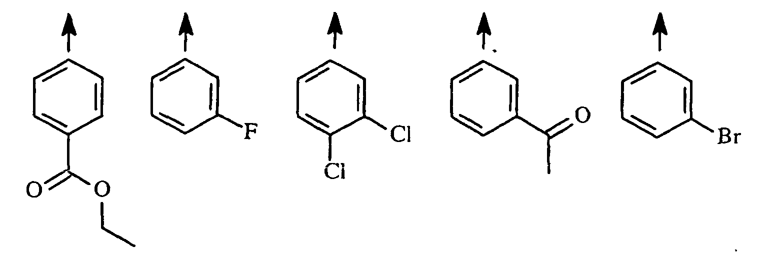 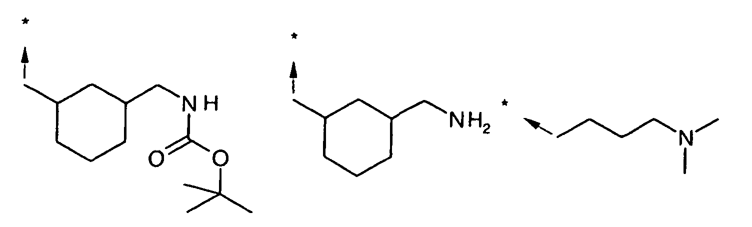 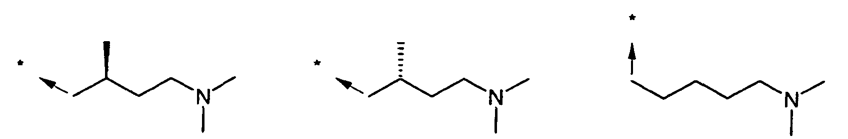 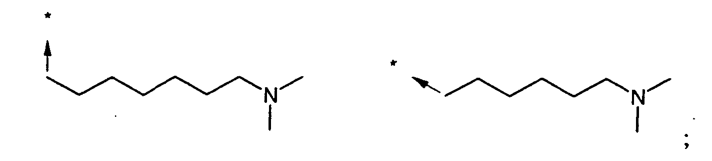
R5 represents H;
or a pharmaceutically acceptable salt of such a compound.

3. Compound according to claim 1, **characterized in that**:
R1 represents the phenyl radical optionally substituted by a halogen atom or a (C₁-C₁₂)alkyl, (C₁-C₁₂)alkoxy or nitro radical;
R2 and R5 each represent H;
R3 represents H or (CH₂)ₚ-Z3;
Z3 represents (C₁-C₁₂)alkyl, (C₃-C₈)cycloalkyl, an optionally substituted carbocyclic or heterocyclic aryl radical, an optionally substituted non-aromatic heterocyclic radical, *bis*-arylalkyl, di-arylalkyl or one of the radicals represented below 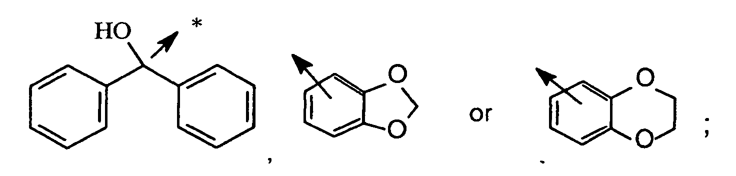
R4 represents (CH₂)ₚ-Z4;
Z4 represents amino, (C₃-C₈)cycloalkyl, (C₁-C₁₂)alkylamino, N,N-di-(C₁-C₁₂)alkylamino, amino(C₃-C₆)cycloalkyl, amino(C₁-C₆)alkyl(C₃-C₆)cycloalkyl(C₁-C₆)alkyl, carbocyclic or heterocyclic aminoaryl, an optionally substituted carbocyclic or heterocyclic aryl radical, an optionally substituted non-aromatic heterocyclic radical, *bis*-arylalkyl, di-arylalkyl or one of the radicals represented below 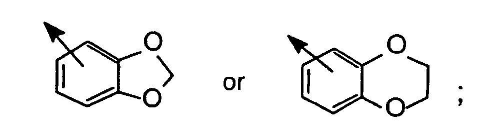 it being understood that an optionally substituted radical or an optionally substituted phenyl is optionally substituted by one or more substituents, each preferably chosen independently from the group constituted by the Cl, F, Br, I, CF₃, NO₂, OH, NH₂, CN, N₃, -OCF₃, (C₁-C₁₂)alkyl, (C₁-C₁₂)alkoxy, -(CH₂)ₚ-phenyl-(X1)_{q}, -NH-CO-(C₁-C₆)alkyl, -NH-C(O)O-(C₁-C₆)alkyl, -S-(C₁-C₆)alkyl, -S-phenyl-(X1)_{q}, -O-(CH₂)ₚ-phenyl-(X1)_{q}, -(CH₂)ₚ-C(O)-O-(C₁-C₆)alkyl, -(CH₂)ₚ-C(O)-(C₁-C₆)alkyl, -O-(CH₂)ₚ-NH₂, -O-(CH₂)ₚ-NH-(C₁-C₆)alkyl, -O-(CH₂)ₚ-N-di-((C₁-C₆)alkyl) and -((C₀-C₁₂))alkyl-(X1)_{q} radicals;
X1, each time that it occurs, is independently chosen from the group constituted by the H, Cl, F, Br, I, CF₃, NO₂, OH, NH₂, CN, N₃, -OCF₃, (C₁-C₁₂)alkyl, (C₁-C₁₂)alkoxy, -S-(C₁-C₆)alkyl, -(CH₂)ₚ-amino, -(CH₂)ₚ-NH-(C₁-C₆)alkyl, -(CH₂)ₚ-N-di-((C₁-C₆)alkyl), -(CH₂)ₚ-phenyl and -(CH₂)ₚ-NH-(C₃-C₆)cycloalkyl radicals;
p each time that it occurs is independently 0 or an integer from 1 to 6; and
q each time that it occurs is independently an integer from 1 to 5.
or a pharmaceutically acceptable salt of such a compound.

4. Compound according to claim 3, **characterized in that**:
R1 represents the phenyl radical optionally substituted by a halogen atom or a (C₁-C₁₂)alkyl, (C₁-C₁₂)alkoxy or nitro radical;
R2 and R5 each represent H;
R3 represents (CH₂)ₚ -Z3,
Z3 representing a (C₃-C₈)cycloalkyl radical or an optionally substituted radical chosen from the phenyl, naphthyl, furannyl, thiophene, indolyl, pyrrolyl and benzothiophene radicals;
R4 represents (CH₂)ₚ -Z4;
Z4 representing amino, (C₁-C₁₂)alkylamino, N,N-di-(C₁-C₁₂)alkylamino or amino(C₁-C₆)alkyl(C₃-C₆)cycloalkyl-(C₁-C₆)alkyl;
X represents S;
p each time that it occurs is independently 0 or an integer from 1 to 6;
m represents 0, 1 or 2; and finally
n represents 0 or 1.
or a pharmaceutically acceptable salt of such a compound.

5. Compound according to claim 4, **characterized in that** it is a compound:
- of general sub-formula **(I)a** represented below: 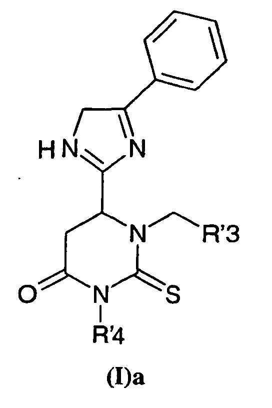 in which:
R'3 represents one of the radicals represented below: 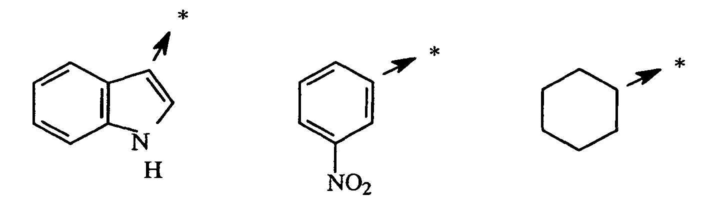 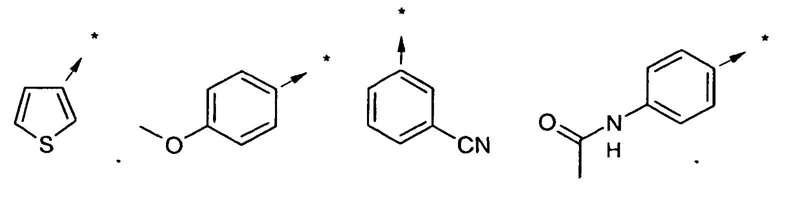
and R'4 represents one of the radicals represented below: 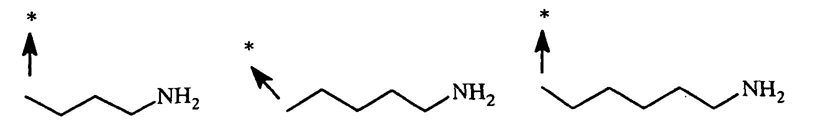 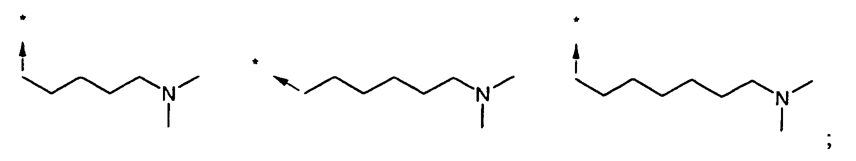
- of general sub-formula **(I)b** represented below: 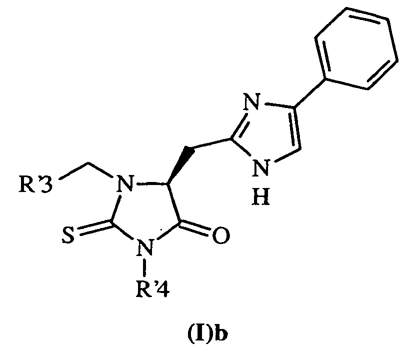 in which:
R'3 represents one of the radicals represented below: 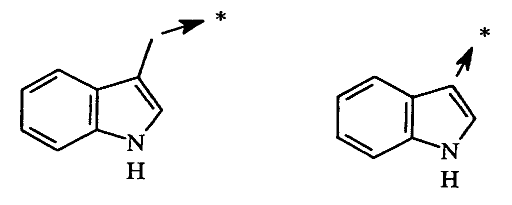
and R'4 represents one of the radicals represented below: 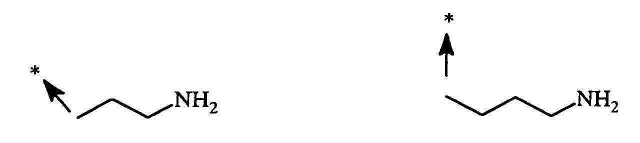 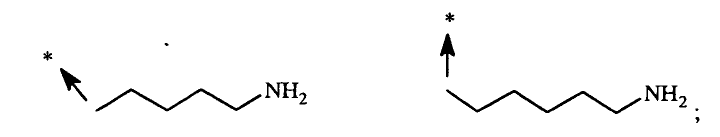
- of general sub-formula **(I)c** represented below: 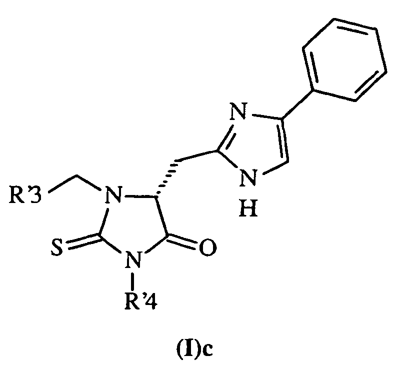 in which:
R3 represents one of the radicals represented below: 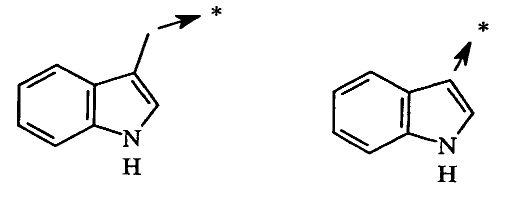
and R'4 represents one of the radicals represented below: 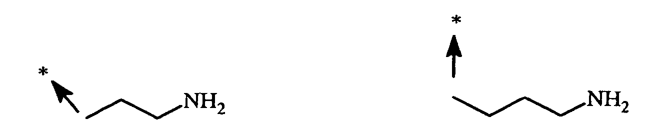 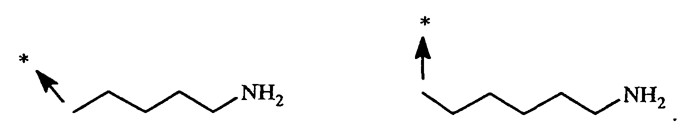
or a pharmaceutically acceptable salt of such a compound.

6. Process for the preparation of a compound of general formula **(I)** according to claim 1 in which n represents 0, **characterized in that** the compound of general formula **(II)** 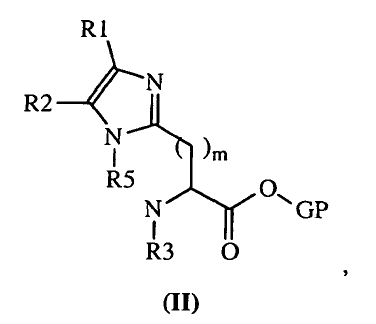 in which m, R1, R2, R3 and R5 have the same meaning as in general formula **(I)** of claim 1, and the O-GP radical is a leaving protective group derived from an alcohol and in particular benzyloxy, methoxy or tert-butoxy, is treated in an aprotic solvent
with an isocyanate or isothiocyanate of general formula **(III)** $\text{R4-N=C=X} \text{(III)}$in which R4 and X have the same meaning as in general formula **(I)** of claim 1,
preferably in the presence of a tertiary base for a duration of approximately 1 to 24 hours and at a temperature preferably comprised between 20 and 70 °C.

7. Process for the preparation of a compound of general formula **(I)** according to claim 1 in which n represents 1, **characterized in that** the compound of general formula **(IV)** 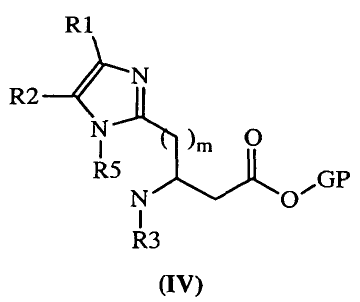 in which m, R1, R2, R3 and R5 have the same meaning as in general formula **(I)** of claim 1, and the O-GP radical is a leaving protective group derived from an alcohol and in particular benzyloxy, methoxy or tert-butoxy, is treated in an aprotic solvent with an isocyanate or isothiocyanate of general formula **(III)** $\text{R4-N=C=X} \text{(III)}$ in which R4 and X have the same meaning as in general formula **(I)** of claim 1,
preferably in the presence of a tertiary base for a duration of approximately 1 to 48 hours and at a temperature preferably comprised between 20 and 70 °C.

8. As a new industrial compound and intermediate in the synthesis process for the compounds of general formula **(I)** according to claim 1, a compound of general formula **(V)**, 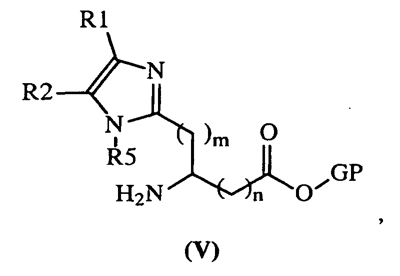 in which:
R1, R2, R5, m and n have the same meaning as in general formula **(I)**;
and the O-GP radical is chosen from the benzyloxy, methoxy or tert-butoxy groups.

9. Compound according to claim 8, **characterized in that** it is:
- benzyl (2*S*)-2-amino-3-[(4-phenyl)-1H-imidazol-2-yl]propanoate;
- benzyl (2*R*)-2-amino-3-[(4-phenyl)-1H-imidazol-2-yl]propanoate;
- benzyl (2*S*)-2-amino-4-[(4-phenyl)-1H-imidazol-2-yl]butanoate; or
- benzyl (2*R*)-2-amino-4-[(4-phenyl)-1H-imidazol-2-yl]butanoate.

10. As a medicament, a compound according to one of claims 1 to 5 or a pharmaceutically acceptable salt of said compound.

11. Pharmaceutical composition comprising as active ingredient a compound according to one of claims 1 to 5 or a pharmaceutically acceptable salt of said compound.

12. Use of a compound according to one of claims 1 to 5 or of a pharmaceutically acceptable salt of said compound for preparing a medicament intended to treat the pathological states or diseases in which one (or more) of the somatostatin receptors are involved.

13. Use according to claim 12, **characterized in that** the pathological states or diseases to be treated are chosen from the group comprising the following pathological states or diseases: acromegaly, hypophyseal adenomas, Cushing's disease, gonadotrophinomas and prolactinomas, catabolic side-effects of glucocorticoids, insulin dependent diabetes, diabetic retinopathy, diabetic nephropathy, syndrome X, dawn phenomena, angiopathy, angioplasty, hyperthyroidism, gigantism, endocrinic gastroenteropancreatic tumours including carcinoid syndrome, VIPoma, insulinoma, nesidioblastosis, hyperinsulinemia, glucagonoma, gastrinoma and Zollinger-Ellison's syndrome, GRFoma as well as acute bleeding of the esophageal varices, ulcers, gastroesophageal reflux, gastroduodenal reflux, pancreatitis, enterocutaneous and pancreatic fistulae but also diarrhoeas, refractory diarrhoeas of acquired immunodeficiency syndrome, chronic secretary diarrhoea, diarrhoea associated with irritable bowel syndrome, diarrhoeas induced by chemotherapy, disorders linked with gastrin releasing peptide, secondary pathologies with intestinal grafts, portal hypertension as well as haemorrhages of the varices in patients with cirrhosis, gastro-intestinal haemorrhage, haemorrhage of the gastroduodenal ulcer, bleeding of grafted vessels, Crohn's disease, systemic scleroses, dumping syndrome, small intestine syndrome, hypotension, scleroderma and medullar thyroid carcinoma, diseases linked with cell hyperproliferation such as cancers and more particularly breast cancer, prostate cancer, thyroid cancer as well as pancreatic cancer and colorectal cancer, fibroses and more particularly fibrosis of the kidney, fibrosis of the liver, fibrosis of the lung, fibrosis of the skin, also fibrosis of the central nervous system as well as that of the nose and fibrosis induced by chemotherapy, and in other therapeutic fields, cephaleas including cephalea associated with hypophyseal tumours, pain, inflammatory disorders such as arthritis, panic attacks, chemotherapy, cicatrization of wounds, renal insufficiency resulting from delayed growth, hyperlipidemia, obesity and delayed growth linked with obesity, delayed uterine growth, dysplasia of the skeleton, Noonan's syndrome, sleep apnea syndrome, Graves' disease, polycystic disease of the ovaries, pancreatic pseudocysts and ascites, leukaemia, meningioma, cancerous cachexia, inhibition of H pylori, psoriasis, chronic rejection of allografts as well as Alzheimer's disease and finally osteoporosis.

14. Use according to claim 13, **characterized in that** the pathological states or diseases to be treated are chosen from the group comprising the following pathological states or diseases: acromegaly, hypophyseal adenomas or endocrinic gastroenteropancreatic tumours including carcinoid syndrome, and gastrointestinal bleeding.
